# EUROPEAN PATENT APPLICATION

(11) **EP 1 959 010 A2**
(43) Date of publication of application: **20.08.2008**
(21) Application number: 07076075.6
(22) Date of filing: 05.12.2005
(51) Int. Cl.: C12N 15/11, A61K 31/713

(54) **Apoptosis-specific eif-5A and polynucleotides encoding same**

(30) Priority: 03.12.2004 US 632514 P; 31.03.2005 US 666626 P; 29.04.2005 US 675884 P; 26.08.2005 US 711397 P
(62) Divisional of application: 05853235.9
(71) Applicant: Senesco Technologies, Inc., New Brunswick, NJ 08901 (US)
(72) Inventor: Thompson, John E., Waterloo Ontario N2K 4A1 (CA); Galton, Bruce C., Madison, NJ 07940 (US); Dinarello, Charles, Boulder, CO 80302 (US); Boone, Adrienne, Waterloo, ON N2T 2V9 (CA); Hopkins, Marianne, New Hambourg, ON N3A 4J2 (CA); Taylor, Catherine, Waterloo, ON N2K 3N1 (CA); Reznikov, Leonid, Aurora, CO 80014 (US)
(74) Representative: Schmidt, Martin Peter

(57) **Abstract**

The present invention relates to apoptosis specific eucaryotic initiation factor 5A (eIF-5A), referred to as apoptosis-specific eIF-5A or eIF5-A1, nucleic acids and polypeptides and methods for increasing or decreasing expression of apoptosis-specific eIF-5A. The invention also relates to methods of increasing or decreasing apoptosis.

## Description

This application claims priority to the following U.S. provisional, which are herein incorporated by reference: 60/632,514 filed on Dec. 2, 2004; 60/666,626 filed on March 21, 2005; 60/675,884 filed on April 29, 2005 and 60/711,397 filed on August 26, 2005. This application is a CIP of U.S. 11/184,982 filed on July 20, 2005, which is a CIP of U.S. 10/861,980 filed on June 7, 2004, which is a CIP of U.S. 10/792,893 filed on March 5, 2004, which is a CIP of U.S. 10/383,614 filed on March 10, 2003, which is a CIP of 10/277,969 filed on October 10, 2002, which is a CIP of 10/200,148 filed on July 23, 2002, which is a CIP of 10/141,647 filed on May 5, 2002, which is a CIP of 09/909,796 filed on July 23, 2001 (now U.S. 6,867,237), which are all herein incorporated by reference in their entireties.

### FIELD OF THE INVENTION

The present invention relates to apoptosis-specific eucaryotic initiation factor ("eIF-5A") or referred to as "apoptosis-specific eIF-5A" or "eIF-5A1."

### BACKGROUND OF THE INVENTION

Apoptosis is a genetically programmed cellular event that is characterized by well-defined morphological features, such as cell shrinkage, chromatin condensation, nuclear fragmentation, and membrane blebbing. Kerr et al. (1972) Br. J. Cancer, 26, 239-257; Wyllie et al. (1980) Int. Rev. Cytol., 68, 251-306. It plays an important role in normal tissue development and homeostasis, and defects in the apoptotic program are thought to contribute to a wide range of human disorders ranging from neurodegenerative and autoimmunity disorders to neoplasms. Thompson (1995) Science, 267, 1456-1462; Mullauer et al. (2001) Mutat. Res, 488, 211-231. Although the morphological characteristics of apoptotic cells are well characterized, the molecular pathways that regulate this process have only begun to be elucidated.

One group of proteins that is thought to play a key role in apoptosis is a family of cysteine proteases, termed caspases, which appear to be required for most pathways of apoptosis. Creagh & Martin (2001) Biochem. Soc. Trans, 29, 696-701; Dales et al. (2001) Leuk. Lymphoma, 41, 247-253. Caspases trigger apoptosis in response to apoptotic stimuli by cleaving various cellular proteins, which results in classic manifestations of apoptosis, including cell shrinkage, membrane blebbing and DNA fragmentation. Chang & Yang (2000) Microbiol. Mol. Biol. Rev., 64, 821-846.

Pro-apoptotic proteins, such as Bax or Bak, also play a key role in the apoptotic pathway by releasing caspase-activating molecules, such as mitochondrial cytochrome c, thereby promoting cell death through apoptosis. Martinou & Green (2001) Nat. Rev. Mol. Cell. Biol., 2, 63-67; Zou et al. (1997) Cell, 90, 405-413. Anti-apoptotic proteins, such as Bcl-2, promote cell survival by antagonizing the activity of the pro-apoptotic proteins, Bax and Bak. Tsujimoto (1998) Genes Cells, 3, 697-707; Kroemer (1997) Nature Med., 3, 614-620. The ratio of Bax:Bcl-2 is thought to be one way in which cell fate is determined; an excess of Bax promotes apoptosis and an excess of Bcl-2 promotes cell survival. Salomons et al. (1997) Int. J. Cancer, 71, 959-965; Wallace-Brodeur & Lowe (1999) Cell Mol. Life Sci.,55, 64-75.

Another key protein involved in apoptosis is a protein that encoded by the tumor suppressor gene p53. This protein is a transcription factor that regulates cell growth and induces apoptosis in cells that are damaged and genetically unstable, presumably through up-regulation of Bax. Bold et al. (1997) Surgical Oncology, 6, 133-142; Ronen et al., 1996; Schuler & Green (2001) Biochem. Soc. Trans., 29, 684-688; Ryan et al. (2001) Curr. Opin. Cell Biol., 13, 332-337; Zörnig et al. (2001) Biochem. Biophys. Acta, 1551, F1-F37.

Alterations in the apoptotic pathways are believed to play a key role in a number of disease processes, including cancer. Wyllie et al. (1980) Int. Rev. Cytol., 68, 251-306; Thompson (1995) Science, 267, 1456-1462; Sen & D'Incalci (1992) FEBS Letters, 307, 122-127; McDonnell et al. (1995) Seminars in Cancer and Biology, 6, 53-60. Investigations into cancer development and progression have traditionally been focused on cellular proliferation. However, the important role that apoptosis plays in tumorigenesis has recently become apparent. In fact, much of what is now known about apoptosis has been learned using tumor models, since the control of apoptosis is invariably altered in some way in tumor cells. Bold et al. (1997) Surgical Oncology, 6, 133-142.

Cytokines also have been implicated in the apoptotic pathway. Biological systems require cellular interactions for their regulation, and cross-talk between cells generally involves a large variety of cytokines. Cytokines are mediators that are produced in response to a wide variety of stimuli by many different cell types. Cytokines are pleiotropic molecules that can exert many different effects on many different cell types, but are especially important in regulation of the immune response and hematopoietic cell proliferation and differentiation. The actions of cytokines on target cells can promote cell survival, proliferation, activation, differentiation, or apoptosis depending on the particular cytokine, relative concentration, and presence of other mediators.

The use of anti-cytokines to treat autoimmune disorders such as psoriasis, rheumatoid arthritis, and Crohn's disease is gaining popularity. The pro-inflammatory cytokines IL-1 and TNF play a large role in the pathology of these chronic disorders. Anti-cytokine therapies that reduce the biological activities of these two cytokines can provide therapeutic benefits (Dinarello and Abraham, 2002).

Interleukin 1 (IL-I) is an important cytokine that mediates local and systemic inflammatory reactions and which can synergize with TNF in the pathogenesis of many disorders, including vasculitis, osteoporosis, neurodegenerative disorders, diabetes, lupus nephritis, and autoimmune disorders such as rheumatoid arthritis. The importance of IL-1β in tumour angiogenesis and invasiveness was also recently demonstrated by the resistance of IL-1β knockout mice to metastases and angiogenesis when injected with melanoma cells (Voronov *et al.,* 2003).

Interleukin 18 (IL-18) is a recently discovered member of the IL-1 family and is related by structure, receptors, and function to IL-1. IL-18 is a central cytokine involved in inflammatory and autoimmune disorders as a result of its ability to induce interferon-gamma (IFN-γ), TNF-α, and IL-1. IL-1β and IL-18 are both capable of inducing production of TNF-α, a cytokine known to contribute to cardiac dysfunction during myocardial ischemia (Maekawa *et al.,* 2002). Inhibition of IL-18 by neutralization with an IL-18 binding protein was found to reduce ischemia-induced myocardial dysfunction in an ischemia/reperfusion model of suprafused human atrial myocardium (Dinarello, 2001). Neutralization of IL-18 using a mouse IL-18 binding protein was also able to decrease IFN-γ, TNF-α, and IL-1β transcript levels and reduce joint damage in a collagen-induced arthritis mouse model (Banda *et al.,* 2003). A reduction of IL-18 production or availability may also prove beneficial to control metastatic cancer as injection of IL-18 binding protein in a mouse melanoma model successfully inhibited metastases (Carrascal *et al.,* 2003). As a further indication of its importance as a pro-inflammatory cytokine, plasma levels of IL-18 were elevated in patients with chronic liver disease and increased levels were correlated with the severity of the disease (Ludwiczek *et al.,* 2002). Similarly, IL-18 and TNF-α were elevated in the serum of diabetes mellitus patients with nephropathy (Moriwaki *et al.,* 2003). Neuroinflammation following traumatic brain injury is also mediated by pro-inflammatory cytokines and inhibition of IL-18 by the IL-18 binding protein improved neurological recovery in mice following brain trauma (Yatsiv *et al.,* 2002).

TNF-α, a member of the TNF family of cytokines, is a pro-inflammatory cytokine with pleiotropic effects ranging from co-mitogenic effects on hematopoietic cells, induction of inflammatory responses, and induction of cell death in many cell types. TNF-α is normally induced by bacterial lipopolysaccharides, parasites, viruses, malignant cells and cytokines and usually acts beneficially to protect cells from infection and cancer. However, inappropriate induction of TNF-α is a major contributor to disorders resulting from acute and chronic inflammation such as autoimmune disorders and can also contribute to cancer, AIDS, heart disease, and sepsis (reviewed by Aggarwal and Natarajan, 1996; Sharma and Anker, 2002). Experimental animal models of disease (i.e. septic shock and rheumatoid arthritis) as well as human disorders (i.e. inflammatory bowel diseases and acute graft-versus-host disease) have demonstrated the beneficial effects of blocking TNF-α (Wallach *et al.,* 1999). Inhibition of TNF-α has also been effective in providing relief to patients suffering autoimmune disorders such as Crohn's disease (van Deventer, 1999) and rheumatoid arthritis (Richard-Miceli and Dougados, 2001). The ability of TNF-α to promote the survival and growth of B lymphocytes is also thought to play a role in the pathogenesis of B-cell chronic lymphocytic leukemia (B-CLL) and the levels of TNF-α being expressed by T cells in B-CLL was positively correlated with tumour mass and stage of the disease (Bojarska-Junak *et al.,* 2002). Interleukin-1β (IL-1β) is a cytokine known to induce TNF-α production.

Thus, since the accumulation of excess cytokines and TNF-α can lead to deleterious consequences on the body, including cell death, there is a need for a method to reduce the levels of cytokines in the body as well as inhibiting or reducing apoptosis. The present invention fulfills these needs.

Deoxyhypusine synthase (DHS) and hypusine-containing eucaryotic translation initiation Factor-5A (eIF-5A) are known to play important roles in many cellular processes including cell growth and differentiation. Hypusine, a unique amino acid, is found in all examined eucaryotes and archaebacteria, but not in eubacteria, and eIF-5A is the only known hypusine-containing protein. Park (1988) J. Biol. Chem., 263, 7447-7449; Schümann & Klink (1989) System. Appl. Microbiol., 11, 103-107; Bartig et al. (1990) System. Appl. Microbiol., 13, 112-116; Gordon et al. (1987a) J. Biol. Chem., 262, 16585-16589. Active eIF-5A is formed in two post-translational steps: the first step is the formation of a deoxyhypusine residue by the transfer of the 4-aminobutyl moiety of spermidine to the α-amino group of a specific lysine of the precursor eIF-5A catalyzed by deoxyhypusine synthase; the second step involves the hydroxylation of this 4-aminobutyl moiety by deoxyhypusine hydroxylase to form hypusine.

The amino acid sequence of eIF-5A is well conserved between species, and there is strict conservation of the amino acid sequence surrounding the hypusine residue in eIF-5A, which suggests that this modification may be important for survival. Park et al. (1993) Biofactors, 4, 95-104. This assumption is further supported by the observation that inactivation of both isoforms of eIF-5A found to date in yeast, or inactivation of the DHS gene, which catalyzes the first step in their activation, blocks cell division. Schnier et al. (1991) Mol. Cell. Biol., 11, 3105-3114; Sasaki et al. (1996) FEBS Lett., 384, 151-154; Park et al. (1998) J. Biol. Chem., 273, 1677-1683. However, depletion of eIF-5A protein in yeast resulted in only a small decrease in total protein synthesis suggesting that eIF-5A may be required for the translation of specific subsets of mRNA's rather than for protein global synthesis. Kang et al. (1993), "Effect of initiation factor eIF-5A depletion on cell proliferation and protein synthesis," in Tuite, M. (ed.), Protein Synthesis and Targeting in Yeast, NATO Series H. The recent finding that ligands that bind eIF-5A share highly conserved motifs also supports the importance of eIF-5A. Xu & Chen (2001) J. Biol. Chem., 276, 2555-2561. In addition, the hypusine residue of modified eIF-5A was found to be essential for sequence-specific binding to RNA, and binding did not provide protection from ribonucleases.

In addition, intracellular depletion of eIF-5A results in a significant accumulation of specific mRNAs in the nucleus, indicating that eIF-5A may be responsible for shuttling specific classes of mRNAs from the nucleus to the cytoplasm. Liu & Tartakoff (1997) Supplement to Molecular Biology of the Cell, 8, 426a. Abstract No. 2476, 37th American Society for Cell Biology Annual Meeting. The accumulation of eIF-5A at nuclear pore-associated intranuclear filaments and its interaction with a general nuclear export receptor further suggest that eIF-5A is a nucleocytoplasmic shuttle protein, rather than a component of polysomes. Rosorius et al. (1999) J. Cell Science, 112, 2369-2380.

The first cDNA for eIF-5A was cloned from human in 1989 by Smit-McBride et al., and since then cDNAs or genes for eIF-5A have been cloned from various eukaryotes including yeast, rat, chick embryo, alfalfa, and tomato. Smit-McBride et al. (1989) J. Biol. Chem., 264, 1578-1583; Schnier et al. (1991) (yeast); Sano, A. (1995) in Imahori, M. et al. (eds), Polyamines, Basic and Clinical Aspects, VNU Science Press, The Netherlands, 81-88 (rat); Rinaudo & Park (1992) FASEB J., 6, A453 (chick embryo); Pay et al. (1991) Plant Mol. Biol., 17, 927-929 (alfalfa); Wang et al. (2001) J. Biol. Chem., 276, 17541-17549 (tomato).

Expression of eIF-5A mRNA has been explored in various human tissues and mammalian cell lines. For example, changes in eIF-5A expression have been observed in human fibroblast cells after addition of serum following serum deprivation. Pang & Chen (1994) J. Cell Physiol., 160, 531-538. Age-related decreases in deoxyhypusine synthase activity and abundance of precursor eIF-5A have also been observed in senescing fibroblast cells, although the possibility that this reflects averaging of differential changes in isoforms was not determined. Chen & Chen (1997) J. Cell Physiol., 170, 248-254.

Studies have shown that eIF-5A may be the cellular target of viral proteins such as the human immunodeficiency virus type 1 Rev protein and human T cell leukemia virus type 1 Rex protein. Ruhl et al. (1993) J. Cell Biol.,123, 1309-1320; Katahira et al. (1995) J. Virol., 69, 3125-3133. Preliminary studies indicate that eIF-5A may target RNA by interacting with other RNA-binding proteins such as Rev, suggesting that these viral proteins may recruit eIF-5A for viral RNA processing. Liu et al. (1997) Biol. Signals, 6, 166-174.

Thus, although eIF-5A and DHS are known, there remains a need in understanding how these proteins are involved in apoptotic pathways as well as cytokine stimulation to be able to modulate apoptosis and cytokine expression. The present invention fulfills this need.

### SUMMARY OF INVENTION

The present invention relates to apoptosis specific eucaryotic initiation factor 5A (eIF-5A), referred to as "apoptosis specific eIF-5A" or "eIF-5A1" and methods for inhibiting or suppressing apoptosis in cells using antisense nucleotides or siRNAs to inhibit expression of apoptosis-specific eIF-5A.

The present invention also relates to methods of increasing apoptosis in cells by increasing expression of apoptosis-specific eIF-5A.

### BRIEF DESCRIPTION OF THE DRAWINGS

Figure 1 depicts the nucleotide sequence (SEQ ID NO: 11) and derived amino acid sequence (SEQ ID NO: 12) of the 3' end of rat apoptosis-specific eIF-5A.
Figure 2 depicts the nucleotide sequence (SEQ ID NO: 15) and derived amino acid sequence (SEQ ID NO: 16) of the 5' end of rat apoptosis-specific eIF-5A cDNA.
Figure 3 depicts the nucleotide sequence of rat corpus luteum apoptosis-specific eIF-5A full-length cDNA (SEQ ID NO: 1). The amino acid sequence is shown in SEQ ID NO: 2.
Figure 4 depicts the nucleotide sequence (SEQ ID NO: 6) and derived amino acid sequence (SEQ ID NO: 7) of the 3' end of rat apoptosis-specific DHS cDNA.
Figure 5 is an alignment of the full-length nucleotide sequence of rat corpus luteum apoptosis-specific eIF-5A cDNA (SEQ ID NO: 20) with the nucleotide sequence of human eIF-5A (SEQ ID NO: 3) (Accession number BC000751 or NM_001970, SEQ ID NO:3).
Figure 6 is an alignment of the full-length nucleotide sequence of rat corpus luteum apoptosis-specific eIF-5A cDNA (SEQ ID NO: 20) with the nucleotide sequence of human eIF-5A (SEQ ID NO: 4) (Accession number NM-020390, SEQ ID NO:4).
Figure 7 is an alignment of the full-length nucleotide sequence of rat corpus luteum apoptosis-specific eIF-5A cDNA (SEQ ID NO: 20) with the nucleotide sequence of mouse eIF-5A (Accession number BC003889). Mouse nucleotide sequence (Accession number BC003889) is SEQ ID NO:5.
Figure 8 is an alignment of the derived full-length amino acid sequence of rat corpus luteum apoptosis-specific eIF-5A (SEQ ID NO: 2) with the derived amino acid sequence of human eIF-5A (SEQ ID NO: 21) (Accession number BC000751 or NM_001970).
Figure 9 is an alignment of the derived full-length amino acid sequence of rat corpus luteum apoptosis-specific eIF-5A (SEQ ID NO: 2) with the derived amino acid sequence of human eIF-5A (SEQ ID NO: 22) (Accession number NM_020390).
Figure 10 is an alignment of the derived full-length amino acid sequence of rat corpus luteum apoptosis-specific eIF-5A (SEQ ID NO: 2) with the derived amino acid sequence of mouse eIF-5A (SEQ ID NO: 23) (Accession number BC003889).
Figure 11 is an alignment of the partial-length nucleotide sequence of rat corpus luteum apoptosis-specific DHS cDNA (residues 1-453 of SEQ ID NO: 6) with the nucleotide sequence of human DHS (SEQ ID NO: 8) (Accession number BC000333, SEQ ID NO:8).
Figure 12 is a Northern blot (top) and an ethidium bromide stained gel (bottom) of total RNA probed with the ³²P-dCTP-labeled 3'-end of rat corpus luteum apoptosis-specific eIF-5A cDNA.
Figure 13 is a Northern blot (top) and an ethidium bromide stained gel (bottom) of total RNA probed with the ³²P-dCTP-labeled 3'-end of rat corpus luteum apoptosis-specific DHS cDNA.
Figure 14 depicts a DNA laddering experiment in which the degree of apoptosis in superovulated rat corpus lutea was examined after injection with PGF-2α.
Figure 15 is an agarose gel of genomic DNA isolated from apoptosing rat corpus luteum showing DNA laddering after treatment of rats with PGF F-2α.
Figure 16 depicts a DNA laddering experiment in which the degree of apoptosis in dispersed cells of superovulated rat corpora lutea was examined in rats treated with spermidine prior to exposure to PGF-2α.
Figure 17 depicts a DNA laddering experiment in which the degree of apoptosis in superovulated rat corpus lutea was examined in rats treated with spermidine and/or PGF-2α.
Figure 18 is a Southern blot of rat genomic DNA probed with ³²P-dCTP-labeled partial-length rat corpus luteum apoptosis-specific eIF-5A cDNA.
Figure 19 depicts pHM6, a mammalian epitope tag expression vector (Roche Molecular Biochemicals).
Figure 20 is a Northern blot (top) and ethidium bromide stained gel (bottom) of total RNA isolated from COS-7 cells after induction of apoptosis by withdrawal of serum probed with the ³²P-dCTP-labeled 3'-untranslated region of rat corpus luteum apoptosis-specific DHS cDNA.
Figure 21 is a flow chart illustrating the procedure for transient transfection of COS-7 cells.
Figure 22 is a Western blot of transient expression of foreign proteins in COS-7 cells following transfection with pHM6.
Figure 23 shows that induction of apoptosis in normal fibroblasts by treatment with sodium nitroprusside up-regulates apoptosis-specific eIF-5A.

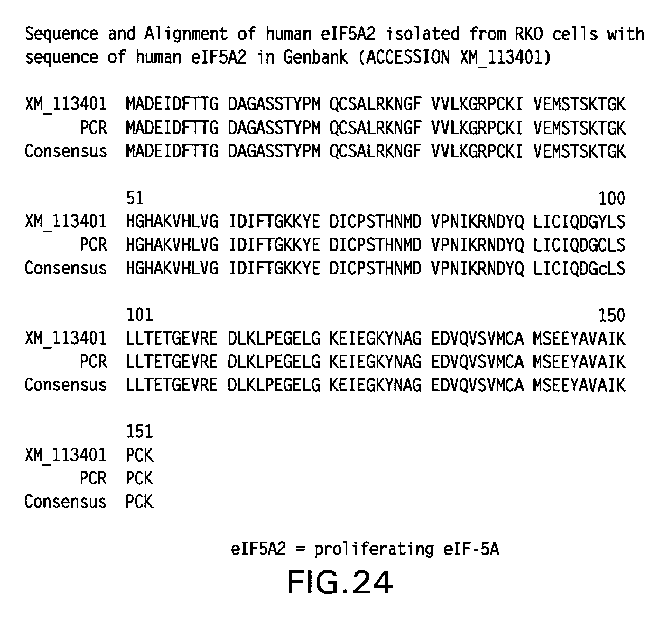

Figure 24 is an alignment of human eIF5A2 isolated from RKO cells (SEQ ID NO: 24) with the sequence of human eIF5A2 (SEQ ID NO: 22) (Genbank accession number XM_113401). The consensus sequence is shown in SEQ ID NO: 28.

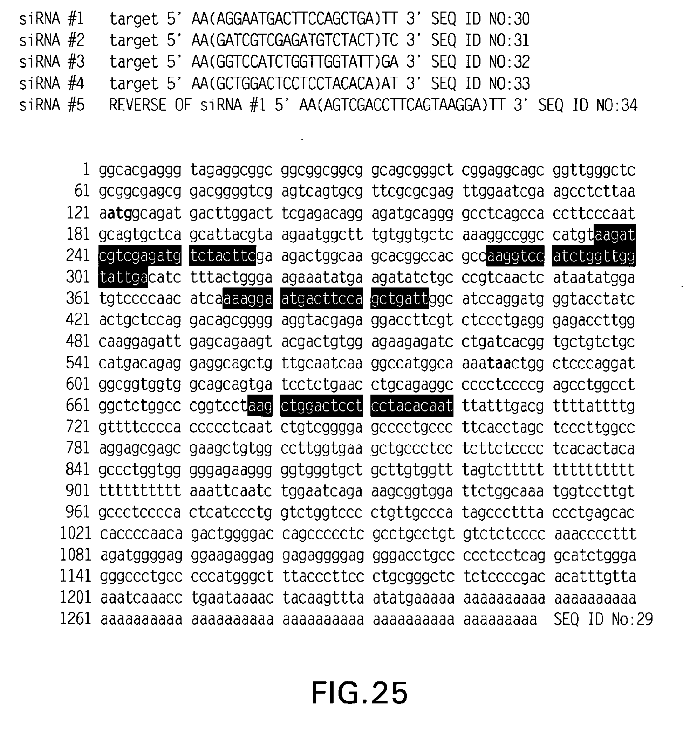

Figure 25 shows the sequence of human apoptosis-specific eIF-5A (SEQ ID NO:29) and the sequences of 5 siRNAs of the present invention (SEQ ID NO:30, 31, 32, 33 and 34).

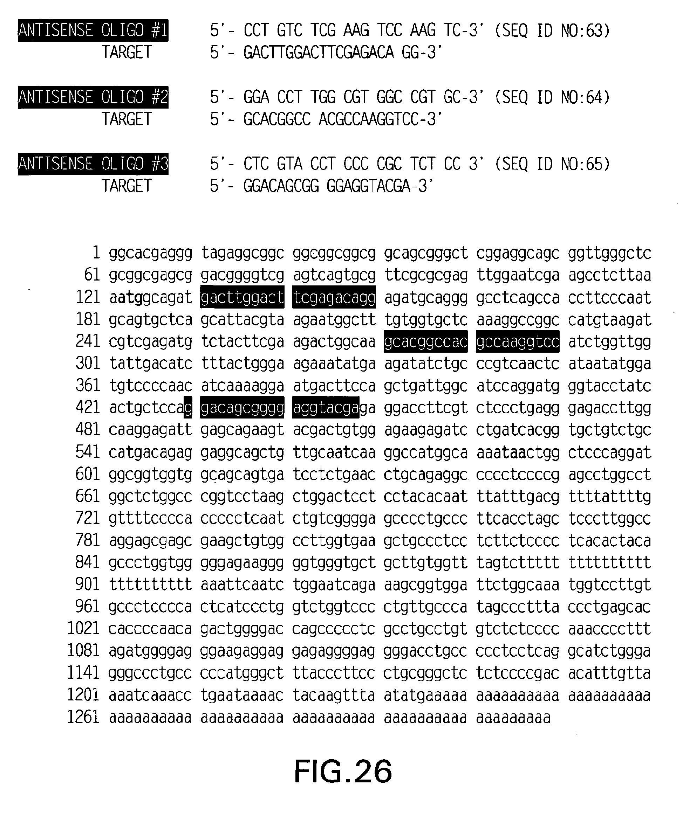

Figure 26 shows the sequence of human apoptosis-specific eIF-5A (SEQ ID NO: 29) and the sequences of 3 antisense oligonucleotides of the present invention (SEQ ID NO:35, 37, and 39, respectively in order of appearance).

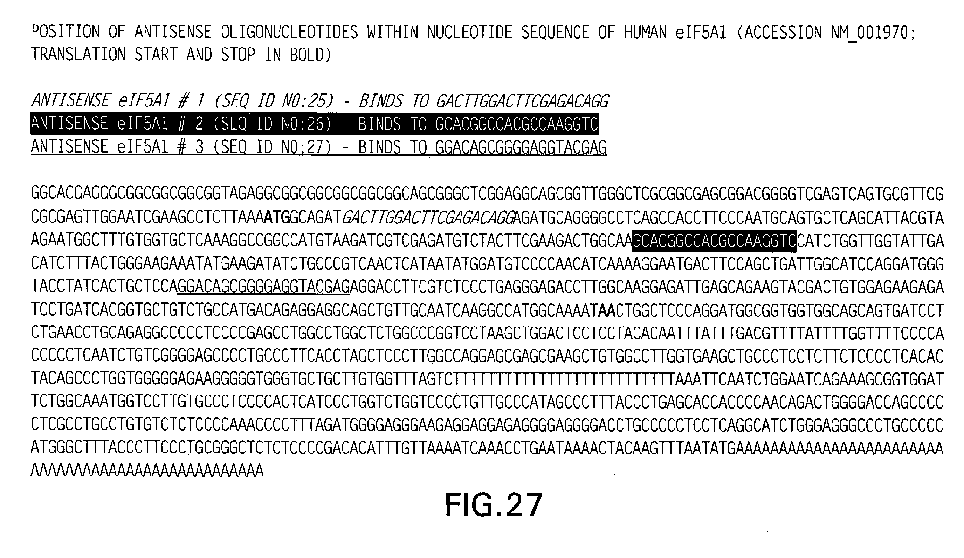

Figure 27 shows the binding position of three antisense oligonucleotides (SEQ ID NO:25-27, respectively in order of appearance) targeted against human apoptosis-specific eIF-5A. The full-length nucleotide sequence is SEQ ID NO: 19.
Figure 28a and b show the nucleotide alignment (SEQ ID NO: 41 and 42, respectively in order of appearance) and amino acid alignment (SEQ ID NO: 43 and 22, respectively in order of appearance) of human apoptosis-specific eIF-5A against human proliferating eIF-5A.

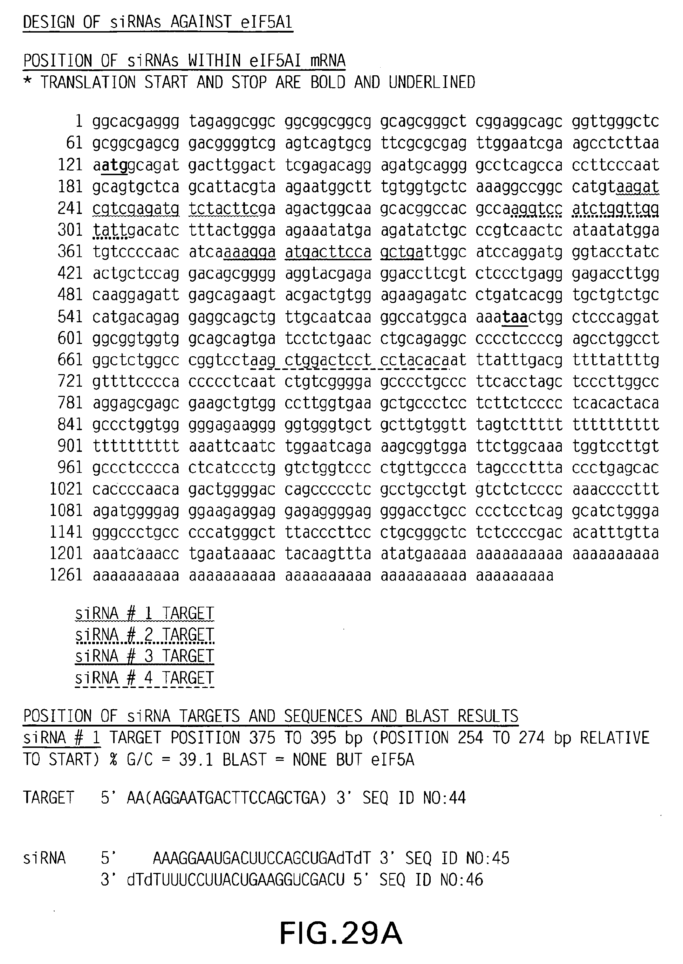

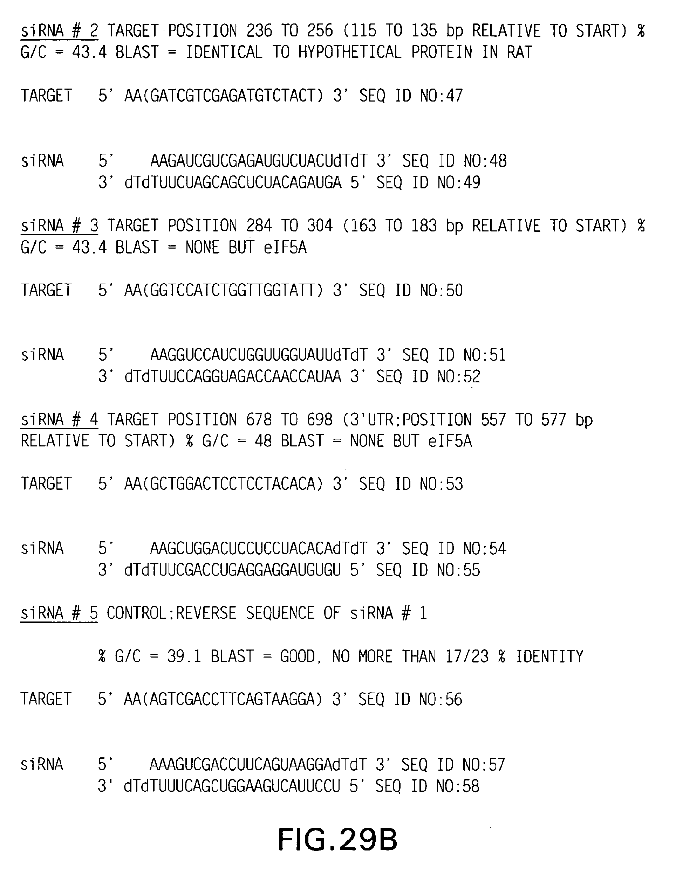

Figure 29 depicts the design of siRNAs against apoptosis-specific eIF-5A. The siRNAs have the SEQ ID NO: 45, 48, 51, 54 and 56. The full-length nucleotide sequence is shown in SEQ ID NO: 29.
Figure 30 shows eIF5A1 expression is increased by genotoxic stress.
Figure 30A provides a Northern blot analysis of eIF5A1 expression in normal colon fibroblasts and
figure 30B provides Western blot of cell lysate isolated from normal colon fibroblasts.
Figure 31 shows that eIF5A1 is not required for cell proliferation.
Figure 32 is a model of eIF5A1 function and regulation. In healthy cells, eIF5A1 is hypusinated by DHS and localized in the cytoplasm. Hypusinated eIF5A1 may support cell growth via some unknown cytoplasmic function. Genotoxic stress or death receptor activation stimulate translocation of eIF5A1 into the nucleus where it participates in the induction or execution of apoptotic cell death. In the event of apoptosis induced by genotoxic stress, nuclear eIF5A1 may function to regulate the expression of p53, possibly by regulating the nuclear export of its mRNA.
Figure 33 shows that HA-tagged eIF5A11 is not hypusinated in vitro.
Figure 34 shows the results of an XTT cell proliferation assay. The results show that siRNA against apoptosis-specific eIF-5A (eIF-5A1) does not inhibit cell division. siRNA directed against cell proliferation eIF-5A (eIF-5A2) inhibits cell division.

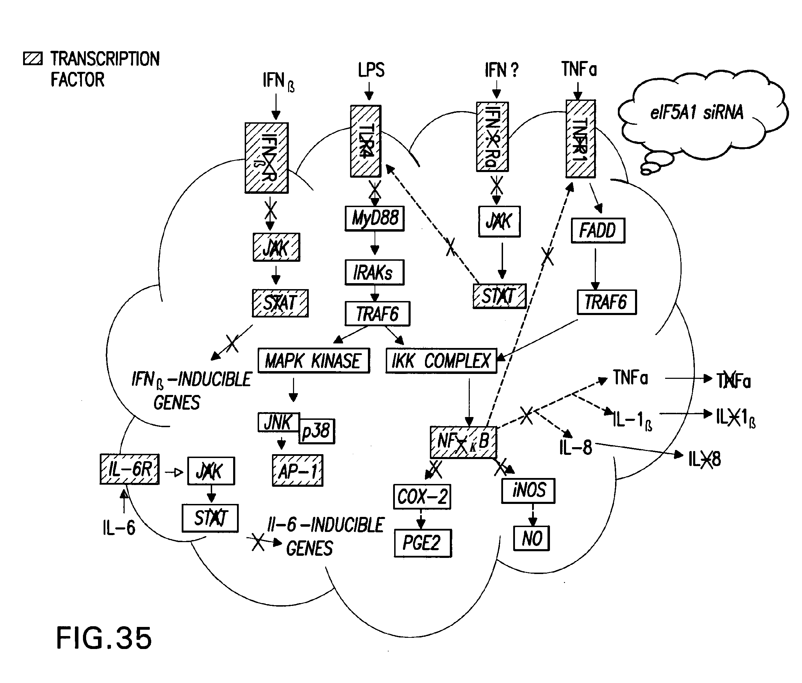

Figure 35 depicts various schemes involved in inflammation and cell apoptosis.
Figures 36-38 are graphs depicting the percentage of apoptosis occurring in RKO and RKO-E6 cells following transient transfection.
Figure 39 provides the results of a flow cytometry analysis of RKO cell apoptosis following transient transfection.
Figure 40 shows the results of an experiment where RKO cells were transfected with apoptosis-specific eIF-5A (eIF-51) siRNA followed by a treatment of Actinomycin D (which induced cells to undergo apoptosis).
Figure 41 provides Western blots of protein extracted from RKO cells treated with 0.25 µg/ml Actinomycin D for 0, 3, 7, 24, and 48 hours.
Figure 42 shows the levels of protein produced by RKO cells after being treated with antisense oligo 1, 2 and 3 (of apoptosis-specific eIF-5A)(SEQ ID NO: 35, 37 and 39, respectively).

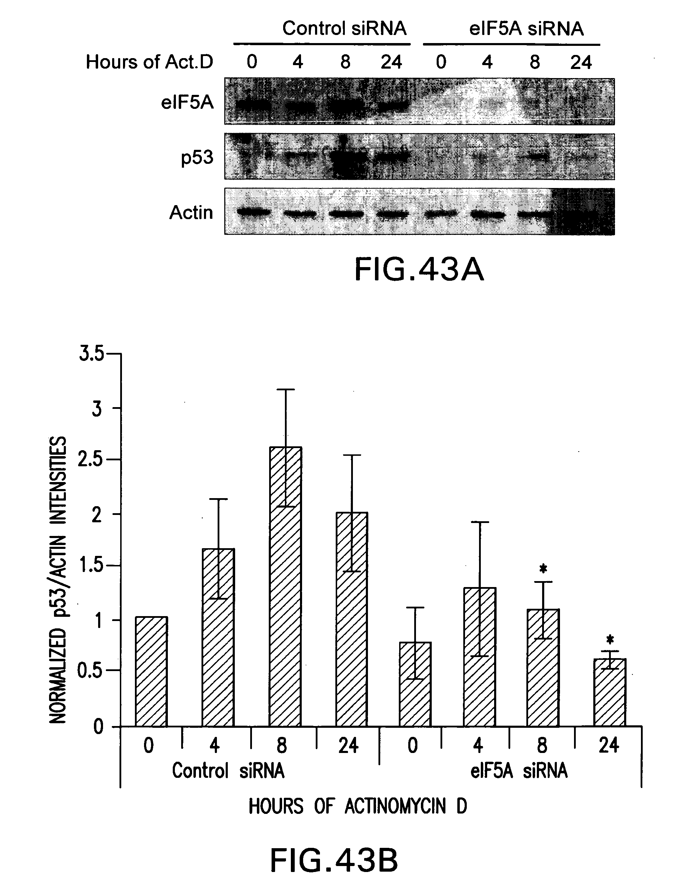

Figure 43 shows that eIF5Al regulates expression of p53 in response to Actinomycin D. RKO cells were transfected with either control siRNA or siRNA directed against eIF5Al. Seventy-two hours after transfection, the cells were treated for 0, 4, 8, or 24 hours with 0.5 µg/ml Actinomycin D. A) Western blot of cell lysates blotted with antibodies against eIF5A1, p53, or β-actin. The result is representative of three independent experiments. B) Plot of the relative intensities of p53 in Western blots that were normalized to the corresponding actin bands. The p53/actin intensity ratios were normalized to the ratio obtained for the 0 hour control, which was set to a value of 1. The values represent means + SE for n = 3. Asterisks (*) denote values considered significantly different from the corresponding control value by paired Student *t*-test (*p* < 0.05).
Figure 44 shows that over-expression of human eIF5A1 induces apoptosis. RKO cells were transfected with pHM6-LacZ or pHM6-eIF5A1. Forty-eight hours after transfection, the cells were fixed and labeled using the TUNEL method to detect DNA fragmentation characteristic of apoptotic cells. The number of apoptotic cells was quantified by flow cytometry analysis. Values are means + SE for n = 3. The asterisk (*) denotes significant difference by paired Student *t*-test (*p* < 0.01).

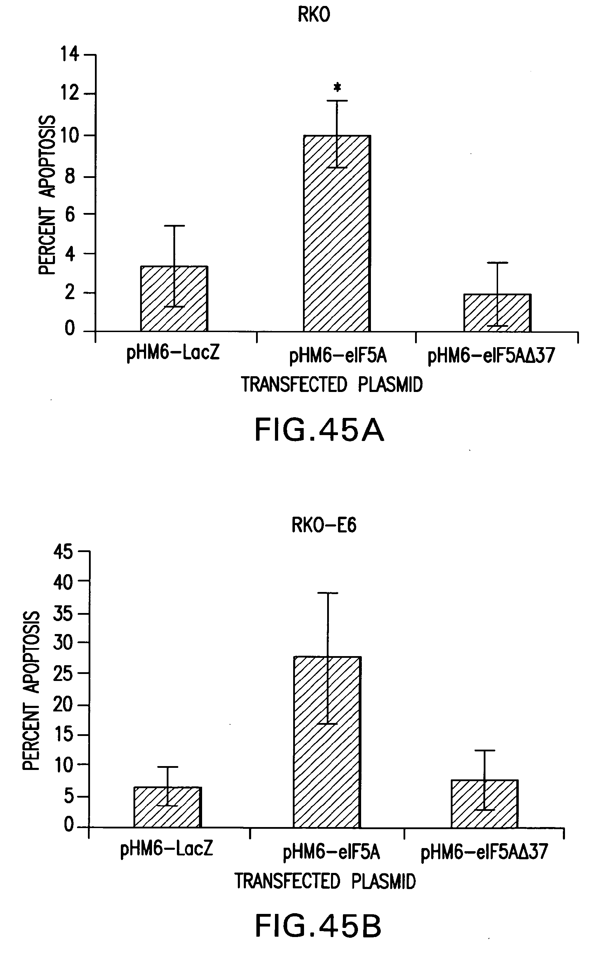

Figure 45 shows that over-expression of human eIF5A1 induces apoptosis independently of p53. RKO cells (A) or RKOE6 cells (B) were transfected with pHM6-LacZ, pHM6 eIF5A1, or pHM6-eIF5A1Δ37 (a 37 amino acid truncation of the C-terminus). Forty-eight hours after transfection, the cells were fixed and labelled using the TUNEL method. The nuclei were stained with Hoescht 33258, and the labelled cells were viewed by fluorescence microscopy. Cells stained bright green were scored as apoptotic. Hoescht-stained nuclei were used to determine the total cell number. Values are means + SE for n = 4 (A) or n = 3 (B). Asterisks (*) denote significant difference from the control (pHM6-LacZ) by paired Student *t*-test (*p* < 0.02).
Figure 46 illustrates enhanced apoptosis as reflected by increased caspase activity when COS-7 cells were transiently transfected with pHM6 containing full-length rat apoptosis-specific eIF-5A in the sense orientation.
Figure 47 illustrates enhanced apoptosis as reflected by increased DNA fragmentation when COS-7 cells were transiently transfected with pHM6 containing full-length rat apoptosis-specific eIF-5A in the sense orientation.
Figure 48 illustrates detection of apoptosis as reflected by increased nuclear fragmentation when COS-7 cells were transiently transfected with pHM6 containing full-length rat apoptosis-specific eIF-5A in the sense orientation.
Figure 49 illustrates enhanced apoptosis as reflected by increased nuclear fragmentation when COS-7 cells were transiently transfected with pHM6 containing full-length rat apoptosis-specific eIF-5A in the sense orientation.
Figure 50 illustrates detection of apoptosis as reflected by phosphatidylserine exposure when COS-7 cells were transiently transfected with pHM6 containing full-length rat apoptosis-specific eIF-5A in the sense orientation.
Figure 51 illustrates enhanced apoptosis as reflected by increased phosphatidylserine exposure when COS-7 cells were transiently transfected with pHM6 containing full-length rat apoptosis-specific eIF-5A in the sense orientation.
Figure 52 illustrates enhanced apoptosis as reflected by increased nuclear fragmentation when COS-7 cells were transiently transfected with pHM6 containing full-length rat apoptosis-specific eIF-5A in the sense orientation.
Figure 53 illustrates enhanced apoptosis when COS-7 cells were transiently transfected with pHM6 containing full-length rat apoptosis-specific eIF-5A in the sense orientation.
Figure 54 illustrates down-regulation of Bcl-2 when COS-7 cells were transiently transfected with pHM6 containing full-length rat apoptosis-specific eIF-5A in the sense orientation. The top photo is the Coomassie-blue-stained protein blot; the bottom photo is the corresponding Western blot.
Figure 55 is a Coomassie-blue-stained protein blot and the corresponding Western blot of COS-7 cells transiently transfected with pHM6 containing full-length rat apoptosis-specific eIF-5A in the antisense orientation using Bcl-2 as a probe.
Figure 56 is a Coomassie-blue-stained protein blot and the corresponding Western blot of COS-7 cells transiently transfected with pHM6 containing full-length rat apoptosis-specific eIF-5A in the sense orientation using c-Myc as a probe.
Figure 57 is a Coomassie-blue-stained protein blot and the corresponding Western blot of COS-7 cells transiently transfected with pHM6 containing full-length rat apoptosis-specific eIF-5A in the sense orientation when p53 is used as a probe.
Figure 58A-C are Coomassie-blue-stained protein blot and the corresponding Western blot of expression of pHM6-full-length rat apoptosis-specific eIF-5A in COS-7 cells using an anti-[HA]-peroxidase probe and a Coomassie-blue-stained protein blot and the corresponding Western blot of expression of pHM6-full-length rat apoptosis-specific eIF-5A in COS-7 cells when a p53 probe is used.
Figure 59 is a bar graph showing that both apoptosis-specific eIF-5A and proliferation eIF-5A are expressed in heart tissue. The heart tissue was taken from patients receiving coronary artery bypass grafts ("CABG"). Gene expression levels apoptosis-specific eIF-5A (light gray bar) are compared to proliferation eIF-5A (dark gray bar). The X-axis is patient identifier numbers.
Figure 60 is a bar graph showing that both apoptosis-specific eIF-5A and proliferation eIF-5A are expressed in heart tissue. The heart tissue was taken from patients receiving valve replacements. Gene expression levels of apoptosis-specific eIF-5A (light gray bar) are compared to proliferation eIF-5A (dark gray bar). The X-axis is patient identifier numbers.
Figure 61 is a bar graph showing the gene expression levels measured by real-time PCR of apoptosis-specific eIF-5A (eIf5a) versus proliferation eIF-5A (eIF5b) in pre-ischemia heart tissue and post ischemia heart tissue.

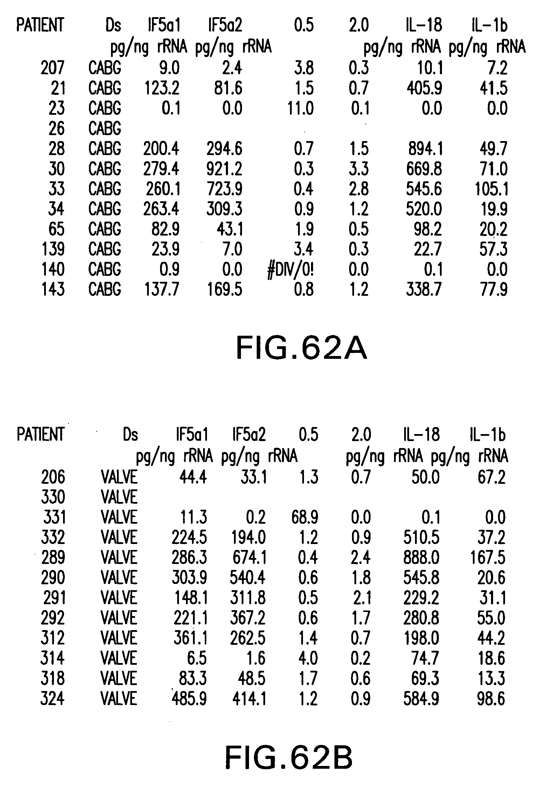

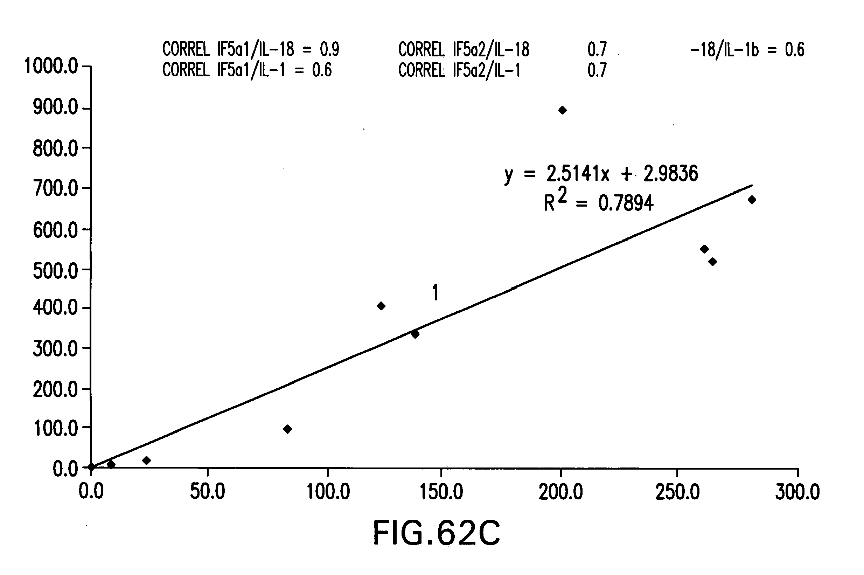

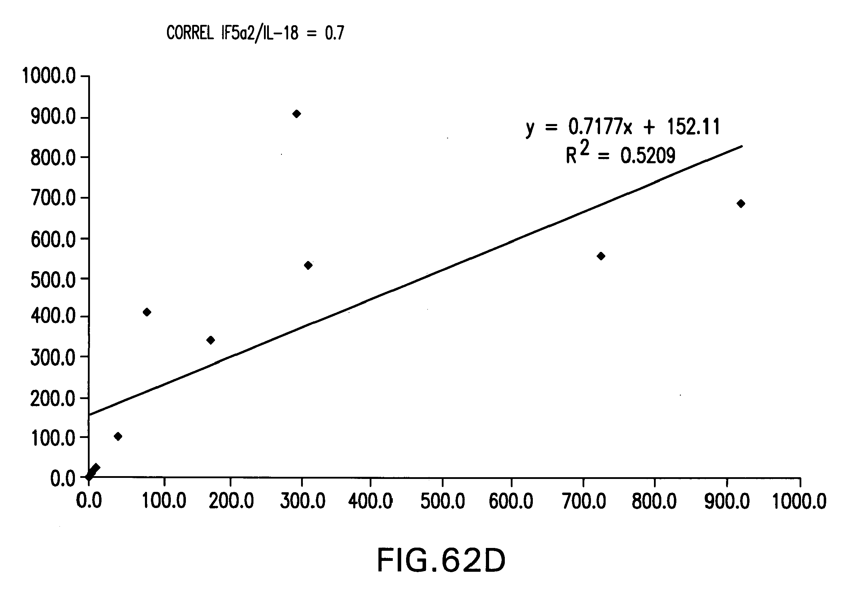

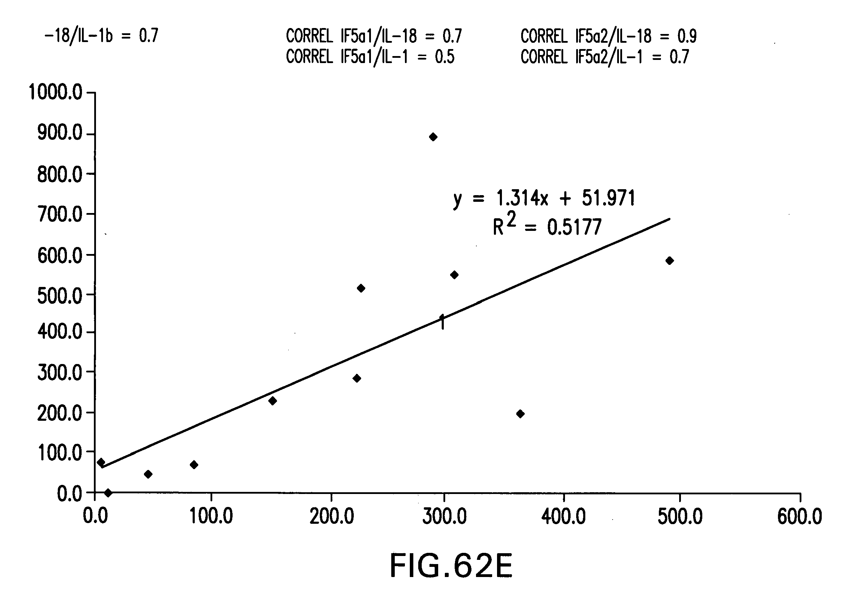

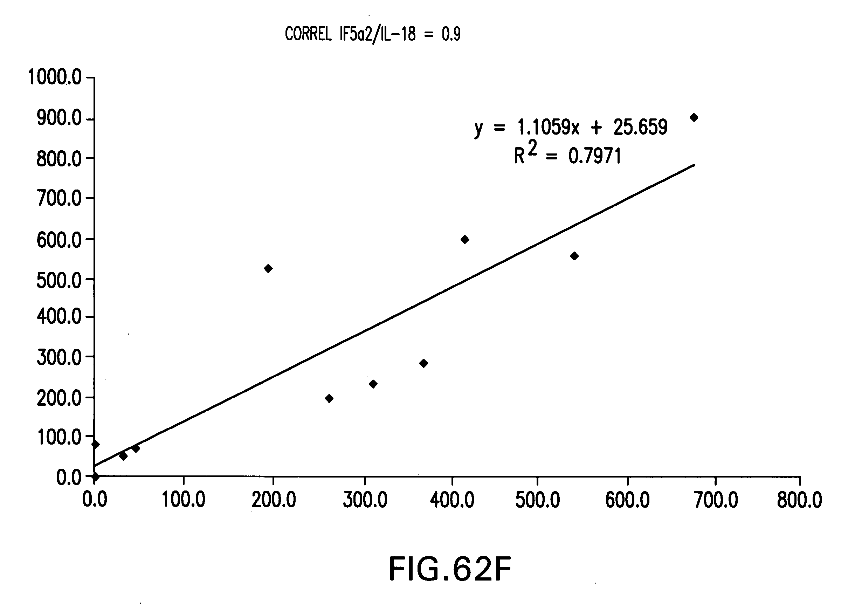

Figures 62A-F report patient data where the levels of apoptosis-specific eIF-5A are correlated with levels of IL-1β and IL-18. Figure 62A is a chart of data obtained from coronary artery bypass graft (CABG) patients. Figure 62B is a chart of data obtained from valve replacement patients. Figure 62C is a graph depicting the correlation of apoptosis-specific eIF-5A to IL-18 in CABG patients. Figure 62D is a graph depicting the correlation of proliferating eIF-5A to IL-18 in CABG patients. Figure 62E is a graph depicting the correlation of apoptosis-specific eIF-5A to IL-18 in valve replacement patients. Figure 62F is a graph depicting the correlation of proliferating eIF-5A to IL-18 in valve replacement patients.

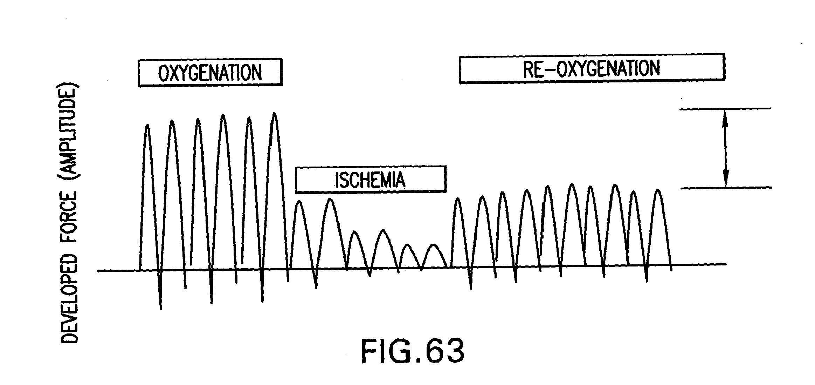

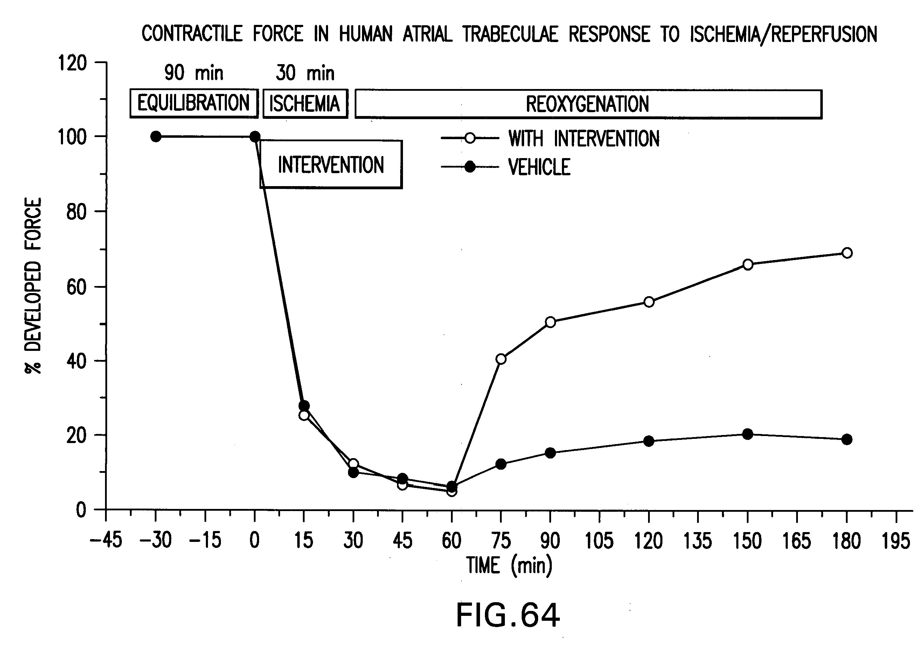

Figure 63-64 shows a diagram of contractile force of heart tissue before, during and after an ischemic event. Post ischemic tissue does not generate as much contractile force as pre-ischemic tissue.
Figure 65 shows localization of I1-18 in human mycocardium.
Figure 66 shows that ischemia/reperfusion induced synthesis of IL-18 in human atrial tissue.
Figure 67 shows that the presence of ICE inhibitor (Interleukin-1β converting enzyme) reduced ischemia/reperfusion injury.
Figure 68 shows that neutralization of IL-18 by IL-18BP (an endogenous inhibitor of IL-18) reduces ischemia/reperfusion injury.
Figure 69 shows that there is a decrease over time in contractile force of heart tissue when exposed to TNF-α.
Figure 70 shows that TNF-α induced myocardial suppression is reduced by IL-18BP.
Figure 71 shows that IL-1β induced mycocardial suppression is reduced by IL-18BP.
Figure 72 shows that creatine kinase activity (CK) is preserved in atrial tissues subjected to ischemia/reperfusion by inhibition of processing of IL-1β and IL-18 or inhibition in IL-1β and IL-18 activity.

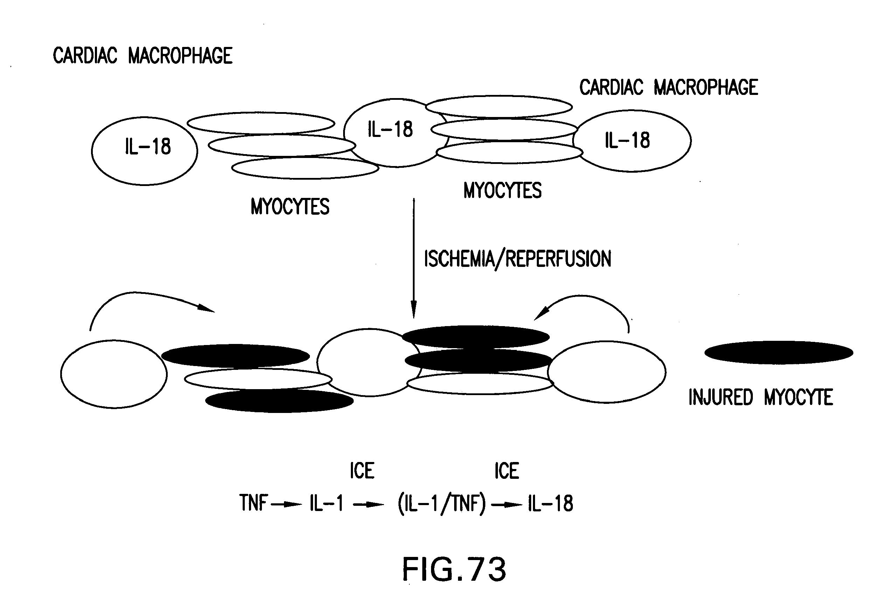

Figure 73 shows a schematic of myocyte injury after ischemia/reperfusion and the TNFα to IL-18 cascade.
Figure 74 shows that apoptosis-specific eIF-5A (eIF-5A1) is up-regulated in ischemic heart tissue in a greater amount than proliferation eIF-5A (eIF-5A2).

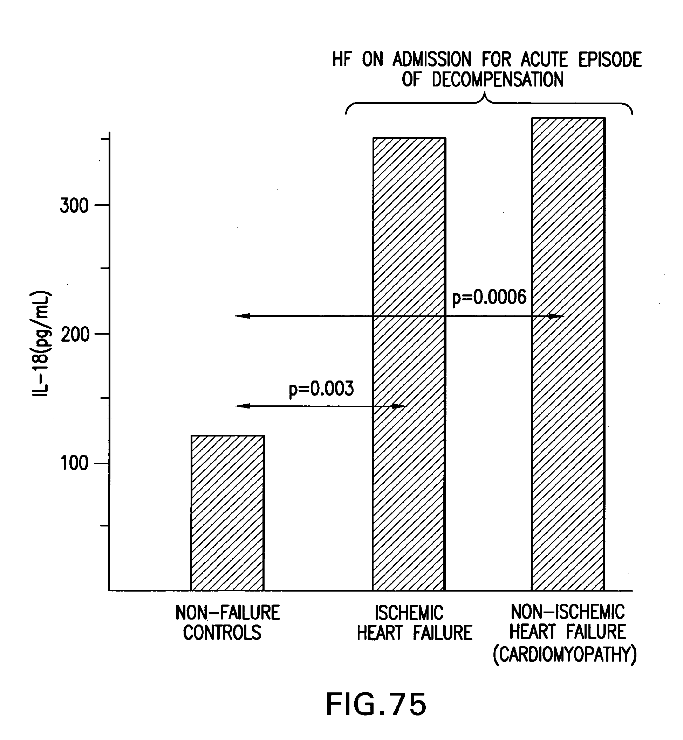

Figure 75 shows that in both ischemic and non-ischemic heart failure, there is an increase in IL-18 expression

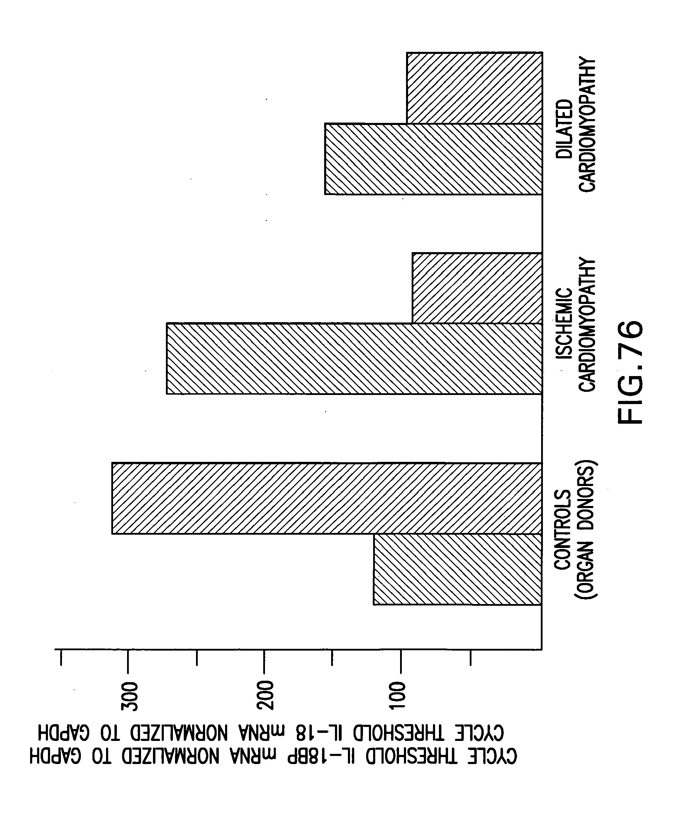

Figure 76 shows that in ischemic and dilated cardiomyopathy the relative expression of IL-18 is increased whereas the expression ofIL-18BP (an endogenous inhibitor ofIL-18) is decreased as compared to normal heart tissue.
Figure 77A and B show uptake of the fluorescently labeled antisense oligonucleotide.
Figures 78 - 82 show a decrease in the percentage of cells undergoing apoptosis in the cells having being treated with antisense apoptosis-specific eIF-5A oligonucleotides as compared to cells not having been transfected with the antisense apoptosis-specific eIF-5A oligonucleotides.
Figure 83 shows that treating lamina cribrosa cells with TNF-α and/or camptothecin caused an increase in the number of cells undergoing apoptosis.
Figure 84 and 85 show a decrease in the percentage of cells undergoing apoptosis in the cells having being treated with antisense apoptosis-specific eIF-5A oligonucleotides as compared to cells not having been transfected with the antisense apoptosis-specific eIF-5A oligonucleotides.
Figure 86A and B show that the lamina cribrosa cells uptake the labeled siRNA either in the presence of serum or without serum.
Figures 87-89 show that lamina cribosa cells transfected with apoptosis-specific eIF-5A siRNA had a lower percentage of cells undergoing apoptosis after exposure to amptothecin and TNF-α than untransfected cells.
Figure 90 are photographs of Hoecht-stained lamina cribrosa cell line # 506 transfected with siRNA and treated with camptothecin and TNF-α from the experiment described in figure 89 and Example 13. The apoptosing cells are seen as more brightly stained cells. They have smaller nucleic because of chromatin condensation and are smaller and irregular in shape.
Figure 91 is a characterization of lamina cribrosa cells by immunofluorescence.
Figure 92 is a graph showing percent apoptosis of lamina cribrosa cell line # 506 in response to treatment with camptothecin and TNF-α.
Figure 93 shows expression levels of against apoptosis-specific eIF-5A during camptothecin or TNF-a plus camptothecin treatment.
Figure 94 shows expression levels of apoptosis-specific eIF-5A in lamina cribosa cell lines # 506 and # 517 following transfection with siRNAs.
Figure 95 shows the percent apoptosis of lamina cribosa cell line # 506 cells transfected with apoptosis-specific eIF-5A siRNAs and treated with TNF-α and camptothecin.
Figure 96 shows percent apoptosis of lamina cribosa cell line #517 cells transfected with apoptosis-specific eIF-5A siRNA # 1 and treated with TNF-α and camptothecin.
Figure 97a-d show TUNEL-labeling of lamina cribosa cell line # 506 cells transfected with apoptosis-specific eIF-5A siRNA # 1 and treated with TNF-α and camptothecin. Panel A represents the slide observed by fluorescence microscopy using a fluorescein filter to visualize TUNEL-labeling of the fragmented DNA of apoptotic cells. Panel B represents the same slide observed by through a UV filter to visualize the Hoescht-stained nuclei.
Figure 98 shows that cells transfected with apoptosis-specific eIF-5A siRNA produced less apoptosis-specific eIF-5A protein and in addition, produced more Bcl-2 protein. A decrease in apoptosis-specific eIF-5A expression correlates with an increase in BCL-2 expression.
Figure 99 shows that cells transfected with apoptosis-specific eIF-5A siRNA produced less apoptosis factor 5a protein.
Figure 100 shows that IL-1 exposed HepG2 cells transfected with apoptosis-specific eIF-5A cells secreted less TNF-α than non-transfected cells.
Figure 101A provides a picture of a Western blot where siRNAs against apoptosis-specific eIF-5A have reduced if not inhibited the production of TNF-α in transfected HT-29 cells. Figure 101B provides the results of an ELISA.
Figure 102 provides the results of an ELISA. TNF-α production was reduced in cells treated with siRNAs against apoptosis-specific eIF-5A as compared to control cells.
Figure 103 is a bar graph showing that IL-8 is produced in response to TNF-α as well as in response to interferon. This graph shows that siRNA against apoptosis-specific eIF-5A blocked almost all IL-8 produced in response to interferon as well as a significant amount of the IL-8 produced as a result of the combined treatment of interferon and TNF.
Figure 104 is another bar graph showing that IL-8 is produced in response to TNF-alpha as well as in response to interferon. This graph shows that siRNA against apoptosis-specific eIF-5A blocked almost all IL-8 produced in response to interferon as well as a significant amount of the IL-8 produced as a result of the combined treatment of interferon and TNF.
Figure 105 is a western blot of HT-29 cells treated with IFN gamma for 8 and 24 hours. This blot shows up-regulation in HT-29 cells (4 fold at 8 hours) of against apoptosis-specific eIF-5A in response to interferon gamma.
Figure 106 shows the results of an experiment where siRNAs directed against apoptosis-specific eIF-5A provided for a reduction in NKkB activation in the presence of interferon gamma and LPS.

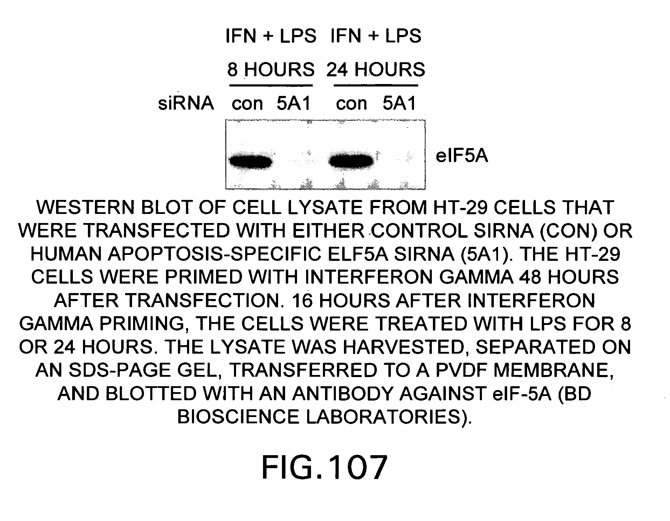

Figure 107 shows a western blot of cell lysate from HT-29 cells that were transfected with either control siRNA or apoptosis-specific eIF-5A siRNAs. This figure shows that siRNAs of apoptosis-specific eIF-5A inhibit expression of apoptosis-specific eIF-5A.
Figure 108 shows that HT-29 cells transfected with apoptosis-specific eIF-5A siRNAs have a reduced level of TNF production.
Figure 109 shows that HT-29 cells transfected with apoptosis-specific eIF-5A siRNAs exhibit a decreased in apoptosis as compared to control cells. Both control and siRNA-transfected cells were primed with interferon gamma and also treated with TNF-α.

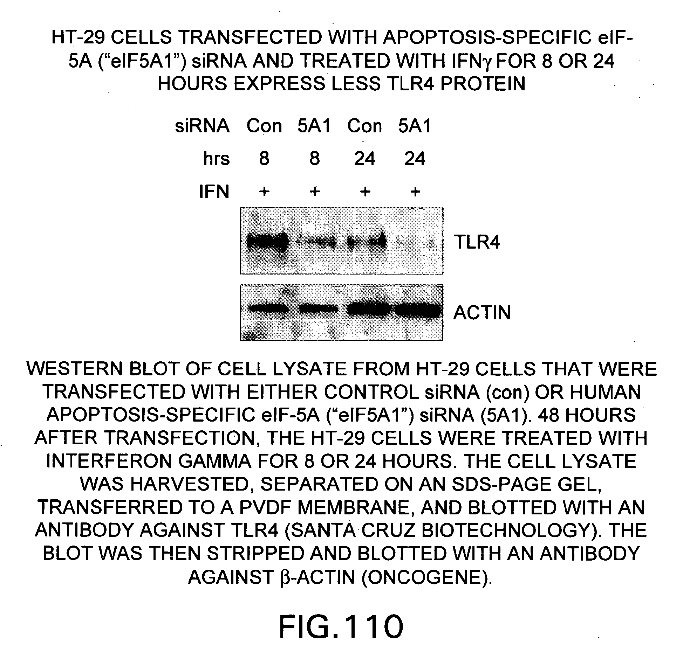

Figure 110 shows that HT-29 cells transfected with apoptosis-specific eIF-5A siRNAs express less TLR4 protein than control cells.
Figure 111 shows that HT-29 cells transfected with apoptosis-specific eIF-5A siRNAs express less TNFR1 protein than control cells.
Figure 112 shows that HT-29 cells transfected with apoptosis-specific eIF-5A siRNAs express less iNOS protein than control cells.
Figure 113 shows that HT-29 cells transfected with apoptosis-specific eIF-5A siRNAs express less TLR4 mRNA than control cells.
Figure 114 shows that in HT-29 cells exposed to IFN-γ and LPS, transfection with siRNAs against apoptosis-specific eIF-5A causes a decrease in NFκB p50 activation and TNF-α production.

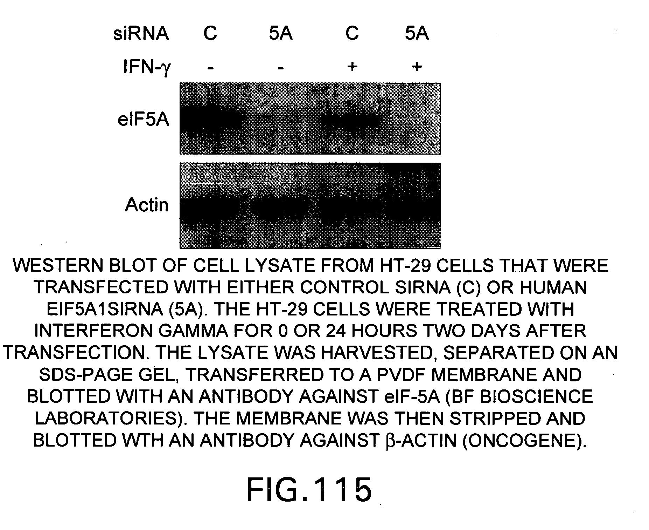

Figure 115 shows that siRNAs against apoptosis-specific eIF-5A suppress expression of endogenous apoptosis-specific eIF-5A in HT-29 cells.
Figure 116 shows that in HT-29, cells siRNA-mediated suppression of apoptosis-specific eIF-5A reduces IFN-γ Receptor-α accumulation in response to IFN-γ.
Figure 117 shows that in HT-29 cells, siRNA-mediated suppression of apoptosis-specific eIF-5A reduces Toll Receptor 4 (TLR4) accumulation in response to IFN-γ.
Figure 118 shows that in HT-29 cells, siRNA-mediated suppression of apoptosis-specific eIF-5A reduces JAK1 and STAT1 phosphorylation in response to IFN-γ.

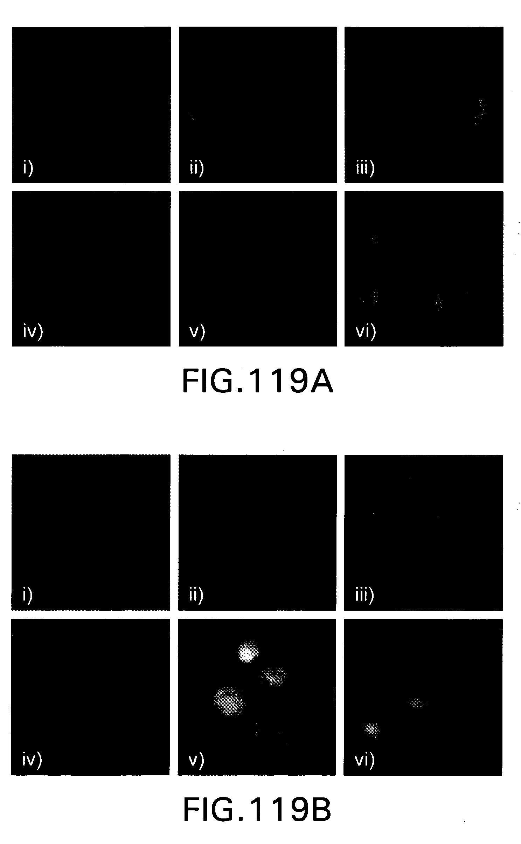

Figure 119 shows mmunofluorescent localization of eIF5A1. The subcellular localization of eIF5A1 protein in HT-29 cells stimulated with IFN-γ and TNF-α (A) or Actinomycin D (B) was determined by indirect immunofluorescence. A) HT-29 cells were either untreated (i) or primed with IFN-γ for 16 hours before stimulating with TNF-α for 0 min. (ii), 10 min. (iii), 30 min. (iv), 90 min.(v), or 8 hours (vi). B) HT-29 cells were either untreated (i) or treated with Actinomycin D for 30 min. (ii), 90 min. (iii), 4 hours (iv), 8 hours (v), or 16 hours (vi). All photographs were taken at 400 X magnification. The results are representative of three independent experiments.
Figure 120 shows the time course for PBMC experiments (see Example 18).
Figure 121 shows a Western blot of a cell lysate from PBMCs collected from two donors over a time course. The PBMCs were treated with PMA and subsequently stimulated with LPS to have an increased apoptosis-specific eIF-5A expression.
Figure 122 shows that PBMCs treated with PMA and subsequently stimulated with LPS have an increased apoptosis-specific eIF-5A expression, which coincides with increased TNF production.
Figure 123 demonstrates that PBMCs respond to LPS without PMA differentiation.
Figure 124 shows that PBMCs transfected with apoptosis-specific eIF-5A siRNAs demonstrate suppression of expression of apoptosis-specific eIF-5A.
Figure 125 shows that PBMCs transfected with apoptosis-specific eIF-5A siRNAs and stimulated with LPS produce less TNF than PBMCs not transfected with apoptosis-specific eIF-5A siRNAs.
Figure 126 shows the time course of the U-937 differentiation experiment. See Example 16.
Figure 127 shows the results of a Western blot showing that apoptosis-specific eIF-5A is up-regulated during monocyte differentiation and subsequence TNF-α secretion.

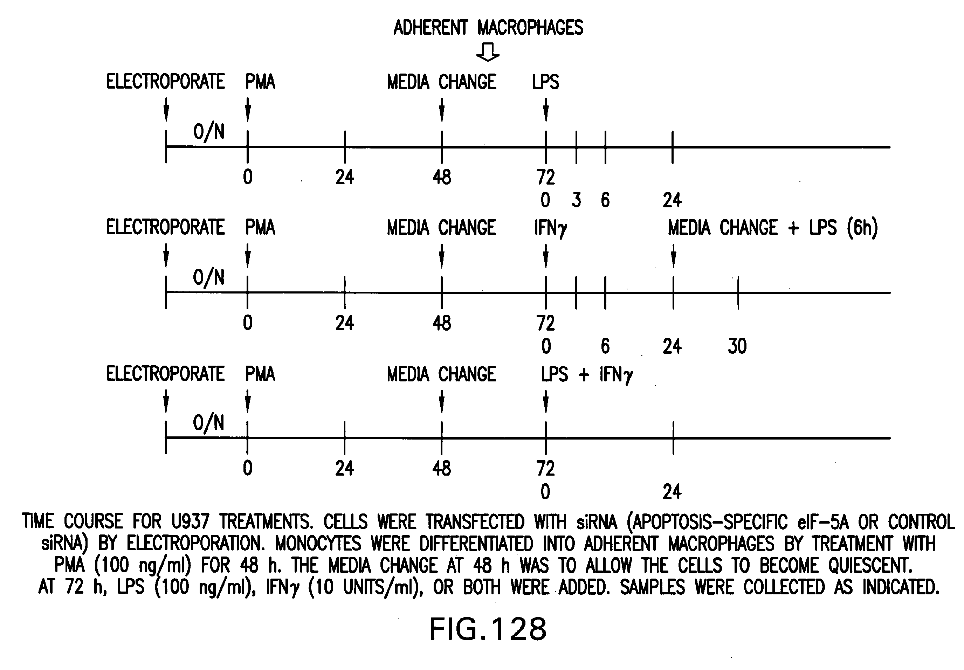

Figure 128 shows the time course for U937 treatments.
Figure 129 shows that apoptosis-specific eIF-5A is upregulated with PMA in U937 cells.
Figure 130 shows that apoptosis-specific eIF-5A is upregulated with LPS in U937 cells.
Figure 131 shows that apoptosis-specific eIF-5A protein expression is still reduced after numerous hours following siRNA treatment.
Figure 132 shows that siRNA mediated down-regulation of apoptosis-specific eIF-5A coincides with a reduction of TLR4.
Figure 133 shows that siRNA mediated down-regulation of apoptosis-specific eIF-5A coincides with fewer glycosylated forms of the interferon gamma receptor in U937 cells.
Figure 134 shows that siRNA mediated down-regulation of apoptosis-specific eIF-5A coincides with a reduction in TNFR1 in U937 cells.
Figure 135 shows that siRNA mediated down-regulation of apoptosis-specific eIF-5A coincides with a reduction in LPS-induced TNF-α, production in U937 cells.
Figure 136 shows that siRNA mediated down-regulation of apoptosis-specific eIF-5A coincides with a reduction in LPS-induced IL-1β production in U937 cells.
Figure 137 shows that siRNA mediated down-regulation of apoptosis-specific eIF-5A coincides with a reduction in LPS-induced IL-8 production in U937 cells.
Figure 138 shows that IL-6 production is independent of siRNA mediated down-regulation of apoptosis-specific eIF-5A in U937 cells.
Figure 139 shows that intraveneous delivery of siRNAs directed against apoptosis-specific eIF-5A cause a decrease in levels of TNF-α in the serum.
Figure 140 shows that transnasal delivery of siRNAs directed against apoptosis-specific eIF-5A cause a decrease in levels of TNF-α in the lung.
Figure 141 shows that transnasal delivery of siRNAs directed against apoptosis-specific eIF-5A cause a decrease in levels of MIP-1 in the lung.
Figure 142 shows that intranasal delivery of siRNAS directed against apoptosis-specific eIF-5A cause a decrease in the levels of IL-1α.
Figure 143 shows that after mice received LPS and eIF-5A1 siRNA intranasaly had a reduced myeloperoxidase activity than mice receiving control siRNA.
Figure 144 shows that nasal-LPS-induced loss of thymocyes is blocked by pre-treatment with apoptosis-specific eIF-5A siRNA.

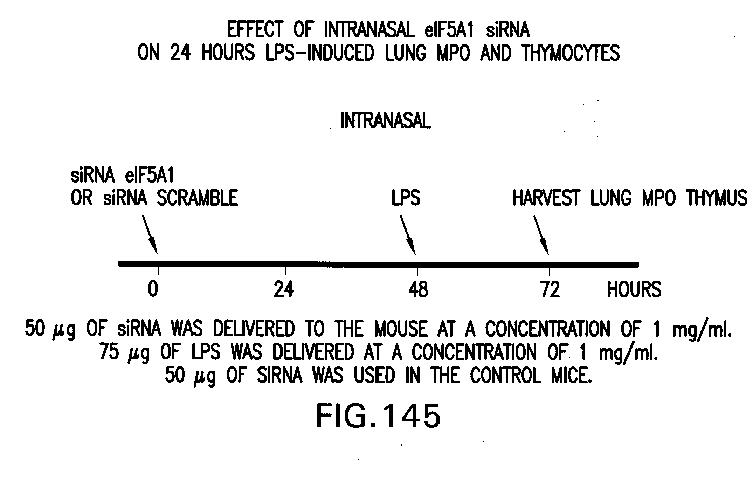

Figure 145 shows the time course for experiments with intranasal delivery of apoptosis-specific eIF-5A siRNA.
Figure 146 shows that nasal-LPS-induced loss of thymocyes is blocked by pre-treatment with apoptosis-specific eIF-5A siRNA.
Figure 147 shows that siRNA against eIF-5A decreased production of IL-6, IFN-γ and Il-1α.
Figure 148 shows that siRNA against eIF-5A is able to reduce the expression of TNFα as a result of treatment with LPS. The top panel shows the raw data and the bottom panel shows the data in a bar graph.
Figure 149 shows the results of an experiment where septic Balb/C mice were treated with different concentrations of siRNA and at different times.
Figure 150 shows the results of figure 133 in a different format.
Figure 151 shows the results of an experiment where septic C57BL/6 mice were treated with different concentrations of siRNA and at different times.
Figure 152 shows the results of figure 151 in a different format.
Figure 153-155 show the results of a combined sepsis survival study in Balb/C mice. This study shows that mice receiving apoptosis-specific eIF-5A survived longer than control mice.
Figures 156-158 show the results of a combined sepsis survival study in C57BL/6 mice. This study shows that mice receiving apoptosis-specific eIF-5A siRNA survived longer than control mice.
Figure 159 summarized the sepsis study, showing that animals treated with apoptosis-specific eIF-5A siRNA had a better chance of survival.
Figure 160 shows the construct of the siRNA used in the septic mice models.
Figure 161 is a picture of a tumor and non-tumor (healthy) tissue treated with control siRNA and showing that in the cancerous tissue, there is little or no apoptosis.
Figures 162-170 show that in tumors treated with apoptosis-specific eIF-5A siRNA there is strong apoptosis but there is none in the non-tumorous tissue. Various tissues are shown (kidney in Figure 167, liver in Figure 168, heart in Figure 169 and spleen in Figure 170)
Figure 171 shows a graph demonstrating that systemically injected ad5orioP.lucerferase is selectively expressed in nasopharyngeal xenograph tumors.

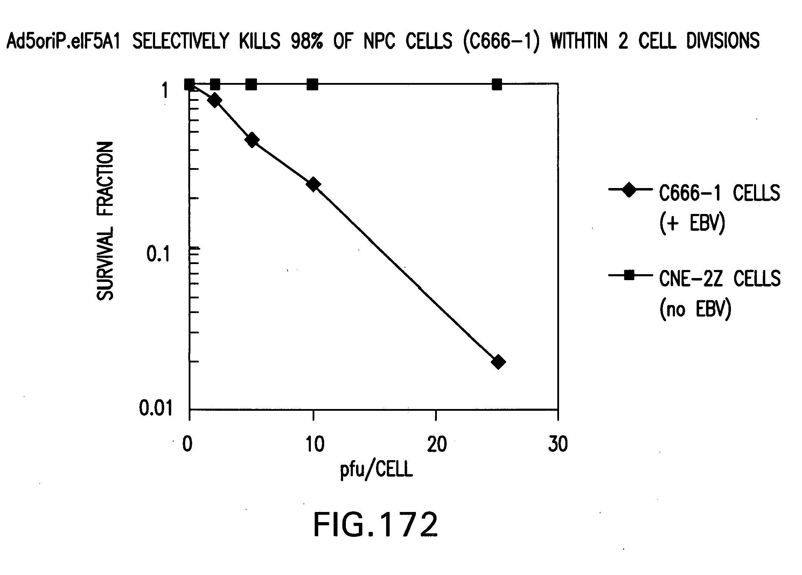

Figure 172 shows that ad5orioP.eIF-5A1 selectively kills 98% of nasopharyngeal cancer cells (C666-1) within two cell divisions.
Figure 173 shows the results of an experiment where cancer cells were injected into mice and resulted in the development of lung cancer. Mice injected with eIF-5A showed a decrease in the amount of cancerous cells as compared to control mice.
Figure 174 shows that cancerous lung tissue treated with apoptosis-specific eIF-5A shows a decrease in the amount of cancerous tissue as compared to control lungs not treated with apoptosis-specific eIF-5A.
Figure 175 shows the weight of the lungs used in the lung cancer experiments.
Figure 176 depicts stem cell differentiation and the use of siRNAs against apoptosis-specific eIF-5A to inhibit cytokine production.

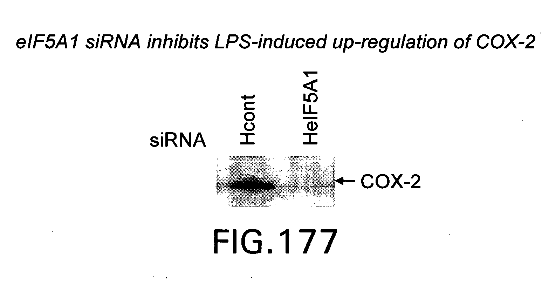

Figure 177 shows that apoptosis-specific eIF-5A siRNA inhibits LPS-induced up-regulation of COX-2.
Figure 178 shows the results of an experiment where eIF-5A1 was able to down-regulate the expression of VEGF.
Figure 179 shows that over-expressing apoptosis-specific eIF-5A in a synovial sarcoma cell line decreases the production of VEGF by the tumor cells.

### DETAILED DESCRIPTION OF THE INVENTION

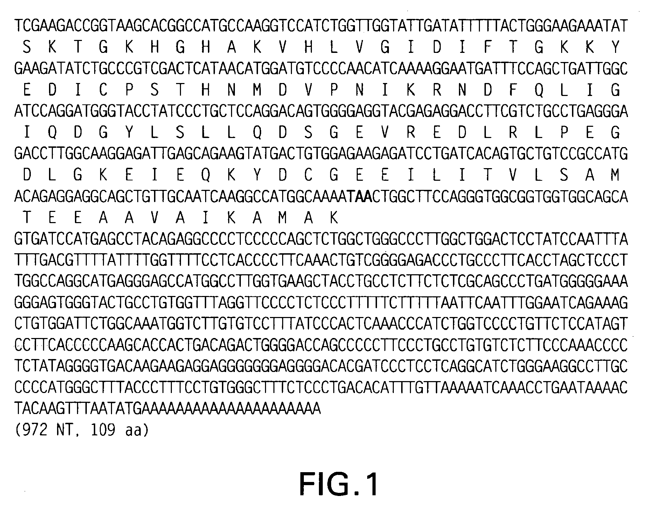

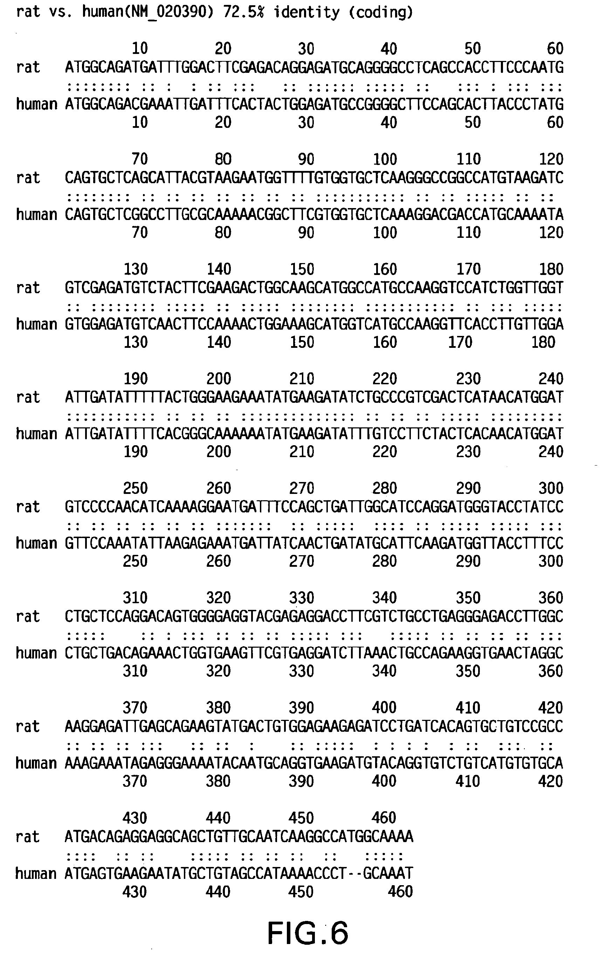

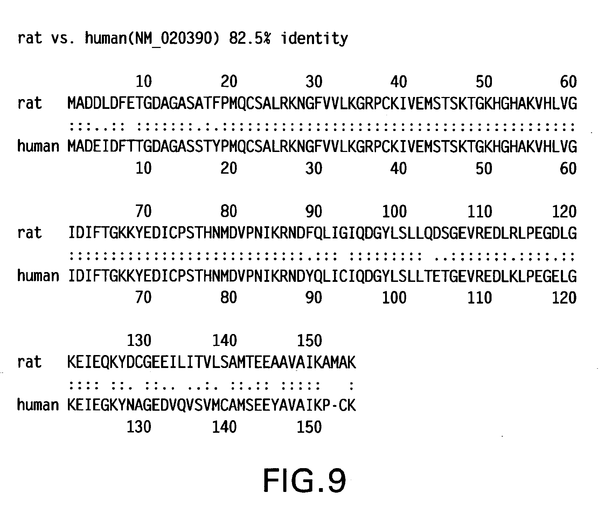

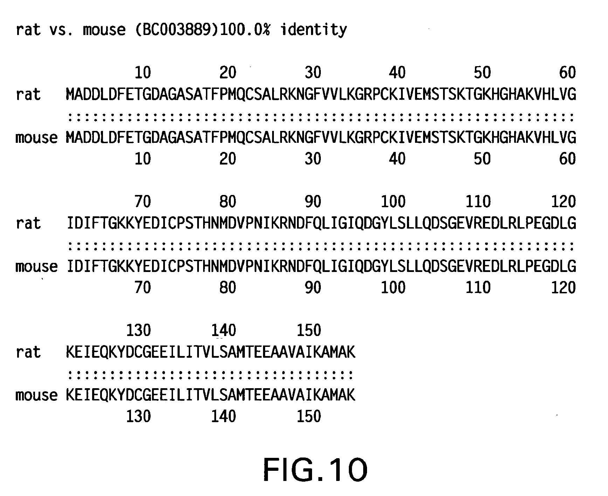

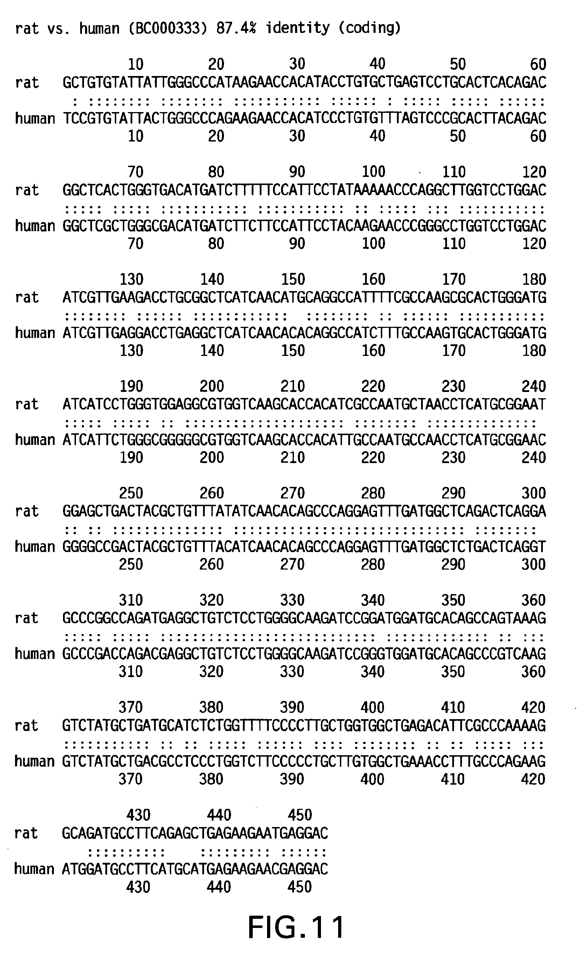

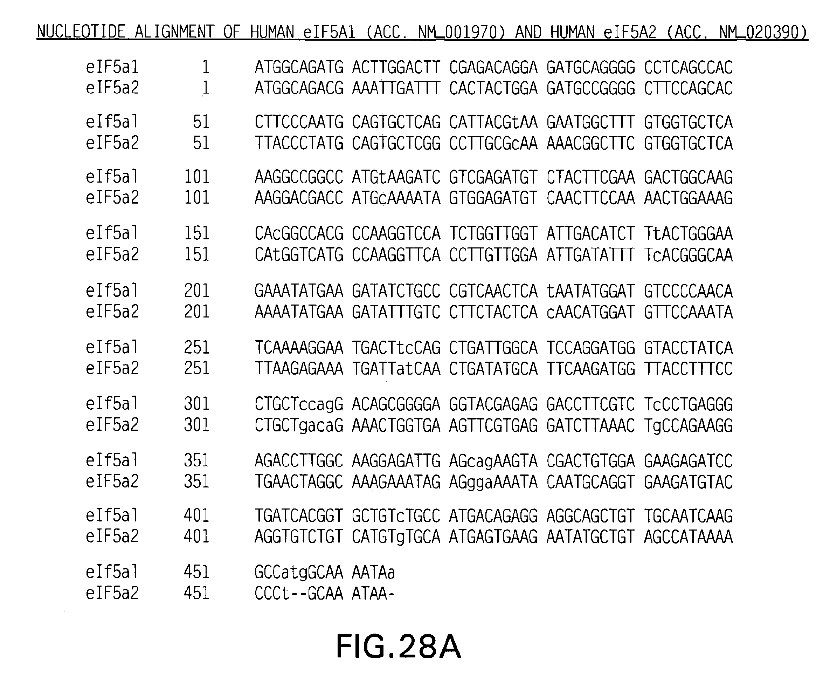

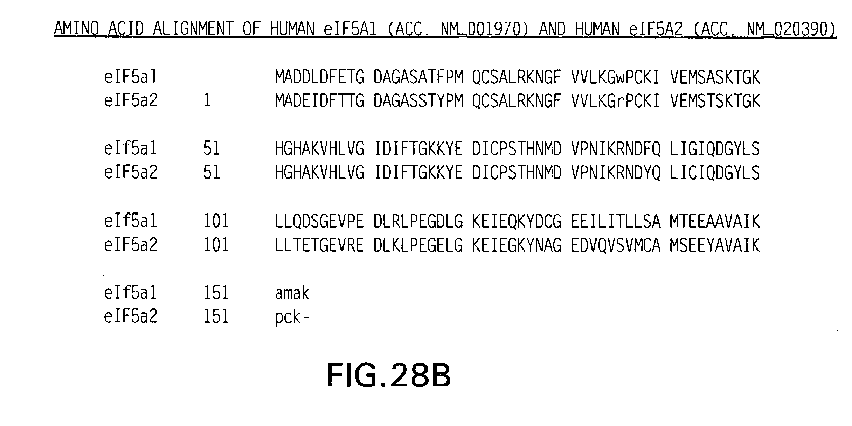

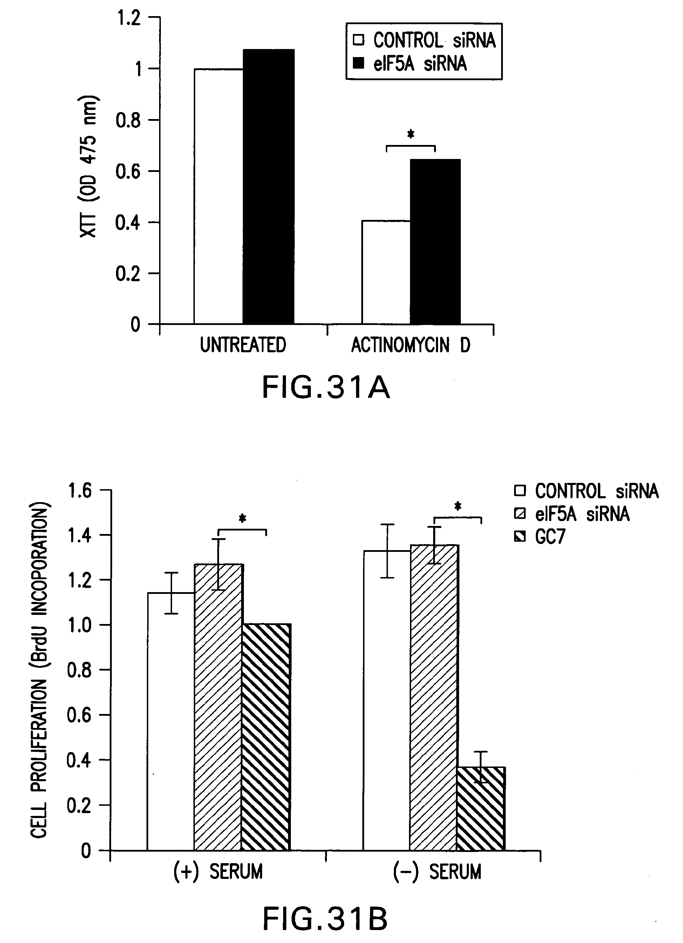

Several isoforms of eukaryotic initiation factor 5A ("eIF-5A") have been isolated and present in published databanks. It was thought that these isoforms were functionally redundant. The present inventors have discovered that one isoform is upregulated immediately before the induction of apoptosis, which they have designated apoptosis-specific eIF-5A or eIF-5A1. The subject of the present invention is apoptosis-specific eIF-5A. Figures 1-11 show the sequence (nucleotide and amino acid) of rat, mouse and human eIF-5A. The other isoform is believed to be involved in cellular proliferation and is names proliferation eIF-5A or eIF-5As. Figure 28 shows the comparison of apoptosis-specific eIF-5A with proliferation eIF-5A (eIF-5A2). Figure 31 shows that apoptosis-specific eIF-5A is not required for cell proliferation. In figure 31A, the metabolic activity of cells transfected with eIF5A1 siRNA was measured using an XTT cell proliferation assay. HT-29 cells were seeded on a 96-well plate 24 hours before transfection with either control siRNA or eIF5A1 siRNA. Twenty-four hours after transfection, the cells were either left untreated or treated with Actinomycin D (1.0 µg/ml) for 48 hours before measuring metabolic activity. Values are means for two experiments performed in quadruplicate and were normalized to the value obtained for the 0 hour control which was set at 1. Figure 31B shows where the proliferative ability of HT-29 cells transfected with control or eIF5A1 siRNA was compared to that of cells incubated with 50 µM GC7 for 72 hours. Cell proliferation was measured by BrdU incorporation. Values are means + SE for n = 4 and were normalized to the value for the GC7 (+) serum sample which was set at 1. Asterisks (*) denote values considered significantly different by paired Student *t*-test (*p* < 0.01).

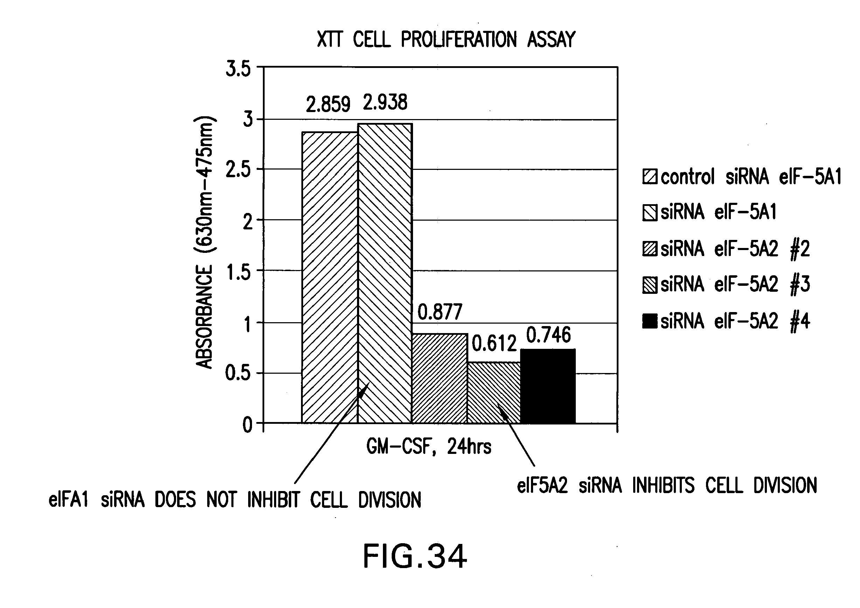

Figure 34 shows that siRNA sgainst apoptosis-specific eIF-5A does not inhibit cell division whereas siRNA directed against cell proliferation eIF-5A inhibits cell division.

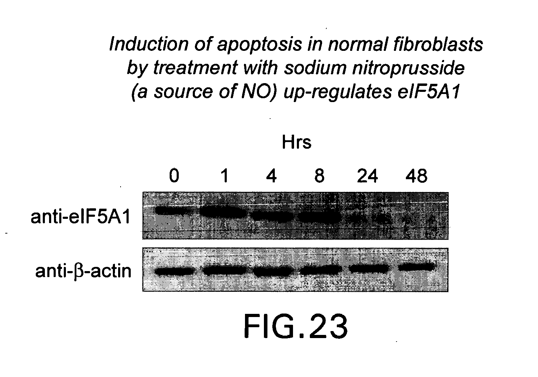

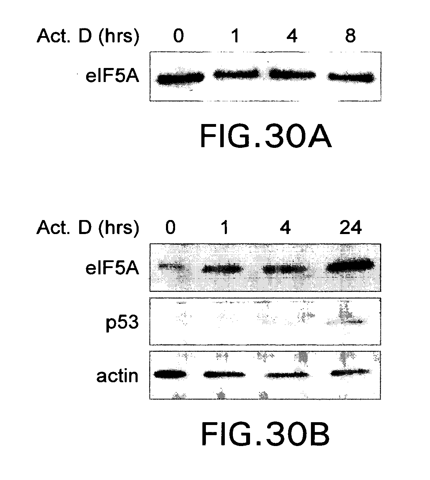

Apoptosis-specific eIF-5A is up-regulated during cellular apoptosis. Figure 23 shows that there is an increase in expression of apoptosis-specific eIF-5A after induction of apoptosis with sodium nitroprusside in normal fibroblasts. Figure 30 shows that apoptosis-specific eIF-5A is up-regulated by genotoxic stress. Figure 30A provides Northern blot analysis of eIF5A1 expression in normal colon fibroblasts treated with 0.5 µg/ml Actinomycin D for 0, 1, 4, and 8 hours. Figure 30B provides Western blot of cell lysate isolated from normal colon fibroblasts treated with 0.5 µg/ml Actinomycin D for 0, 1, 4, and 24 hours. The blot was probed with antibodies against eIF5A1, p53, and β-actin. This figures shows that there is an increase in eIF-5A protein after treatment with actinomycin D.

Apoptosis-specific eIF-5A is likely to be a suitable target for intervention in apoptosis-causing disease states since it appears to act at the level of post-transcriptional regulation of downstream effectors and transcription factors involved in the apoptotic pathway. Specifically, apoptosis-specific eIF-5A appears to selectively facilitate the translocation of mRNAs encoding downstream effectors and transcription factors of apoptosis from the nucleus to the cytoplasm, where they are subsequently translated. The ultimate decision to initiate apoptosis appears to stem from a complex interaction between internal and external pro- and anti-apoptotic signals. Lowe & Lin (2000) Carcinogenesis, 21, 485-495. Through its ability to facilitate the translation of downstream apoptosis effectors and transcription factors, apoptosis-specific eIF-5A appears to tip the balance between these signals in favor of apoptosis.

Accordingly, the present invention provides a method of suppressing or reducing apoptosis in a cell by administering an agent that inhibits or reduces expression of apoptosis-specific eIF-5A. One agent that can inhibit or reduce expression of apoptosis-specific eIF-5A are antisense oligonucleotides of apoptosis-specific eIF-5A. By reducing or inhibiting expression of apoptosis-specific eIF-5A, cellular apoptosis can be delayed or inhibited.

Antisense oligonucleotides have been successfully used to accomplish both *in vitro* as well as *in vivo* gene-specific suppression. Antisense oligonucleotides are short, synthetic strands of DNA (or DNA analogs), RNA (or RNA analogs), or DNA/RNA hybrids that are antisense (or complimentary) to a specific DNA or RNA target. Antisense oligonucleotides are designed to block expression of the protein encoded by the DNA or RNA target by binding to the target mRNA and halting expression at the level of transcription, translation, or splicing. By using modified backbones that resist degradation (Blake *et al.,* 1985), such as replacement of the phosphodiester bonds in the oligonucleotides with phosphorothioate linkages to retard nuclease degradation (Matzura and Eckstein, 1968), antisense oligonucleotides have been used successfully both in cell cultures and animal models of disease (Hogrefe, 1999). Other modifications to the antisense oligonucleotide to render the oligonucleotide more stable and resistant to degradation are known and understood by one skilled in the art. Antisense oligonucleotide as used herein encompasses double stranded or single stranded DNA, double stranded or single stranded RNA, DNA/RNA hybrids, DNA and RNA analogs, and oligonucleotides having base, sugar, or backbone modifications. The oligonucleotides may be modified by methods known in the art to increase stability, increase resistance to nuclease degradation or the like. These modifications are known in the art and include, but are not limited to modifying the backbone of the oligonucleotide, modifying the sugar moieties, or modifying the base.

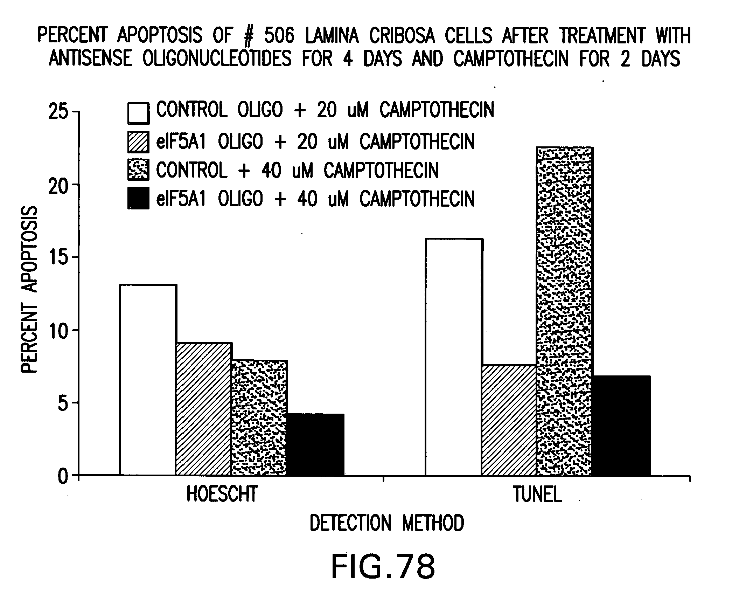

Preferably, the antisense oligonucleotides of the present invention have a nucleotide sequence encoding a portion or the entire coding sequence of an apoptosis-specific eIF-5A polypeptide. The inventors have transfected various cell lines with antisense nucleotides encoding a portion of an apoptosis-specific eIF-5A polypeptide as described below and measured the number of cells undergoing apoptosis. The cell populations that were transfected with the antisense oligonucleotides showed a decrease in the number of cells undergoing apoptosis as compared to like cell populations not having been transfected with the antisense oligos. Figures 78-82 show a decrease in the percentage of cells undergoing apoptosis in the cells having being treated with antisense apoptosis-specific eIF-5A oligonucleotides as compared to cells not having been transfected with the antisense apoptosis-specific eIF-5A oligonucleotides.

The present invention contemplates the use of many suitable nucleic acid sequences encoding an apoptosis-specific eIF-5A polypeptide. For example, the present invention provides antisense oligonucleotides of the following apoptosis-specific eIF-5A nucleic acid sequences (SEQ ID NOS:1, 3, 4, 5, 11, 12, 15, 16, 19, 20, and 21) as well as other antisense nucleotides described herein. Antisense oligonucleotides of the present invention need not be the entire length of the provided SEQ ID NOs. They need only be long enough to be able to bind to inhibit or reduce expression of apoptosis-specific eIF-5A. "Inhibition or reduction of expression" or "suppression of expression" refers to the absence or detectable decrease in the level of protein and/or mRNA product from a target gene, such as apoptosis-specific eIF-5A.

Exemplary antisense oligonucleotides of apoptosis-specific eIF-5A that do not comprise the entire coding sequence are antisense oligonucleotides of apoptosis-specific eIF-5A having the following SEQ ID NO: 35, 37, and 39.

"Antisense oligonucleotide of apoptosis-specific eIF-5A" includes oligonucleotides having substantial sequence identity or substantial homology to apoptosis-specific eIF-5A. Additional antisense oligonucleotides of apoptosis-specific eIF-5A of the present invention include those that have substantial sequence identity to those enumerated above (i.e. 90% homology) or those having sequences that hybridize under highly stringent conditions to the enumerated SEQ ID NOs. As used herein, the term "substantial sequence identity" or "substantial homology" is used to indicate that a sequence exhibits substantial structural or functional equivalence with another sequence. Any structural or functional differences between sequences having substantial sequence identity or substantial homology will be *de minimus;* that is, they will not affect the ability of the sequence to function as indicated in the desired application. Differences may be due to inherent variations in codon usage among different species, for example. Structural differences are considered *de minimus* if there is a significant amount of sequence overlap or similarity between two or more different sequences or if the different sequences exhibit similar physical characteristics even if the sequences differ in length or structure. Such characteristics include, for example, the ability to hybridize under defined conditions, or in the case of proteins, immunological crossreactivity, similar enzymatic activity, etc. The skilled practitioner can readily determine each of these characteristics by art known methods.

Additionally, two nucleotide sequences are "substantially complementary" if the sequences have at least about 70 percent or greater, more preferably 80 percent or greater, even more preferably about 90 percent or greater, and most preferably about 95 percent or greater sequence similarity between them. Two amino acid sequences are substantially homologous if they have at least 70%, more preferably at least 80%, even more preferably at least 90%, and most preferably at least 95% similarity between the active, or functionally relevant, portions of the polypeptides.

To determine the percent identity of two sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, at least 30%, 40%, 50%, 60%, 70%, 80%, or 90% or more of the length of a reference sequence is aligned for comparison purposes. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity and similarity between two sequences can be accomplished using a mathematical algorithm. (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991).

The nucleic acid and protein sequences of the present invention can further be used as a "query sequence" to perform a search against sequence databases to, for example, identify other family members or related sequences. Such searches can be performed using the NBLAST and XBLAST programs (version 2.0) of Altschul, et al. (1990) J. Mol Biol. 215:403-10. BLAST nucleotide searches can be performed with the NBLAST program. BLAST protein searches can be performed with the XBLAST program to obtain amino acid sequences homologous to the proteins of the invention. To obtain gapped alignments for comparison purposes, Gapped BLAST can be utilized as described in Altschul et al. (1997) Nucleic Acids Res. 25(17):3389-3402. When utilizing BLAST and gapped BLAST programs, the default parameters of the respective programs (e.g., XBLAST and NBLAST) can be used.

The term "apoptosis-specific eIF-5A" includes functional derivatives thereof. The term "functional derivative" of a nucleic acid is used herein to mean a homolog or analog of the amino acid or nucleotide sequence. A functional derivative retains the function of the given gene, which permits its utility in accordance with the invention. "Functional derivatives" of the apoptosis-specific eIF-5A polypeptide or functional derivatives of antisense oligonucleotides of apoptosis-specific eIF-5A as described herein are fragments, variants, analogs, or chemical derivatives of apoptosis-specific eIF-5A that retain apoptosis-specific eIF-5A activity or immunological cross reactivity with an antibody specific for apoptosis-specific eIF-5A. A fragment of the apoptosis-specific eIF-5A polypeptide refers to any subset of the molecule.

Functional variants can also contain substitutions of similar amino acids that result in no change or an insignificant change in function. Amino acids that are essential for function can be identified by methods known in the art, such as site-directed mutagenesis or alanine-scanning mutagenesis (Cunningham et al. (1989) Science 244:1081-1085). The latter procedure introduces single alanine mutations at every residue in the molecule. The resulting mutant molecules are then tested for biological activity such as kinase activity or in assays such as an in vitro proliferative activity. Sites that are critical for binding partner/substrate binding can also be determined by structural analysis such as crystallization, nuclear magnetic resonance or photoaffinity labeling (Smith et al. (1992) J. Mol. Biol. 224:899-904; de Vos et al. (1992) Science 255:306-312).

A "variant" refers to a molecule substantially similar to either the entire gene or a fragment thereof, such as a nucleotide substitution variant having one or more substituted nucleotides, but which maintains the ability to hybridize with the particular gene or to encode mRNA transcript which hybridizes with the native DNA. A "homolog" refers to a fragment or variant sequence from a different animal genus or species. An "analog" refers to a non-natural molecule substantially similar to or functioning in relation to the entire molecule, a variant or a fragment thereof.

Variant peptides include naturally occurring variants as well as those manufactured by methods well known in the art. Such variants can readily be identified/made using molecular techniques and the sequence information disclosed herein. Further, such variants can readily be distinguished from other proteins based on sequence and/or structural homology to the eIF-5A of the present invention. The degree of homology/identity present will be based primarily on whether the protein is a functional variant or non-functional variant, the amount of divergence present in the paralog family and the evolutionary distance between the orthologs.

Non-naturally occurring variants of the eIF-5A polynucleotides, antisense oligonucleotides, or proteins of the present invention can readily be generated using recombinant techniques. Such variants include, but are not limited to deletions, additions and substitutions in the nucleotide or amino acid sequence. For example, one class of substitutions are conserved amino acid substitutions. Such substitutions are those that substitute a given amino acid in a protein by another amino acid of like characteristics. Typically seen as conservative substitutions are the replacements, one for another, among the aliphatic amino acids Ala, Val, Leu, and Ile; interchange of the hydroxyl residues Ser and Thr; exchange of the acidic residues Asp and Glu; substitution between the amide residues Asn and Gln; exchange of the basic residues Lys and Arg; and replacements among the aromatic residues Phe and Tyr. Guidance concerning which amino acid changes are likely to be phenotypically silent are found in Bowie et al., Science 247:1306-1310 (1990).

The term "hybridization" as used herein is generally used to mean hybridization of nucleic acids at appropriate conditions of stringency as would be readily evident to those skilled in the art depending upon the nature of the probe sequence and target sequences. Conditions of hybridization and washing are well known in the art, and the adjustment of conditions depending upon the desired stringency by varying incubation time, temperature and/or ionic strength of the solution are readily accomplished. *See, e.g.* Sambrook, J. et al., Molecular Cloning: A Laboratory Manual, 2nd edition, Cold Spring Harbour Press, Cold Spring Harbor, New York, 1989.

The choice of conditions is dictated by the length of the sequences being hybridized, in particular, the length of the probe sequence, the relative G-C content of the nucleic acids and the amount of mismatches to be permitted. Low stringency conditions are preferred when partial hybridization between strands that have lesser degrees of complementarity is desired. When perfect or near perfect complementarity is desired, high stringency conditions are preferred. High stringency conditions means that the hybridization solution contains 6X S.S.C., 0.01 M EDTA, 1X Denhardt's solution and 0.5% SDS. Hybridization is carried out at about 68°C for about 3 to 4 hours for fragments of cloned DNA and for about 12 to 16 hours for total eucaryotic DNA. For lower stringencies, the temperature of hybridization is reduced to about 42° C below the melting temperature (Tₘ) of the duplex. The Tₘ is known to be a function of the G-C content and duplex length as well as the ionic strength of the solution.

As used herein, the phrase "hybridizes to a corresponding portion" of a DNA or RNA molecule means that the molecule that hybridizes, e.g., oligonucleotide, polynucleotide, or any nucleotide sequence (in sense or antisense orientation) recognizes and hybridizes to a sequence in another nucleic acid molecule that is of approximately the same size and has enough sequence similarity thereto to effect hybridization under appropriate conditions. For example, a 100 nucleotide long sense molecule will recognize and hybridize to an approximately 100 nucleotide portion of a nucleotide sequence, so long as there is about 70% or more sequence similarity between the two sequences. It is to be understood that the size of the "corresponding portion" will allow for some mismatches in hybridization such that the "corresponding portion" may be smaller or larger than the molecule which hybridizes to it, for example 20-30% larger or smaller, preferably no more than about 12-15% larger or smaller.

The present invention also provides other agents that can inhibit or reduce expression of apoptosis-specific eIF-5A. One such agent includes small inhibitory RNAs ("siRNA"). siRNA technology has been emerging as a viable alternative to antisense oligonucleotides since lower concentrations are required to achieve levels of suppression that are equivalent or superior to those achieved with antisense oligonucleotides (Thompson, 2002). Long double-stranded RNAs have been used to silence the expression of specific genes in a variety of organisms such as plants, nematodes, and fruit flies. An RNase-III family enzyme called Dicer processes these long double stranded RNAs into 21-23 nucleotide small interfering RNAs which are then incorporated into an RNA-induced silencing complex (RISC). Unwinding of the siRNA activates RISC and allows the single-stranded siRNA to guide the complex to the endogenous mRNA by base pairing. Recognition of the endogenous mRNA by RISC results in its cleavage and consequently makes it unavailable for translation. Introduction of long double stranded RNA into mammalian cells results in a potent antiviral response, which can be bypassed by use of siRNAs. (Elbashir *et al.,* 2001). siRNA has been widely used in cell cultures and routinely achieves a reduction in specific gene expression of 90 % or more.

The use of siRNAs has also been gaining popularity in inhibiting gene expression in animal models of disease. A recent study demonstrated that an siRNA against luciferase was able to block luciferase expression from a co-transfected plasmid in a wide variety of organs in post-natal mice. (Lewis *et al.,* 2002). An siRNA against Fas, a receptor in the TNF family, injected hydrodynamically into the tail vein of mice was able to transfect greater than 80 % of hepatocytes and decrease Fas expression in the liver by 90 % for up to 10 days after the last injection (Song *et al.,* 2003). The Fas siRNA was also able to protect mice from liver fibrosis and fulminant hepatitis. The development of sepsis in mice treated with a lethal dose of lipopolysaccharide was inhibited by the use of an siRNA directed against TNF-α (Sørensen *et al.,* 2003). SiRNA has the potential to be a very potent drug for the inhibition of specific gene *expression in vitro* in light of their long-lasting effectiveness in cell cultures and their ability to transfect cells *in vivo* and their resistance to degradation in serum *in vivo* (Bertrand *et al.,* 2002) in vivo.

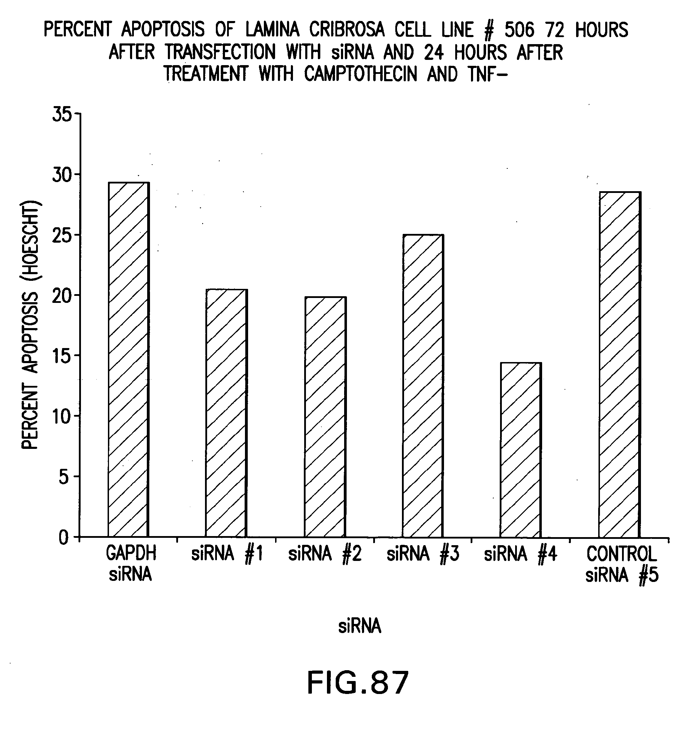

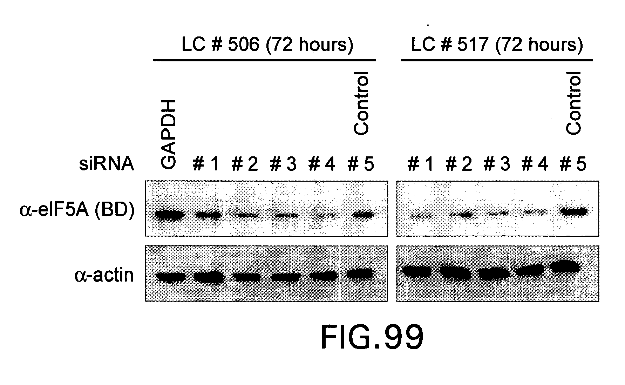

The present inventors have transfected cells with siRNAs of apoptosis-specific eIF-5A and studied the effects on expression of apoptosis-specific eIF-5A. Figure 99 shows that cells transfected with apoptosis-specific eIF-5A siRNA produced less apoptosis-specific eIF-5A protein. Figures 87-89 show that cell populations transfected with apoptosis-specific eIF-5A siRNAs have a lower percentage of cells undergoing apoptosis after exposure to amptothecin and TNF-α as compared to cells not having been transfected with apoptosis-specific eIF-5A siRNAs. Thus, one embodiment of the present invention provides for inhibiting expression of apoptosis-specific eIF-5A in cells by transfecting the cells with a vector comprising a siRNA of apoptosis-specific eIF-5A.

Preferred siRNAs of apoptosis-specific eIF-5A include those that have SEQ ID NO: 31, 31, 32, and 33. Additional siRNAs include those that have substantial sequence identity to those enumerated (i.e. 90% homology) or those having sequences that hybridize under highly stringent conditions to the enumerated SEQ ID NOs. What is meant by substantial sequence identity and homology is described above with respect to antisense oligonucleotides of the present invention. The term "siRNAs of apoptosis-specific eIF-5A" include functional variants or derivatives as described above with respect to antisense oligonucleotides of the present invention.

Delivery of siRNA and expression constructs/vectors comprising siRNA are known by those skilled in the art. U.S. applications 2004/106567 and 2004/0086884, which are herein incorporated by reference in their entirety, provide numerous expression constructs/vectors as well as delivery mechanism including viral vectors, non viral vectors, liposomal delivery vehicles, plasmid injection systems, artificial viral envelopes and poly-lysine conjugates to name a few.

One skilled in the art would understand regulatory sequences useful in expression constructs/vectors with antisense oligonucleotides or siRNA. For example, regulatory sequences may be a constitutive promoter, an inducible promoter, a tissue-specific promoter, or a combination thereof.

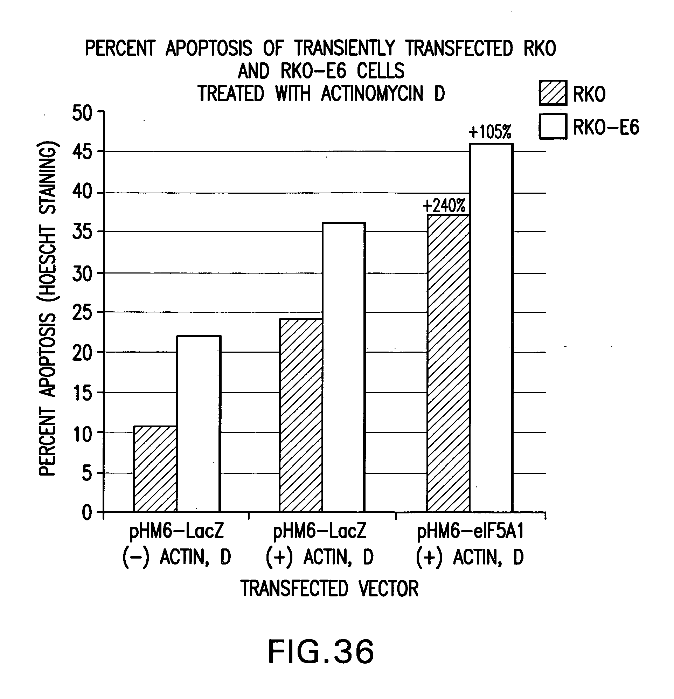

By decreasing expression of apoptosis-specific eIF-5A in a cell in a mammal with either antisense polynucleotides or siRNA apoptosis-specific eIF-5A, there is a decrease in cellular apoptosis. For example, RKO and RKO-E6 cells were transiently transfected with pHM6-LacZ or pHM6-apoptosis-specific eIF-5A. RKO cells treated with Actinomycin D and transfected with pHM6-apoptosis-specific eIF-5A showed a 240% increase in apoptosis relative to cells transfected with pHM6-LacZ that were not treated with Actinomycin D. RKO-E6 cells treated with Actinomycin D and transfected with pHM6-apoptosis-specific eIF-5A showed a 105% increase in apoptosis relative to cells transfected with pHM6-LacZ that were not treated with Actinomycin D. See figure 36.

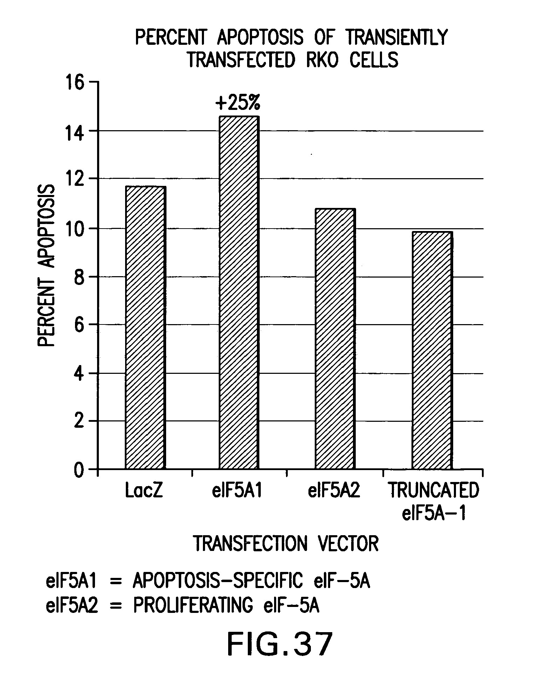

Figure 37 is a graph depicting the percentage of apoptosis occurring in RKO cells following transient transfection. RKO cells were transiently transfected with pHM6-LacZ, pHM6-apoptosis-specific eIF-5A, pHM6-eIF5A2, or pHM6-truncated apoptosis-specific eIF-5A. Cells transfected with pHM6-apoptosis-specific eIF-5A showed a 25% increase in apoptosis relative to control cells transfected with pHM6-LacZ. This increase was not apparent for cells transfected with pHM6-eIF5A2 or pHM6-truncated apoptosis-specific eIF-5A.

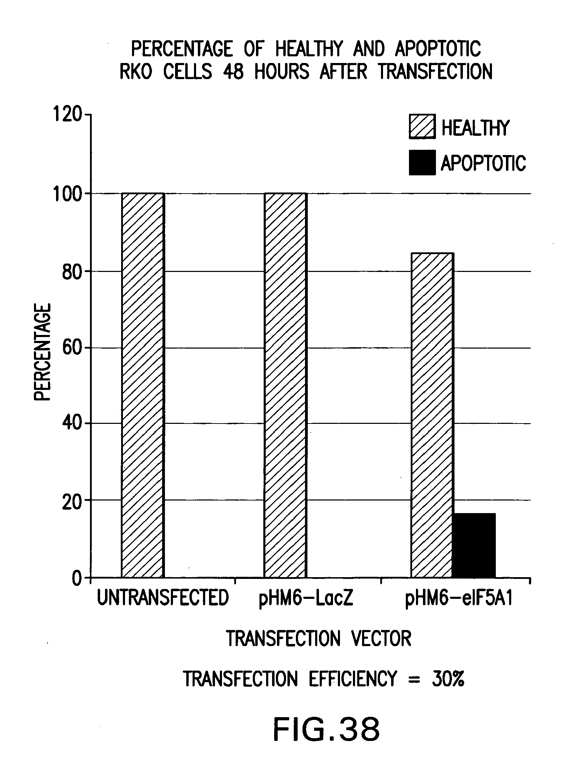

Figure 38 is a graph depicting the percentage of apoptosis occurring in RKO cells following transient transfection. RKO cells were either left untransfected or were transiently transfected with pHM6-LacZ or pHM6-apoptosis-specific eIF-5A. After correction for transfection efficiency, 60 % of the cells transfected with pHM6-apoptosis-specific eIF-5A were apoptotic.

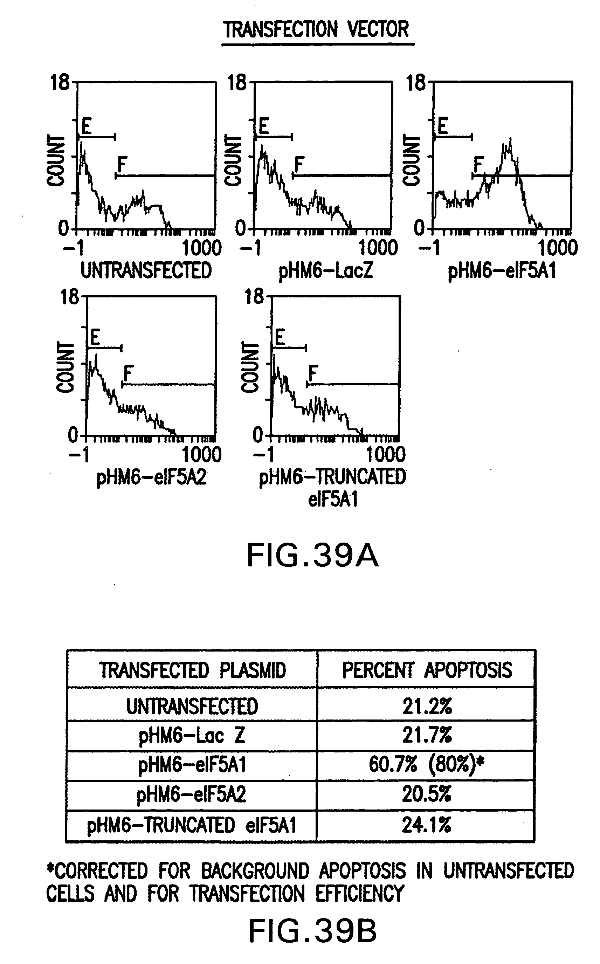

Figure 39 provides the results of a flow cytometry analysis of RKO cell apoptosis following transient transfection. RKO cells were either left untransfected or were transiently transfected with pHM6-LacZ, pHM6-apoptosis-specific eIF-5A, pHM6-eIF5A2, or pHM6-truncated apoptosis-specific eIF-5A. The table depicts the percentage of cells undergoing apoptosis calculated based on the area under the peak of each gate. After correction for background apoptosis in untransfected cells and for transfection efficiency, 80% of cells transfected with pHM6-apoptosis-specific eIF-5A exhibited apoptosis. Cells transfected with pHM6-LacZ, pHM6-eIF5A2 or pHM6-truncated apoptosis-specific eIF-5A exhibited only background levels of apoptosis.

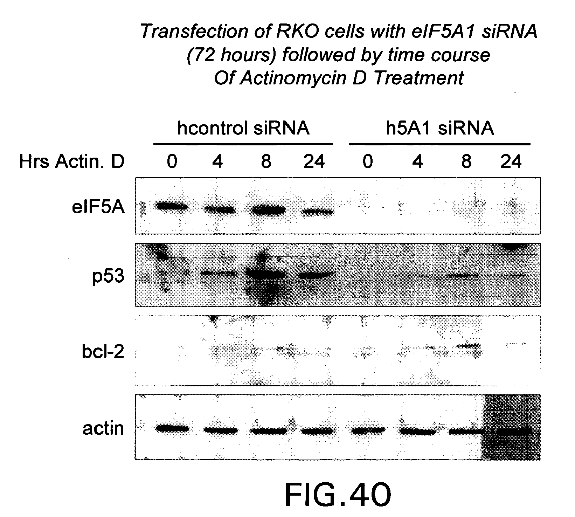

Figure 40 shows the results of an experiment where RKO cells were transfected with apoptosis-specific eIF-5A (eIF-51) siRNA followed by a treatment of Actinomycin D (which induced cells to undergo apoptosis). Cells having been transfected with the siRNA show less expression of eIF-5A, less expression of p53, more expression of bcl-2 and the same expression levels of the control gene, actin.

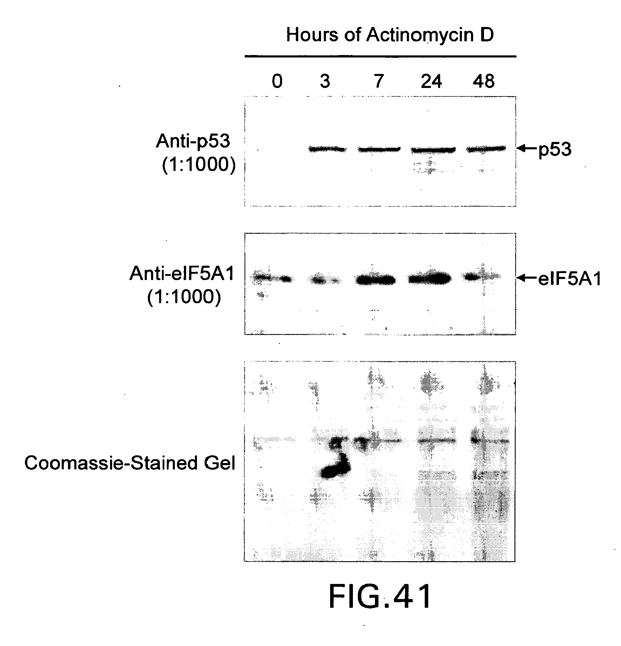

Figure 41 provides Western blots of protein extracted from RKO cells treated with 0.25 µg/ml Actinomycin D for 0, 3, 7, 24, and 48 hours. The top panel depicts a Western blot using anti-p53 as the primary antibody. The middle panel depicts a Western blot using anti-apoptosis-specific eIF-5A as the primary antibody. The bottom panel depicts the membrane used for the anti-apoptosis-specific eIF-5A blot stained with Coomassie blue following chemiluminescent detection to demonstrate equal loading. p53 and apoptosis-specific eIF-5A are both up-regulated by treatment with Actinomycin D.

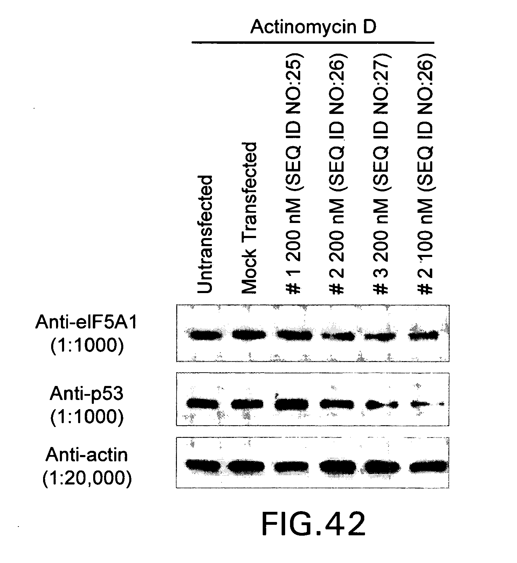

Figure 42 shows the levels of protein produced by RKO cells after being treated with antisense oligo 1, 2 and 3 (of apoptosis-specific eIF-5A)(SEQ ID NO: 35, 37 and 39, respectively). The RKO cells produced less apoptosis-specific eIF-5A as well as less p53 after having been transfected with the antisense apoptosis-specific eIF-5A oligonucleotides.

Thus, figures 36-42 show that when expression of apoptosis-specific eIF-5A is reduced in a cell population that is later treated with a compound known to induce apoptosis, the cells expressing less apoptosis-specific eIF-5A undergo less apoptosis.

In addition to causing a decrease in expression of apoptosis-specific eIF-5A, the antisense polynucleotide or siRNA of the present invention also cause the following responses: decreasing expression of TLR4, IFN-γRa, TNF-α, IL-8, TNFR-1, p53, iNOS and IL-1, IL-12, IFN-γ, IL-6, and IL-18, decreasing phosphorylation of STAT1α and JAK1 response, decreasing NF-κB p50 activation, decreasing levels of myleloperoxidase, decreasing levels of MIP-1α and increasing BCL-2 expression.

Many important human diseases are caused by abnormalities in the control of apoptosis. These abnormalities can result in either a pathological increase in cell number (e.g. cancer) or a damaging loss of cells (e.g. degenerative diseases). As non-limiting examples, the methods and compositions of the present invention can be used to prevent or treat a subject having the following apoptosis-associated diseases and disorders by decreasing or inhibiting expression in a mammal, mammalian cell or mammalan tissue of apoptosis-specific eIF-5A through the use of antisense polynucleotides or siRNA directed against apoptosis specific eIF-5A to cause reduced expression of apoptosis specific eIF-5A: neurological/ neurodegenerative disorders (e.g., Alzheimer's, Parkinson's, Huntington's, Amyotrophic Lateral Sclerosis (Lou Gehrig's Disease), autoimmune disorders (e.g., rheumatoid arthritis, systemic lupus erythematosus (SLE), multiple sclerosis), Duchenne Muscular Dystrophy (DMD), motor neuron disorders, ischemia, heart ischemia, chronic heart failure, stroke, infantile spinal muscular atrophy, cardiac arrest, renal failure, atopic dermatitis, sepsis and septic shock, AIDS, hepatitis, glaucoma, diabetes (type 1 and type 2), asthma, retinitis pigmentosa, osteoporosis, xenograft rejection, and burn injury.

One such disease caused by abnormalities in the control of apoptosis is glaucoma. Apoptosis in various optical tissues is a critical factor leading to blindness in glaucoma patients. Glaucoma is a group of eye conditions arising from damage to the optic nerve that results in progressive blindness. Apoptosis has been shown to be a direct cause of this optic nerve damage.

Early work in the field of glaucoma research has indicated that elevated intraocular pressure ("IOP") leads to interference in axonal transport at the level of the lamina cribosa (a perforated, collagenous connective tissue) that is followed by the death of retinal ganglion cells. Quigley and Anderson (1976) Invest. Ophthalmol. Vis. Sci., 15, 606-16; Minckler, Bunt, and Klock, (1978) Invest. Ophthalmol. Vis. Sci., 17, 33-50; Anderson and Hendrickson, (1974) Invest. Ophthalmol. Vis. Sci., 13, 771-83; Quigley et al., (1980) Invest. Ophthalmol. Vis. Sci., 19, 505-17. Studies of animal models of glaucoma and post-mortem human tissues indicate that the death of retinal ganglion cells in glaucoma occurs by apoptosis. Garcia-Valenzuela et al., (1995) Exp. Eye Res., 61, 33-44; Quigley et al., (1995) Invest. Ophthalmol. Vis. Sci., 36, 774-786; Monard, (1998) In: Haefliger IO, Flammer J (eds) Nitric Oxide and Endothelin in the Pathogenesis of Glaucoma, New York, NY, Lippincott-Raven, 213-220. The interruption of axonal transport as a result of increased IOP may contribute to retinal ganglion cell death by deprivation of trophic factors. Quigley, (1995) Aust N Z J Ophthalmol, 23(2), 85-91. Optic nerve head astrocytes in glaucomatous eyes have also been found to produce increased levels of some neurotoxic substances. For example, increased production of tumor necrosis factor-α (TNF-α) (Yan et al., (2000) Arch. Ophthalmol., 118, 666-673), and nitric oxide synthase (Neufeld et al., (1997) Arch. Ophthalmol., 115, 497-503), the enzyme which gives rise to nitric oxide, has been found in the optic nerve head of glaucomatous eyes. Furthermore, increased expression of the inducible form of nitric oxide synthase (iNOS) and TNF-α by activated retinal glial cells have been observed in rat models of hereditary retinal diseases. Cotinet et al., (1997)Glia, 20, 59-69; de Kozak et al., (1997) Ocul. Immunol. Inflamm., 5, 85-94; Goureau et al., (1999) J. Neurochem, 72, 2506-2515. In the glaucomatous optic nerve head, excessive nitric oxide has been linked to the degeneration of axons of retinal ganglion cells. Arthur and Neufeld, (1999) Surv Ophthalmol, 43 (Suppl 1), S129-S135. Finally, increased production of TNF-α by retinal glial cells in response to simulated ischemia or elevated hydrostatic pressure has been shown to induce apoptosis in co-cultured retinal ganglion cells. Tezel and Wax, (2000) J. Neurosci., 20(23), 8693-8700.

Protecting retinal ganglion cells from degeneration by apoptosis is under study as a potential new treatment/prevention for blindness due to glaucoma. Antisense oligonucleotides have been used successfully in animal models of eye disease. In a model of transient global retinal ischemia, expression of caspase 2 was increased during ischemia, primarily in the inner nuclear and ganglion cell layers of the retina. Suppression of caspase using an antisense oligonucleotide led to significant histopathologic and functional improvement as determined by electroretinogram. Singh et al., (2001) J. Neurochem., 77(2), 466-75. Another study demonstrated that, upon transfection of the optic nerve, retinal ganglion cells up-regulate the pro-apoptotic protein Bax and undergo apoptosis. Repeated injections of a Bax antisense oligonucleotide into the temporal superior retina of rats inhibited the local expression of Bax and increased the number of surviving retinal ganglion cells following transaction of the optic nerve. Isenmann et al., (1999) Cell Death Differ., 6(7). 673-82.

Delivery of antisense oligonucleotides to retinal ganglion cells has been improved by encapsulating the oligonucleotides in liposomes, which were then coated with the envelope of inactivated hemagglutinating virus of Japan (HVJ; Sendai virus) by fusion (HVJ liposomes). Intravitreal injection into mice of FITC-labeled antisense oligonucleotides encapsulated in HVJ liposomes resulted in high fluorescence within 44 % of the cells in the ganglion layer which lasted three days while fluorescence with naked FITC-labeled antisense oligonucleotide disappeared after one day. Hangai et al., (1998) Arch Ophthalmol, 116(7), 976.

One method of the present invention is directed to preventing or reducing apoptosis in cells and tissues of the eye, such as but not limited to, astrocytes, retinal ganglion, retinal glial cells and lamina cribosa. Death of retinal ganglion cells in glaucoma occurs by apoptosis and which leads to blindness. Thus, providing a method of inhibiting or reducing apoptosis in retinal ganglion cells or by protecting retinal ganglion cells from degeneration by apoptosis provides a novel treatment for prevention of blindness due to glaucoma. This method involves suppressing expression of apoptosis-specific eIF-5A to reduce apoptosis. Apoptosis-specific eIF-5A is a powerful gene that appears to regulate the entire apoptotic process. Thus, controlling apoptosis in the optic nerve head by blocking expression of apoptosis-specific eIF-5A provides a treatment for glaucoma.

Suppression of expression of apoptosis-specific eIF-5A is accomplished by administering an antisense oligonucleotide or a siRNA of human apoptosis-specific eIF-5A to cells of the eye such as, but not limited to lamina cribrosa, astrocytes, retinal ganglion, or retinal glial cells. Antisense oligonucleotides and siRNAs are as defined above, i.e. have a nucleotide sequence encoding at least a portion of an apoptosis-specific eIF-5A polypeptide. Exemplary antisense oligonucleotides useful in this aspect of the invention comprise SEQ ID NO:26 or 27 or oligonucleotides that bind to a sequence complementary to SEQ ID NO:26 or 27 under high stringency conditions and which inhibit expression of apoptosis-specific eIF-5A.

Another embodiment of the invention provides a method of suppressing expression of apoptosis-specific eIF-5A in lamina cribosa cells, astrocyte cells, retinal ganglion cells or retinal glial cells. Antisense oligonucleotides or siRNAs, such as but not limited to, SEQ ID NO:26 and 27, targeted against human apoptosis-specific eIF-5A are administered to lamina cribosa cells, astrocyte cells, retinal ganglion cells or retinal glial cells. The cells may be of human origin.

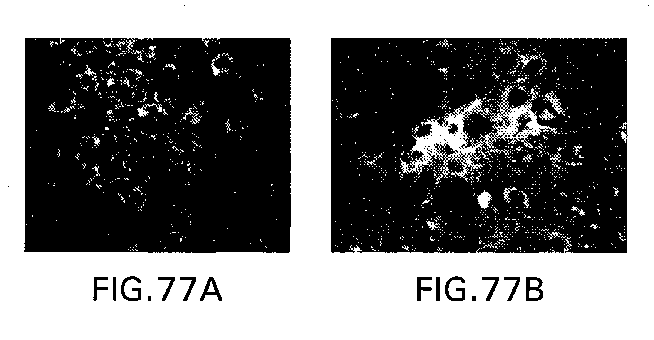

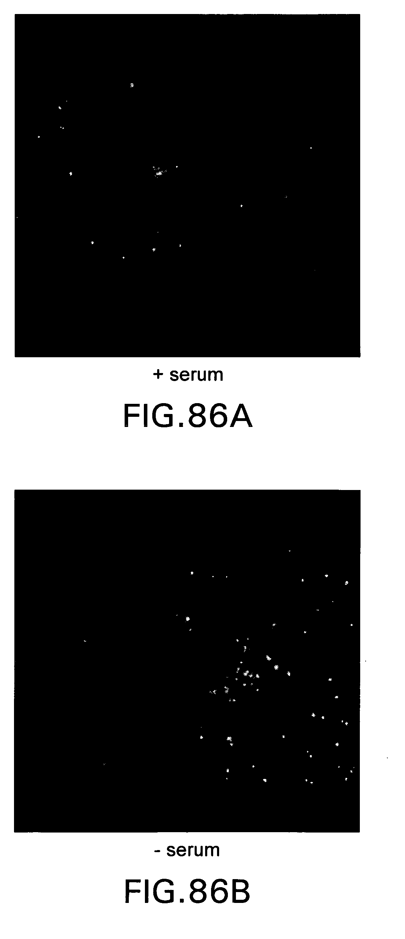

Figure 77A and B shows successful uptake of the fluorescently labeled antisense oligonucleotide in lamina cribosa cells. Figure 86A and B show that the lamina cribrosa cells uptake the labeled siRNA either in the presence of serum or without serum.

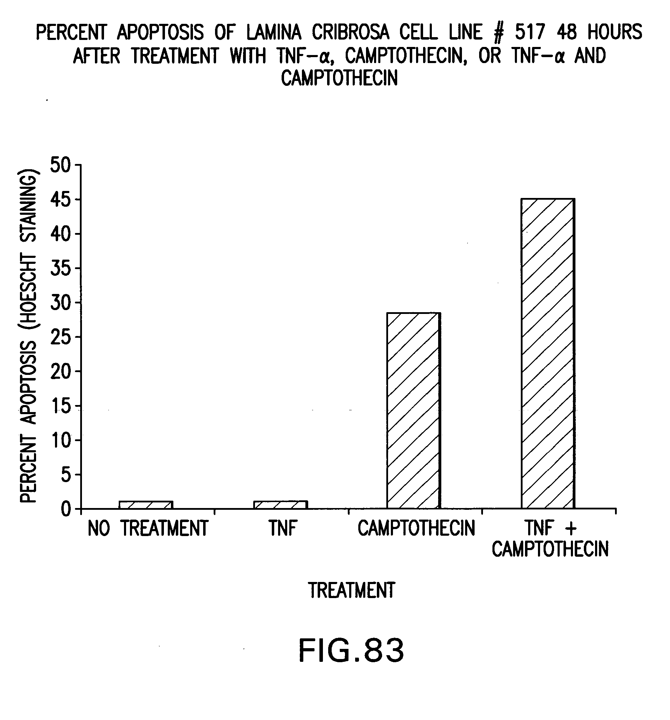

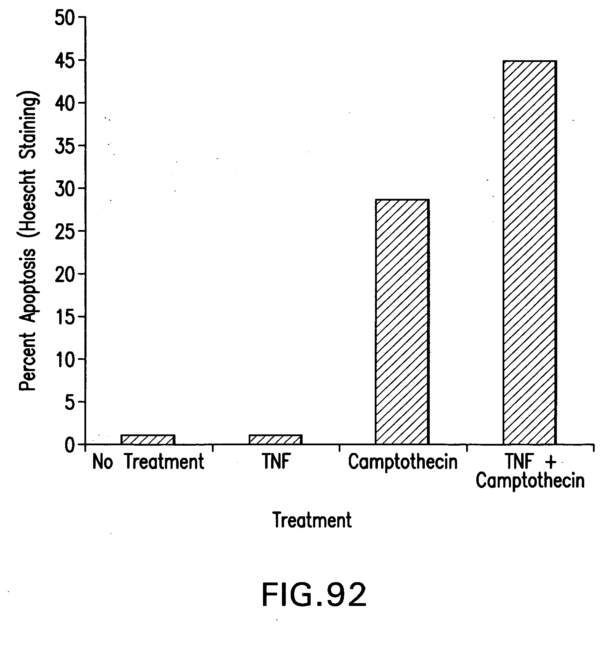

Figures 78 - 82, 84-85, and 88 show the results of several experiments where lamina cribosa cells were treated with antisense polynucleotides against apoptosis-specific eIF-5A and camptothecin (an agent that induced apoptosis). The results show that there is a decrease in the percentage of cells undergoing apoptosis in the cells having being treated with antisense apoptosis-specific eIF-5A oligonucleotides as compared to cells not having been transfected with the antisense apoptosis-specific eIF-5A oligonucleotides. As the figures indicate, different time courses and concentration of camptothecin were used. Also several different lamina cribosa cell lines were tested (cell lien 506 and 517). As a control, figure lamina cribosa cells were treated with TNF-α and/or camptothecin, which caused an increase in the number of cells undergoing apoptosis. See figure 83 and 92. Lamina cribrosa cell line # 506 cells were seeded at 40,000 cells per well onto an 8-well culture slide. Three days later the confluent LC cells were treated with either 10 ng/ml TNF-α, 50 µM camptothecin, or 10 ng/ml TNF-α plus 50 µM camptothecin. An equivalent volume of DMSO, a vehicle control for camptothecin, was added to the untreated control cells. The cells were stained with Hoescht 33258 48 hours after treatment and viewed by fluorescence microscopy using a UV filter. Cells with brightly stained condensed or fragmented nuclei were counted as apoptotic.

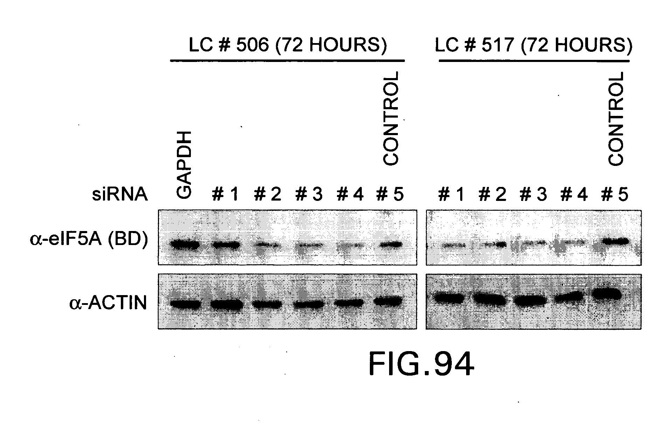

Figure 94 shows that cells treated with siRNAs of apoptosis-specific eIF-5A produce less apoptosis-specific eIF-5A protein. Lamina cribrosa cell # 506 and #517 cells were seeded at 10,000 cells per well onto a 24-well plate. Three days later the LC cells were transfected with either GAPDH siRNA, apoptosis-specific eIF-5A siRNAs #1-4 (SEQ ID NO:30-33) or control siRNA # 5 (SEQ ID NO:34). Three days after transfection the protein lysate was harvested and 5 µg of protein from each sample was separated by SDS-PAGE, transferred to a PVDF membrane, and Western blotted with anti-eIF-5A antibody. The bound antibody was detected by chemiluminescence and exposed to x-ray film. The membrane was then stripped and re-blotted with anti-β-actin as an internal loading control.

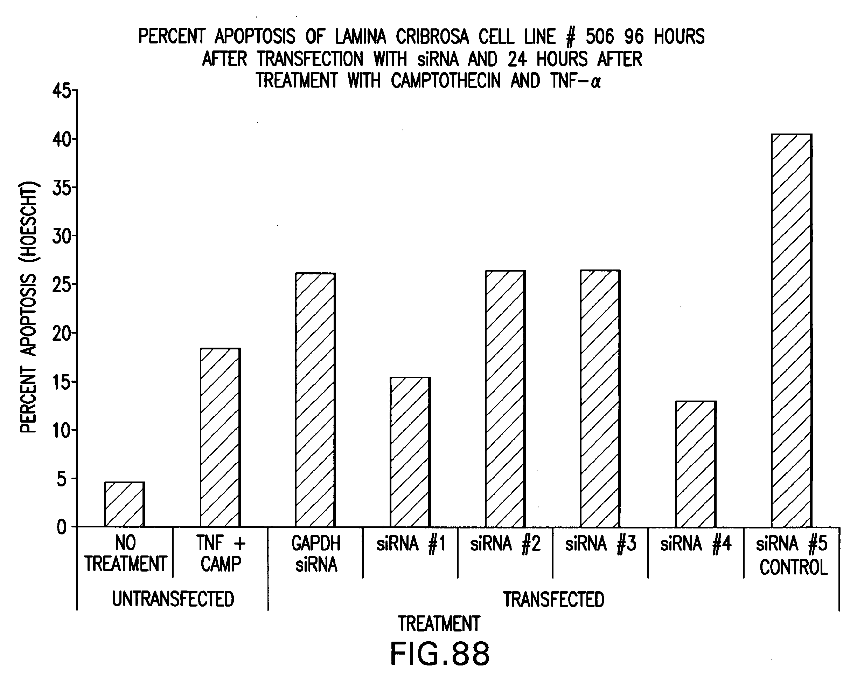

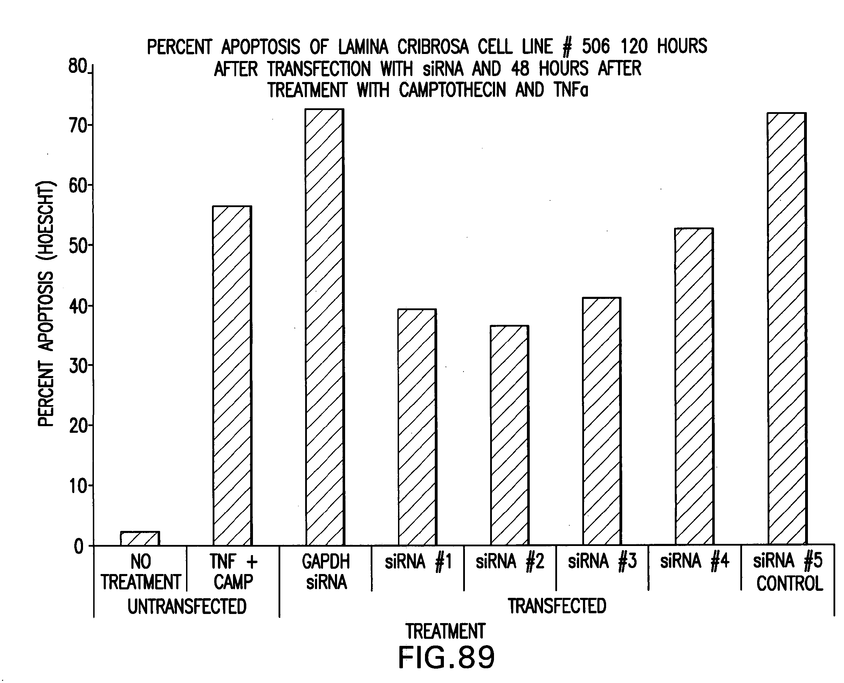

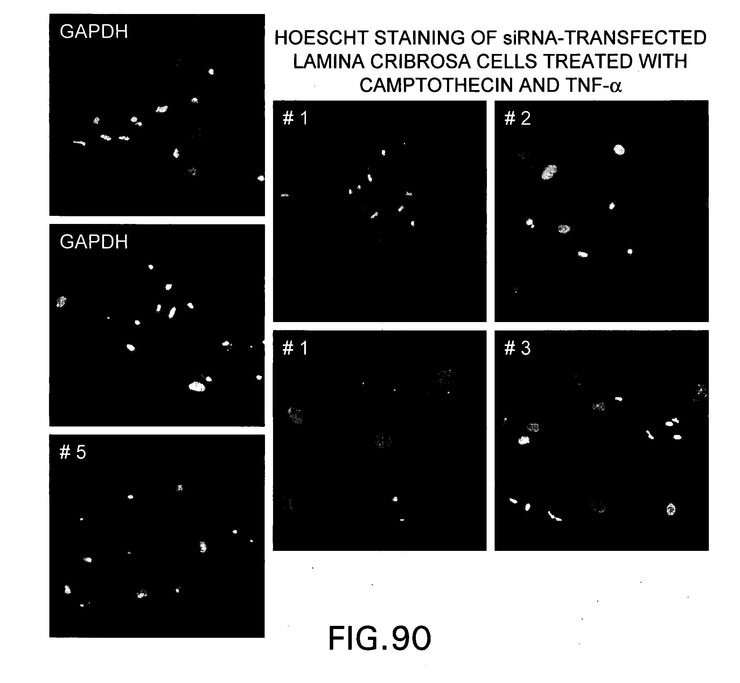

Thus as expected, similar to the results seen with antisense polynucleotides, siRNAs against apoptosis-specific eIF-5A, which led to a decreased expression of apoptosis-specific eIF-5A also led to a decrease in apoptosis as compared to controls. See figures 88-89. Figure 90 shows photographs of Hoescht-stained lamina cribrosa cell line # 506 transfected with siRNA and treated with camptothecin and TNF-α from the experiment described in figure 89 and Example 13. The apoptosing cells are seen as more brightly stained cells. They have smaller nucleic because of chromatin condensation and are smaller and irregular in shape.

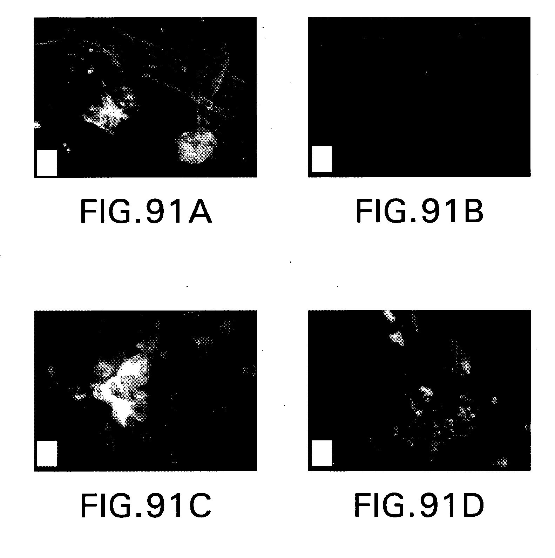

Figure 91 is a characterization of lamina cribrosa cells by immunofluorescence. Lamina cribrosa cells (# 506) isolated from the optic nerve head of an 83-year old male were characterized by immunofluorescence. Primary antibodies were a) actin; b) fibronectin; c) laminin; d) GFAP. All pictures were taken at 400 times magnification.

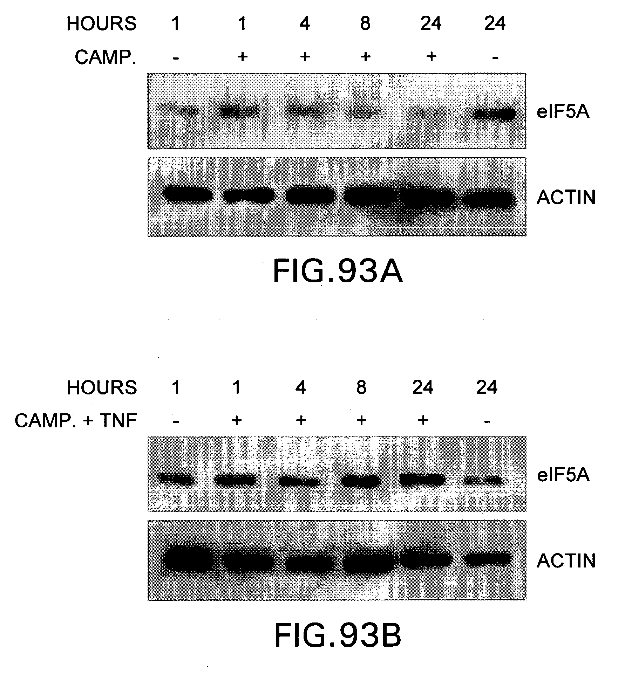

Figure 93 shows expression levels of apoptosis-specific eIF-5A during camptothecin or TNF-α plus camptothecin treatment. This figure shows that apoptosis-specific eIF-5A is up-regulated as a result of the camptotheci and TNF-α treatment whereas the expression levels of the control gene, actin remained constant. Lamina cribrosa cell # 506 cells were seeded at 40,000 cells per well onto a 24-well plate. Three days later the LC cells were treated with either 50 µM camptothecin or 10 ng/ml TNF-α plus 50 µM camptothecin and protein lysate was harvested 1, 4, 8, and 24 hours later. An equivalent volume of DMSO was added to control cells as a vehicle control and cell lysate was harvested 1 and 24 hours later. 5 µg of protein from each sample was separated by SDS-PAGE, transferred to a PVDF membrane, and Western blot with anti-apoptosis-specific eIF-5A antibody. The bound antibody was detected by chemiluminescence and exposed to x-ray film. The membrane was then stripped and re-blotted with anti-β-actin as an internal loading control.

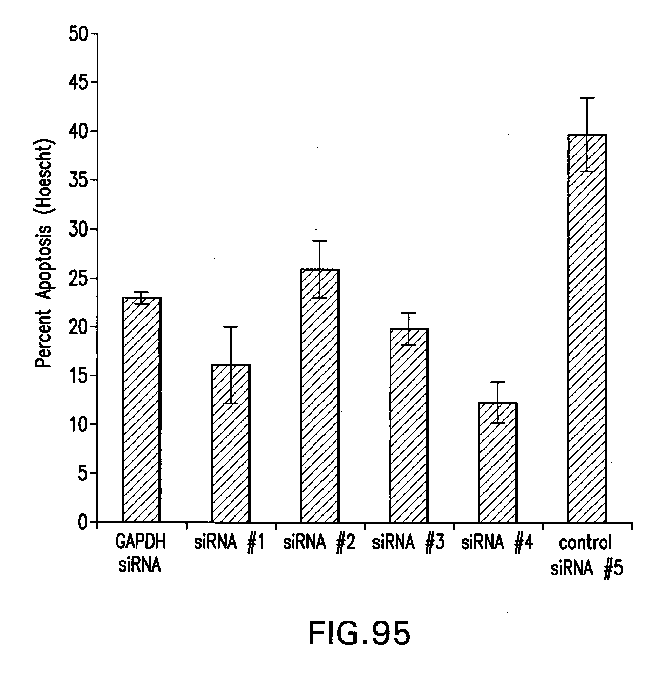

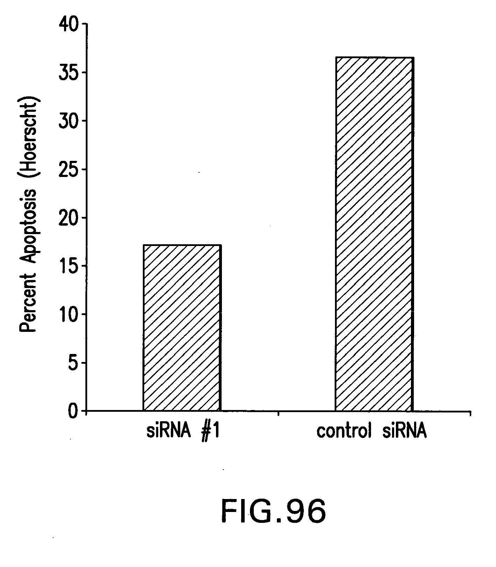

Figure 95 (cell line #506) and figure 96 (cell line 517) show that cells treated with siRNAs of apoptosis-specific eIF-5A show a smaller percentage of apoptosis upon treatment with camptothecin and TNF as compared to cells not transfected with siRNAs of apoptosis-specific eIF-5A. Lamina cribrosa cell line # 506 cells were seeded at 7500 cells per well onto an 8-well culture slide. Three days later the LC cells were transfected with either GAPDH siRNA, apoptosis-specific eIF-5A siRNAs #1-4 (SEQ ID NO:30-33), or control siRNA # 5 (SEQ ID NO:34). 72 hours after transfection, the transfected cells were treated with 10 ng/ml TNF-α plus 50 µM camptothecin. Twenty-four hours later the cells were stained with Hoescht 33258 and viewed by fluorescence microscopy using a UV filter. Cells with brightly stained condensed or fragmented nuclei were counted as apoptotic. This graph represents the average of n=4 independent experiments.

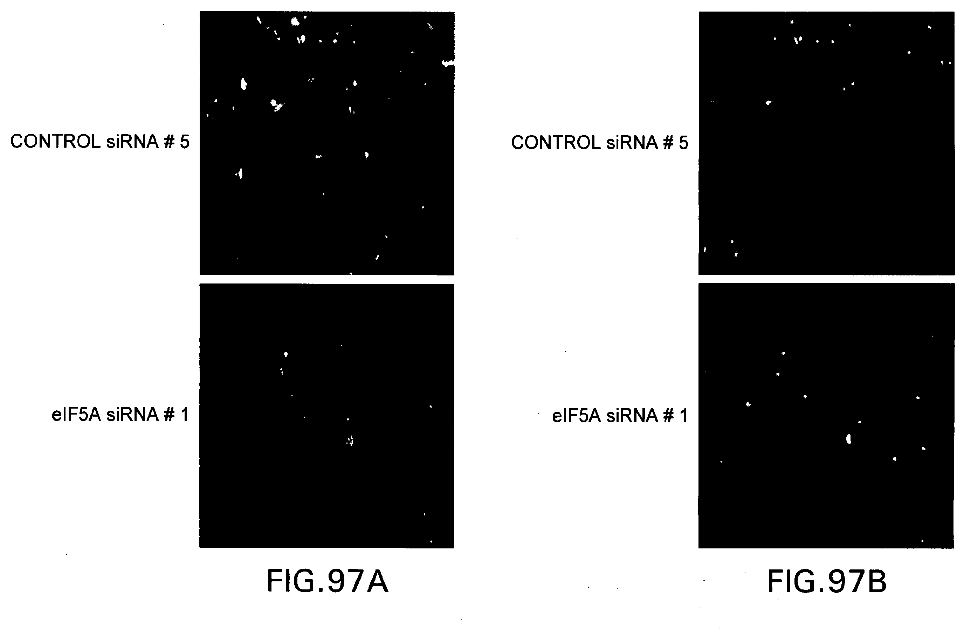

Figure 97a-d show TUNEL-labeling of lamina cribosa cell line # 506 cells transfected with apoptosis-specific eIF-5A siRNA # 1 and treated with TNF-α and camptothecin. This figure shows that there is less apoptosis in siRNA treated cells (less nuclear fragmentation). Lamina cribrosa cell line # 506 cells were seeded at 7500 cells per well onto an 8-well culture slide. Three days later the LC cells were transfected with either apoptosis-specific eIF-5A siRNA #1 (SEQ ID NO:30) or control siRNA # 5 (SEQ ID NO:34). 72 hours after transfection, the transfected cells were treated with 10 ng/ml TNF-α plus 50 µM camptothecin. Twenty-four hours later the cells were stained with Hoescht 33258 and DNA fragmentation was evaluated in situ using the terminal deoxynucleotidyl transferase-mediated dUTP-digoxigenin nick end labeling (TUNEL) method. Panel A represents the slide observed by fluorescence microscopy using a fluorescein filter to visualize TUNEL-labeling of the fragmented DNA of apoptotic cells. Panel B represents the same slide observed by through a UV filter to visualize the Hoescht-stained nuclei. The results are representative of two independent experiments. All pictures were taken at 400 times magnification.

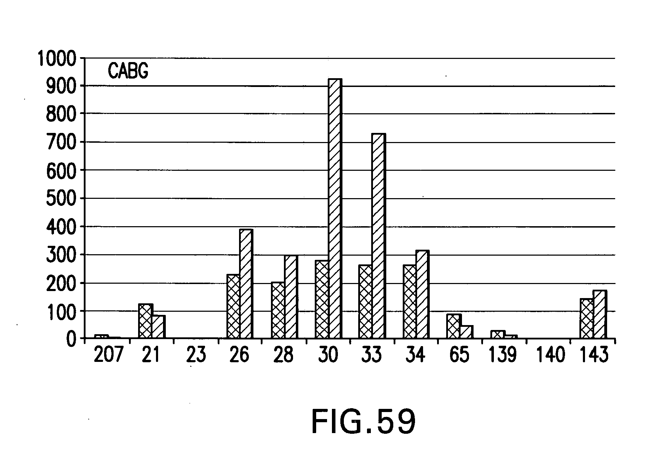

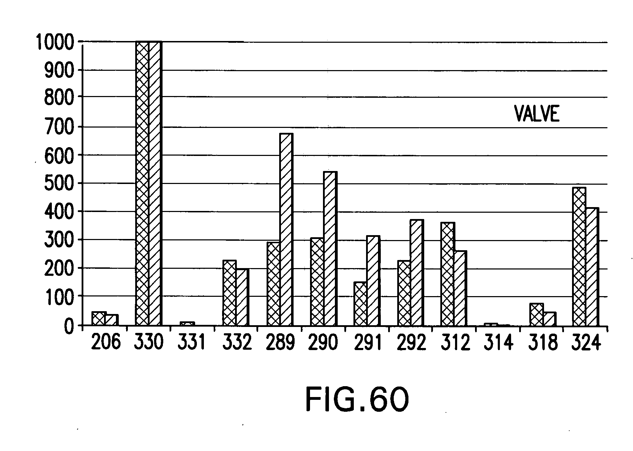

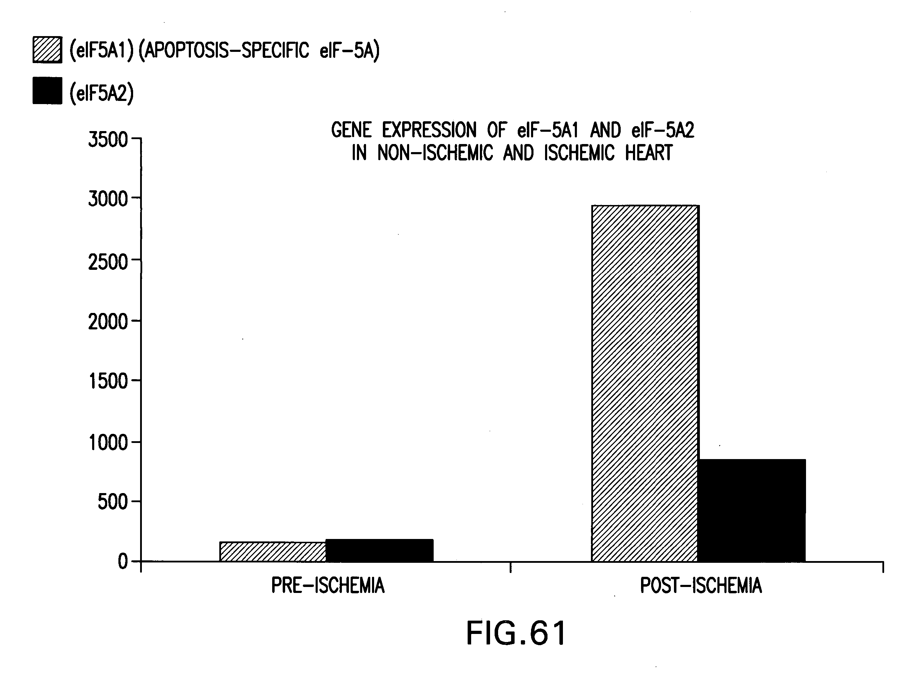

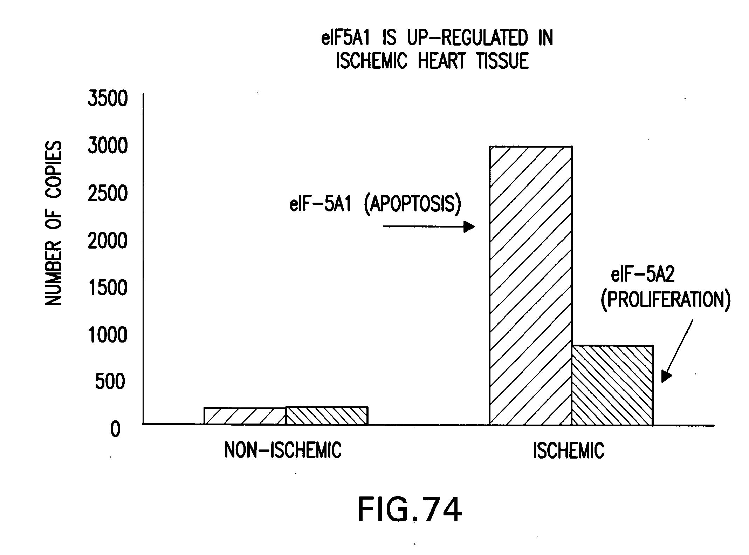

Figures 59 and 60 are bar graphs showing that both apoptosis-specific eIF-5A and proliferation eIF-5A are expressed in heart tissue. The present inventors have discovered that apoptosis-specific eIF-5A levels correlate with elevated levels of two cytokines (Interleukin 1-beta "IL-1β" and interleukin 18 "IL-18") in ischemic heart tissue, thus further proving that apoptosis-specific eIF-5A is involved in cell death as it is present in ischemic heart tissue. This apoptosis-specific eIF-5A/interleukin correlation is not seen in non-ischemic heart tissue. Figure 61 and 74 are bar graphs showing the gene expression levels measured by real-time PCR of apoptosis-specific eIF-5A (eIF-SA1) versus proliferation eIF-5A (eIF-5A2) in pre-ischemia heart tissue and post ischemia heart tissue. The Y-axis is pg/ng of 18s (picograms of message RNA over nanograms of ribosomal RNA 18S). The results depicted in these figures show that apoptosis-specific eIF-5A is preferentially up-regulated in ischemic heart tissue.

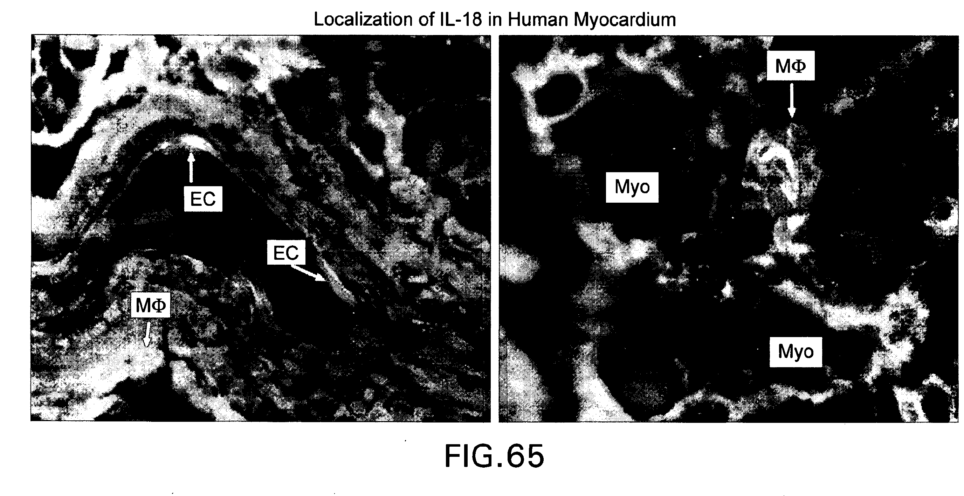

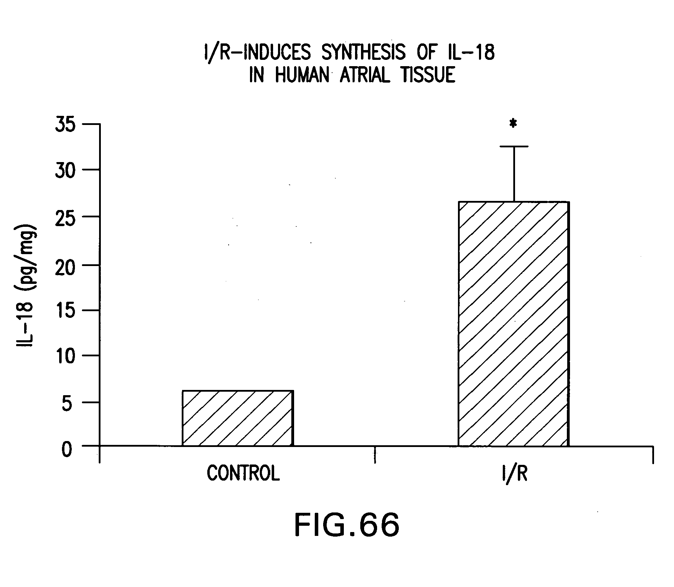

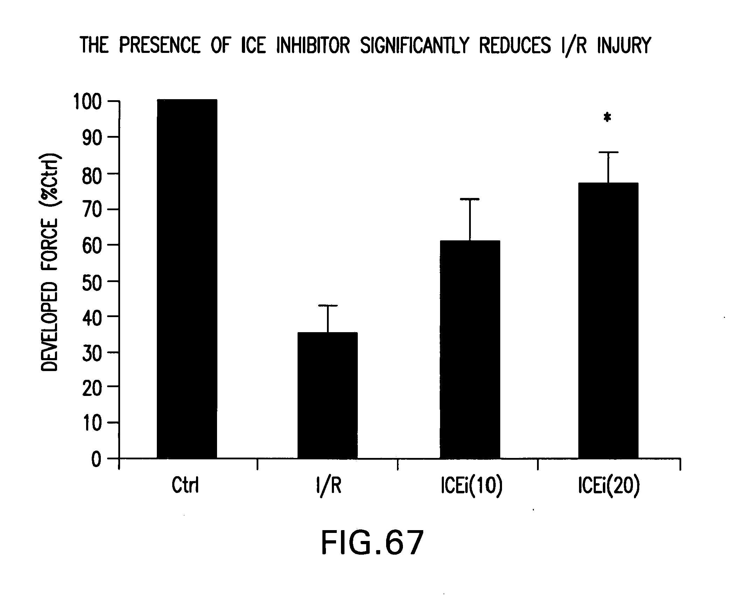

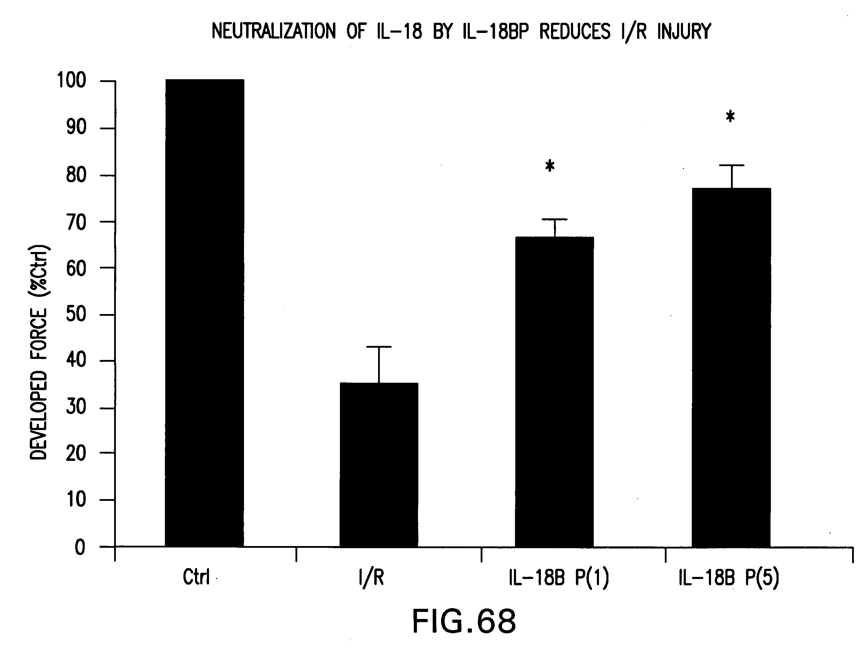

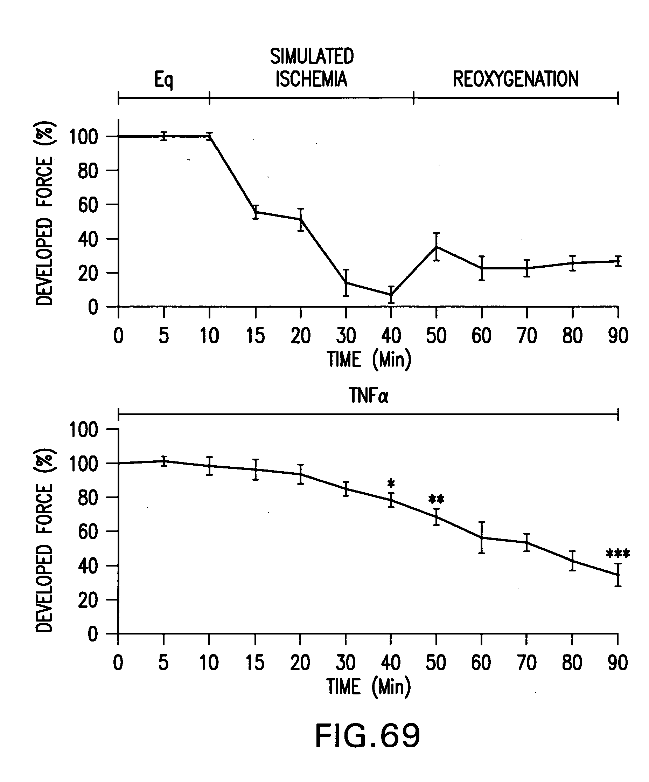

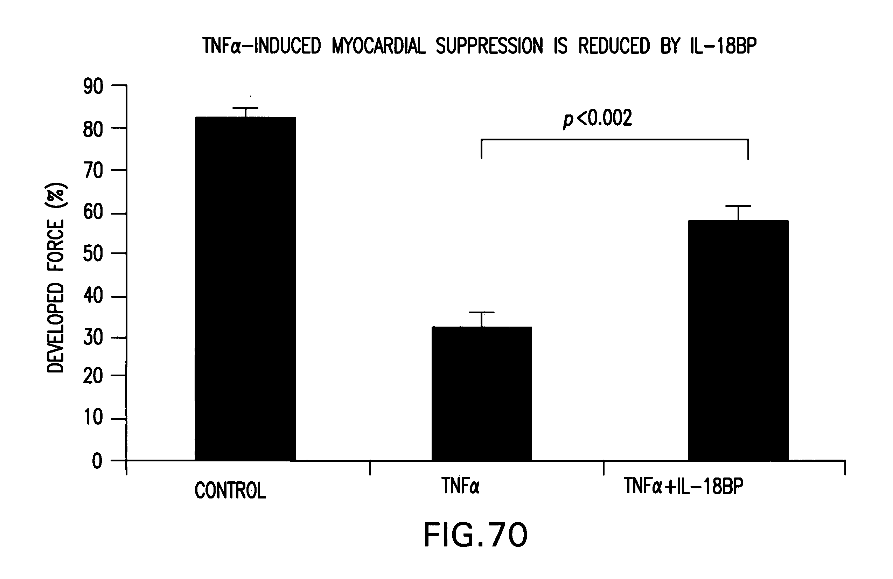

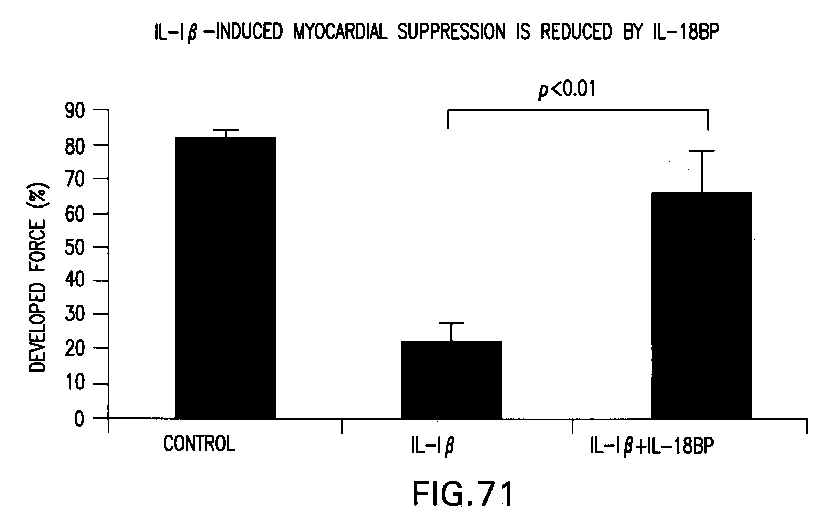

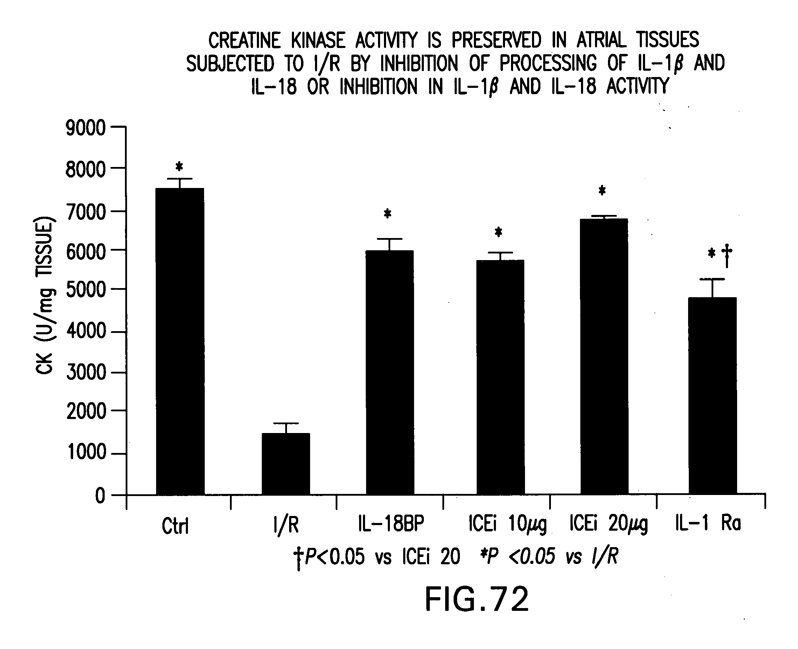

Figure 65 shows localization of IL-18 in human mycocardium. Figure 66 shows that ischemia/reperfusion induced synthesis of IL-18 in human atrial tissue. Figures 62A-F show a correlation between apoptosis-specific eIF-5A and IL-1β and IL-18. Using PCR measurements, levels of apoptosis-specific eIF-5A, and proliferating eIF-5A ("eIF-5A2") - another isoform), IL-1β, and IL-18 were measured and compared in various ischemic heart tissue (from coronary bypass graft and valve (mitral and atrial valve) replacement patients). Thus, it appears that increased levels of apoptosis-specific eIF-5A correlated with increased levels of IL-1β and IL-18. The correlation of apoptosis-specific eIF-5A to these potent interleukins further suggests that the inflammation and apoptosis pathways in ischemia may be controlled via controlling levels of apoptosis-specific eIF-5A. Modulating/decreasing/preventing up-regulation of apoptosis-specific eIF-5A (i.e. with antisense polynucleotides or siRNA of the present invention), would lead to a reduced up-regulation of IL-18. Levels of IL-18 and other cytokines lead to damage in ischemic heart, and thus reducing levels of IL-18 and other cytokines would lead to less ischemic damage. (See Figure 67 showing that the presence of ICE inhibitor (Interleukin-1β converting enzyme) reduced ischemia/reperfusion injury; figure 68 showing that neutralization of IL-18 by IL-18BP (an endogenous inhibitor of IL-18) reduces ischemia/reperfusion injury; figure 69 showing that there is a decrease over time in contractile force of heart tissue when exposed to TNF-α; figure 70 showing that TNF-α induced myocardial suppression is reduced by IL-18BP; figure 71 showing that IL-1β induced mycocardial suppression is reduced by IL-18BP; and figure 72 showing that creatine kinase activity (CK) is preserved in atrial tissues subjected to ischemia/reperfusion by inhibition of processing of IL-1β and IL-18 or inhibition in IL-1β and IL-18 activity). Thus, by reducing levels of apoptosis-specific eIF-5A with antisense nucleotides or sIRNA, less IL-18 and other cyotokines are produced in ischemic tissue, and would thus lead to a reduction in tissue damage from the ischemia.

Further evidence that apoptosis-specific eIF-5A is involved in the immune response is suggested by the fact that human peripheral blood mononuclear cells (PBMCs) normally express very low levels of eIF-5A, but upon stimulation with T-lymphocyte-specific stimuli expression of apoptosis-specific eIF-5A increases dramatically (Bevec *et al.,* 1994). This suggests a role for apoptosis-specific eIF-5A in T-cell proliferation and/or activation. Since activated T cells are capable of producing a wide variety of cytokines, it is also possible that apoptosis-specific eIF-5A may be required as a nucleocytoplasmic shuttle for cytokine mRNAs. The authors of the above referenced article also found elevated levels of eIF5A in the PBMCs of HIV-1 patients, which may contribute to efficient HIV replication in these cells as eIF5A has been demonstrated to be a cellular binding factor for the HIV Rev protein and required for HIV replication (Ruhl *et al.,* 1993).

More recently, eIF-5A expression was found to be elevated during dendritic cell maturation (Kruse *et al.,* 2000). Dendritic cells are antigen-presenting cells that sensitize helper and killer T cells to induce T cell-mediated immunity (Steinman, 1991). Immature dendritic cells lack the ability to stimulate T cells and require appropriate stimuli (i.e. inflammatory cytokines and/or microbial products) to mature into cells capable of activating T cells. An inhibitor of deoxyhypusine synthase, the enzyme required to activate apoptosis-specific eIF-5A, was found to inhibit T lymphocyte activation by dendritic cells by preventing CD83 surface expression (Kruse *et al.,* 2000). Thus, apoptosis-specific eIF-5A may facilitate dendritic cell maturation by acting as a nucleocytoplasmic shuttle for CD83 mRNA.

In both of these studies (Bevec *et al.,* 1994; Kruse *et al.,* 2000) implicating a role for eIF-5A in the immune system, the authors did not specify nor identify which isoform of eIF-5A they were examining, nor did they have a reason to. As discussed above, humans are known to have two isoforms of eIF5A, apoptosis-specific eIF-5A ("eIF-5A1") and proliferating eIF-5A ("eIF-5A2"), both encoded on separate chromosomes. Prior to the present inventors discoveries, it was believed that both of these isoforms were functional redundant. The oligonucleotide described by Bevec *et al.* that was used to detect eIF5A mRNA in stimulated PBMCs had 100 % homology to human apoptosis-specific eIF-5A and the study pre-dates the cloning of proliferating eIF-5A. Similarly, the primers described by Kruse *et al.* that were used to detect eIF5A by reverse transcription polymerase chain reaction during dendritic cell maturation had 100 % homology to human apoptosis-specific eIF-5A.

The present invention relates to controlling the expression of apoptosis-specific eIF-5A to control the rate of dendritic cell maturation and PBMC activation, which in turn may control the rate of T cell-mediated immunity. Monocytes and macrophages are central to the immune system as they can recognize and become activated/stimulated by foreign invaders and produce cytokines to alert the rest of the immune system.

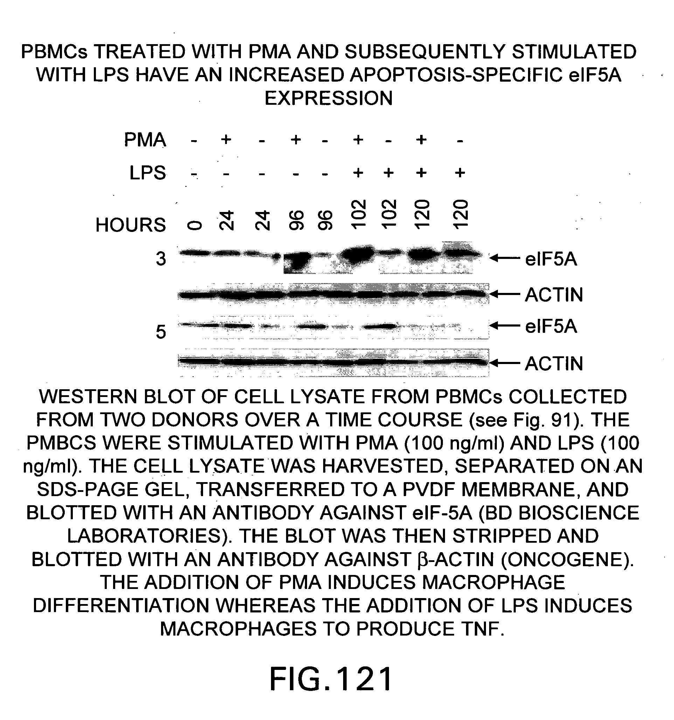

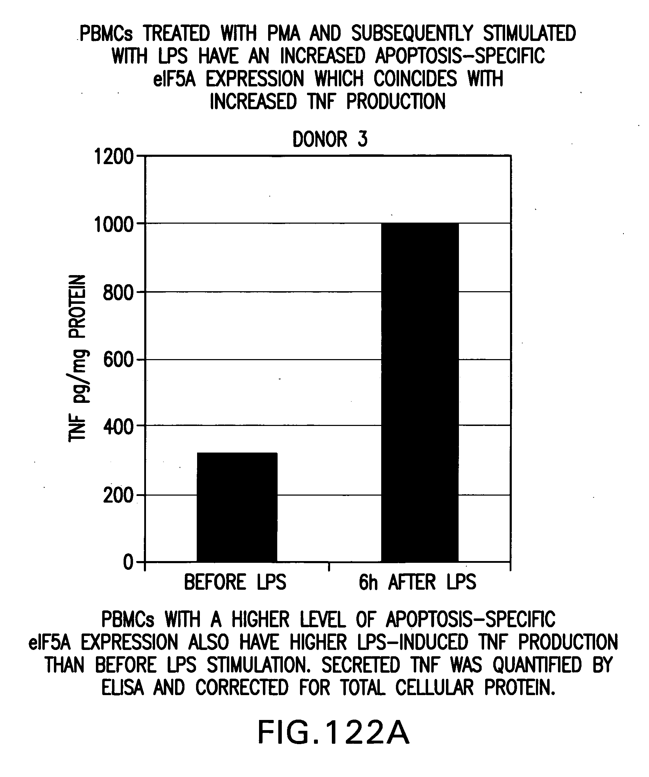

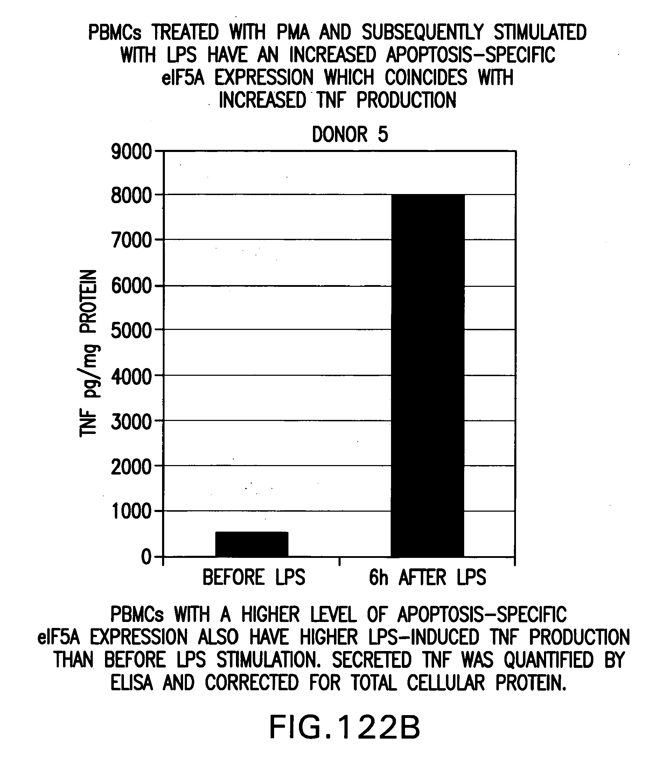

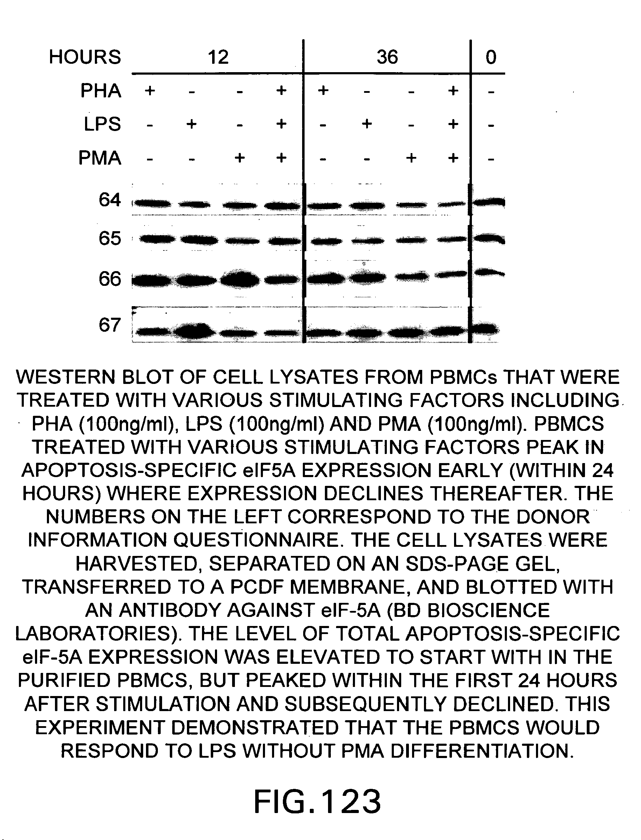

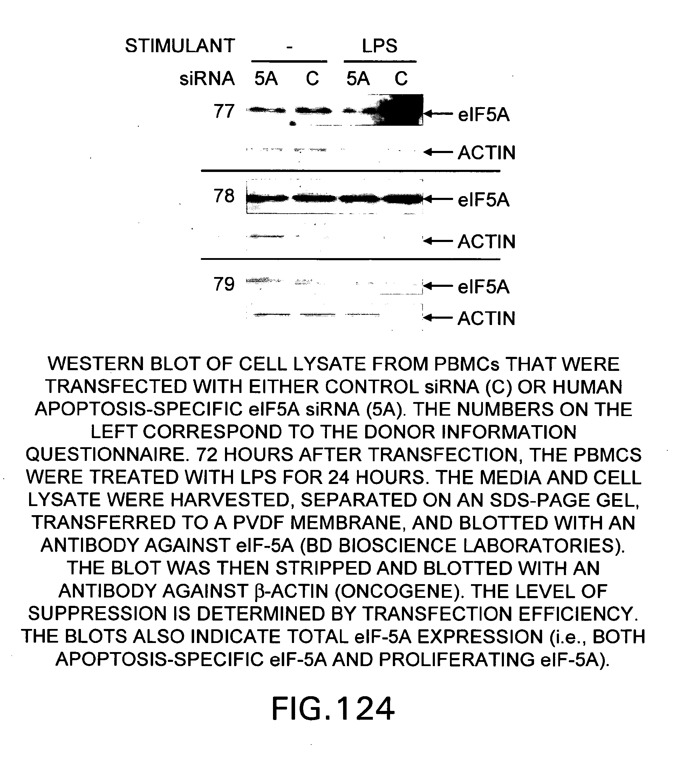

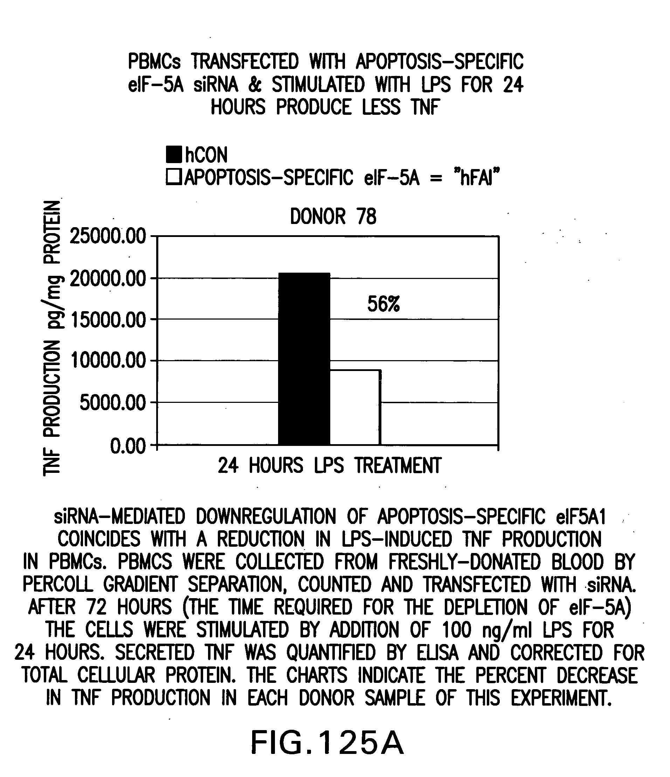

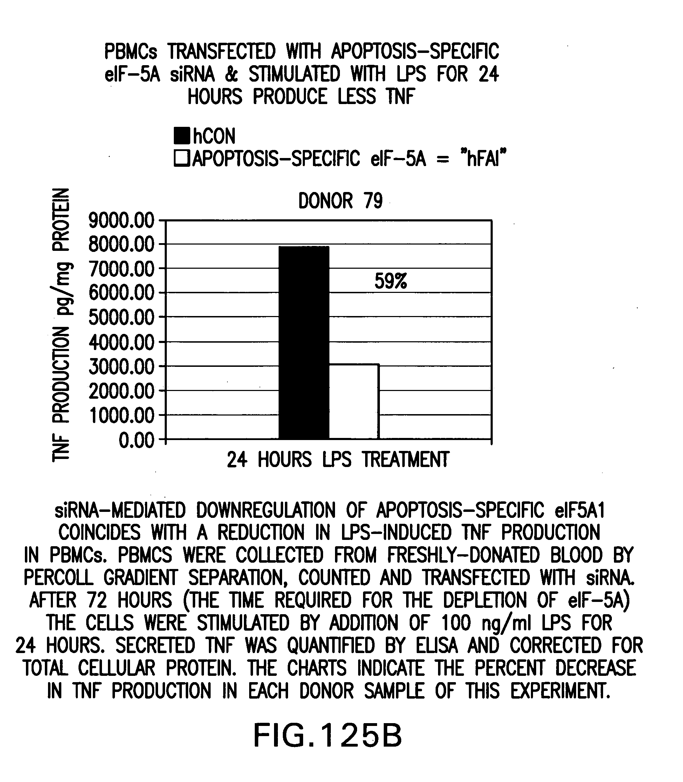

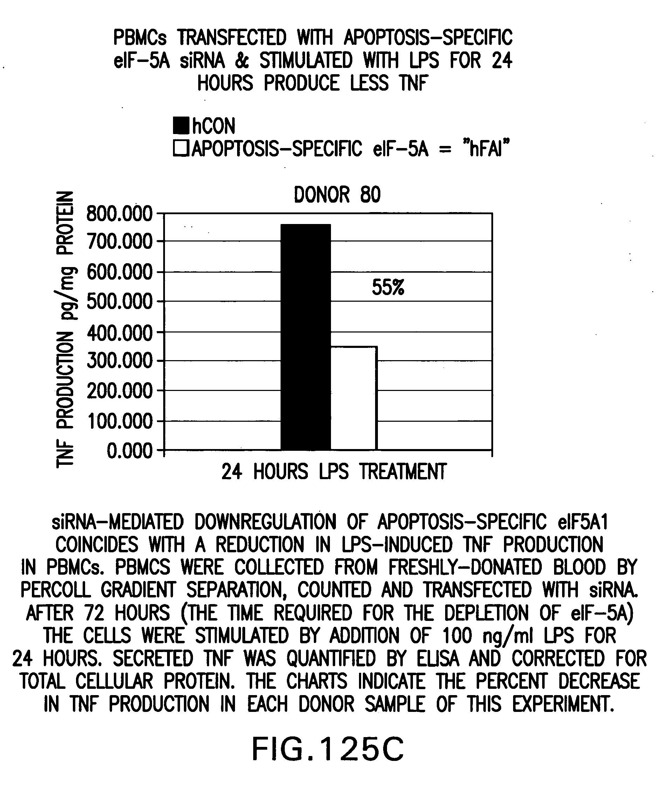

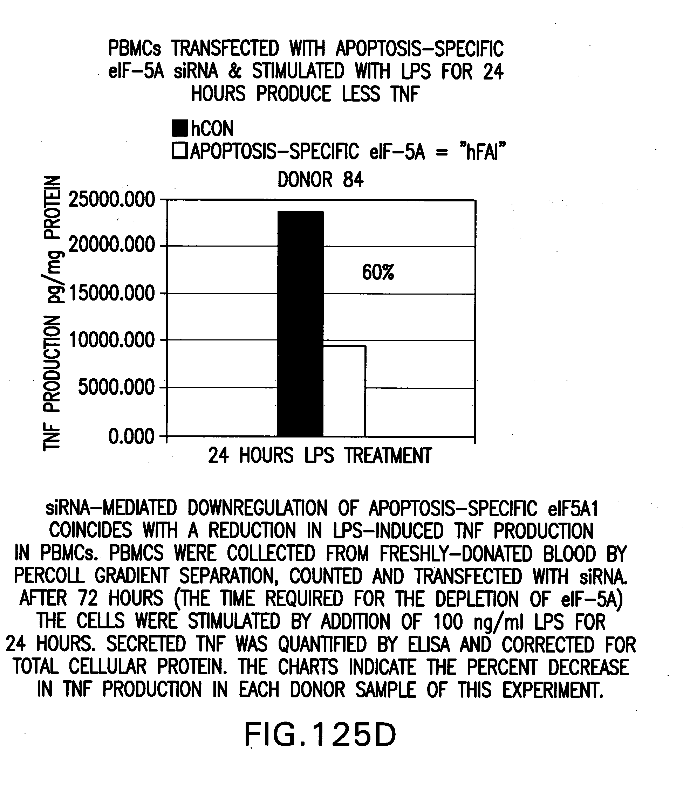

The PBMCs were treated with PMA and subsequently stimulated with LPS to have an increased apoptosis-specific eIF-5A expression. See Figure 121 for the Western blot. Figure 122 shows that this increased expression coincides with increased TNF production. Figure 123 demonstrates that PBMCs respond to LPS without PMA differentiation. Figure 124 shows that PBMCs transfected with apoptosis-specific eIF-5A siRNAs demonstrate suppression of expression of apoptosis-specific eIF-5A and figure 125 shows that suppression apoptosis-specific eIF-5A siRNAs coincides with less production of TNF. Accordingly, the present invention provides a method of decreasing expression of apoptosis-specific eIF-5A in PBMS using antisense polynucleotides or siRNAs of the present invention, which in turn leads to a decreased expression or down-regulation of TNF.

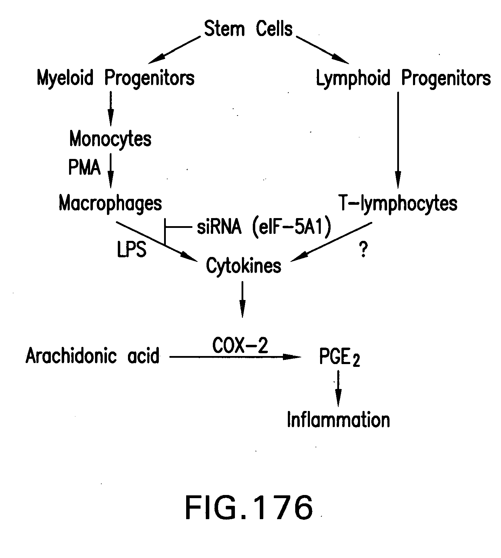

The present inventors also studied the role of apoptosis-specific eIF-5A in the differentiation of monocytes into adherent macrophages using the U-937 cell line, as U-937 is known to express eIF-5A mRNA (Bevec *et al.,* 1994). U-937 is a human monocyte cell line that grows in suspension and will become adherent and differentiate into macrophages upon stimulation with PMA. When PMA is removed by changing the media, the cells become quiescent and are then capable of producing cytokines (Barrios-Rodiles et al., J. Immunol., 163:963-969 (1999)). In response to lipopolysaccharide (LPS), a factor found on the outer membrane of many bacteria and known to induce a general inflammatory response, the macrophages produce both TNF-α and IL-1β (Barrios-Rodiles *et al.,* 1999). See Figure 176 showing a chart of stem cell differentiation and the resultant production of cytokines. The U-937 cells also produce IL-6 and IL-10 following LPS-stimulation (Izeboud et al., J. Receptor & Signal Transduction Research, 19(1-4):191-202. (1999)).

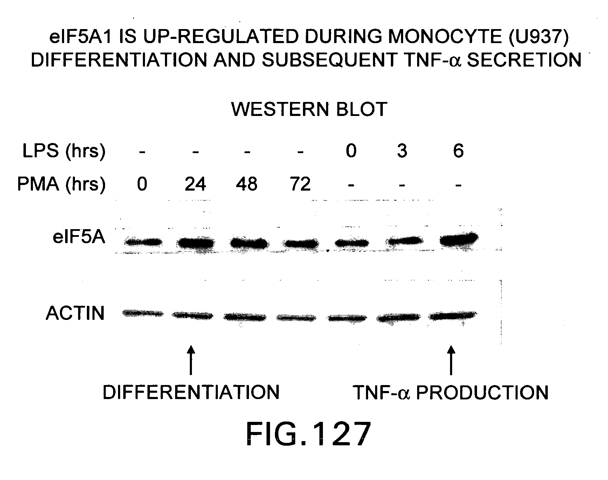

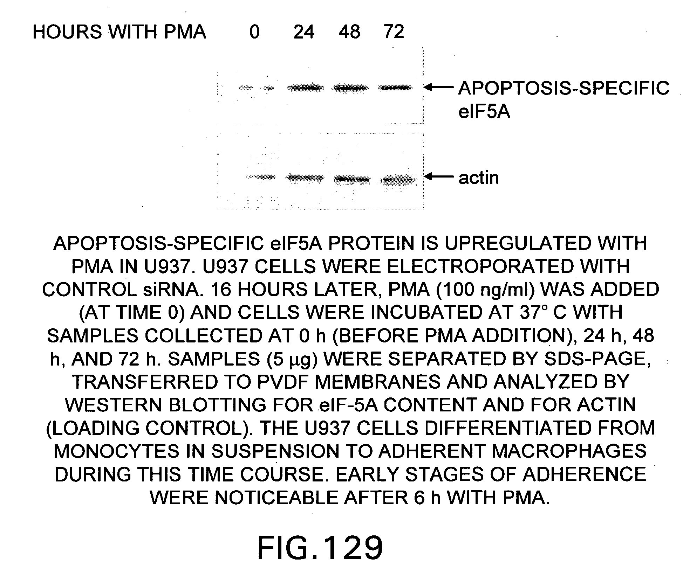

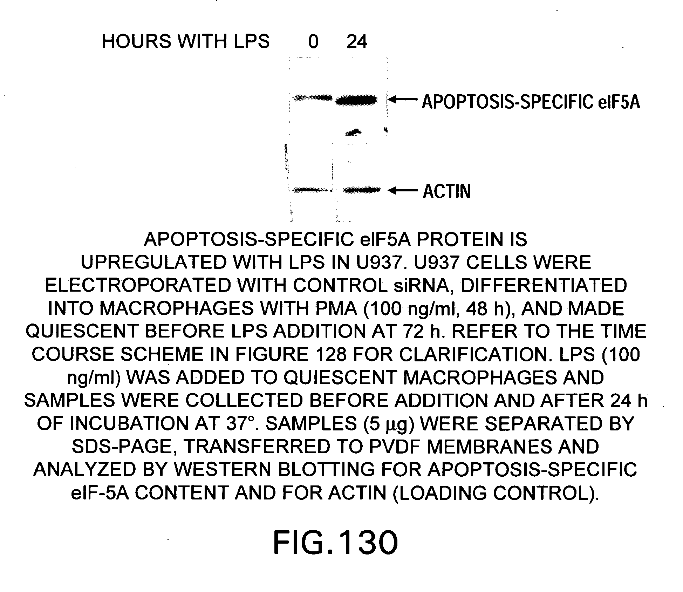

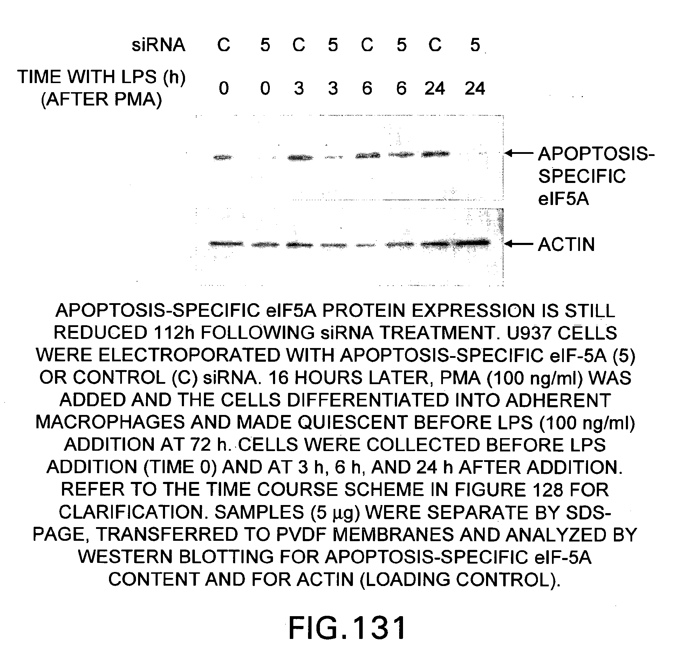

Using U-937 cells, it was shown that apoptosis-specific eIF-5A is upregulated during monocyte differentiation and TNF-α secretion. See Figure 127, and 129-130. Apoptosis-specific eIF-5A protein expression was suppressed with apoptosis-specific eIF-5A siRNA. See figure 131. Control siRNA and apoptosis-specific eIF-5A siRNA-treated cells were compared by Western blotting for the expression of apoptosis-specific eIF-5A, toll-like receptor 4 (TLR4), tumor necrosis factor receptor (TNF-R1), and interferon γ receptor (IFNγ-Rα). The cytokines, TNF, interleukin-1β (IL-1β), IL-6, and IL-8 were quantified by ELISAs and by liquid-phase electrochemiluminescence (ECL).

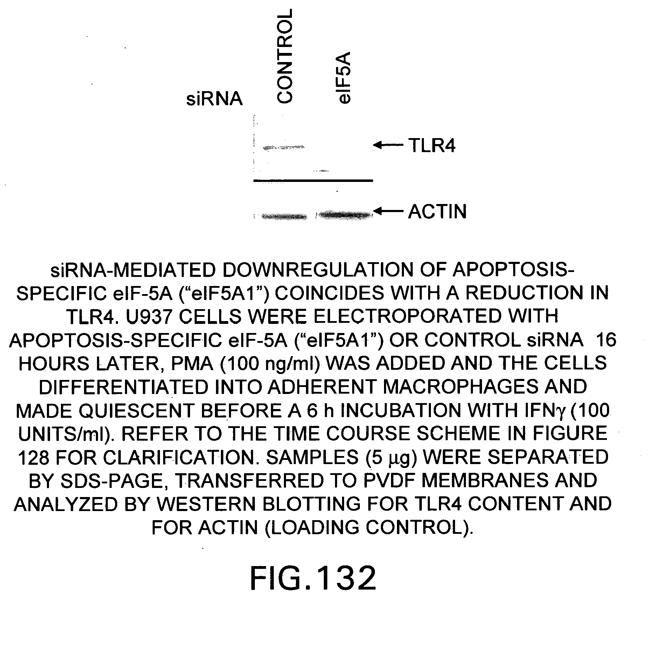

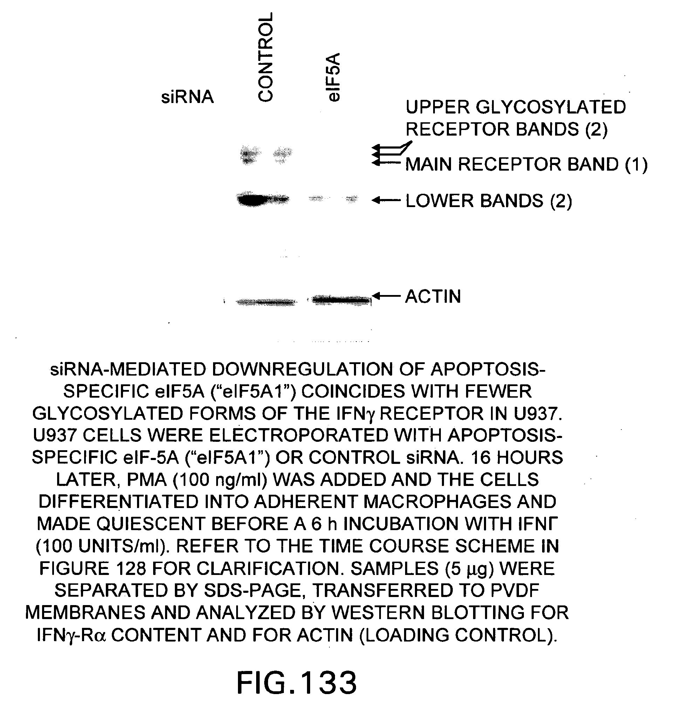

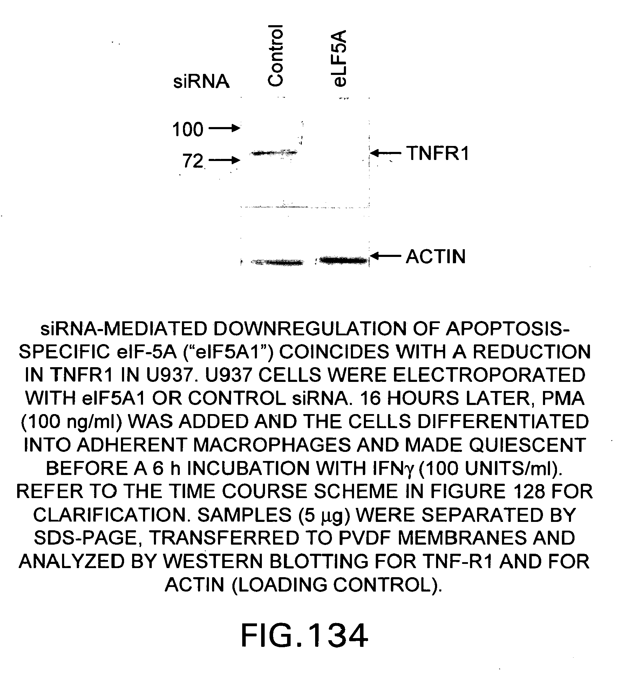

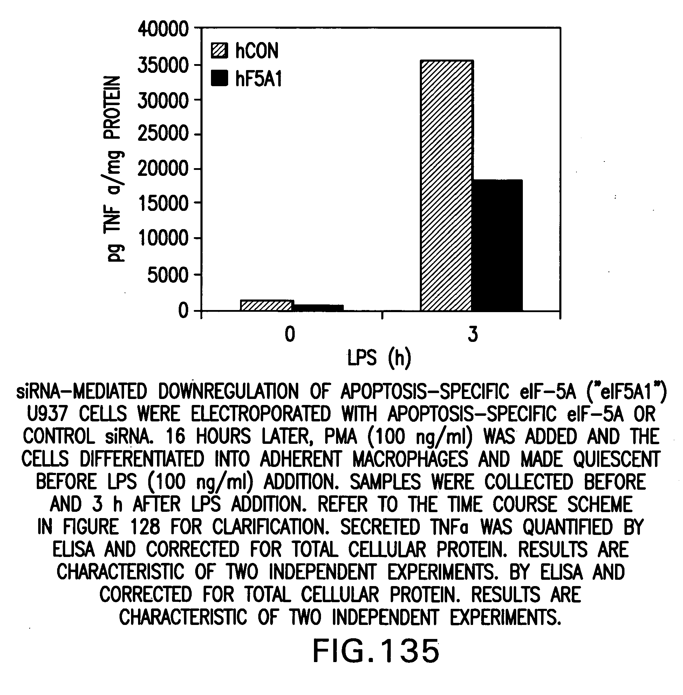

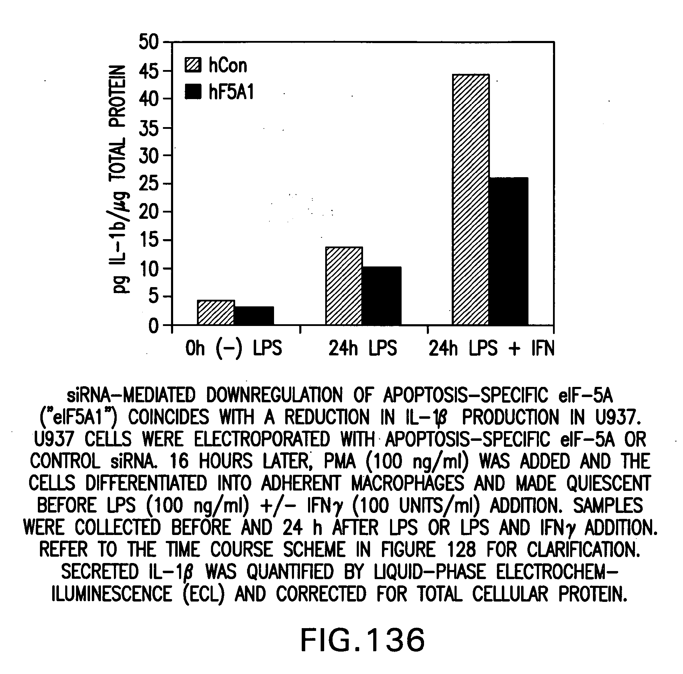

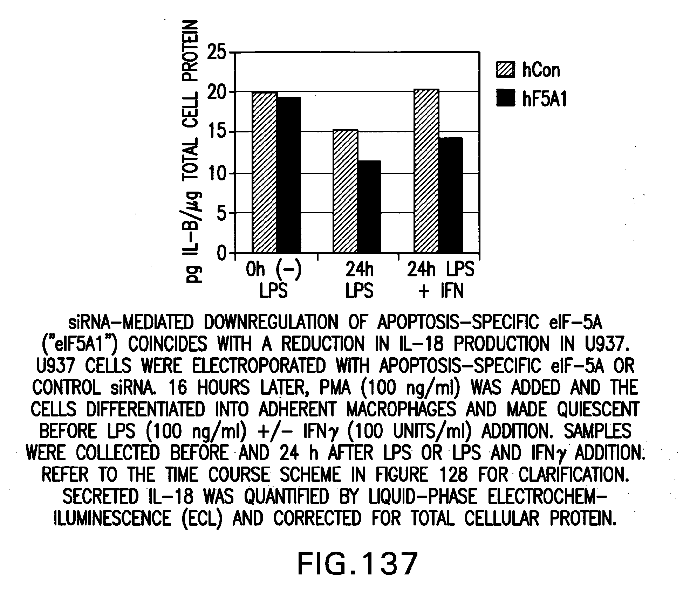

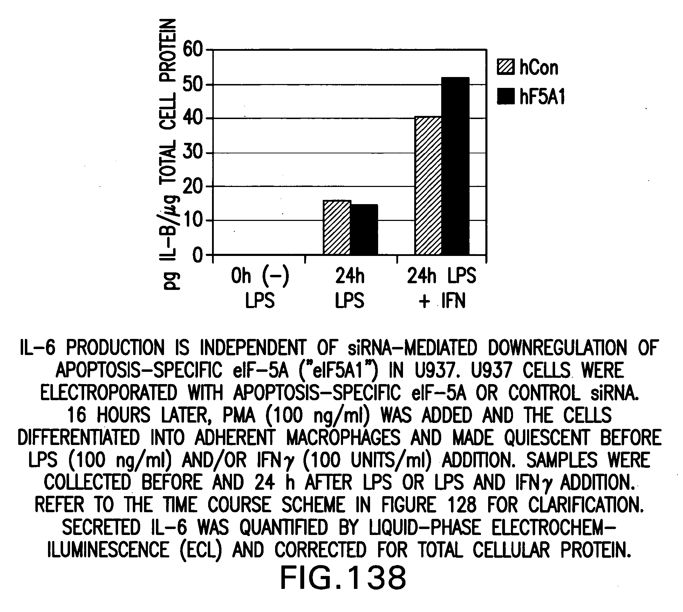

The results show that treatment with apoptosis-specific eIF-5A siRNA specifically down-regulated apoptosis-specific eIF-5A protein expression by more than 80% relative to cells treated with control siRNA. PMA, LPS, and IFN-γ treatment induced apoptosis-specific eIF-5A protein expression. Cells with reduced apoptosis-specific eIF-5A expression also showed reduced protein expression of TLR4 (figure 132), TNF-R1 (figure 134), and IFNγ-Rα (figure 133). Initial experiments also suggest that in cells with reduced apoptosis-specific eIF-5A expression, the LPS-induced TNF α expression was reduced at 3 h (figure 135), and LPS-induced IL-1β (figure 136) and IL-8 production were reduced at 24h (figure 137). These studies suggest that apoptosis-specific eIF-5A may be involved in the post-transcriptional regulation of a number of key cytokine signaling molecules including receptors (TLR4, TNF-R1, and IFNγ-Rα), and cytokines (TNFα, IL-1β, and IL-8). Figure 138 shows that Il-6 production is independent of siRNA-mediated down-regulation of apoptosis-specific eIF-5A.

Accordingly, one aspect of the invention provides for a method of inhibiting or delaying maturation of macrophages to inhibit or reduce the production of cytokines. This method involves providing an agent that is capable of reducing the expression of apoptosis-specific eIF-5A. Since, apoptosis-specific eIF-5A is upregulated during monocyte differentiation and TNF-α secretion, it is believed that apoptosis-specific eIF-5A is necessary for these events to occur. Thus, by reducing apoptosis-specific eIF-5A expression, monocyte differentiation and TNF-α secretion can be reduced. Any agent capable of reducing the expression apoptosis-specific eIF-5A may be used and includes, but is not limited to, and is preferably antisense oligonucleotides or siRNAs against apoptosis-specific eIF-5A as described herein.

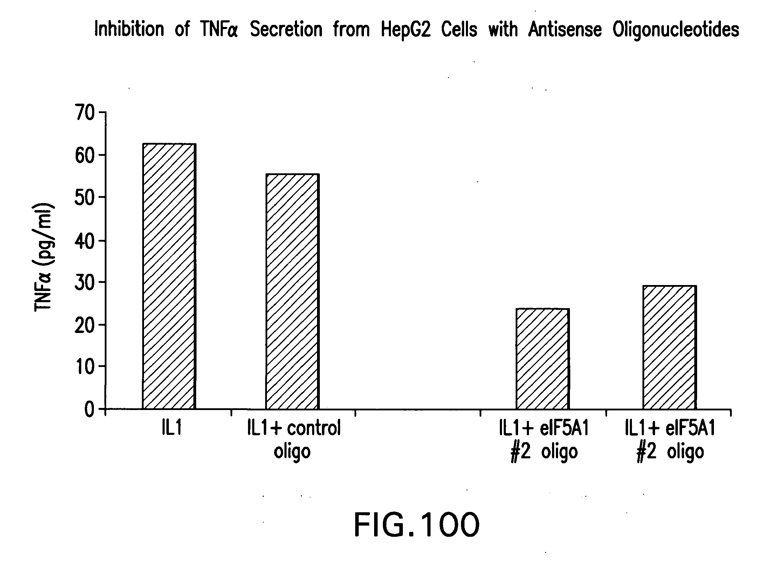

The present inventors have also studied the ability of human apoptosis-specific eIF-5A to promote translation of cytokines by acting as a nucleocytoplasmic shuttle for cytokine mRNAs *in vitro* using a cell line known to predictably produce cytokine(s) in response to a specific stimulus. Some recent studies have found that human liver cell lines can respond to cytokine stimulation by inducing production of other cytokines. HepG2 is a well characterized human hepatocellular carcinoma cell line found to be sensitive to cytokines. In response to IL-1β, HepG2 cells rapidly produce TNF-α, mRNA and protein in a dose-dependent manner (Frede *et al.,* 1996; Rowell *et al.,* 1997; Wordemann *et al.,* 1998). Thus, HepG2 cells were used as a model system to study the regulation of TNF-α production. The present inventors have shown that inhibition of human apoptosis-specific eIF-5A expression in HepG2 cells caused the cells to produce less TNF-α after having been transfected with antisense oligonucleotide directed toward apoptosis-specific eIF-5A. See figure 100.

Thus, the methods of the present invention may be used to reduce levels of a cytokine. The method involves administering an agent capable of reducing expression of apoptosis-specific eIF-5A. Reducing expression of apoptosis-specific eIF-5A also reduces expression of the cytokine and thus leads to a decreased amount of the cytokine produced by cell. The cytokine is a preferably a pro-inflammatory cytokine, including, but not limited to IL-1, IL-18, IL-6 and TNF-α. Suitable agents are discussed above, and include antisense oligonucleotides of apoptosis-specific eIF-5A and siRNA of human apoptosis-specific eIF-5A.

Further, the present invention provides a method of treating pathological conditions characterized by an increased IL-1, TNF-alpha, IL-6 or IL-18 level comprising administering to a mammal having said pathological condition, agents to reduce expression of apoptosis-specific eIF-5A as described above (antisense oligonucleotides and siRNA). Known pathological conditions characterized by an increase in IL-1, TNF-alpha, or Il-6 levels include, but are not limited to, arthritis-rheumatoid and osteo arthritis, asthma, allergies, arterial inflammation, crohn's disease, inflammatory bowel disease, (ibd), ulcerative colitis, coronary heart disease, cystic fibrosis, diabetes, lupus, multiple sclerosis, graves disease, periodontitis, glaucoma and macular degeneration, ocular surface diseases including keratoconus, organ ischemia- heart, kidney, repurfusion injury, sepsis, multiple myeloma, organ transplant rejection, psoriasis and eczema. For example, inflammatory bowel disease is characterized by tissue damage caused, in part, by pro-inflammatory cytokines and chemokines released by intestinal epithelial cells.

Interferon gamma (IFN-γ) is a cytokine produced by natural killer (NK) and T lymphocytes, which plays a central role in the cytokine network. IFN-γ induces a variety of responses in sensitive cells including anti-viral, anti-proliferative, and immunoregulatory activity. Binding of IFN-γ to its receptor (IFN-γR) leads to autophosphorylation of the Janus kinases JAK1 and JAK2. Phosphorylation of JAK1 at tyrosine residues 1022 and 1023 is believed to involved in the activation of catalytic events (Liu et al., Curr. Biol. (7): 817-826 (1997)). The IFN-γR is composed of at least two chains, designated IFN-γRα and IFN-γRβ. Binding of the receptor to its ligand, IFN-y, results in autophosphorylation of JAK1, which leads to recruitment and tyrosine phosphorylation of signal transducer and activator of transcription (STAT) transcription factors. Phosphorylation of STAT transcription factors leads to dimerization and nuclear translocation of STAT where it subsequently binds to elements upstream of target promoters to regulate transcription. The JAK-STAT pathway represents a good target for anti-inflammatory therapies since altering JAK-STAT signaling can reduce cytokine-induced pro-inflammatory responses and inappropriate expression of IFN-γ is thought to contribute to autoimmune disorders.

Intestinal epithelial cells, under normal physiological conditions, are hyporesponsive to the products, including lipopolysaccharide (LPS), of the natural intestinal flora. IFN-γ appears to be able to render intestinal epithelial cells responsive to LPS, as it leads to the production of cytokines such as IL-8 and TNF-α. One mechanism by which IFN-γ may be able to restore LPS sensitivity to intestinal epithelial cells is to increase LPS uptake by increasing expression of MD-2 and Toll receptor 4 (TLR4) - two proteins required for LPS recognition (Suzuki et al., Infection and Immunity; (71): 3503-3511 (2003)). Augmented production of Th1 cytokines such as IFN-γ, which results in altered responsiveness of intestinal epithelial cells to microbial products of commensal bacteria, is thought to contribute to the chronic inflammation that characterizes inflammatory bowel disease.

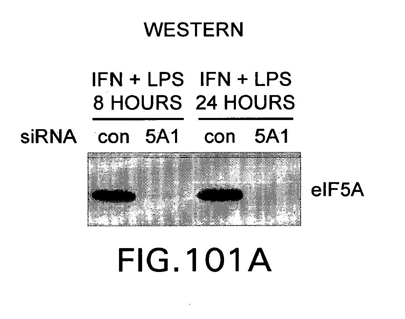

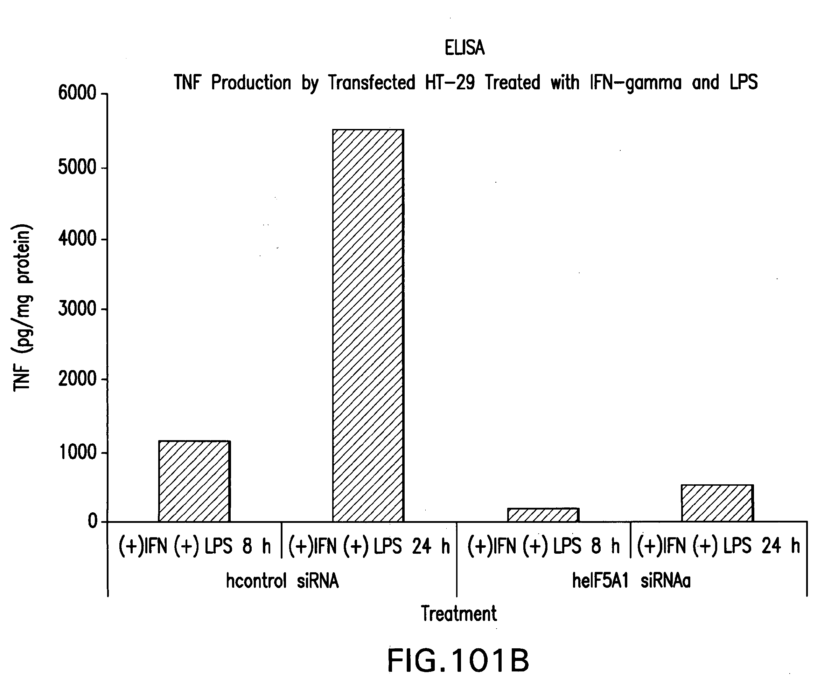

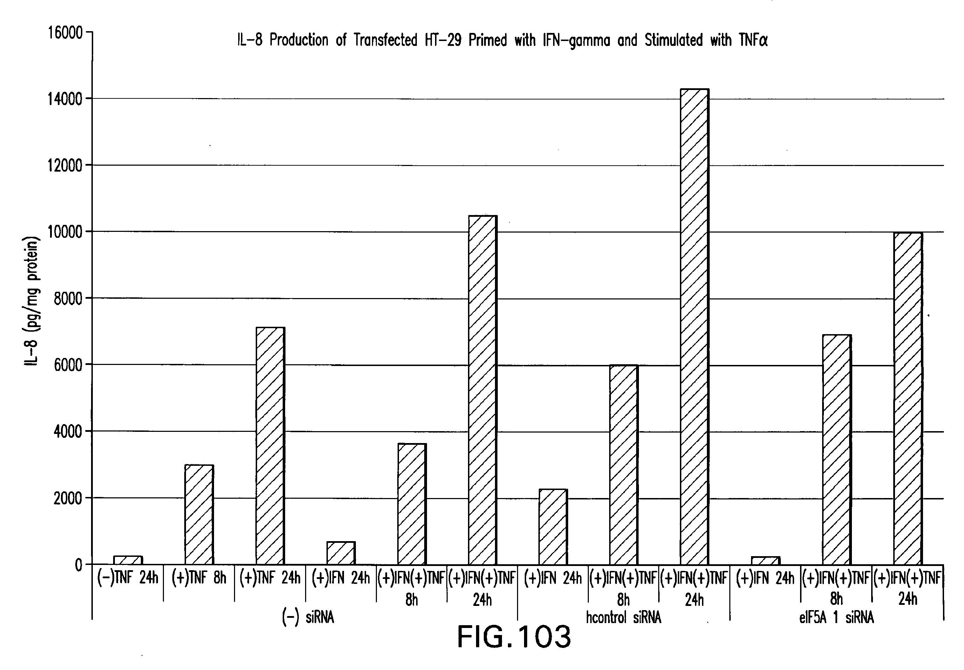

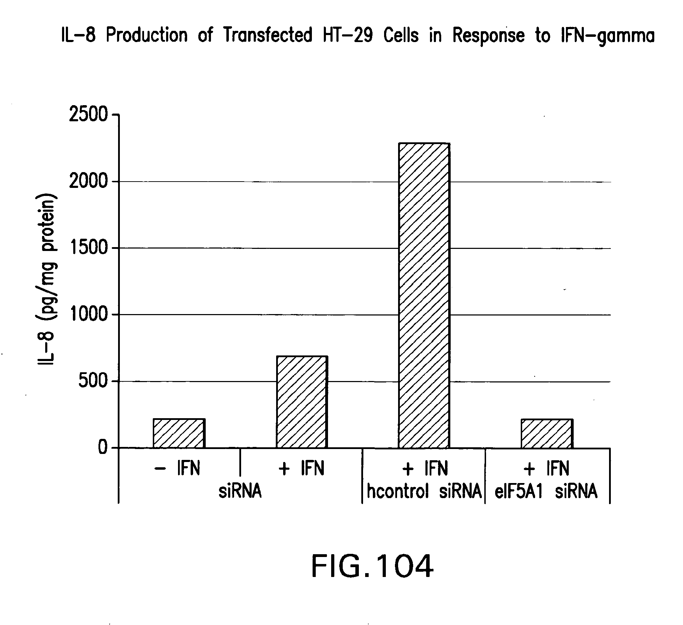

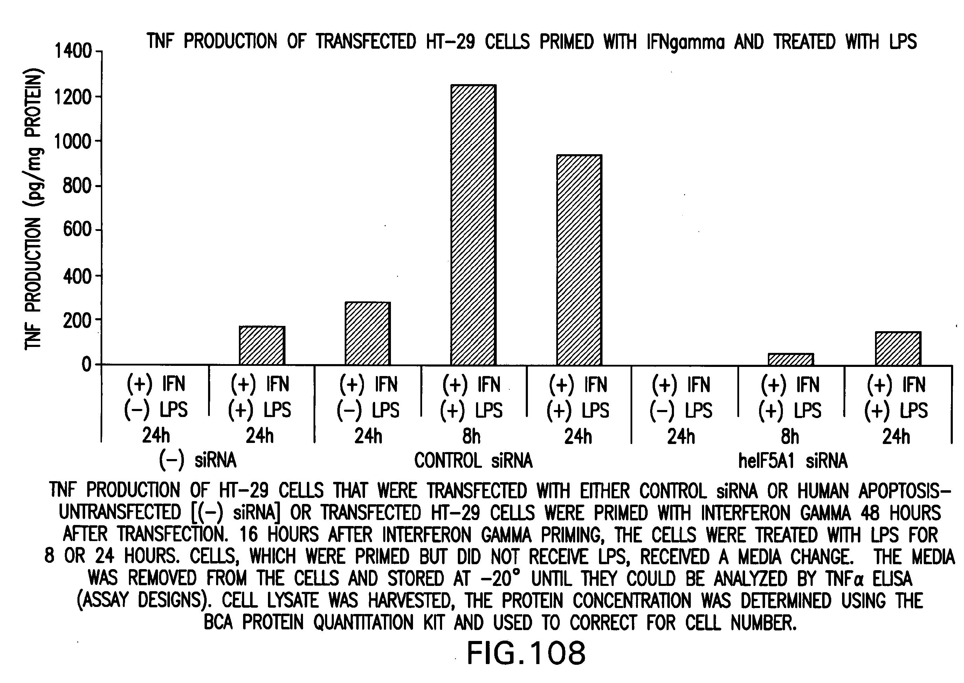

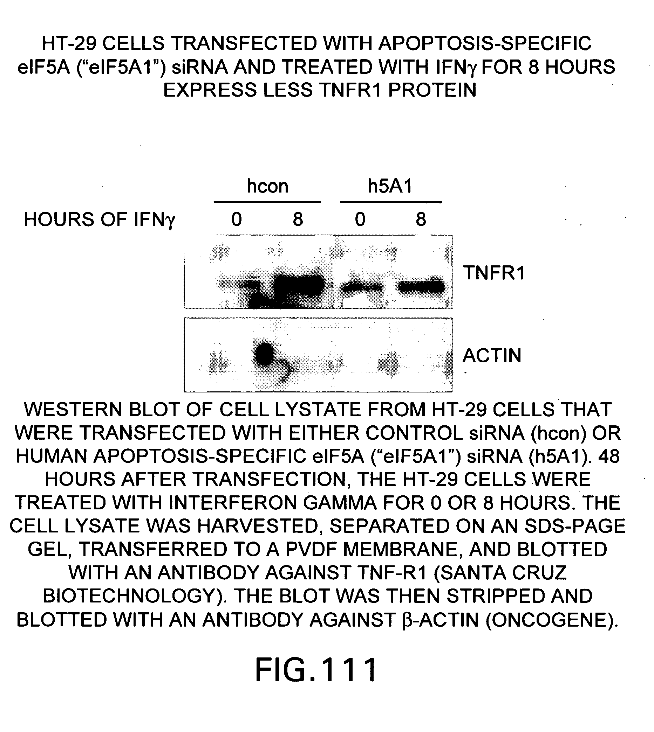

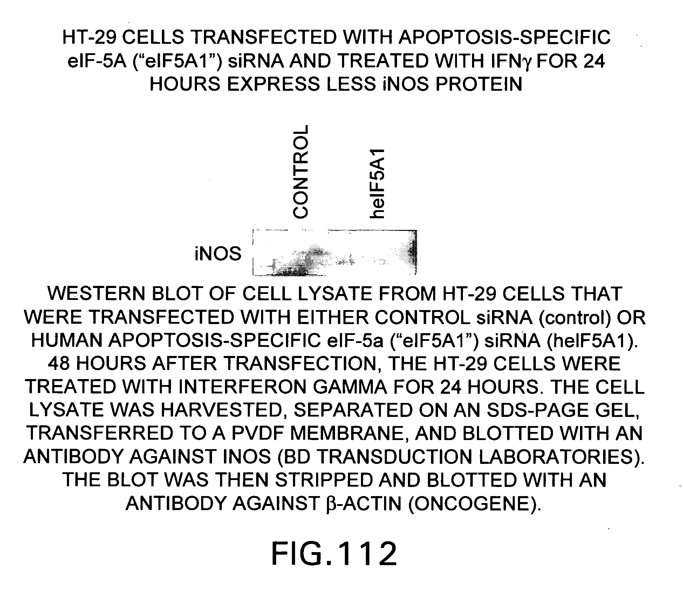

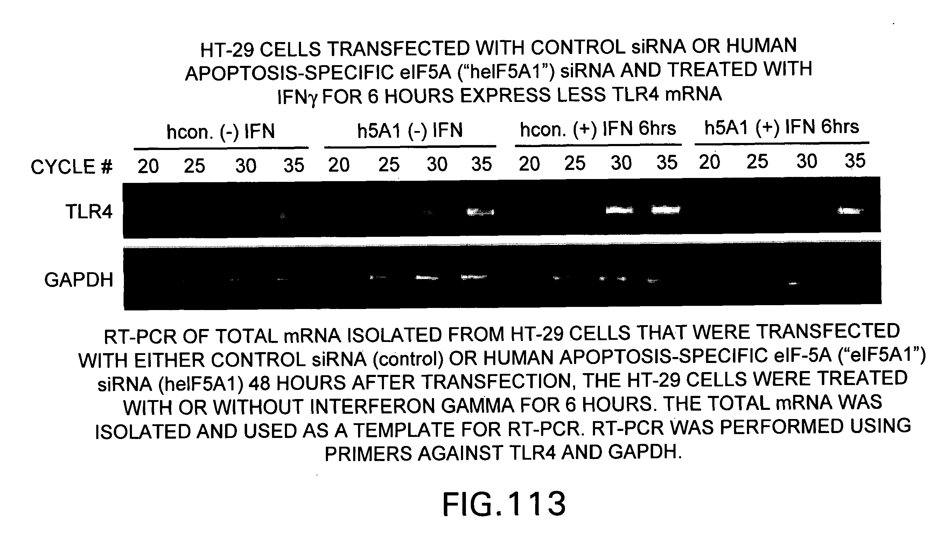

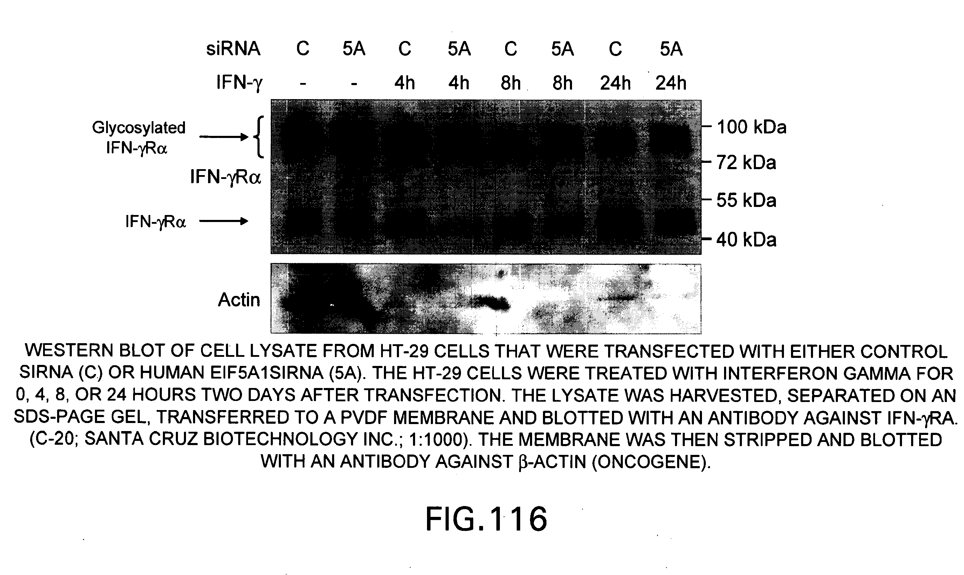

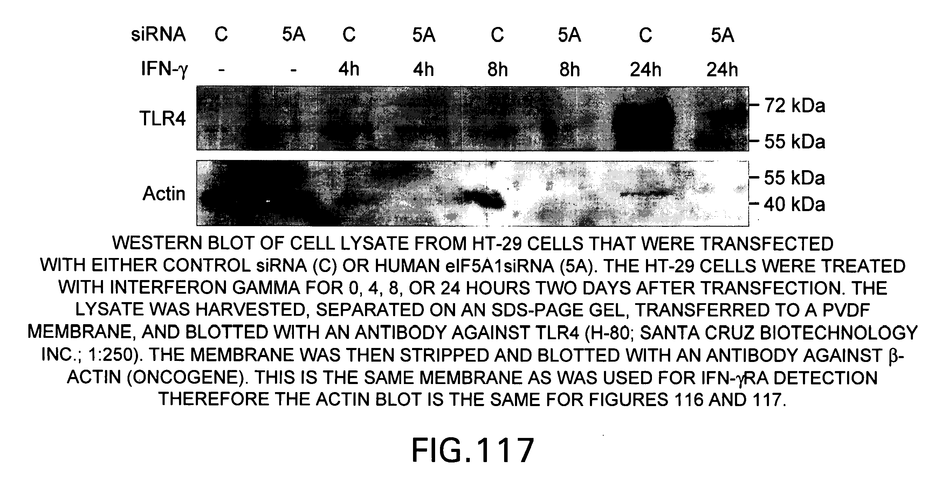

The present inventors have shown that siRNAs against apoptosis-specific eIF-5A leads to a decreased expression of TNF-α when HT-29 cells (a human epithelial cell line) are exposed to IFN-γ and LPS (figures 101-102, 108). Further, the present inventors have shown that siRNAs against apoptosis-specific eIF-5A leads to a decreased expression of IL-8 when HT-29 cells (a human epithelial cell line) are exposed to IFN-γ and TNF-α (figure 103 and 104). The present inventors have also shown that HT-29 cells transfected with siRNA against apoptosis-specific eIF-5A and exposed to IFN express less TNFR1 (figure 111), less iNOs protein (figure 112), less TLR4 (figure 113 and 117), and less IFN-γRa (figure 116) mRNA.

Another molecule involved in inflammation is NFκ-β (also referred to as or NKκ-beta or NFkB). NFκβ is a major cell-signaling molecule for inflammation as its activation induces the expression of COX-2, which leads to tissue inflammation. The expression of the COX-2-encoding gene, believed to be responsible for the massive production of prostaglandins at inflammatory sites, is transcriptionaly regulated by NFkB. NFkB resides in the cytoplasm of the cell and is bound to its inhibitor. Injurious and inflammatory stimuli release NFkB from the inhibitor. NFkB moves into the nucleus and activates the genes responsible for expressing COX-2. Thus, by reducing levels of NFk beta, inflammation can be reduced.

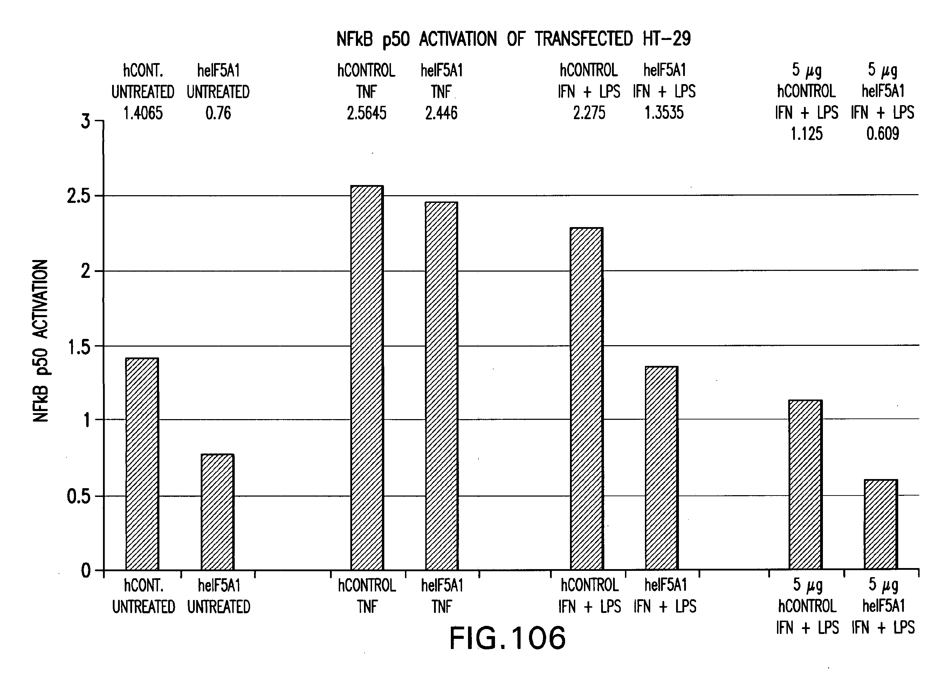

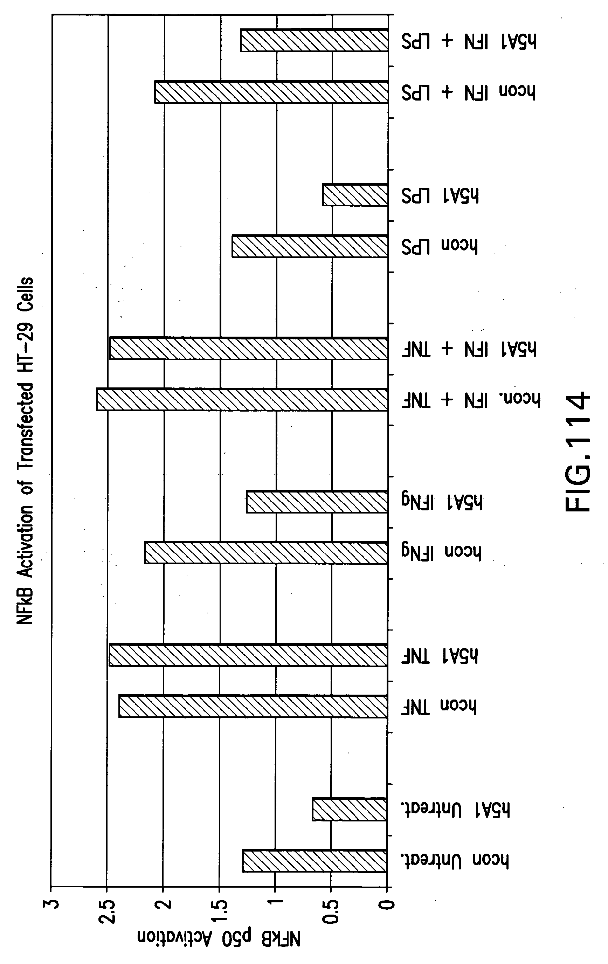

In one experiment by the present inventors, human epithelial cells (HT-29 cells) were treated with siRNA targeted at apoptosis-specific eIF-5A. Inflammation was then induced by NFkB by the addition of TNF or interferon gamma and LPS for one hour. The results of this experiment show that inhibiting the expression of apoptosis-specific eIF-5A with siRNAs provided for a reduction in the levels of NFkB that were activated by the gamma interferon and LPS. See figure 106 and 114.

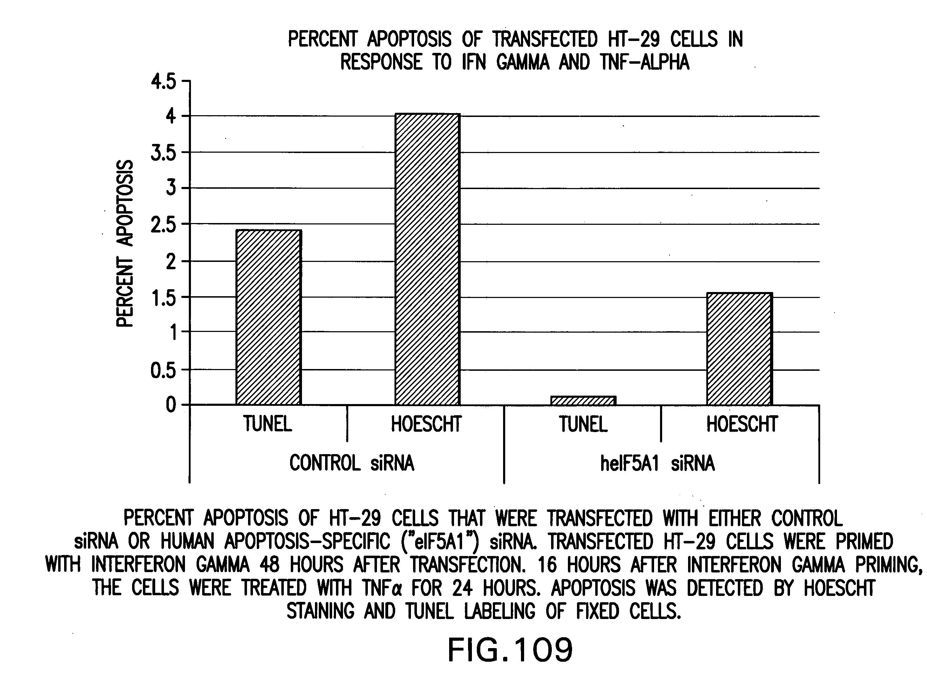

The present inventors also demonstrated through Hoechst staining and TUNEL labeling that HT-29 cells transfected with siRNAs against apoptosis-eIF-5A show a decrease in apoptosis after being exposed to IFN-γ and TNF-α. See figure 109.

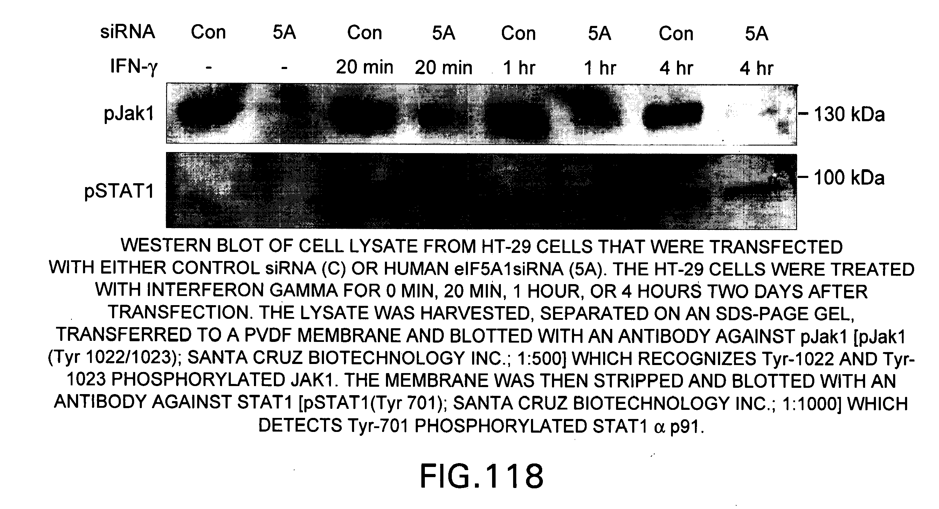

Figure 118 also demonstrates that siRNA-mediated suppression of apoptosis-specific eIF-5A expression results in decreased phosphorylation of STAT1α and JAK1 in response to IFN-γ treatment. The decrease in IFN-γ-stimulated upregulation of TLR4 with apoptosis-specific eIF-5A siRNA is consistent with the previous data that demonstrates that apoptosis-specific eIF-5A siRNA decreases NF-κB p50 activation and TNF-α production in HT-29 cells in response to IFN-γ and LPS. The data is consistent with the theory that apoptosis-specific eIF-5A is regulating IFN-γ signaling through the JAK-STAT pathway. Interfering with apoptosis-specific eIF-5A expression therefore prevents IFN-γ stimulated upregulation of TLR4 (which is required for colon epithelial cells to detect LPS) and the cells thus remain hyporesponsive to LPS. As a result, NFκB p50 is not activated in response to LPS binding by TLR4 and cytokine production (TNF-α and IL-8) is inhibited.

In further support of the idea that apoptosis-specific eIF-5A regulates IFN-γ signaling is the finding that apoptosis-specific eIF-5A siRNA dramatically decreases the phosphorylation of STAT1α and JAK1 - two important steps in the transduction of IFN-γ signals. Although a decrease in the IFN-γ receptor α upregulation upon stimulation with IFN-γ is seen, the amount of IFN-γ appears to be the same before IFN-γ treatment whether it is treated with control siRNA or apoptosis-specific eIF-5A siRNA. Although it is possible that the IFN-γRβ chain could be affected by apoptosis-specific eIF-5A siRNA, the data suggests that IFN-γ binding to it's receptor may be unaffected by apoptosis-specific eIF-5A siRNA. This suggests that apoptosis-specific eIF-5A may be required for post-transcriptional regulation of JAK1 or a protein which regulates JAK1 expression or phosphorylation. It is clear that proper function of the JAK-STAT pathway (at least through JAK1 and STAT1α), and thereby IFN-γ signaling, requires apoptosis-specific eIF-5A.

It is also worth noting that the control siRNA was able to elicit a response in HT-29 cells that may be a result of double stranded RNA detection through TLR3. Specifically, HT-29 cells treated with control siRNA produced significantly more TNF-α and IL-8 than untransfected cells. The apoptosis-specific eIF-5A siRNA did not produce this response. Also, Jak1 was phosphorylated in control siRNA-transfected cells that were not treated with IFN-γ (see figure 118). It is possible that this could reflect activation of the JAK-STAT pathway, which is involved in the interferon response resulting from double stranded RNA recognition by TLR3. The data did not show JAK1 phosphorylation in apoptosis-specific eIF-5A siRNA-treated cells even in the absence of IFN-γ treatment, which suggests the possibility that apoptosis-specific eIF-5A siRNA may be able to block the interferon response triggered by detection of double stranded RNA which also occurs through the Jak-STAT pathway.

The present inventors inhibited expression of apoptosis-specific eIF-5A in HT-29 cells using siRNA to examine the effects on interferon gamma (IFN-γ) signaling. Apoptosis-specific eIF-5A siRNA reduced the ability of HT-29 cells to secrete TNF-α in response to IFN-γ and LPS by greater than 90%. Apoptosis-specific eIF-5A siRNA also inhibited IL-8 secretion in response to IFN-γ but not in response to TNF-α. Likewise, apoptosis-specific eIF-5A siRNA was found to decrease NF-κB p50 activation in an IFN-γ-specific manner and decrease IFN-γ-stimulated expression of TLR4 and TNFR1. Of further interest is the finding that transfection with the control siRNA significantly increased TNF-α secretion compared to mock-transfected controls when cells were stimulated with IFN-γ and apoptosis-specific eIF-5A siRNA was able to significantly reduce this response. These results indicate that apoptosis-specific eIF-5A may be a post-transcriptional regulator of the IFN-γ-signaling pathway and could also be involved in the cellular response to double-stranded RNA. Inhibition of apoptosis-specific eIF-5A by siRNA interferes with IFNγ signaling and reduces the ability of intestinal epithelial cells to respond to LPS and TNF-α via TLR4 and TNFR1, respectively. Thus, inhibition of apoptosis-specific eIF-5A appears to have a direct immunoregulatory effect on intestinal epithelial cells and may be a therapeutic target for inflammatory bowel disease.

Accordingly, one embodiment of the present invention provides methods of inhibiting or reducing a pro-inflammatory response by inhibiting or reducing expression of endogenous apoptosis-specific eIF-5A. Inhibiting expression of apoptosis-specific eIF-5A is preferably carried out by the use of antisense polynucleotides or siRNAs of apoptosis-specific eIF-5A of the present invention as described previously. As presented above, the present inventors have shown that when reduction of expression of endogenous apoptosis-specific eIF-5A occurs, expression of various biomolecules involved in the inflammation cascade are also reduced. Reducing levels of these biomolecules or reducing activation of these biomolecules necessary for the inflammation cascade causes a decrease in inflammation. Decreasing the ability of a cell to enter into the inflammation cascade may prove useful in treating diseases/conditions related to chronic inflammation such as, but not limited to, inflammatory bowel disease, arthritis, Chron's disease, and lupus.

Accordingly, the present invention also provides a method of decreasing levels of p53, decreasing levels of pro-inflammatory cytokines, decreasing levels of active NFκβ; TLR4; TNFR-1, IFN-γRα, iNOS, or TNF-α, and reducing phosphorylation of STAT1 and JAK1 by inhibiting or suppressing expression of apoptosis-specific eIF-5A using antisense or siRNAs directed against apoptosis-specific eIF-5A.

In addition to decreasing expression of various deleterious biomolecules involved in the inflammation cascade, the present invention is also directed to a method for reducing the expression of p53. This method involves administering an agent capable of reducing expression of apoptosis-specific eIF-5A, such as the antisense oligonucleotides or the siRNAs described above. Reducing expression of apoptosis-specific eIF-5A with antisense oligonucleotides (SEQ ID NO:26 and 27) reduces expression of p53 as shown in figure 42 and example 10.

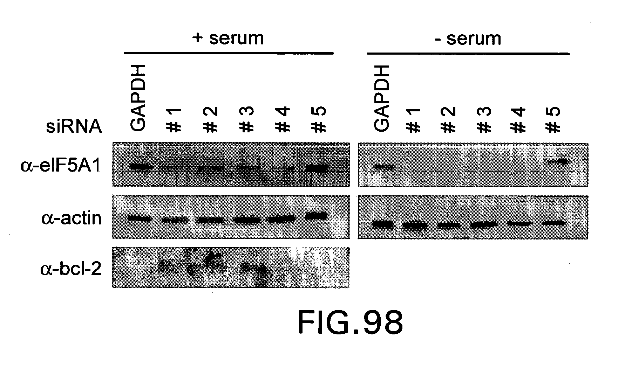

The present invention is also directed to a method for increasing the expression of Bcl-2. This method entails administering an agent capable of reducing expression of human apoptosis-specific eIF-5A. Preferred agents include antisense oligonucleotides and siRNAs described above. Reducing expression of apoptosis-specific eIF-5A increases expression of Bcl-2 as shown in figure 98 and example 13. Figure 98 shows that cells transfected with apoptosis-specific eIF-5A siRNA produced less apoptosis-specific eIF-5A protein and in addition, produced more Bcl-2 protein. A decrease in apoptosis-specific eIF-5A expression correlates with an increase in BCL-2 expression.

The present invention also provides a method of delivering siRNA to mammalian lung cells *in vivo*. siRNAs directed against apoptosis-specific eIF-5A were administered intranasally (mixed with water) to mice. 24 hours after administration of the siRNA against apoptosis-specific eIF-5A, lipopolysaccharide (LPS) was administered intranasally to the mice. LPS is a macromolecular cell surface antigen of bacteria that when applied *in vivo* triggers a network of inflammatory responses. Intranasally delivering LPS causes an increase in the number of neutrophils in the lungs. One of the primary events is the activation of mononuclear phagocytes through a receptor-mediated process, leading to the release of a number of cytokines, including TNF-α. In turn, the increased adherence of neutrophils to endothelial cells induced by TNF-α leads to massive infiltration in the pulmonary space.

After another 24 hours, the right lung was removed and myeloperoxidase was measured. Myeloperoxidase ("MPO") is a lysosomal enzyme that is found in neutrophils. MPO uses hydrogen peroxidase to convert chloride to hypochlorous acid. The hypochlorous acid reacts with and destroys bacteria. Myeloperoxidase is also produced when arteries are inflamed. Thus, it is clear that myeloperoxidase is associated with neutrophils and the inflammation response. The mouse apoptosis-specific eIF-5A siRNA suppressed myeloperoxidase by nearly 90% as compared to the control siRNA. In the study, there were 5 mice in each group. The results of this study show that siRNA can be delivered successfully *in vivo* to lung tissue in mammals, and that siRNA directed against apoptosis-specific eIF-5A inhibits the expression of apoptosis-specific eIF-5A resulting in a suppression of myeloperoxidase production.

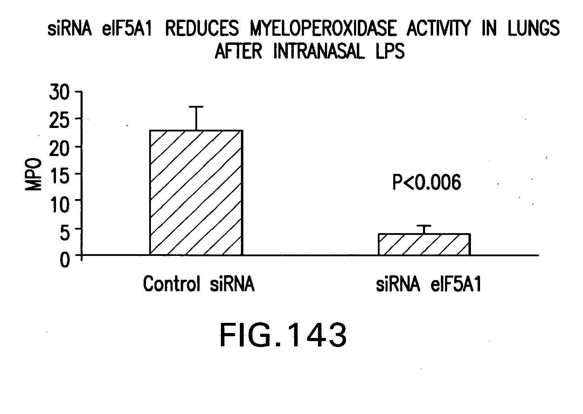

The present inventors have thus demonstrated that down regulating apoptosis-specific eIF-5A with siRNAs decreases levels of myeloperoxidase in lung tissue after exposure to LPS (which normally produces an inflammatory response involving the production of myeloperoxidase), and thus decrease or suppress the inflammation response. See figure 143 showing that after mice received LPS and eIF-5A1 siRNA intranasaly they had a reduced myeloperoxidase activity as compared to mice receiving control siRNA. Accordingly, one embodiment of the present invention provides a method of reducing levels of MPO in lung tissue by delivering siRNAs against apoptosis-specific eIF-5A to inhibit or reduce expression of apoptosis-specific eIF-5A. The reduction in the expression of apoptosis-specific eIF-5A leads to a reduction of MPO. Delivery of the siRNA apoptosis-specific eIF-5A may be intranasal.

MPO levels are a critical predictor of heart attacks and cytokine-induced inflammation caused by autoimmune disorders. This ability to decrease or suppress the inflammation response may serve useful in treating inflammation related disorders such as auto-immune disorders. In addition, the ability to lower MPO could be a means of protecting patients from ischemic events and heart attacks.

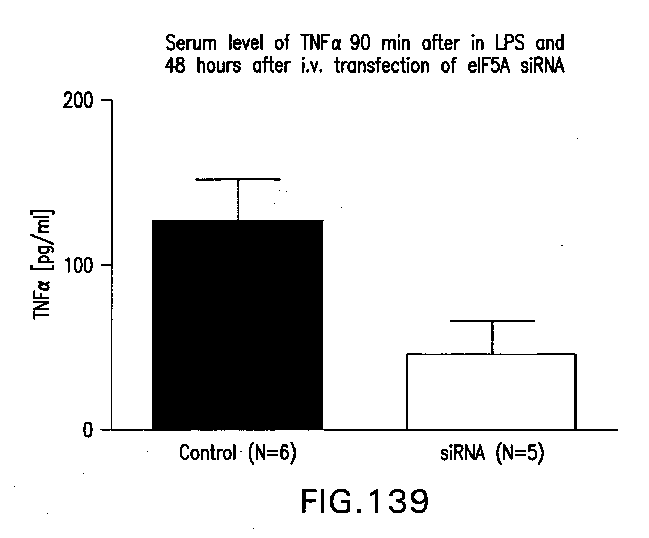

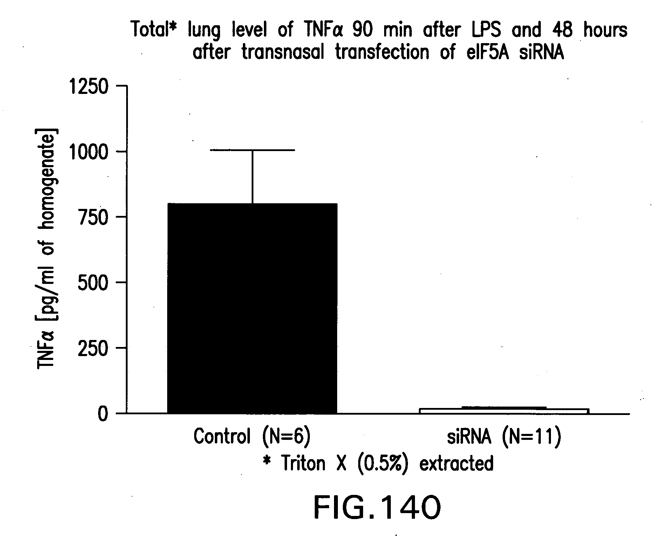

Figure 139 shows the results of an experiment performed in mice where siRNAs against apoptosis-specific eIF-5A were able to decrease the level of TNF-α in the mice serum. The siRNAs were delivered intravenously into a tail vein of the mice. The TNFα serum levels were measured 90 minutes after administration of LPS and 48 hours after intravenous transfection of siRNAs against apoptosis-specific eIF-5A. Figure 140 shows the results of an experiment performed in mice where the siRNAs were delivered trans-nasally (as described above). Total levels of TNF-α were measured in the serum of the mice. The siRNAs against apoptosis-specific eIF-5A caused a decrease in the amount of TNFα. Accordingly, one embodiment of the present invention provides a method of reducing levels of TNF-α in serum by delivering siRNAs against apoptosis-specific eIF-5A to inhibit or reduce expression of apoptosis-specific eIF-5A. The reduction in the expression of apoptosis-specific eIF-5A leads to a reduction of TNF-α in the serum.

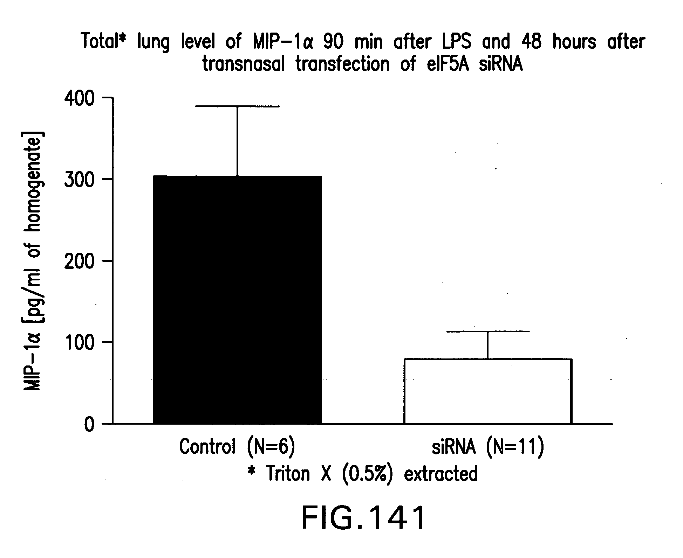

Figure 141 shows that levels of macrophage inflammatory protein 1-alpha (MIP-1α) were also decreased. MIP-1α is a low molecular weight chemokine that belongs to the RANTES (regulated on activation normal T cell expressed and secreted) family of cytokines and binds to receptors CCR1, CCR5 and CCR9. Accordingly, one embodiment of the present invention provides a method of reducing levels of MIP-1α in lung tissue by delivering siRNAs against apoptosis-specific eIF-5A to inhibit or reduce expression of apoptosis-specific eIF-5A. The reduction in the expression of apoptosis-specific eIF-5A leads to a reduction of MIP-1α.

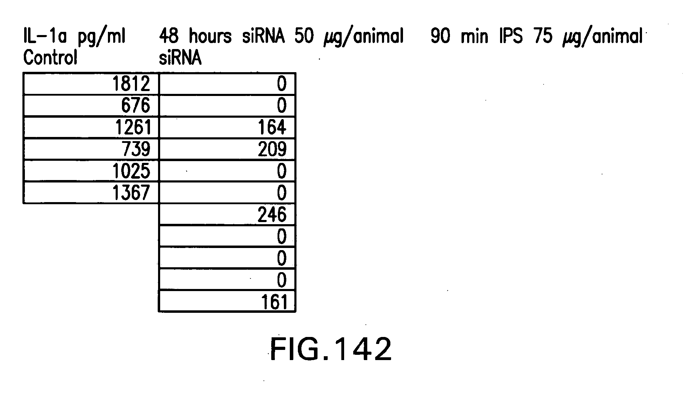

Figure 142 shows the results of an experiment where mice were treated with siRNAs against apoptosis-specific eIF-5A (intranasal/transnasal delivery). The results show that 90 minutes after treatment with LPS and 48 hours after being treated with the siRNAs, there was a marked decrease in levels of Il-1a measured the mice lungs as compared to mice lungs not having been treated with siRNAs against apoptosis-specific eIF-5A. Accordingly, one embodiment of the present invention provides a method of reducing levels of Il-1α in lung tissue by delivering siRNAs against apoptosis-specific eIF-5A to inhibit or reduce expression of apoptosis-specific eIF-5A. The reduction in the expression of apoptosis-specific eIF-5A leads to a reduction of Il-1α.

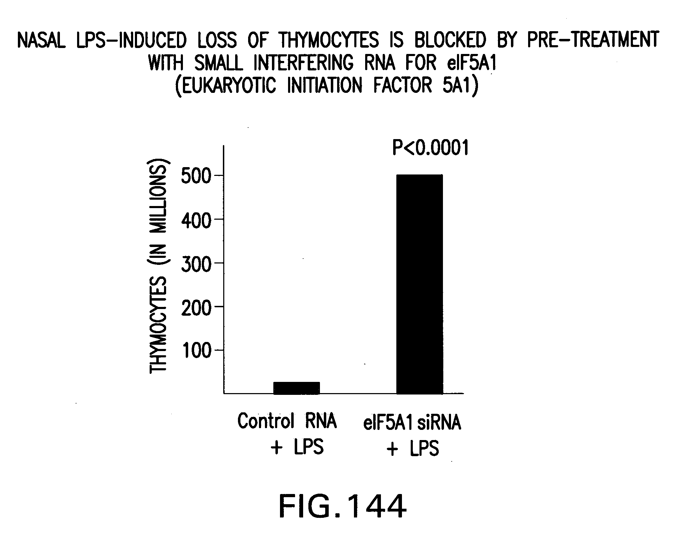

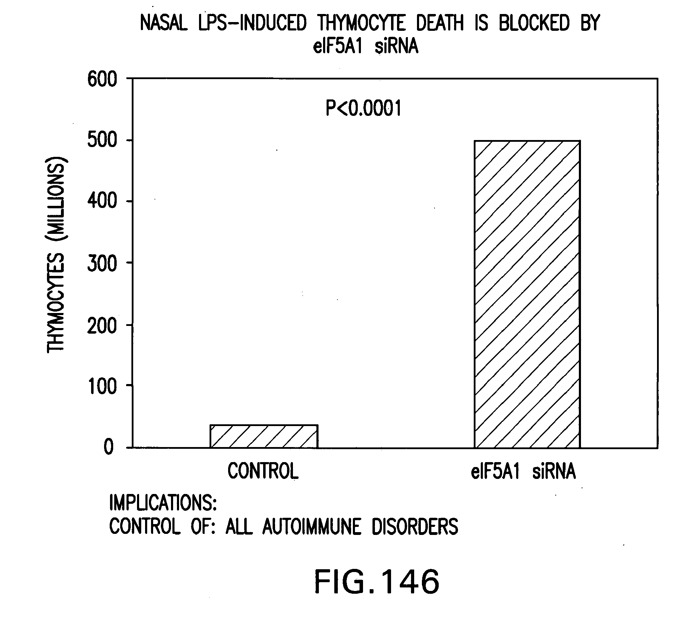

Figure 144 and 146 show that nasal-LPS-induced loss of thymocyes is blocked by pre-treatment with apoptosis-specific eIF-5A siRNA. Accordingly, one method of the present invention provides a method of protecting against LPS-induced thymocyte apoptosis, wherein siRNA against apoptosis-specific eIF-5A is delivered to a mammal intranasaly.

Thymocyte T cell development is a complex event involving distinct stages of proliferation and cell death. Bacterial infections result in the release of bacterial cell wall components such as LPS, lipoteichoic acid, and peptidoglycans. These cell wall components lead to the production of cytokines such as OL-1β, IL-6, IL-8 and TNF-α, each of which contributes to the increased risk of spesis progressing to sepsis syndrome, shock and death. In animal models of systemic inflammatory conditions, the administration of microbial products such as LPS, thymocyte apoptosis is observed.

Pulmonary infection caused by Gram-negative bacteria activates alveolar macrophages resulting in the production of cytokines such as IL-1 and TNF-α. In turn, these cytokines recruit polymorphonuclear neutrophils into the inflammatory site and in late stages of severe infection, septic shock may develop. Increasing evidence suggests that apoptosis occurs in many organs during sepsis, including the thymus. Thus, the effect of intranasal LPS administration on thymocyte apoptosis was studied. The results of the study show that mice treated with LPS intranasally have reduced thymus cellularity. Thymic cellularity was significantly lower 24 hours after intranasal LPS and returned to control levels after 48 hours. Similarly, peak apoptosis was observed 24 hours after LPS administration (32%) and recovered by 48 hours. These observations are similar to what observed after intraperitoneal injection of LPS, where peak apoptosis was reached 24 hours after LPS administration (28%) as well as what we have previously observed after intravenous conA injection (46%).

Fas and FasL are expressed in the thymus and LPS-induced thymocyte apoptosis is mediated by glucocorticoids, which is in turn, increase the expression of Fas/FasL. It is possible that siRNA eIF5A reduced LPS-induced apoptosis by down regulating thymocyte Fas/FasL. In addition, LPS activates NF-kB, which leads to the synthesis and release of a number of proinflammatory mediators, including IL-1, IL-6, IL-8, and TNF-a (37). Because TNF-α and IFN-γ are both critical mediators in thymus atrophy and thymoctyte apoptosis induced systemic inflammation, the mechanism by which siRNA inhbits LPS-induced thymocyte apoptosis could be due to lower levels of TNF-α and other proinflammatory cytokines since siRNA eIF-5A strongly inhibits TNF-α production by IFN-γ primited HT-29 cells in response to LPS. Therefore, the mechanism by which siRNA eIF-5A suppresses LPS-induced thymocyte apoptosis could be the result of decreased synthesis of TNF-α and IFN-γ, indicating that eIF-5A may be an important target for the development of anti-inflammatory therapeutics.

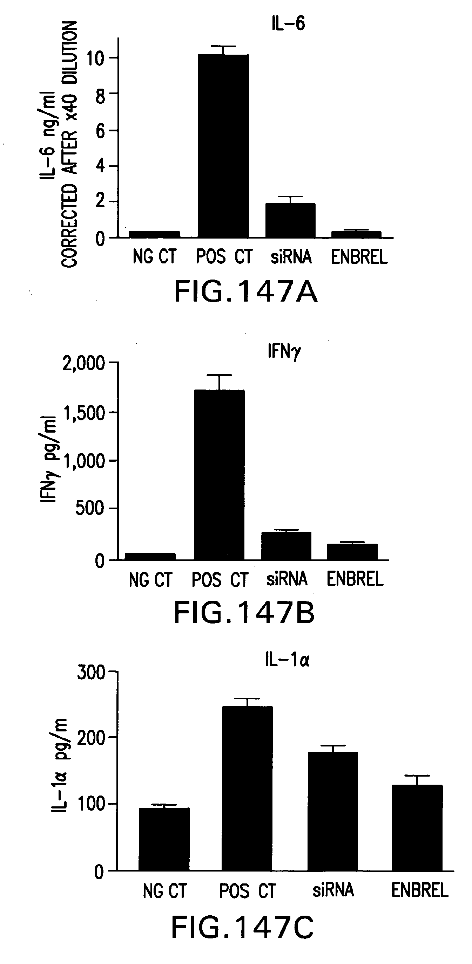

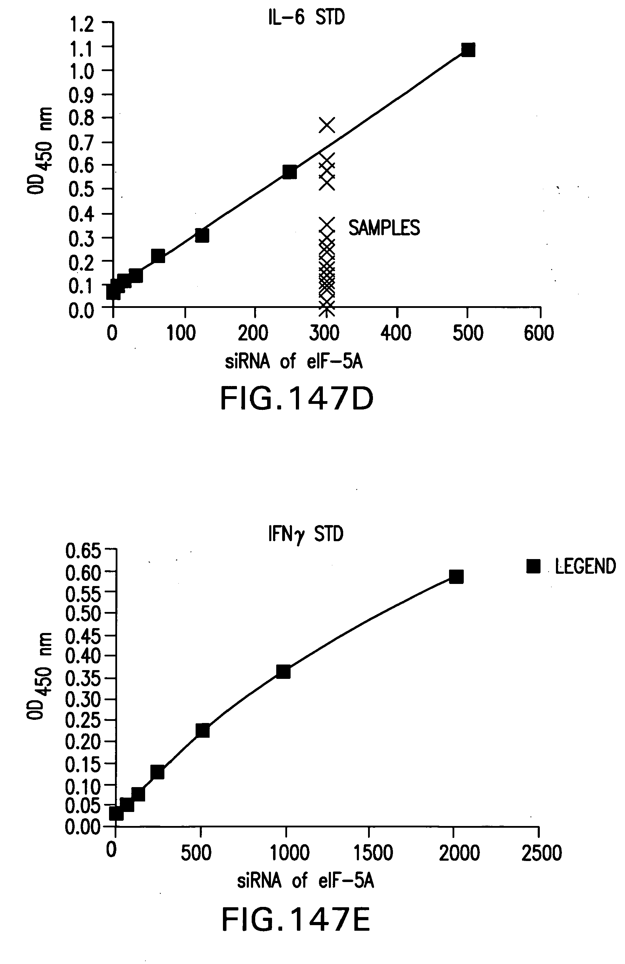

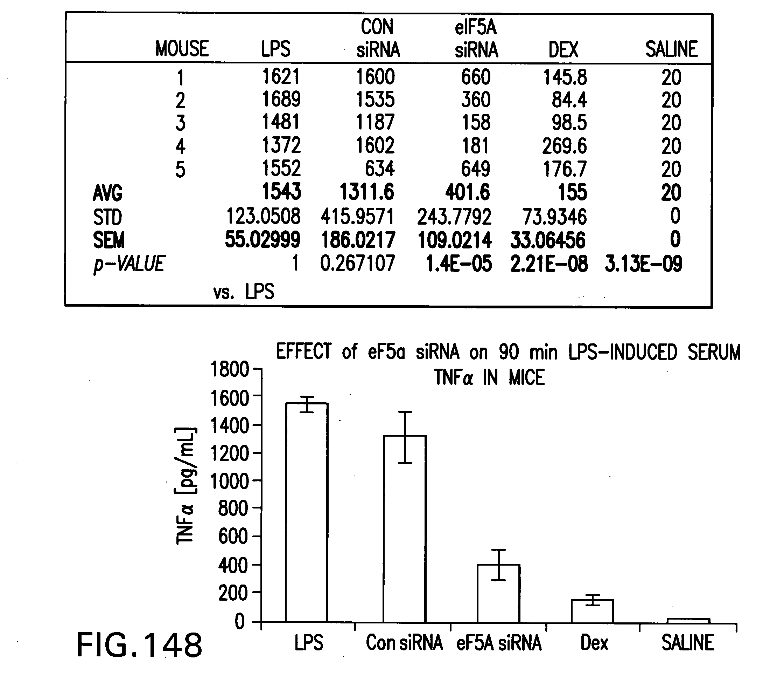

Figure 147 shows that siRNA against eIF-5A delivered intranasaly decreased production of IL-6, IFN-γ and Il-1α in mice. Figure 148 shows that siRNA against eIF-5A is able to reduce the expression of TNFα as a result of treatment with LPS. The top panel shows the raw data and the bottom panel shows the data in a bar graph.

Thus, the present inventors shown the correlation between apoptosis-specific eIF-5A and the immune response, as well as shown that siRNAs against apoptosis-specific eIF-5A suppress the production of myeloperoxidase (i.e. part of the inflammation response). The inventors have also shown that it is possible to deliver siRNAs *in vivo* to lung tissue by simple intranasal delivery. The siRNAs were mixed only in water. This presents a major breakthrough and discovery as others skilled in the art have attempted to design acceptable delivery methods for siRNA.

In another experiment, mice were similarly treated with siRNAs directed against apoptosis-specific eIF-5A. Lipopolysaccharide (LPS) was administered to the mice to induce inflammation and an immune system response. Under control conditions, LPS kills thymocytes, which are important immune system precursor cells created in the thymus to fend off infection. However, using the siRNAs directed against apoptosis-specific eIF-5A allowed approximately 90% survivability of the thymocytes in the presence of LPS. When thymocytes are destroyed, since they are precursors to T cells, the body's natural immunity is compromised by not being able to produce T cells and thus can't ward off bacterial infections and such. Thus, siRNAs against apoptosis-specific eIF-5A can be used to reduce inflammation (as shown by a lower level of MPO in the first example) without destroying the body's natural immune defense system.

Another embodiment of the present invention provides a method to treat sepsis by administering siRNA against apoptosis-specific eIF-5A. Sepsis is also known as systemic inflammatory response syndrome ("SIRS"). Sepsis is caused by bacterial infection that can originate anywhere in the body. Sepsis can be simply defined as a spectrum of clinical conditions caused by the immune response of a patient to infection that is characterized by systemic inflammation and coagulation. It includes the full range of response from systemic inflammatory response (SIRS) to organ dysfunction to multiple organ failure and ultimately death.

Sepsis is a very complex sequence of events and much work still needs to be done to completely understand how a patient goes from SIRS to septic shock. Patients with septic shock have a biphasic immunological response. Initially they manifest an overwhelming inflammatory response to the infection. This is most likely due to the pro-inflammatory cytokines Tumor Necrosis Factor (TNF), IL-1, IL-12, Interferon gamma (IFNgamma), and IL-6. The body then regulates this response by producing anti-inflammatory cytokines (IL-10), soluble inhibitors [TNF receptors, IL-1 receptor type II, and IL-1RA (an inactive form of IL-1)], which is manifested in the patient by a period of immunodepression. Persistence of this hyporesponsiveness is associated with increased risk of nosocomial infection and death.

This systemic inflammatory cascade is initiated by various bacterial products. These bacterial products (gram-negative bacteria = endotoxin, formyl peptides, exotoxins, and proteases; gram-positive bacteria = exotoxins, superantigens (toxic shock syndrome toxin (TSST), streptococcal pyrogenic exotoxin A (SpeA)), enterotoxins, hemolysins, peptidoglycans, and lipotechoic acid, and fungal cell wall material) bind to cell receptors on the host's macrophages and activate regulatory proteins such as Nuclear Factor Kappa B (NFkB). Endotoxin activates the regulatory proteins by interacting with several receptors. The CD receptors pool the LPS-LPS binding protein complex on the surface of the cell and then the TLR receptors translate the signal into the cells.

The pro-inflammatory cytokines produced are tumor necrosis factor (TNF), Interleukins 1, 6 and 12 and Interferon gamma (IFNgamma). These cytokines can act directly to affect organ function or they may act indirectly through secondary mediators. The secondary mediators include nitric oxide, thromboxanes, leukotrienes, platelet-activating factor, prostaglandins, and complement. TNF and IL-1 (as well as endotoxin) can also cause the release of tissue-factor by endothelial cells leading to fibrin deposition and disseminated intravascular coagulation (DIC).

Then these primary and secondary mediators cause the activation of the coagulation cascade, the complement cascade and the production of prostaglandins and leukotrienes. Clots lodge in the blood vessels which lowers profusion of the organs and can lead to multiple organ system failure. In time this activation of the coagulation cascade depletes the patient's ability to make clot resulting in DIC and ARDS.

The cumulative effect of this cascade is an unbalanced state, with inflammation dominant over antiinflammation and coagulation dominant over fibrinolysis. Microvascular thrombosis, hypoperfusion, ischemia, and tissue injury result. Severe sepsis, shock, and multiple organ dysfunction may occur, leading to death.

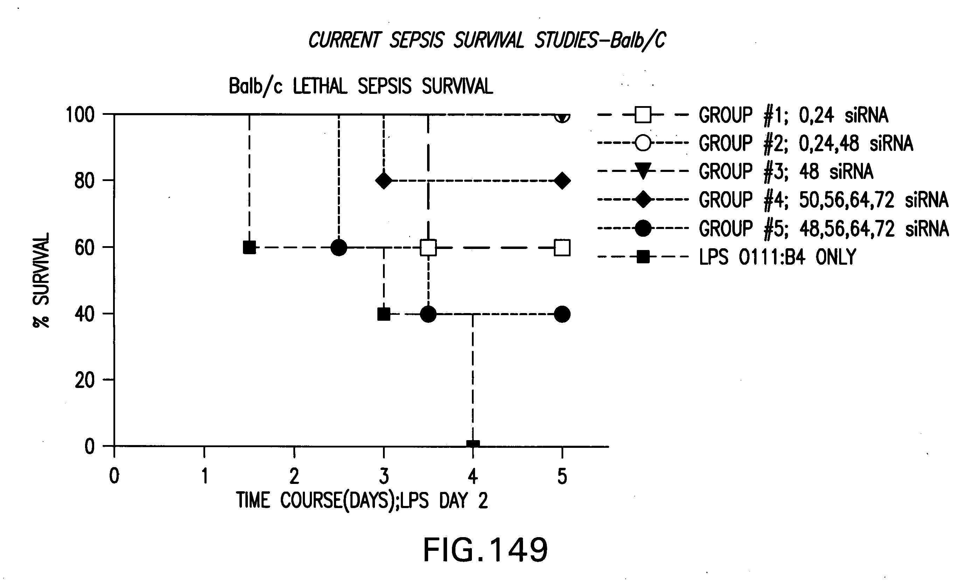

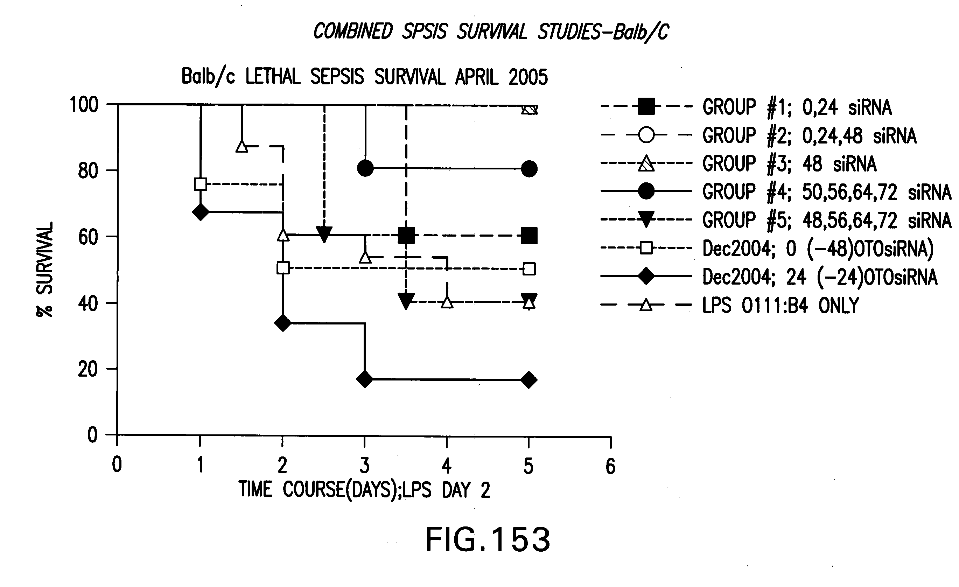

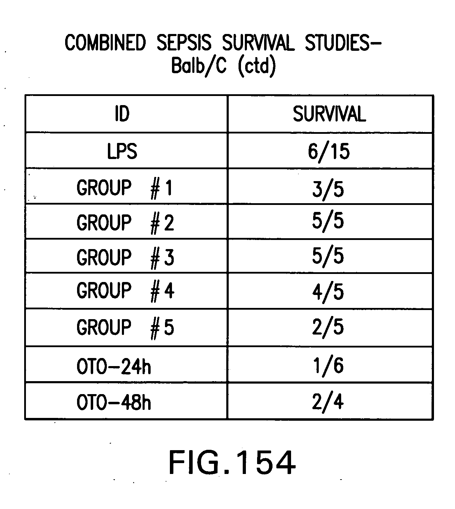

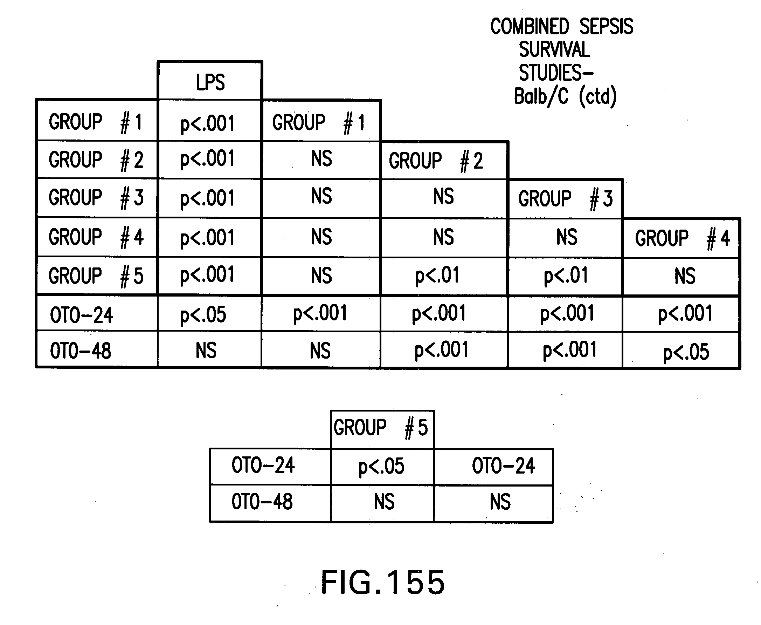

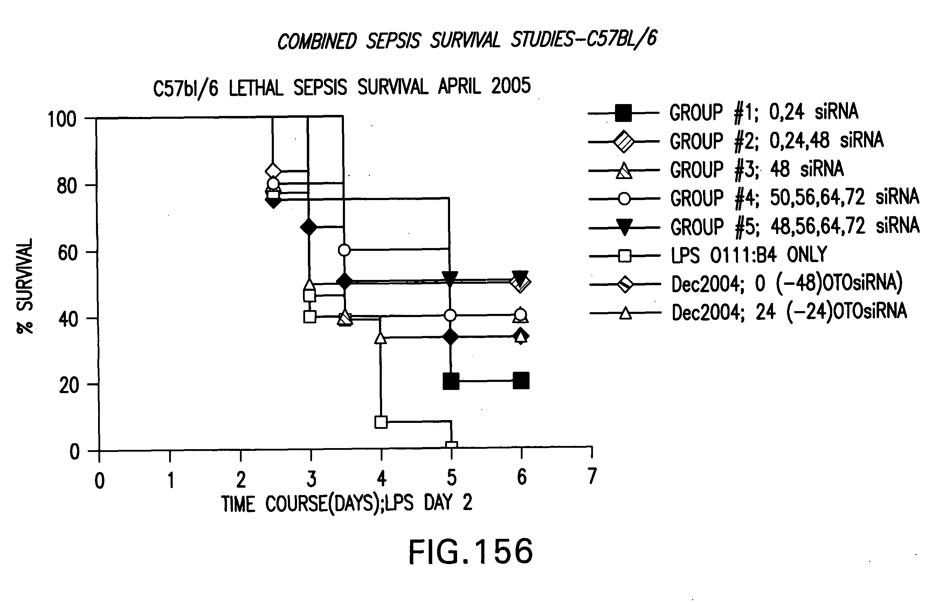

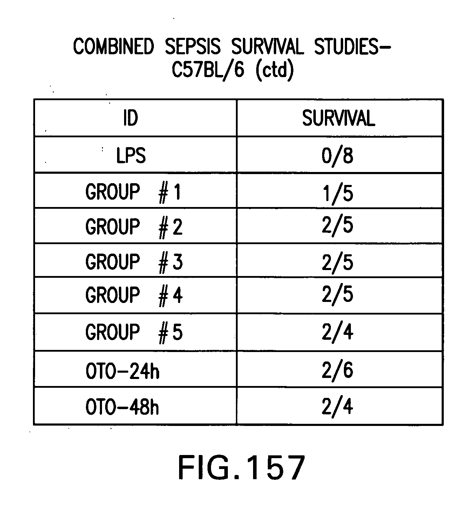

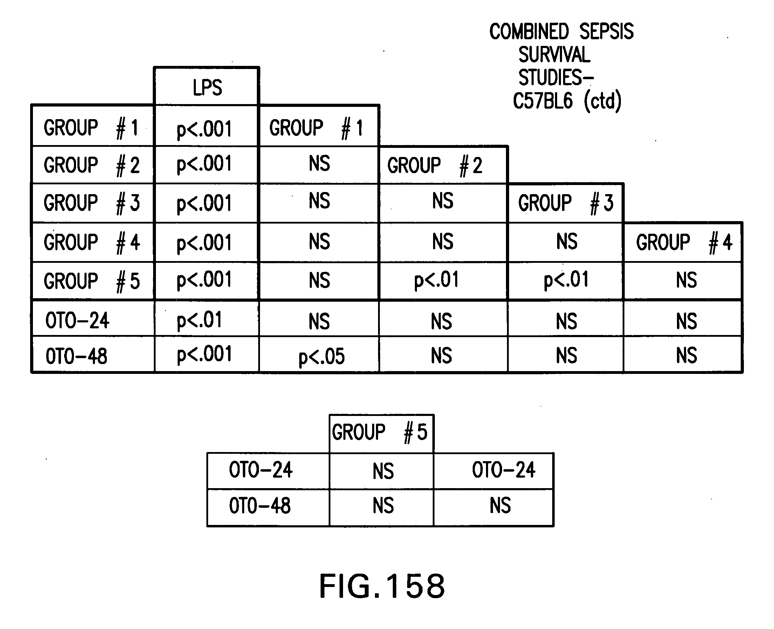

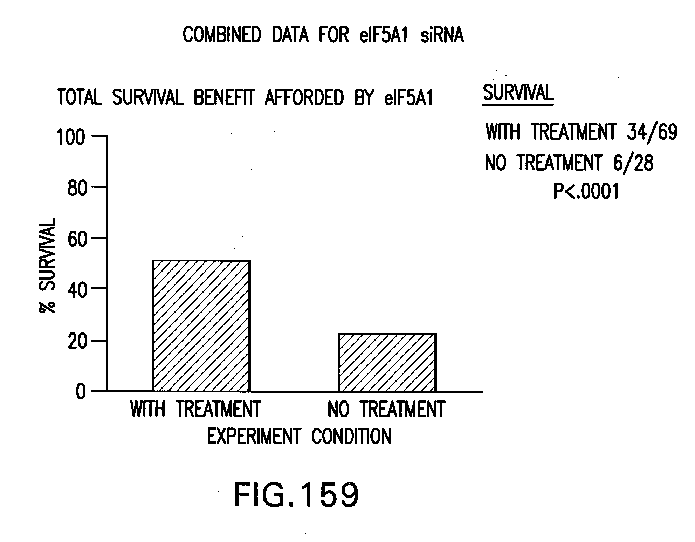

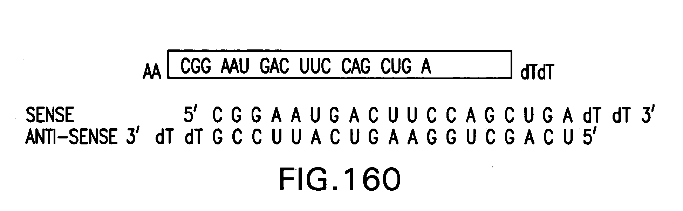

The inventors have previously shown (and presented above) that siRNA against eIF-5A was able to reduce the expression of various inflammation cytokines, such as TNF-α. In a study that involved administering siRNA against apoptosis-specific eIF-5A to treat sepsis in mice, the present inventors have further shown that the siRNA can be used to treat sepsis *in vivo*. See Example 21 and figures 149-160. In this study, the mice were given a dose of LPS that induces sepsis and death in the animal within 48 hours after the LPS is administered. siRNA (3'- GCC UUA CUG AAG GUC GAC U -5') was administered intraperitoneally to mice at different time periods before and after LPS administration. In some test groups, all five mice who received siRNA survived. It is believed that the use of siRNA was able to shut down the inflammation cascade and thus prevent sepsis in the mice.

Accordingly, one embodiment of the present invention provides a siRNA oligonucleotide of apoptosis-specific eIF-5A wherein said siRNA oligonucleotide suppresses endogenous expression of apoptosis-specific eIF-5A in a cell and having the sequence of 3'- GCC UUA CUG AAG GUC GAC U -5'. By suppressing expression of apoptosis-specific eIF-5A, the production of inflammatory cytokines is inhibited or reduced such that the inflammation cascade does not begin and result in septic shock.

The apoptosis-specific eIF-5A is believed to shuttle subsets of mRNA out of the nucleus that are involved in apoptosis and inflammation. If the amount of eIF-5A is reduced or completely eliminated, there is no shuttle available to shuttle mRNAs of various inflammatory and cell death cytokines out of the nucleus. This results in a decreased amount of inflammatory cytokines produced by the cell and thus, inhibits the beginning of the inflammation cascade. Since sepsis and septic shock are a result of the inflammation cascade, shutting down the cascade provides a method of treating or preventing sepsis/septic shock. Accordingly, another embodiment of the present invention provides a method for treating sepsis in a mammal, comprising administering the siRNAs described previously to a mammal.

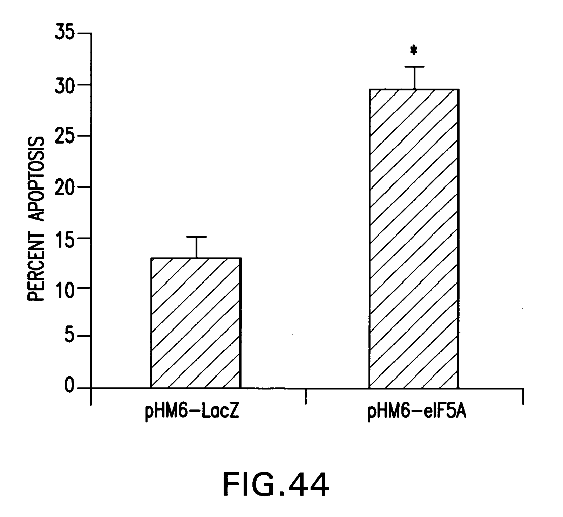

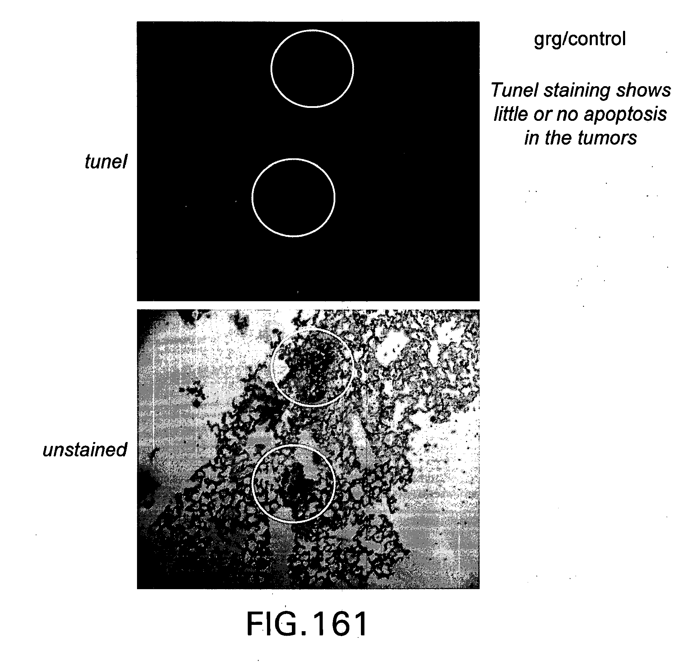

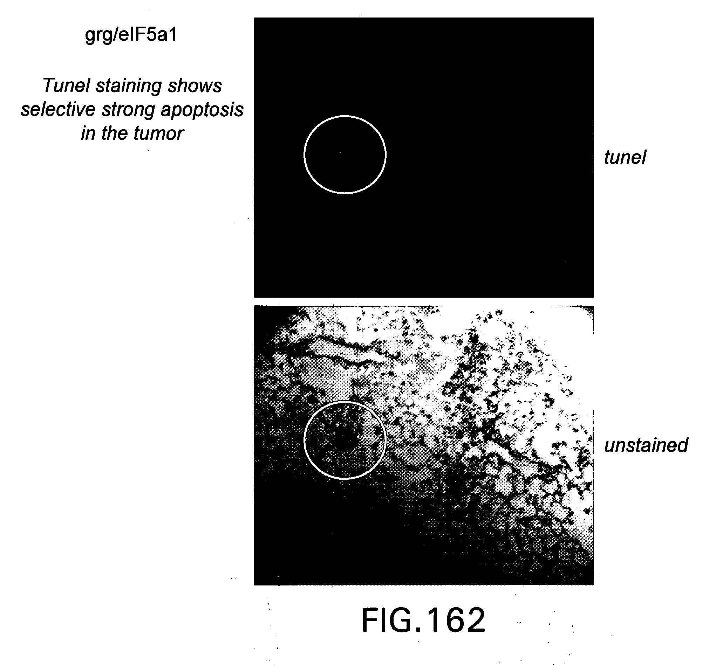

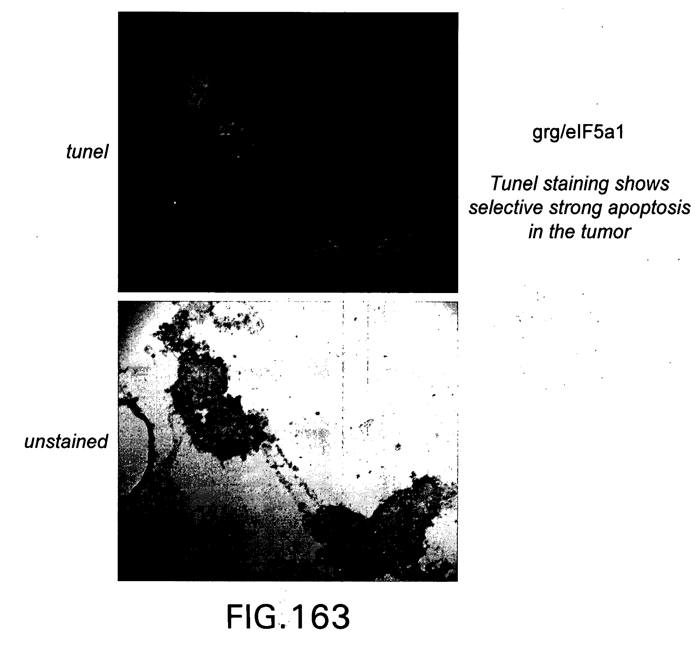

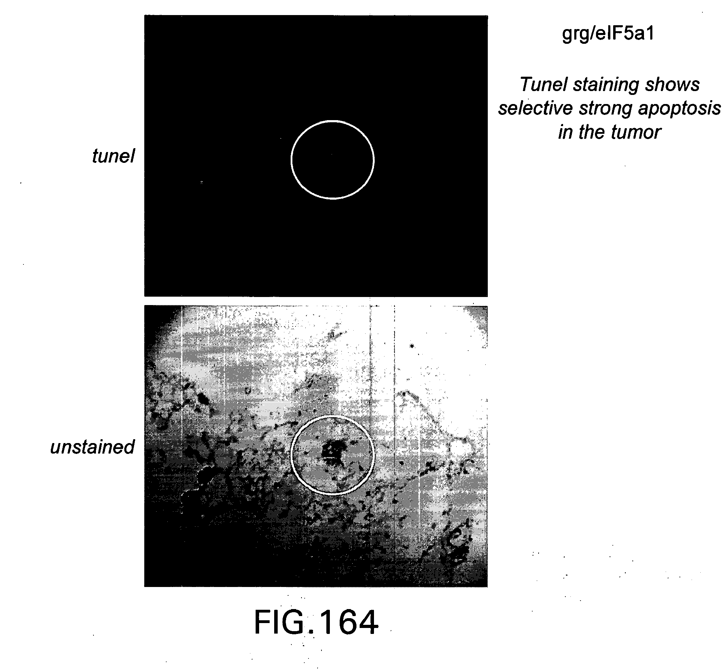

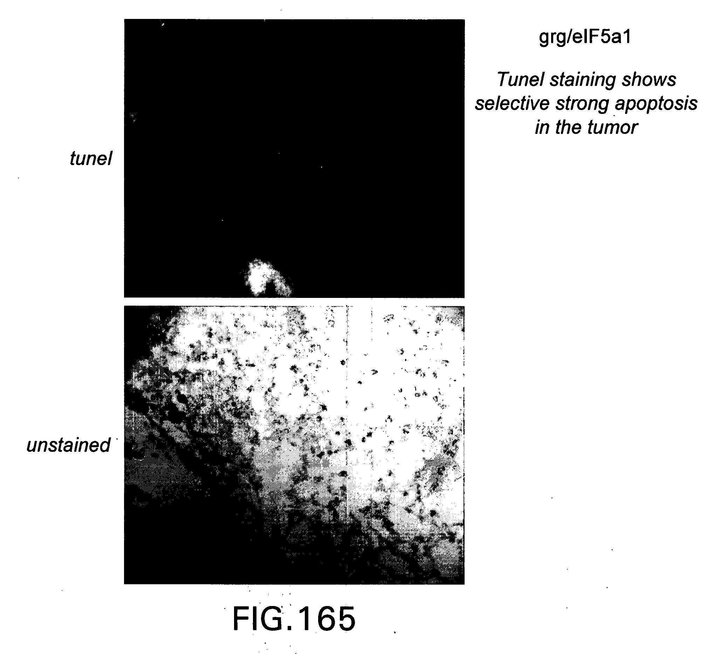

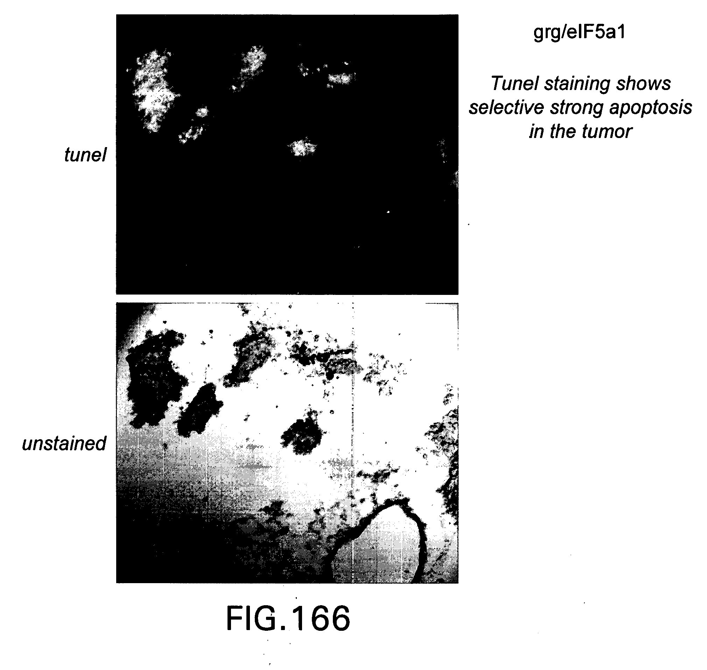

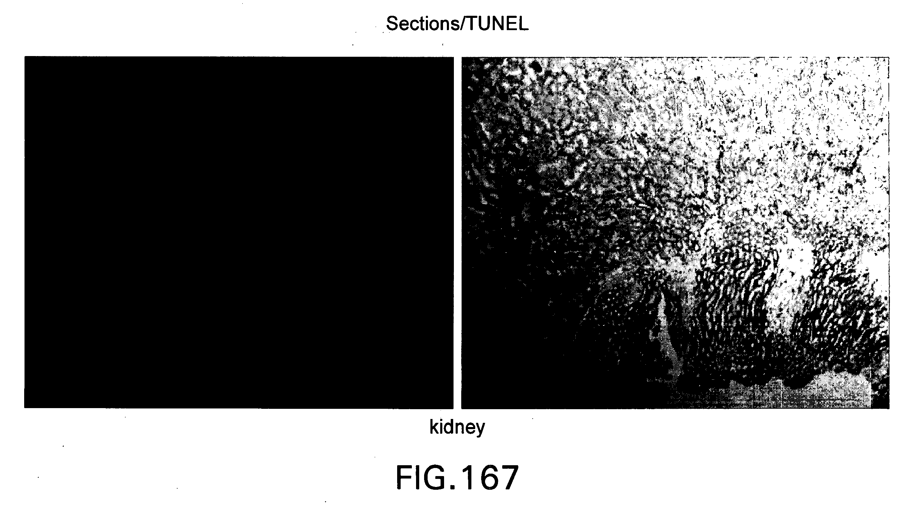

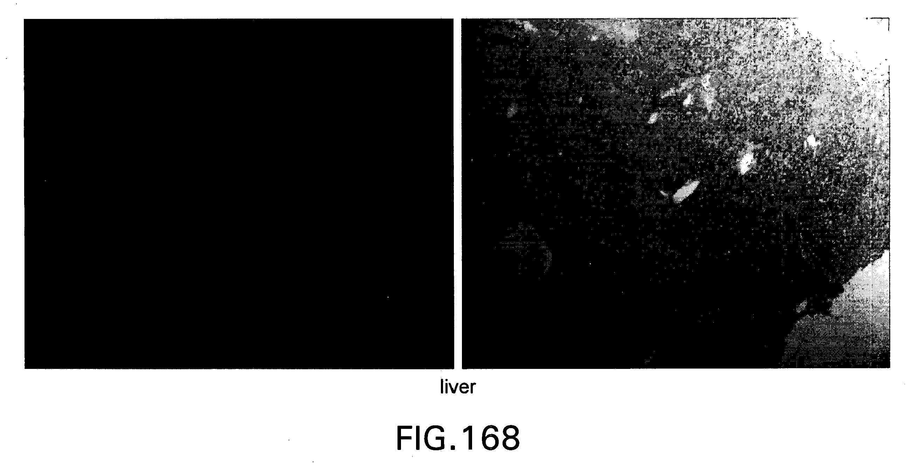

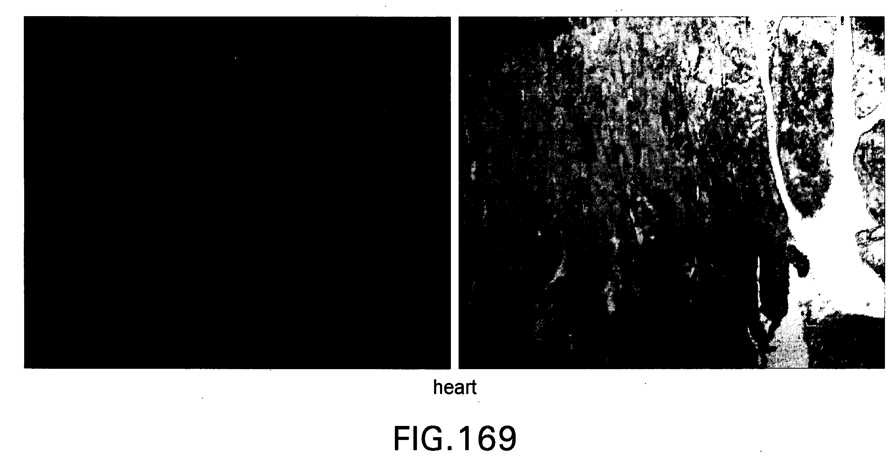

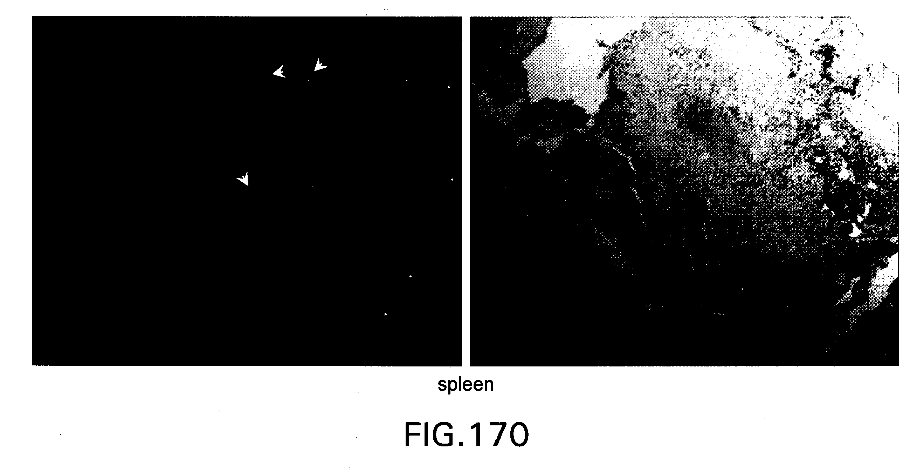

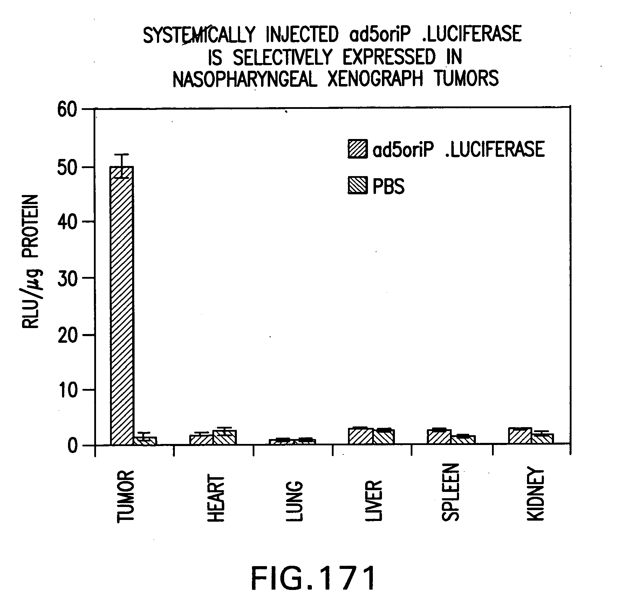

The present invention also provides a method of treating cancer and/or decreasing angiogenesis (or a medicament to of treating cancer and/or decreasing angiogenesis) by administering a polynucleotide encoding an apoptosis-specific eIF-5A to increase apoptosis in the cancer/tumor. The present inventors have shown that over expression of apoptosis-specific eIF-5A induces apoptosis. See figures 44-58. The present inventors have shown that apoptosis-specific eIF-5A polynucleotides increase apoptosis in several tumor/cancer models and further, does not appear to induce apoptosis in surrounding non-cancerous tissues. See figures 161-171.

In a nasopharyngeal cancer cell model, the present inventors have demonstrated that ad5orioP.eIF-5A1 selectively kills 98% of nasopharyngeal cancer cells (C666-1) within two cell divisions.

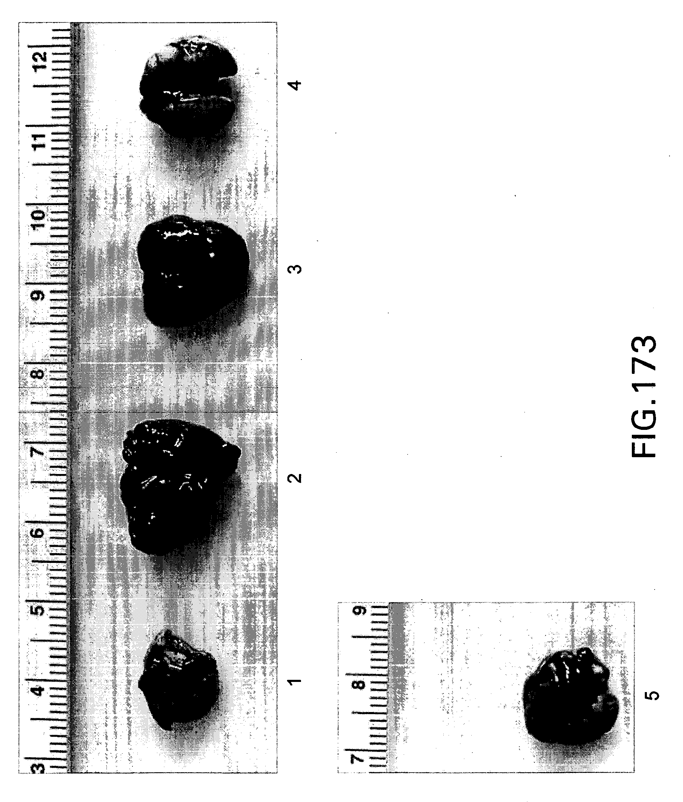

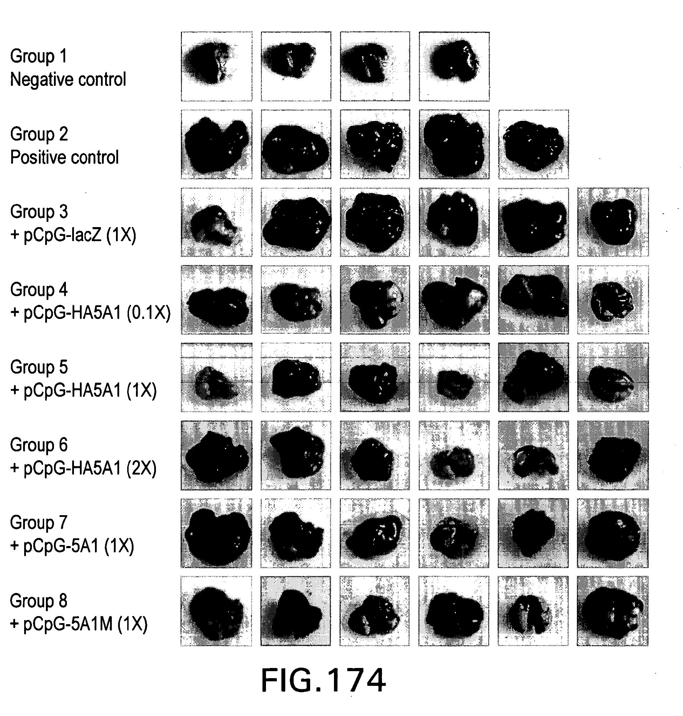

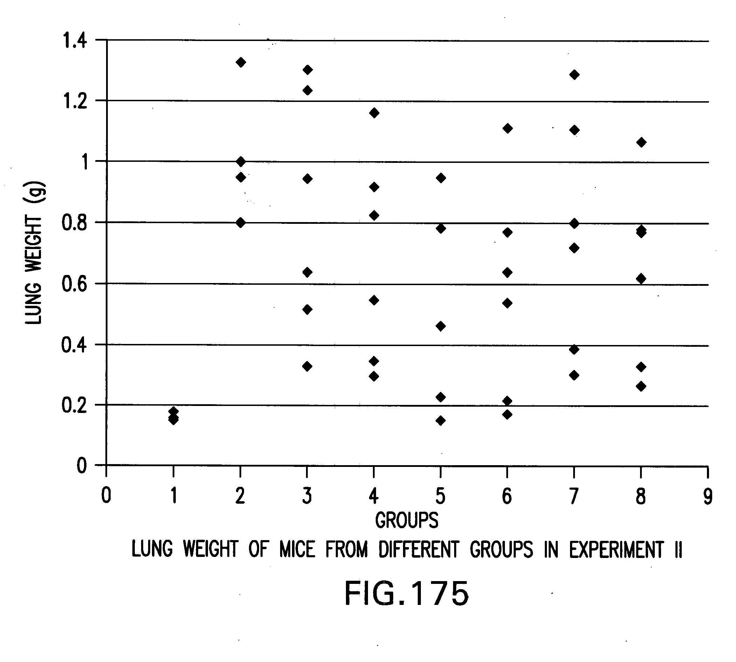

In a lung cancer model, mice were treated with a type of melanoma having an affinity for lung tissue. After three weeks of treatment, the lungs of the treated and untreated mice were compared by weight to assess tumor load. "Treated mice" were injected with a plasmid containing eIF-5A nucleotides (see Example 25). The mice that received the EIF-5A showed an average of 41 % reduction in tumor weights relative to the untreated mice. Additionally, nearly half of the treated mice had lung weights that were statistically comparable to control (healthy) mice that did not have any tumors. See figures 173-175.

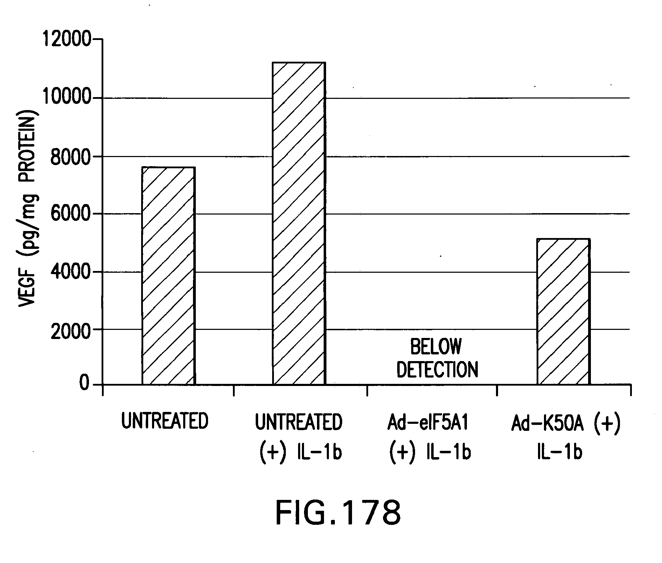

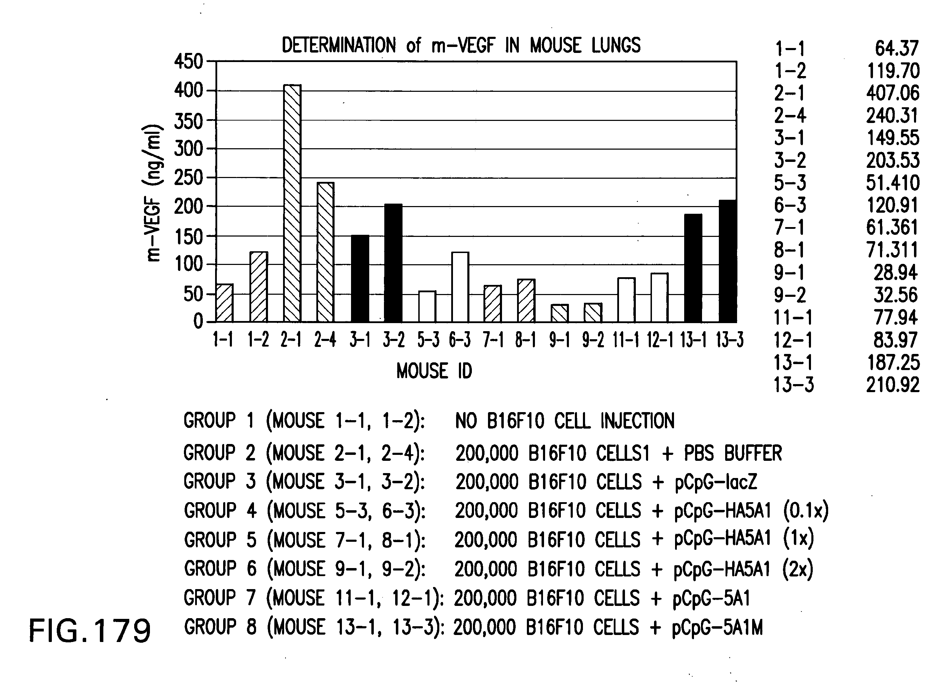

The inventors have also shown that when cancer cells are induced to over-express apoptosis-specific eIF-5A through the use of exogenous apoptosis-specific eIF-5A polynucleotides, the cancer cells show a decrease in VEGF expression. See Example 24 and Figures 178-179. VEGF is a cytokine that mediates endothelial cells growth and angiogenesis. VEGF is believed to be important in pathological angiogenesis as a great number of cells lines secrete VEGF. VEGF has been found to be upregulated in the majority of human tumors.

The present invention also provides a method to induce apoptosis in cancer cells. Since cancer cells have seemingly circumvented the normal cell death pathways, it is desirable to have a mechanism to induce apoptosis in cancer cells. The present inventors have used siRNA against eIF5A1 to induce apoptosis in cancer cells. In addition, the present inventors have discovered that the unhypusinated form of eIF5A1 is able to induce apoptosis, where it had been previously thought that eIF5A1 must be activated by hypusination by DHS. See Example 22.

The present inventors have examined the role of eIF5A1 during apoptosis and observed that over-expression of eIF5A1 in colon carcinoma cell lines induced apoptosis in a p53-independent manner. A dynamic translocation of eIF5A1 protein from the cytoplasm to the nucleus was also observed following induction of apoptosis mediated by tumour necrosis factor α (TNF-α) death receptor activation or by genotoxic stress induced by Actinomycin D, suggesting that eIF5A1 may have important nuclear functions related to apoptosis.

Previous work in by the inventors of the present invention has demonstrated that treatment of human lamina cribrosa cells with TNF-α upregulates eIF5A1 and induces apoptosis. In addition, an siRNA against eIF5A1 protected the lamina cribrosa cells from apoptosis induced by this cytokine¹². These observations indicate that eIF5A1 plays a role in TNF-α-induced apoptosis. To examine the possibility that eIF5A1 may also be involved in DNA damage-induced apoptosis, normal colon fibroblast cells were treated with Actinomycin D, an anti-neoplastic agent that inhibits topoisomerase II, and eIF5A1 expression was examined by Northern and Western blotting (Figure 30). Northern blot analysis indicated that the transcript for eIF5A1 is constitutively expressed in these cells and that treatment with Actinomycin D had no effect on eIF5A1 transcript abundance (Figure 30A). eIF5A1 protein was present at moderate levels in the fibroblasts prior to treatment, and a modest increase in eIF5A1 protein expression was observed within 1 hour of Actinomycin D treatment (Figure 30B). The protein continued to accumulate for at least 24 hours after treatment (Figure 30B). The accumulation of eIF5A1 protein in the absence of increased transcript levels suggests that eIF5A1 may be post-transcriptionally regulated. The expression of p53 in response to Actinomycin D was also examined in these cells and found to increase in parallel with eIF5A1 and continue increasing for at least 24 hours after treatment (Figure 30B). Thus, eIF5A1 may be involved in cell death induced by DNA damage.

The requirement for eIF5A1 in Actinomycin D-induced cytotoxicity was examined using the human colon adenocarcinoma cell line, HT-29. Treatment with Actinomycin D reduced the viability of HT-29 cells by 60 % after 48 hours (Figure 31A). Suppression of eIF5A1 by transfection with siRNA reduced the cytotoxic effects of Actinomycin D. A 40 % increase in cell viability was observed after Actinomycin D treatment for eIF5A1-suppressed cells (Figure 31A) relative to cells transfected with the control siRNA. Therefore, the cytotoxic effects of Actinomycin D appear to be partly dependent on the presence of eIF5Al indicating a role for eIF5Al in genotoxic stress pathways.

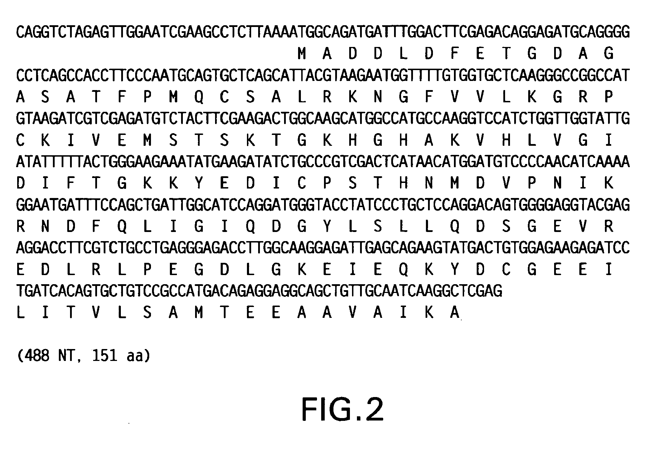

Numerous reports suggest that eIF5A1 may also be involved in cell proliferation. For example, blocking the hypusination of eIF5A1 with inhibitors of DHS such as GC7 induces cell cycle arrest and apoptosis in various tumour cell lines^{3,6,8,19}. In an effort to clarify the proposed involvement of eIF5A1 in cell proliferation, the effects of siRNA-mediated suppression of eIF5A1 on cell growth was examined. HT-29 cells were used for these studies, and cell proliferation was measured by BrdU incorporation. Depletion of eIF5A1 protein by transfection of HT-29 cells with eIF5A1 siRNA, which reduced eIF5A1 expression by >90% (data not shown), had no effect on cell growth and viability (Figure 31A and 2B). No negative effect was observed on cell growth in any of the cell lines transfected with this siRNA. The effect of eIF5A1 siRNA on cell proliferation was compared to that of GC7 using HT-29 cells that were grown in the presence or absence of serum (Figure 31B). Treatment of the cells with GC7 inhibited the proliferation of HT-29 cells in agreement with previous reports of the ability of this DHS inhibitor to arrest tumour cell growth, and this effect was dramatically increased by serum starvation (Figure 31B). In contrast, depletion of eIF5A1 protein from the cells by transfection with siRNA did not produce any noticeable effect on cell proliferation when compared to control siRNA (Figure 31B). Thus, reduction of eIF5A1 protein levels had no effect on the ability of HT-29 cells to proliferate as measured by metabolic activity or new DNA synthesis, indicating that eIF5A1 is not required for cell viability and growth. These results suggest that the reported cytostatic activities of GC7 and other DHS inhibitors are not related to reduced levels of hypusinated eIF5A1 and that eIF5A1 is not required for cell growth.

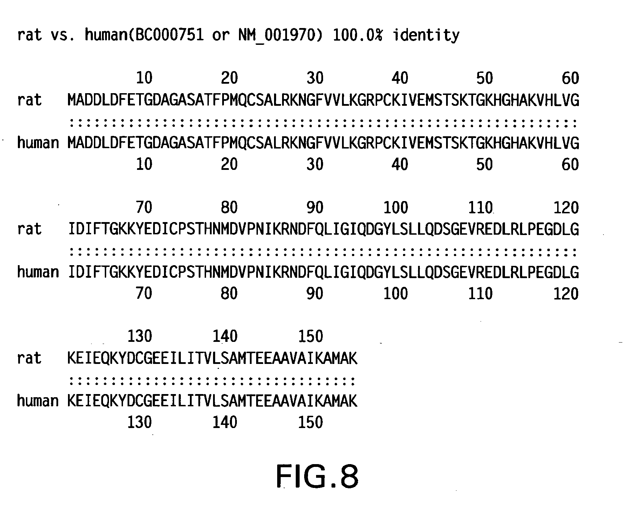

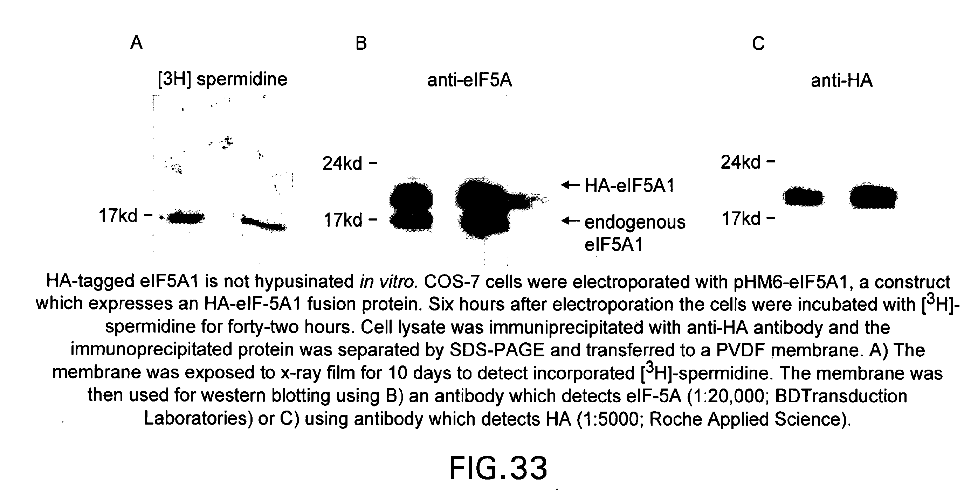

Recent work in the present inventors' lab has demonstrated that the HA-tagged eIF5A1 is not hypusinated *in vitro.* In order to determine whether the HA-tagged eIF5A1 was capable of being hypusinated, the pHM6-eIF5A1 construct which expresses the HA-eIF5A1 fusion protein was electroporated into COS-7 cells. The electroporated cells were than incubated with [³H]-spermidine for two days since spermidine is the substrate used by DHS to modify the conserved lysine in eIF5A1 to hypusine. The HA-tagged eIF5A1 was immunoprecipitated from the cell lysate using an anti-HA antibody and the immunoprecipitated protein was separated by SDS-PAGE. The separated proteins were transferred to a membrane and exposed to x-ray film in order to detect the incorporation of [³H] into eIF5A1 protein. A labeled band was detected at 17 kDa which corresponds to the predicted size of eIF5A1 (Figure 33A). The location of the eIF5A1 protein on the membrane was determined by Western blotting with an anti-eIF5A1 antibody (Figure 8B) and an anti-HA antibody (Figure 33C). Interestingly, two bands were observed in the anti-eIF5A1 western (Figure 33B). The top band (~ 20 kDa) in the anti-eIF5A1 western corresponded to the size of the single band observed in the anti-HA western (Figure 33C) indicating that the top band of the doublet is the HA-tagged form of eIF5A1 while the bottom band of the doublet (~17 kDa) must be the endogenous eIF5A1. It is interesting that the endogenous form of eIF5A1 was immunoprecipitated by the anti-HA antibody since it indicates that the endogenous eIF5A1 was bound to the HA-tagged eIF5A1 and was co-precipitated, suggesting that eIF5A1 may normally exist as a multimer within the cell. The 17 kDa [³H]-labeled band in Figure 30A must therefore be the hypusinated form of the endogenous eIF5A1 protein. Since no corresponding band has been observed at 20 kDa, it appears that the HA-tagged eIF5A1 that was introduced into the cell is either unhypusinated or hypusinated to a much lesser degree than the endogenous eIF5A1.

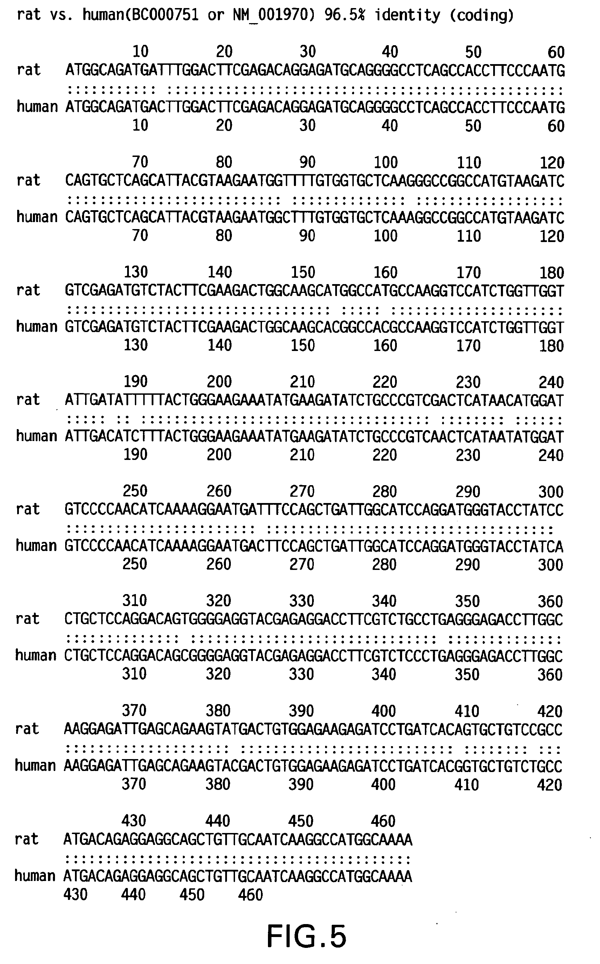

The fact that the HA-tagged eIF5A1 is not hypusinated in vitro is of interest because over-expression of this construct is capable of inducing apoptosis in cancer cell lines. Previous work in our lab has demonstrated that over-expression of human eIF5A1 in cancer cell lines is associated with increased apoptosis, suggesting that eIF5A1 is a pro-apoptotic protein that is capable of inducing apoptosis in cancer cells. Transfection of human colon carcinoma cell lines, RKO and RKO-E6, with pHM6-eIF5A1, a construct expressing an HA-tagged form of eIF5A1 resulted in a greater than 200 % increase in the incidence of apoptosis (Figure 5A and 5B). These results strongly support the view that unhypusinated eIF5A1 is capable of inducing apoptosis in cancer cell lines.

The observations that expression of eIF5A1 appears to be up-regulated during DNA damage-induced apoptosis in parallel to p53 (Figure 30B), and that suppression of eIF5A1 expression partially protects HT-29 cells from the cytotoxic effects of Actinomycin D (Figure 31 A), raised the possibility that there could be a relationship between eIF5Al and p53. In order to determine whether eIF5Al might be required for proper expression of p53 during DNA damage-induced apoptosis, Actinomycin D-induced up-regulation of p53 in transfected RKO cells was examined. RKO cells are a human colorectal carcinoma cell line and were used for this experiment because they are known to have functional p53 tumor suppressor protein 18. It was found that p53 protein levels are normally below detection in these cells, but accumulate quickly upon treatment with Actinomycin D (Figure 43A). Transfection with eIF5A1 siRNA significantly reduced eIF5A1 protein levels (Figure 43A) and also suppressed p53 accumulation in response to Actinomycin D treatment (Figures 43A and 43B). Suppression of eIF5A1 by siRNA transfection decreased levels of p53 protein by 58 % relative to control siRNA 8 hours after Actinomycin D treatment and by 68 % at 24 hours (Figure 43B), indicating that eIF5A1 is required for proper p53 expression in response to DNA damage.

In order to further validate a role for eIF5A1 in apoptosis, the apoptotic response of RKO cells to over-expression of eIF5A1 was examined. Human eIF5A1 was cloned from RKO cells using RT-PCR and subcloned into the expression vector, pHM6, under the control of the strong CMV promoter. The resulting PCR product was found to have the same amino acid sequence as previously reported for human eIF5A1. RKO cells were transiently transfected with either pHM6-LacZ or pHM6-eIF5A1, and 48 hours after transfection the cells were fixed, TUNEL-stained and analyzed by flow cytometry (Figure 44). Transfection efficiencies of 30 % to 40 % were routinely obtained. TUNEL staining indicated that 29.3 % of cells transfected with the plasmid containing eIF5A1 were undergoing apoptosis, whereas only 12.8 % of cells transfected with pHM6-LacZ were apoptotic (Figure 44). Thus, overexpression of eIF5A1 induces apoptosis in RKO cells.

The C-terminal end of eIF5A1 has been proposed to be involved in RNA binding based on its similarity to an oligonucleotide binding fold²⁰. In order to determine whether the C-terminal domain may be important for apoptosis, a plasmid (pHM6-eIF5A1Δ37) containing a truncated eIF5A1 cDNA was constructed in which the last 37 amino acids of the C-terminus were deleted. Apoptosis in transiently transfected RKO cells was scored by TUNEL using fluorescence microscopy (Figure 45A). Cells transiently transfected with the truncated eIF5A1 construct were found to have apoptotic levels similar to those of cells transfected with the control vector. In contrast, cells transfected with pHM6-eIF5A1 exhibited a more than two-fold higher level of apoptosis than cells transfected with the control vector (Figure 45A).

In view of the observation that eIF5A1 appears to regulate the expression of p53 (Figure 43), the susceptibility of a cell line lacking functional p53 to apoptosis induced by eIF5A1 overexpression was examined. RKO-E6, a cell line derived from RKO which contains a stably integrated human papilloma virus E6 oncogene and lacks appreciable functional p53, was used for this purpose. Over-expression of eIF5A1 in RKO-E6 resulted in a greater than three-fold increase in the number of apoptotic cells compared to control cells transfected with pHM6-LacZ (Figure 45B), suggesting that overexpression of eIF5A1 can induce apoptosis independently of p53. Similar to the results obtained with the RKO cell line, the level of apoptosis in RKO-E6 cells transfected with truncated eIF5A1 was not significantly different from that for cells transfected with pHM6-LacZ, indicating that the last 37 amino acids of eIF5A1 are required for its apoptotic activity (Figure 45B). Given that the C-terminus of eIF5A1 is believed to be involved in RNA binding²⁰, these results support the notion that eIF5A1 functions as a nucleocytoplasmic shuttle protein during apoptosis. Although the level of apopotosis in RKO-E6 cells transfected with pHM6-eIF5A1 was on average >3-fold higher than that for corresponding cells transfected with pHM6-LacZ, this difference was not statistically significant as determined by a paired t-test (Figure 45B). This reflects variation among experiments in the degree of apoptosis induced in transfected RKO-E6. However, when this variation is normalized by setting the levels of apoptosis for control cells transfected with pHM6-LacZ at 1 within experiments, the increase in apoptosis for cells transfected with pHM6-eIF5A1 relative to these normalized control values was on average 3.25- fold with a significance probability of < 0.03 for the data illustrated in Figure 45B.

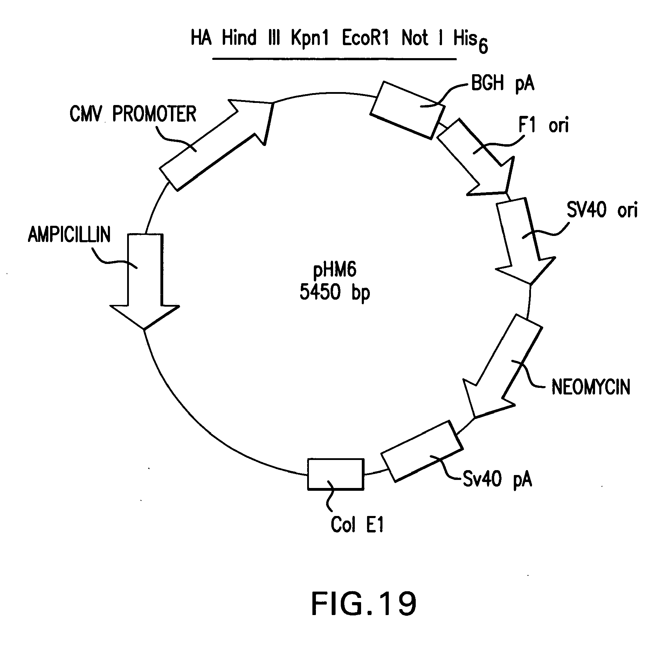

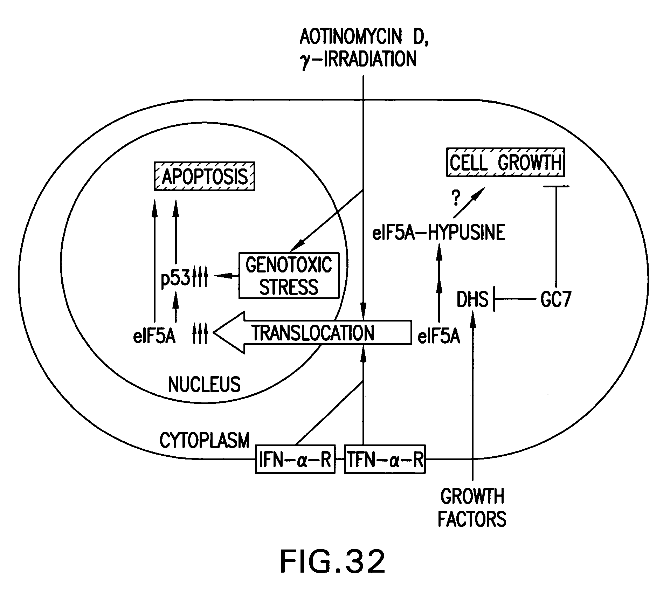

The role of eIF5A1 as a nucleocytoplasmic shuttle protein has often been questioned because it has repeatedly been found to be localized in the cytoplasm and perinuclear region²¹⁻²⁵, and this localization does not change with cell cycle²¹. If eIF5A1 is involved in the recruitment of mRNAs from the nucleus, one would expect it to be at least transiently localized in the nucleus. In light of the inventors previous findings that eIF5A1 is involved in apoptosis and not cell division (figures 31 and 45), a study was done to determine whether the subcellular localization of eIF5A1 is altered following treatment with agents known to induce apoptosis. Apoptosis can be induced in HT-29 cells by incubation with TNF-α after sensitization with IFN-γ26-30. TUNEL staining of IFN-γ-primed HT-29 cells demonstrated that approximately 30 % of the cells were undergoing apoptosis after 24 hours of stimulation with TNF-α (data not shown). Co-stimulation is necessary for apoptosis as neither IFN-γ nor TNF-α is capable of inducing apoptosis independently in this cell type²⁶⁻²⁹. Accordingly, HT-29 cells were primed with IFN-γ for 16 hours and then treated with TNF-α. The cells were fixed with formaldehyde at intervals ranging from 10 minutes to 8 hours after the initiation of TNF-α treatment, and eIF5A1 localization was observed by indirect immunofluorescence using a commercial anti-eIF5A1 antibody. In agreement with previous reports, eIF5A1 was predominantly localized in the cytoplasm of untreated cells [Figure 19A (i), 6B (i)], and this localization was not altered by IFN-γ treatment alone [Figure 19A (ii)]. However, there was a dynamic shift in the localization of eIF5A1 from predominantly cytoplasmic to primarily nuclear within 10 minutes of TNF-α treatment in IFN-γ- primed cells [Figure 19A (iii)]. IFN-γ sensitization of the cells was required for the translocation of eIF5A1 in response to TNF-α treatment as eIF5A1 did not localize to the nucleus in cells which were stimulated with TNF-α without IFN-γ-priming (data not shown). eIF5A1 retained its nuclear localization for at least 8 hours after IFN-γ/TNF-α treatment [Figure 19A (vi)]. In order to determine whether a shift in eIF5A1 localization might also occur in response to genotoxic stress, HT-29 cells were incubated with Actinomycin D for increasing periods of time (Figure 19B). Incubation with Actinomycin D for 24 hours induced apoptosis in approximately 10 % of HT-29 cells (data not shown). In this case, eIF5A1 retained its cytoplasmic distribution for at least 30 minutes after the initiation of Actinomycin D treatment [Figure 19B (ii)], but within 90 minutes [Figure 19B (iii)] was predominantly found in the nucleus and remained there for at least 16 hours [Figure 19B (vi)]. No fluorescent signal was observed when the fixed cells were incubated with only secondary antibody indicating that the observed fluorescence (Figure 19) is due to recognition of eIF5A1 by the primary antibody (data not shown). These observations support the notion that eIF5A1 may indeed function as a nucleocytoplasmic shuttle protein during apoptosis induced by death receptor activation as well as genotoxic stress, and that localization of eIF5A1 may play a role in the regulation of apoptosis (Figure 32).

eIF5A1 is unique in that it is the only known protein to contain the unusual amino acid, hypusine. The hypusine residue is formed posttranslationally in two enzymatic reactions catalyzed by deoxyhypusine synthase (DHS) and function of eIF5A1 has not been elucidated, it has been proposed to function as a nucleocytoplasmic shuttle protein^{5,10-11,20,31} Numerous studies with DHS inhibitors, such as GC7 used in the present study, have demonstrated the ability of these inhibitors to block cell proliferation, prompting the view that hypusinated eIF5A1 facilitates the translation of mRNAs involved in cell division^{3,6,8,19}. This proposal is further supported by experiments with yeast demonstrating that inactivation of both eIF5A1 isoforms or DHS blocks cell division^{2,4,7,9}. However, this view is not consistent with the data reported in the present study, as we observed that siRNA-mediated suppression of eIF5A1 had no effect on cell viability or proliferation of a colon adenocarcinoma cell line. Moreover, in an earlier study with a leukemic cell line it was found that inhibition of eIF5A1 expression with an antisense oligonucleotide actually enhanced the stimulating effect of GM-CSF on cell growth²⁴. An apparent lack of correlation between eIF5A1 expression and proliferation has also been observed in two lung adenocarcinoma cell lines³². Furthermore, in the present study we observed a reduction in cell proliferation as a result of treatment with GC7, but cells in which eIF5A1 protein levels had been depleted by > 90 % were able to proliferate normally. Although it is possible that the residual amount of eIF5A1 protein remaining after transfection with siRNA was sufficient to support growth, these data would appear to challenge the view that the inhibitory effects of DHS inhibitors on cell growth are related to a reduction in the levels of hypusine-modified eIF5A1. Indeed, these findings are consistent with a recent report³³ indicating that a novel DHS inhibitor had no effect on cell viability or growth and suggest the antiproliferative effects of DHS inhibitors used in previous studies are independent of their ability to inhibit hypusination of eIF5A1 and may be due to unrelated effects on cellular metabolism.

Several recent studies have indicated that eIF5A1 may be involved in apoptotic pathways. For example, siRNAs against eIF5A1 protected human lamina cribrosa cells from TNF-α-induced apoptosis¹². In another study, the present inventors demonstrated that over-expression of eIF5A1 resulted in increased apoptosis of a lung cancer cell line¹³. The results of the current study also support a role for eIF5A1 in apoptosis. Firstly, expression of eIF5A1 protein was correlated with p53 accumulation induced by Actinomycin D, and siRNA-mediated suppression of eIF5A1 reduced the cytotoxic effects of Actinomycin D on HT-29 cells. This is in agreement with previous reports of enhanced eIF5A1 expression during apoptosis induced by the cytokines IFN-α¹⁹ and TNF-α¹². Secondly, overexpression of eIF5A1 induced apoptosis in human colorectal carcinoma cells regardless of their p53 status. This is in contrast with a recent report that over-expression of eIF5A1 induced apoptosis in H460 (p53+/+) cells but not in p53-null H1299 cells¹³, and could reflect differences in the cell types used. We also observed that the C-terminal domain of eIF5A1, which has been proposed to contain an oligonucleotide-binding fold²⁰, is essential for its apoptotic activity. These results suggest that binding of RNA, perhaps mRNAs required for apoptosis, may be important in the pro-apoptotic function of eIF5A1. Indeed, in the present study we demonstrated a requirement for eIF5A1 in the proper expression of p53 in response to DNA damage by Actinomycin D in RKO cells. The dependence of p53 expression on eIF5A1 has also been reported for COS-7 cells¹³. Furthermore, eIF5A1 protein expression has been found to be positively correlated with nuclear accumulation of p53 in lung adenocarcinomas³². Another RNA binding protein, HuR, has been shown to bind the transcript of p53 and enhance p53 expression in response to ultraviolet light³⁴ in RKO cells. It seems likely, therefore, that expression of p53 in response to genotoxic stress requires one or more RNA binding proteins. The enhanced expression of p53 protein observed in response to genotoxic stresses such as gamma radiation is thought to be due to enhanced translation of p53 transcript resulting from relief of the inhibitory effect of its 3'UTR, perhaps as a result of interaction with one or more RNA binding proteins^{17,35}. The present inventors' data suggest that eIF5A1 could be one of the RNA binding proteins responsible for enhanced translation of p53 in response to genotoxic stress.

The proposed role of eIF5A1 as a nucleocytoplasmic shuttle protein has been confused by localization studies. Although there are a few reports of eIF5A1 being distributed throughout the cytoplasm and nucleus³⁶⁻³⁸, the majority of studies indicate that eIF5A1 is localized in the cytoplasm and perinuclear region, and is largely absent from the nucleus²¹⁻²⁵. Shi *et al.* (1996b) in a particularly detailed study of eIF5A1 subcellular localization reported that the protein was largely restricted to the cytoplasm and perinuclear regions of the cell with less than 1 % in the nucleus. They also found that the subcellular localization of eIF5A1 was not altered during changes in the cell cycle or following viral oncogene transformation. This localization pattern is not consistent with the proposal that eIF5A1 functions as a nucleocytoplasmic shuttle for transcripts required for cell growth⁴⁻⁵. In the present study, eIF5A1 expression was restricted to the cytoplasm and perinuclear region of HT-29 cells under normal growth conditions. However, a very rapid translocation of eIF5A1 protein into the nucleus was observed when HT-29 cells that had been sensitized with IFN-γ were treated with TNF-α, a treatment known to induce apoptosis in this cell line²⁶⁻³⁰. This translocation to the nucleus occurred within the first ten minutes of stimulation indicating that death receptor signalling initiates rapid transport of eIF5A1 protein from the cytoplasm to the nucleus. Similarly, the results show that Actinomycin D stimulated transport of eIF5A1 into the nucleus, although not as quickly as TNF-α. Previous studies have reported that eIF5A1 localization is not affected by Actinomycin D^{22,38}. Although the reasons for this discrepancy are not clear, differences in cell lines and concentrations of Actinomycin D used to stimulate the cells (4-5 µg/mL versus 1 µg/mL used in the present study) could account for the different findings. eIF5A1 has been reported to enter the nucleus only by passive diffusion^{31,38-39}. However, evidence is provided here of regulated nuclear import of eIF5A1 under conditions which are associated with apoptosis induced by death receptor activation or genotoxic stress.

Increased levels of unhypusinated eIF5Al have been correlated with the induction of apoptosis^{2,19,24,40-43}. These observations have led to the suggestion that the accumulation of unmodified eIF5A1 may play a role in the induction of certain types of apoptosis²⁴. It has also been reported that only unmodified eIF5A1 is capable of nuclear localization, suggesting that the unmodified form of eIF5A1 may have an apoptotic function which takes place in the nucleus²⁴. Under normal growth conditions, virtually all of the cellular eIF5A1 protein is hypusinated almost immediately after synthesis¹. It seems likely, therefore, that eIF5A1 is hypusinated and retained in the cytoplasm until an apoptotic stimulus triggers its translocation to the nucleus where it may have pro-apoptotic functions. Although hypusination is considered to be an irreversible modification⁴⁴, it is conceivable that a protein with de-hypusinating activity may be inactive under normal physiological conditions.

In conclusion, eIF5A1 appears to be a pro-apoptotic protein with nuclear functions during apoptosis induced by both death receptor activation and genotoxic stress. Greater understanding of the apoptotic functions of this unique protein could lead to new therapeutic interventions for the treatment of cancer.

The antisense polynucleotides or siRNA of the present invention can be used to make a medicament to decrease expression of apoptosis-specific eIF-5A in a mammal, mammalian cell or mammalian tissue. By decreasing expression of apoptosis specific eIF-5A, a decrease in cellular apoptosis results.

Alternatively, the methods and compositions of the present invention can be used to treat a subject having a tumor or cancer by increasing expression in a mammal, mammalian cell or mammalan tissue of apoptosis-specific eIF-5A through the use of polynucleotides encoding apoptosis specific eIF-5A to cause an increase in expression of apoptosis specific eIF-5A.

Further, polynucleotides encoding apoptosis-specific eIF-5A can be used to make a medicament to increase expression of apoptosis-specific eIF-5A in a mammal, mammalian cell or mammalian tissue. By increase expression of apoptosis specific eIF-5A, an increase in cellular apoptosis results. Thus, the medicament can be used to treat cancer by decreasing cancer cell or tumor cell growth or by inducing apoptosis in the cancer cell or tumor.

It is understood that the antisense nucleic acid and siRNAs of the present invention, where used in an animal for the purpose of prophylaxis or treatment, will be administered in the form of a composition additionally comprising a pharmaceutically acceptable carrier. Suitable pharmaceutically acceptable carriers include, for example, one or more of water, saline, phosphate buffered saline, dextrose, glycerol, ethanol and the like, as well as combinations thereof. Pharmaceutically acceptable carriers can further comprise minor amounts of auxiliary substances such as wetting or emulsifying agents, preservatives or buffers, which enhance the shelf life or effectiveness of the binding proteins. The compositions of the injection can, as is well known in the art, be formulated so as to provide quick, sustained or delayed release of the active ingredient after administration to the mammal.

The compositions of this invention can be in a variety of forms. These include, for example, solid, semi-solid and liquid dosage forms, such as tablets, pills, powders, liquid solutions, dispersions or suspensions, liposomes, suppositories, injectable and infusible solutions. The preferred form depends on the intended mode of administration and therapeutic application.

Such compositions can be prepared in a manner well known in the pharmaceutical art. In making the composition the active ingredient will usually be mixed with a carrier, or diluted by a carrier, and/or enclosed within a carrier which can, for example, be in the form of a capsule, sachet, paper or other container. When the carrier serves as a diluent, it can be a solid, semi-solid, or liquid material, which acts as a vehicle, excipient or medium for the active ingredient. Thus, the composition can be in the form of tablets, lozenges, sachets, cachets, elixirs, suspensions, aerosols (as a solid or in a liquid medium), ointments containing for example up to 10% by weight of the active compound, soft and hard gelatin capsules, suppositories, injection solutions, suspensions, sterile packaged powders and as a topical patch.

Having now generally described the invention, the same will be more readily understood through reference to the following examples, which are provided by way of illustration. The Examples are set forth to aid in understanding the invention but are not intended to, and should not be construed to, limit its scope in any way. The examples do not include detailed descriptions of conventional methods. Such methods are well known to those of ordinary skill in the art and are described in numerous publications. Detailed descriptions of conventional methods, such as those employed in the construction of vectors and plasmids, the insertion of nucleic acids encoding polypeptides into such vectors and plasmids, the introduction of plasmids into host cells, and the expression and determination thereof of genes and gene products can be obtained from numerous publication, including Sambrook, J. et al., (1989) Molecular Cloning: A Laboratory Manual, 2nd ed., Cold Spring Harbor Laboratory Press. All references mentioned herein are incorporated in their entirety.

### EXAMPLES

### EXAMPLE 1

### Visualization of Apoptosis in Rat Corpus Luteum by DNA Laddering

The degree of apoptosis was determined by DNA laddering. Genomic DNA was isolated from dispersed corpus luteal cells or from excised corpus luteum tissue using the QIAamp DNA Blood Kit (Qiagen) according to the manufacturer's instructions. Corpus luteum tissue was excised before the induction of apoptosis by treatment with PGF-2α, 1 hour and 24 hours after induction of apoptosis. The isolated DNA was end-labeled by incubating 500 ng of DNA with 0.2 µCi [α-³²P]dCTP, 1 mM Tris, 0.5 mM EDTA, 3 units of Klenow enzyme, and 0.2 pM each of dATP, dGTP, and dTTP at room temperature for 30 minutes. Unincorporated nucleotides were removed by passing the sample through a 1 ml Sepadex G-50 column according to Sambrook et al. The samples were then resolved by Tris-acetate-EDTA (1.8 %) gel electrophoresis. The gel was dried for 30 minutes at room temperature under vacuum and exposed to x-ray film at - 80° C for 24 hours.

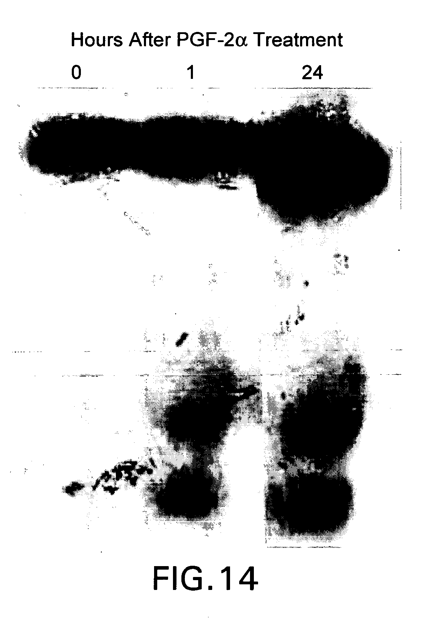

In one experiment, the degree of apoptosis in superovulated rat corpus lutea was examined either 0, 1, or 24 hours after injection with PGF-2α. In the 0 hour control, the ovaries were removed without PGF-2α injection. Laddering of low molecular weight DNA fragments reflecting nuclease activity associated with apoptosis is not evident in control corpus luteum tissue excised before treatment with PGF-2α, but is discernible within 1 hour after induction of apoptosis and is pronounced by 24 hours after induction of apoptosis, which is shown in figure 14. In this figure, the top panel is an autoradiograph of the Northern blot probed with the ³²P-dCTP-labeled 3'-untranslated region of rat corpus luteum apoptosis-specific DHS cDNA. The lower panel is the ethidium bromide stained gel of total RNA. Each lane contains 10 µg RNA. The data indicate that there is down-regulation of apoptosis-specific eIF-5A transcript following serum withdrawal.

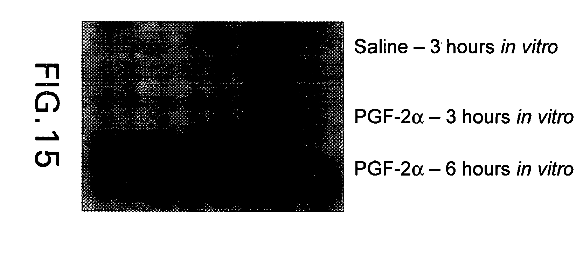

In another experiment, the corresponding control animals were treated with saline instead of PGF-2α. Fifteen minutes after treatment with saline or PGF-2α, corpora lutea were removed from the animals. Genomic DNA was isolated from the corpora lutea at 3 hours and 6 hours after removal of the tissue from the animals. DNA laddering and increased end labeling of genomic DNA are evident 6 hours after removal of the tissue from the PGF-2α-treated animals, but not at 3 hours after removal of the tissue. See figure 15. DNA laddering reflecting apoptosis is also evident when corpora lutea are excised 15 minutes after treatment with PGF-2α and maintained for 6 hours under *in vitro* conditions in EBSS (Gibco). Nuclease activity associated with apoptosis is also evident from more extensive end labeling of genomic DNA.

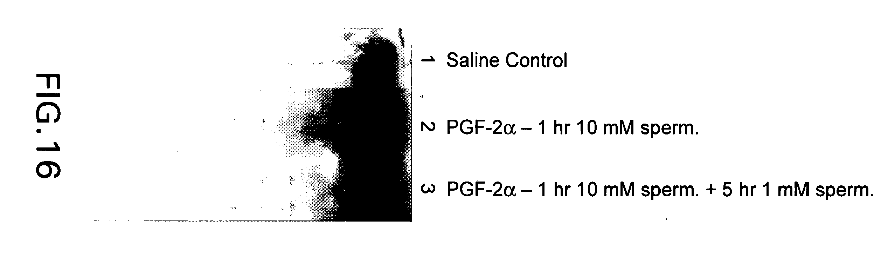

In another experiment, superovulation was induced by subcutaneous injection with 500 µg of PGF-2α. Control rats were treated with an equivalent volume of saline solution. Fifteen to thirty minutes later, the ovaries were removed and minced with collagenase. The dispersed cells from rats treated with PGF-2α and were incubated in 10 mm glutamine + 10 mm spermidine for 1 hour and for a further 5 hours in 10 mm glutamine without spermidine (lane 2) or in 10 mm glutamine + 10 mm spermidine for 1 hour and for a further 5 hours in 10 mm glutamine + 1 mm spermidine (lane 3). Control cells from rats treated with saline were dispersed with collagenase and incubated for 1 hour and a further 5 hours in glutamine only (lane 1). Five hundred nanograms of DNA from each sample was labeled with [α-³²P]-dCTP using klenow enzyme, separated on a 1.8 % agarose gel, and exposed to film for 24 hours. Results are shown in figure 16.

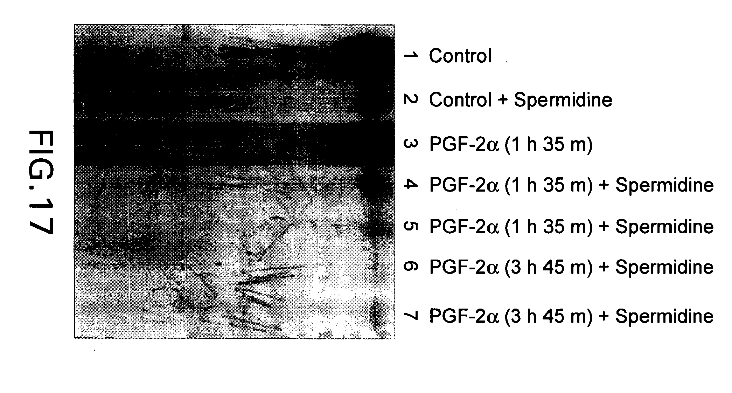

In yet another experiment, superovulated rats were injected subcutaneously with 1 mg/100 g body weight of spermidine, delivered in three equal doses of 0.333 mg/100 g body weight, 24, 12, and 2 hours prior to a subcutaneous injection with 500 µg PGF-2α. Control rats were divided into three sets: no injections, three injections of spermidine but no PGF-2α; and three injections with an equivalent volume of saline prior to PGF-2α treatment. Ovaries were removed front the rats either 1 hour and 35 minutes or 3 hours and 45 minutes after prostaglandin treatment and used for the isolation of DNA. Five hundred nanograms of DNA from each sample was labeled with [α-³²P]-dCTP using Klenow enzyme, separated on a 1.8 % agarose gel, and exposed to film for 24 hours (see figure 17): lane 1, no injections (animals were sacrificed at the same time as for lanes 3-5); lane 2, three injections with spermidine (animals were sacrificed at the same time as for lanes 3-5); lane 3, three injections with saline followed by injection with PGF-2α (animals were sacrificed 1 h and 35 min after treatment with PGF-2α); lane 4, three injections with spermidine followed by injection with PGF-2α (animals were sacrificed 1 h and 35 min after treatment with PGF-2α); lane 5, three injections with spermidine followed by injection with PGF-2α (animals were sacrificed 1 h and 35 min after treatment with PGF-2α); lane 6, three injections with spermidine followed by injection with PGF-2α (animals were sacrificed 3 h and 45 min after treatment with PGF-2α); lane 7, three injections with spermidine followed by injection with PGF-2α (animals were sacrificed 3 h and 45 min after treatment with PGF-2α).

### RNA Isolation

Total RNA was isolated from corpus luteum tissue removed from rats at various times after PGF-2α induction of apoptosis. Briefly, the tissue (5 g) was ground in liquid nitrogen. The ground powder was mixed with 30 ml guanidinium buffer (4 M guanidinium isothiocyanate, 2.5 mM NaOAc pH 8.5, 0.8% β-mercaptoethanol). The mixture was filtered through four layers of Miracloth and centrifuged at 10,000g at 4° C for 30 minutes. The supernatant was then subjected to cesium chloride density gradient centrifugation at 11,200g for 20 hours. The pelleted RNA was rinsed with 75% ethanol, resuspended in 600 ml DEPC-treated water and the RNA precipitated at -70° C with 1.5 ml 95% ethanol and 60 ml of 3M NaOAc.

### Genomic DNA Isolation and Laddering

Genomic DNA was isolated from extracted corpus luteum tissue or dispersed corpus luteal cells using the QIAamp DNA Blood Kit (Qiagen) according to the manufacturer's instructions. The DNA was end-labeled by incubating 500 ng of DNA with 0.2 µCi [α-³²P]dCTP, 1 mM Tris, 0.5 mM EDTA, 3 units of Klenow enzyme, and 0.2 pM each of dATP, dGTP, and dTTP, at room temperature for 30 minutes. Unincorporated nucleotides were removed by passing the sample through a 1-ml Sephadex G-50 column according to the method described by Maniatis et al. The samples were then resolved by Tris-acetate-EDTA (2 %) gel electrophoresis. The gel was dried for 30 minutes at room temperature under vacuum and exposed to x-ray film at - 80° C for 24 hours.

### Plasmid DNA Isolation, DNA Sequencing

The alkaline lysis method described by Sambrook et al., *supra,* was used to isolate plasmid DNA. The full-length positive cDNA clone was sequenced using the dideoxy sequencing method. Sanger et al., Proc. Natl. Acad. Sci. USA, 74:5463-5467. The open reading frame was compiled and analyzed using BLAST search (GenBank, Bethesda, MD) and sequence alignment was achieved using a BCM Search Launcher: Multiple Sequence Alignments Pattern-Induced Multiple Alignment Method (see F. Corpet, Nuc. Acids Res., 16:10881-10890, (1987). Sequences and sequence alignments are shown in figures 5-11.

### Northern Blot Hybridization of Rat Corpus Luteum RNA

Twenty milligrams of total RNA isolated from rat corpus luteum at various stages of apoptosis were separated on 1% denatured formaldehyde agarose gels and immobilized on nylon membranes. The full-length rat apoptosis-specific eIF-5A cDNA (SEQ ID NO: 1) labeled with ³²P-dCTP using a random primer kit (Boehringer) was used to probe the membranes 7 x 10⁷. Alternatively, full length rat DHS cDNA (SEQ ID NO:6) labeled with ³²P-dCTP using a random primer kit (Boehringer) was used to probe the membranes (7 x 10⁷ cpm). The membranes were washed once with 1 x SSC, 0.1% SDS at room temperature and three times with 0.2x SSC, 0.1% SDS at 65°C. The membranes were dried and exposed to X-ray film overnight at -70° C.

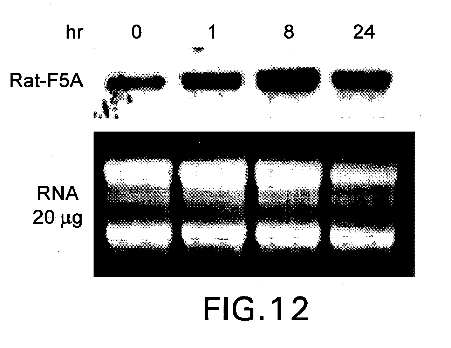

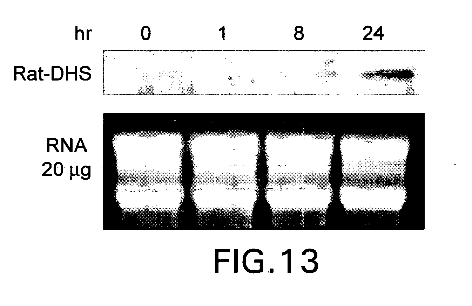

As can be seen, apoptosis-specific eIF-5A and DHS are both upregulated in apoptosing corpus luteum tissue. Expression of apoptosis-specific eIF-5A is significantly enhanced after induction of apoptosis by treatment with PGF-2α-low at time zero, increased substantially within 1 hour of treatment, increased still more within 8 hours of treatment and increased slightly within 24 hours of treatment (figure 12). Expression of DHS was low at time zero, increased substantially within 1 hour of treatment, increased still more within 8 hours of treatment and increased again slightly within 24 hours of treatment (figure 13).

### Generation of an Apoptosing Rat Corpus Luteum RT-PCR Product Using Primers Based on Yeast, Fungal and Human eIF-5A Sequences

A partial-length apoptosis-specific eIF-5A sequence (SEQ ID NO: 11) corresponding to the 3' end of the gene was generated from apoptosing rat corpus luteum RNA template by RT-PCR using a pair of oligonucleotide primers designed from yeast, fungal and human apoptosis-specific eIF-5A sequences. The upstream primer used to isolate the 3'end of the rat apoptosis-specific eIF-5A gene is a 20 nucleotide degenerate primer: 5'TCSAARACHGGNAAGCAYGG 3' (SEQ ID NO:9), wherein S is selected from C and G; R is selected from A and G; H is selected from A, T, and C; Y is selected from C and T; and N is any nucleic acid. The downstream primer used to isolate the 3'end of the rat eIF-5A gene contains 42 nucleotides: 5'GCGAAGCTTCCATGG CTCGAGTTTTTTTTTTTTTTTTTTTTT 3' (SEQ ID NO:10). A reverse transcriptase polymerase chain reaction (RT-PCR) was carried out. Briefly, using 5 mg of the downstream primer, a first strand of cDNA was synthesized. The first strand was then used as a template in a RT-PCR using both the upstream and downstream primers.

Separation of the RT-PCR products on an agarose gel revealed the presence a 900 bp fragment, which was subcloned into pBluescript^{™} (Stratagene Cloning Systems, LaJolla, CA) using blunt end ligation and sequenced (SEQ ID NO: 11). The cDNA sequence of the 3' end is SEQ ID NO:11 and the amino acid sequence of the 3' end is SEQ ID NO:12. See figures 1-2.

A partial-length apoptosis-specific eIF-5A sequence (SEQ ID NO:15) corresponding to the 5' end of the gene and overlapping with the 3' end was generated from apoptosing rat corpus luteum RNA template by RT-PCR. The 5' primer is a 24-mer having the sequence, 5' CAGGTCTAGAGTTGGAATCGAAGC 3' (SEQ ID NO:13), that was designed from human eIF-5A sequences. The 3' primer is a 30-mer having the sequence, 5' ATATCTCGAGCCTT GATTGCAACAGCTGCC 3' (SEQ ID NO:14) that was designed according to the 3' end RT-PCR fragment. A reverse transcriptase-polymerase chain reaction (RT-PCR) was carried out. Briefly, using 5 mg of the downstream primer, a first strand of cDNA was synthesized. The first strand was then used as a template in a RT-PCR using both the upstream and downstream primers.

Separation of the RT-PCR products on an agarose gel revealed the presence a 500 bp fragment, which was subcloned into pBluescript^{™} (Stratagene Cloning Systems, LaJolla, CA) using XbaI and XhoI cloning sites present in the upstream and downstream primers, respectively, and sequenced (SEQ ID NO:15). The cDNA sequence of the 5' end is SEQ ID NO:15, and the amino acid sequence of the 5' end is SEQ ID NO:16. See figure 2.

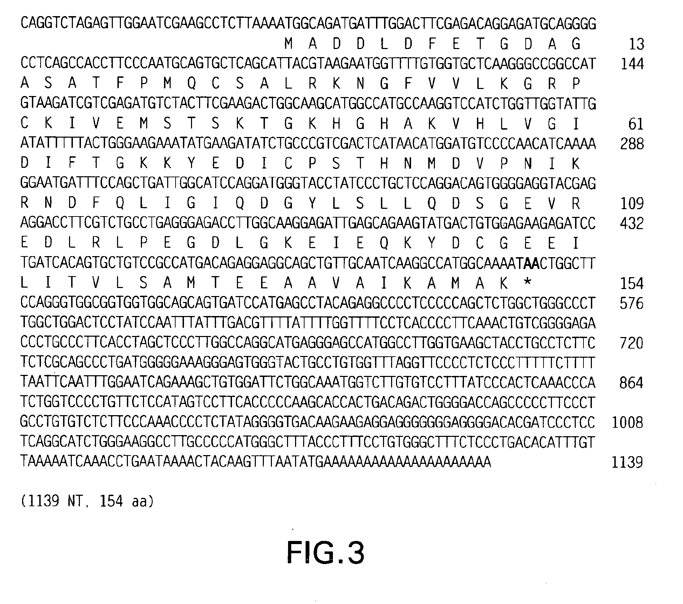

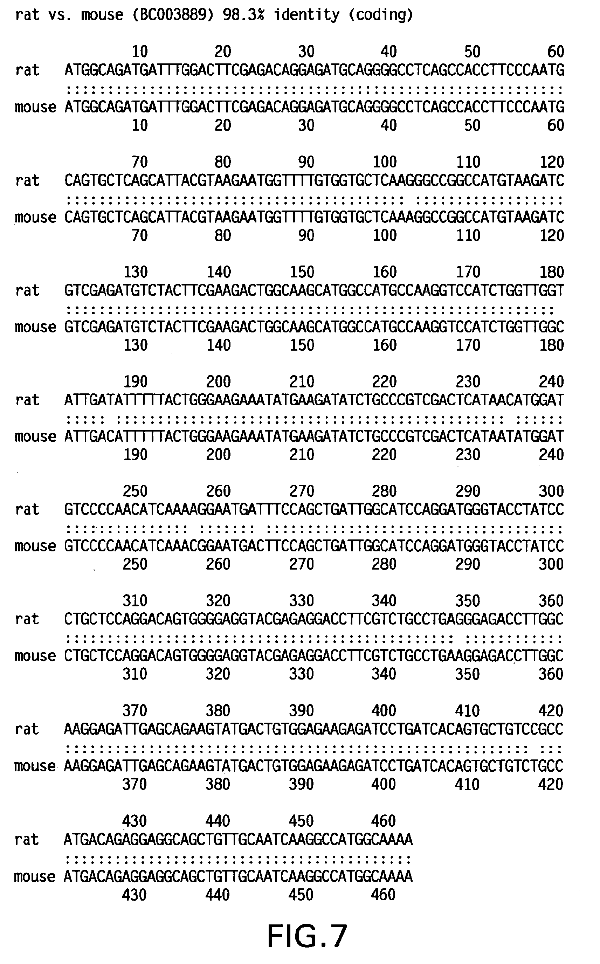

The sequences of the 3' and 5' ends of the rat apoptosis-specific eIF-5A (SEQ ID NO:11 and SEQ ID NO:15, respectively) overlapped and gave rise to the full-length cDNA sequence (SEQ ID NO: 1). This full-length sequence was aligned and compared with sequences in the GeneBank data base. See figures 1-2. The cDNA clone encodes a 154 amino acid polypeptide (SEQ ID NO:2) having a calculated molecular mass of 16.8 KDa. The nucleotide sequence, SEQ ID NO:1, for the full length cDNA of the rat apoptosis-specific corpus luteum eIF-5A gene obtained by RT-PCR is depicted in figure 3 and the corresponding derived amino acid sequence is SEQ ID NO:9. The derived full-length amino acid sequence of eIF-5A was aligned with human and mouse eIF-5a sequences. See figures 7-9.

### Generation of an Apoptosing Rat Corpus Luteum RT-PCR Product Using Primers Based on a Human DHS Sequence

A partial-length DHS sequence (SEQ ID NO:6) corresponding to the 3' end of the gene was generated from apoptosing rat corpus luteum RNA template by RT-PCR using a pair of oligonucleotide primers designed from a human DHS sequence. The 5' primer is a 20-mer having the sequence, 5' GTCTGTGTATTATTGGGCCC 3' (SEQ ID NO. 17).; the 3' primer is a 42-mer having the sequence, 5' GCGAAGCTTCCATGGC TCGAGTTTTTTTTTTTTTTTTTTTTT 3' (SEQ ID NO:18). A reverse transcriptase polymerase chain reaction (RT-PCR) was carried out. Briefly, using 5 mg of the downstream primer, a first strand of cDNA was synthesized. The first strand was then used as a template in a RT-PCR using both the upstream and downstream primers.

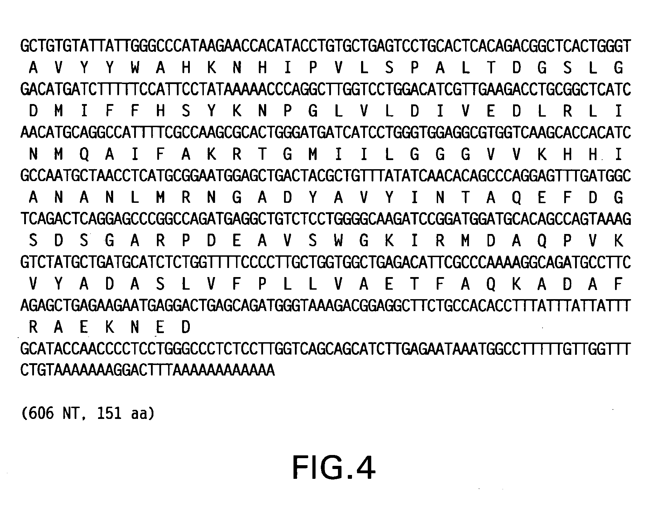

Separation of the RT-PCR products on an agarose gel revealed the presence a 606 bp fragment, which was subcloned into pBluescript^{™} (Stratagene Cloning Systems, LaJolla, CA) using blunt end ligation and sequenced (SEQ ID NO:6). The nucleotide sequence (SEQ ID NO:6) for the partial length cDNA of the rat apoptosis-specific corpus luteum DHS gene obtained by RT-PCR is depicted in figure 4 and the corresponding derived amino acid sequence is SEQ ID NO.7.

### Isolation of Genomic DNA and Southern Analysis

Genomic DNA for southern blotting was isolated from excised rat ovaries. Approximately 100 mg of ovary tissue was divided into small pieces and placed into a 15 ml tube. The tissue was washed twice with 1 ml of PBS by gently shaking the tissue suspension and then removing the PBS using a pipette. The tissue was resuspended in 2.06 ml of DNA-buffer (0.2 M Tris-HCl pH 8.0 and 0.1 mM EDTA) and 240 µl of 10 % SDS and 100 µl of proteinase K (Boehringer Manheim; 10 mg/ml) was added. The tissue was placed in a shaking water bath at 45° C overnight. The following day another 100 µl of proteinase K (10 mg/ml) was added and the tissue suspension was incubated in a water-bath at 45 °C for an additional 4 hours. After the incubation the tissue suspension was extracted once with an equal volume of phenol:chloroform:iso-amyl alcohol (25:24:1) and once with an equal volume of chloroform:iso-amyl alcohol (24:1). Following the extractions 1/10th volume of 3M sodium acetate (pH 5.2) and 2 volumes of ethanol were added. A glass pipette sealed and formed into a hook using a Bunsen burner was used to pull the DNA threads out of solution and to transfer the DNA into a clean microcentrifuge tube. The DNA was washed once in 70 % ethanol and air-dried for 10 minutes. The DNA pellet was dissolved in 500 µl of 10 mM Tris-HCl (pH 8.0), 10 µl of RNase A (10 mg/ml) was added, and the DNA was incubated for 1 hour at 37 °C. The DNA was extracted once with phenol:chloroform:iso-amyl alcohol (25:24:1) and the DNA was precipitated by adding 1/10th volume of 3 M sodium acetate (pH 5.2) and 2 volumes of ethanol. The DNA was pelleted by centrifugation for 10 minutes at 13,000 x g at 4° C. The DNA pellet was washed once in 70 % ethanol and dissolved in 200 µl 10 mM Tris-HCl (pH 8.0) by rotating the DNA at 4 °C overnight.

For Southern blot analysis, genomic DNA isolated from rat ovaries was digested with various restriction enzymes that either do not cut in the endogenous gene or cut only once. To achieve this, 10 µg genomic DNA, 20 µl 10X reaction buffer and 100 U restriction enzyme were reacted for five to six hours in a total reaction volume of 200 µl. Digested DNA was loaded onto a 0.7 % agarose gel and subjected to electrophoresis for 6 hours at 40 volts or overnight at 15 volts. After electrophoresis, the gel was depurinated for 10 minutes in 0.2 N HCl followed by two 15-minute washes in denaturing solution (0.5 M NaOH, 1.5 M NaCl) and two 15 minute washes in neutralizing buffer (1.5 M NaCl, 0.5 M Tris-HCl pH 7.4). The DNA was transferred to a nylon membrane, and the membrane was prehybridized in hybridization solution (40 % formamide, 6 X SSC, 5 X Denhart's, solution (1 X Denhart's solution is 0.02 % Ficoll, 0.02 % PVP, and 0.02 % BSA), 0.5 % SDS, and 1.5 mg of denatured salmon sperm DNA). A 700 bp PCR fragment of the 3' UTR of rat eIF-5A cDNA (650 bp of 3' UTR and 50 bp of coding) was labeled with [a-32P]-dCTP by random priming and added to the membrane at 1 X 106 cpm/ml.

Similarly, a 606 bp PCR fragment of the rat DHS cDNA (450 bp coding and 156 bp 3' UTR) was random prime labeled with [α-³²P]-dCTP and added at 1 X 10 6 cpm/ml to a second identical membrane. The blots were hybridized overnight at 42° C and then washed twice with 2 X SSC and 0.1 % SDS at 42° C and twice with 1 X SSC and 0.1 % SDS at 42° C. The blots were then exposed to film for 3-10 days.

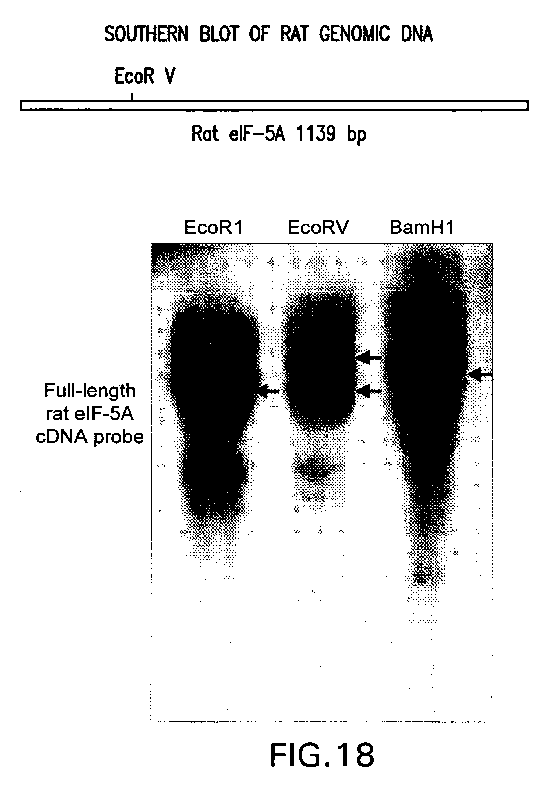

Rat corpus genomic DNA was cut with restriction enzymes as indicated on figure 18 and probed with ³²P-dCTP-labeled full-length eIF-5A cDNA. Hybridization under high stringency conditions revealed hybridization of the full-length cDNA probe to several restriction fragments for each restriction enzyme digested DNA sample, indicating the presence of several isoforms of eIF-5A. Of particular note, when rat genomic DNA was digested with EcoRV, which has a restriction site within the open reading frame of apoptosis-specific eIF-5A, two restriction fragments of the apoptosis-specific isoform of eIF-5A were detectable in the Southern blot. The two fragments are indicated with double arrows in figure 18. The restriction fragment corresponding to the apoptosis-specific isoform of eIF-5A is indicated by a single arrow in the lanes labeled EcoR1 and BamH1, restriction enzymes for which there are no cut sites within the open reading frame. These results suggest that the apoptosis-specific eIF-5A is a single copy gene in rat. As shown in figures 5-11, the eIF-5A gene is highly conserved across species, and so it would be expected that there is a significant amount of conservation between isoforms within any species.

Figure 18 shows a Southern blot of rat genomic DNA probed with ³²P-dCTP-labeled partial-length rat corpus luteum DHS cDNA. The genomic DNA was cut with EcoRV, a restriction enzyme that does not cut the partial-length cDNA used as a probe. Two restriction fragments are evident indicating that there are two copies of the gene or that the gene contains an intron with an EcoRV site.

### EXAMPLE 2

The present example demonstrates modulation of apoptosis apoptosis-specific eIF-5A (increasing apoptosis with apoptosis-specific eIF-5A in sense orientation)

### Culturing of COS-7 Cells and Isolation of RNA

COS-7, an African green monkey kidney fibroblast-like cell line transformed with a mutant of SV40 that codes for wild-type T antigen, was used for all transfection-based experiments. COS-7 cells were cultured in Dulbecco's Modified Eagle's medium (DMEM) with 0.584 grams per liter of L-glutamine, 4.5 g of glucose per liter, and 0.37 % sodium bicarbonate. The culture media was supplemented with 10 % fetal bovine serum (FBS) and 100 units of penicillin/streptomycin. The cells were grown at 37° C in a humidified environment of 5 % CO₂ and 95 % air. The cells were subcultured every 3 to 4 days by detaching the adherent cells with a solution of 0.25 % trypsin and 1 mM EDTA. The detached cells were dispensed at a split ratio of 1:10 in a new culture dish with fresh media.

COS-7 cells to be used for isolation of RNA were grown in 150-mm tissue culture treated dishes (Corning). The cells were harvested by detaching them with a solution of trypsin-EDTA. The detached cells were collected in a centrifuge tube, and the cells were pelleted by centrifugation at 3000 rpm for 5 minutes. The supernatant was removed, and the cell pellet was flash-frozen in liquid nitrogen. RNA was isolated from the frozen cells using the GenElute Mammalian Total RNA Miniprep kit (Sigma) according to the manufacturer's instructions.

### Construction of Recombinant Plasmids and Transfection of COS-7 Cells

Recombinant plasmids carrying the full-length coding sequence of rat apoptosis-specific eIF-5A in the sense orientation and the 3' untranslated region (UTR) of rat apoptosis-specific eIF-5A in the antisense orientation were constructed using the mammalian epitope tag expression vector, pHM6 (Roche Molecular Biochemicals), which is illustrated in figure 19. The vector contains the following: CMV promoter - human cytomegalovirus immediate-early promoter/enhancer; HA - nonapeptide epitope tag from influenza hemagglutinin; BGH pA - Bovine growth hormone polyadenylation signal; f1 ori - f1 origin; SV40 ori - SV40 early promoter and origin; Neomycin - Neomycin resistance (G418) gene; SV40 pA - SV40 polyadenylation signal; Col E1-CoIE1 origin; Ampicillin- Ampicillin resistance gene. The full-length coding sequence of rat apoptosis-specific eIF-5A and the 3' UTR of rat apoptosis-specific eIF-5A were amplified by PCR from the original rat eIF-5A RT-PCR fragment in pBluescript (SEQ ID NO: 1). To amplify the full-length eIF-5A the primers used were as follows: Forward 5' GCCAAGCTTAATGGCAGATGATTT GG 3' (SEQ ID NO: 59) (Hind3) and Reverse 5' CTGAATTCCAGT TATTTTGCCATGG 3' (SEQ ID NO:60) (EcoR1). To amplify the 3'UTR rat apoptosis-specific eIF-5A the primers used were as follows: forward 5' AATGAATTCCGCCATGACAGAGGAGGC 3' (SEQ ID NO: 61) (EcoR1) and reverse 5'GCGAAGCTTCCATGGCTCGAGTTTTTTTTTTTTTTTTTTTTT 3' (SEQ ID NO: 62) (Hind3).

The full-length rat apoptosis-specific eIF-5A PCR product isolated after agarose gel electrophoresis was 430 bp in length while the 3' UTR rat apoptosis-specific eIF-5A PCR product was 697 bp in length. Both PCR products were subcloned into the Hind 3 and EcoR1 sites of pHM6 to create pHM6-full-length apoptosis-specific eIF-5A and pHM6-antisense 3'UTR eIF-5A. The full-length rat apoptosis-specific eIF-5A PCR product was subcloned in frame with the nonapeptide epitope tag from influenza hemagglutinin (HA) present upstream of the multiple cloning site to allow for detection of the recombinant protein using an anti-[HA]-peroxidase antibody. Expression is driven by the human cytomegalovirus immediate-early promoter/enhancer to ensure high level expression in mammalian cell lines. The plasmid also features a neomycin-resistance (G418) gene, which allows for selection of stable transfectants, and a SV40 early promoter and origin, which allows episomal replication in cells expressing SV40 large T antigen, such as COS-7.

COS-7 cells to be used in transfection experiments were cultured in either 24 well cell culture plates (Coming) for cells to be used for protein extraction, or 4 chamber culture slides (Falcon) for cells to be used for staining. The cells were grown in DMEM media supplemented with 10 % FBS, but lacking penicillin/streptomycin, to 50 to 70 % confluency. Transfection medium sufficient for one well of a 24-well plate or culture slide was prepared by diluting 0.32 µg of plasmid DNA in 42.5 µl of serum-free DMEM and incubating the mixture at room temperature for 15 minutes. 1.6 µl of the transfection reagent, LipofectAMINE (Gibco, BRL), was diluted in 42.5 µl of serum-free DMEM and incubated for 5 minutes at room temperature. After 5 minutes the LipofectAMINE mixture was added to the DNA mixture and incubated together at room temperature for 30 to 60 minutes. The cells to be transfected were washed once with serum-free DMEM before overlaying the transfection medium and the cells were placed back in the growth chamber for 4 hours.

After the incubation, 0.17 ml of DMEM + 20 % FBS was added to the cells. The cells were the cultured for a further 40 hours before either being induced to undergo apoptosis prior to staining or harvested for Western blot analysis. As a control, mock transfections were also performed in which the plasmid DNA was omitted from the transfection medium.

### Protein Extraction and Western Blotting

Protein was isolated for Western blotting from transfected cells by washing the cells twice in PBS (8 g/L NaCl, 0.2 g/L KCl, 1.44 g/L Na₂HPO₄, and 0.24 g/L KH₂PO₄) and then adding 150 µl of hot SDS gel-loading buffer (50 mM Tris-HCl pH 6.8, 100 mM dithiothreitol, 2 % SDS, 0.1 % bromophenol blue, and 10 % glycerol). The cell lysate was collected in a microcentrifuge tube, heated at 95° C for 10 minutes, and then centrifuged at 13,000 x g for 10 minutes. The supernatant was transferred to a fresh microcentrifuge tube and stored at -20° C until ready for use.

For Western blotting, 2.5 or 5 µg of total protein was separated on a 12 % SDS-polyacrylamide gel. The separated proteins were transferred to a polyvinylidene difluoride membrane. The membrane was then incubated for one hour in blocking solution (5 % skim milk powder, 0.02 % sodium azide in PBS) and washed three times for 15 minutes in PBS-T (PBS + 0.05 % Tween-20). The membrane was stored overnight in PBS-T at 4 °C. After being warmed to room temperature the next day, the membrane was blocked for 30 seconds in 1 µg/ml polyvinyl alcohol. The membrane was rinsed 5 times in deionized water and then blocked for 30 minutes in a solution of 5 % milk in PBS. The primary antibody was preincubated for 30 minutes in a solution of 5 % milk in PBS prior to incubation with the membrane.

Several primary antibodies were used. An anti-[HA]-peroxidase antibody (Roche Molecular Biochemicals) was used at a dilution of 1:5000 to detect expression of the recombinant proteins. Since this antibody is conjugated to peroxidase, no secondary antibody was necessary, and the blot was washed and developed by chemiluminescence. The other primary antibodies that were used are monoclonal antibodies from Oncogene that recognize p53 (Ab-6), Bcl-2 (Ab-1), and c-Myc (Ab-2). The monoclonal antibody to p53 was used at a dilution of 0.1 µg/ml, and the monoclonal antibodies to Bcl-2 and c-Myc were both used at a dilution of 0.83 µg/ml. After incubation with primary antibody for 60 to 90 minutes, the membrane was washed 3 times for 15 minutes in PBS-T. Secondary antibody was then diluted in 1 % milk in PBS and incubated with the membrane for 60 to 90 minutes. When p53 (Ab-6) was used as the primary antibody, the secondary antibody used was a goat anti-mouse IgG conjugated to alkaline phosphatase (Rockland) at a dilution of 1:1000. When Bcl-2 (Ab-1) and c-Myc (Ab-2) were used as the primary antibody, a rabbit anti-mouse IgG conjugated to peroxidase (Sigma) was used at a dilution of 1:5000. After incubation with the secondary antibody, the membrane was washed 3 times in PBS-T.

Two detection methods were used to develop the blots, a colorimetric method and a chemiluminescent method. The colorimetric method was used only when p53 (Ab-6) was used as the primary antibody in conjunction with the alkaline phosphatase-conjugated secondary antibody. Bound antibody was visualized by incubating the blot in the dark in a solution of 0.33 mg/mL nitro blue tetrazolium, 0.165 mg/mL 5-bromo-4-chloro-3-indolyl phosphate, 100 mM NaCl, 5 mM MgCl₂, and 100 mM Tris-HCl (pH 9.5). The color reaction was stopped by incubating the blot in 2 mM EDTA in PBS. A chemiluminescent detection method was used for all other primary antibodies, including anti-[HA]-peroxidase, Bcl-2 (Ab-1), and c-Myc (Ab-2). The ECL Plus Western blotting detection kit (Amersham Pharmacia Biotech) was used to detect peroxidase-conjugated bound antibodies. In brief, the membrane was lightly blotted dry and then incubated in the dark with a 40:1 mix of reagent A and reagent B for 5 minutes. The membrane was blotted dry, placed between sheets of acetate, and exposed to X-ray film for time periods varying from 10 seconds to 10 minutes.

### Induction of Apoptosis in COS 7 Cells

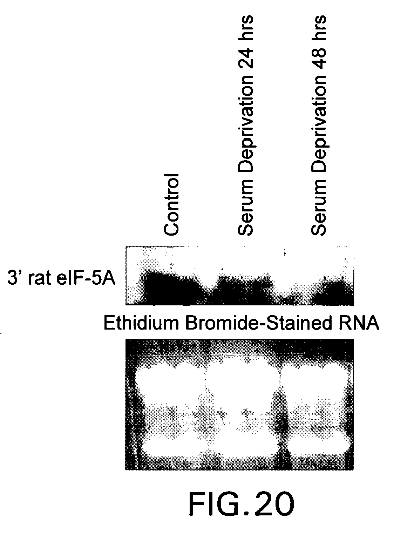

Two methods were used to induce apoptosis in transfected COS-7 cells, serum deprivation and treatment with Actinomycin D, *streptomyces* sp (Calbiochem). For both treatments, the medium was removed 40 hours post-transfection. For serum starvation experiments, the media was replaced with serum- and antibiotic-free DMEM. Cells grown in antibiotic-free DMEM supplemented with 10 % FBS were used as a control. For Actinomycin D induction of apoptosis, the media was replaced with antibiotic-free DMEM supplemented with 10 % FBS and 1 µg/ml Actinomycin D dissolved in methanol. Control cells were grown in antibiotic-free DMEM supplemented with 10 % FBS and an equivalent volume of methanol. For both methods, the percentage of apoptotic cells was determined 48 hours later by staining with either Hoescht or Annexin V-Cy3. Induction of apoptosis was also confirmed by Northern blot analyses, as shown in figure 20.

### Hoescht Staining

The nuclear stain, Hoescht, was used to label the nuclei of transfected COS-7 cells in order to identify apoptotic cells based on morphological features such as nuclear fragmentation and condensation. A fixative, consisting of a 3:1 mixture of absolute methanol and glacial acetic acid, was prepared immediately before use. An equal volume of fixative was added to the media of COS-7 cells growing on a culture slide and incubated for 2 minutes. The media/fixative mixture was removed from the cells and discarded, and 1 ml of fixative was added to the cells. After 5 minutes the fixative was discarded, and 1 ml of fresh fixative was added to the cells and incubated for 5 minutes. The fixative was discarded, and the cells were air-dried for 4 minutes before adding 1 ml of Hoescht stain (0.5 µg/ml Hoescht 33258 in PBS). After a 10-minute incubation in the dark, the staining solution was discarded and the slide was washed 3 times for 1 minute with deionized water. After washing, 1 ml of McIlvaine's buffer (0.021 M citric acid, 0.058 M Na₂HPO₄.7H₂O; pH 5.6) was added to the cells, and they were incubated in the dark for 20 minutes. The buffer was discarded, the cells were air-dried for 5 minutes in the dark and the chambers separating the wells of the culture slide were removed. A few drops of Vectashield mounting media for fluorescence (Vector Laboratories) was added to the slide and overlaid with a coverslip. The stained cells were viewed under a fluorescence microscope using a UV filter. Cells with brightly stained or fragmented nuclei were scored as apoptotic.

### Annexin V-Cy3 Staining

An Annexin V-Cy3 apoptosis detection kit (Sigma) was used to fluorescently label externalized phosphatidylserine on apoptotic cells. The kit was used according to the manufacturer's protocol with the following modifications. In brief, transfected COS-7 cells growing on four chamber culture slides were washed twice with PBS and three times with 1 X Binding Buffer. 150 µl of staining solution (1 µg/ml AnnCy3 in 1 X Binding Buffer) was added, and the cells were incubated in the dark for 10 minutes. The staining solution was then removed, and the cells were washed 5 times with 1 X Binding Buffer. The chamber walls were removed from the culture slide, and several drops of 1 X Binding Buffer were placed on the cells and overlaid with a coverslip. The stained cells were analyzed by fluorescence microscopy using a green filter to visualize the red fluorescence of positively stained (apoptotic) cells. The total cell population was determined by counting the cell number under visible light.

### EXAMPLE 3

The present example demonstrates modulation of apoptosis apoptosis-specific eIF-5A.

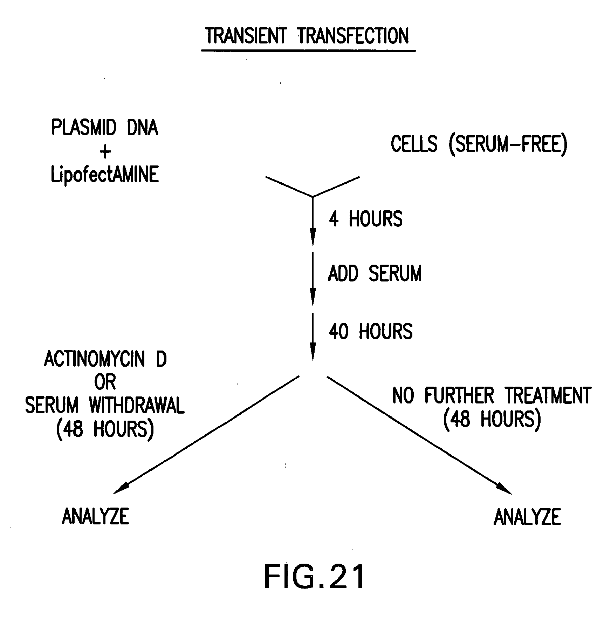

Using the general procedures and methods described in the previous examples, figure 21 is a flow chart illustrating the procedure for transient transfection of COS-7 cells, in which cells in serum-free medium were incubated in plasmid DNA in lipofectAMINE for 4 hours, serum was added, and the cells were incubated for a further 40 hours. The cells were then either incubated in regular medium containing serum for a further 48 hours before analysis (i.e. no further treatment), deprived of serum for 48 hours to induce apoptosis before analysis, or treated with actinomycin D for 48 hours to induce apoptosis before analysis.

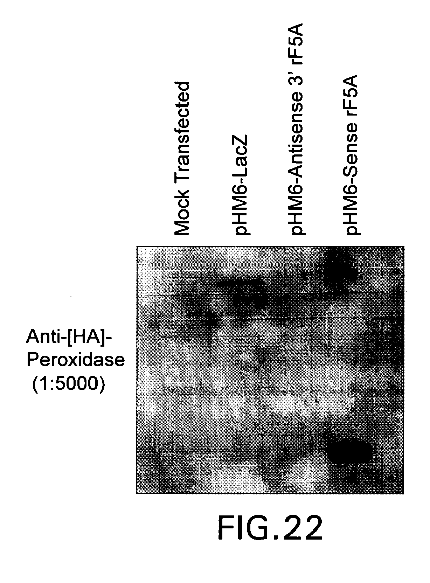

Figure 22 is a Western blot illustrating transient expression of foreign proteins in COS-7 cells following transfection with pHM6. Protein was isolated from COS-7 cells 48 hours after either mock transfection, or transfection with pHM6-LacZ, pHM6-Antisense 3'rF5A (pHM6-Antisense 3' UTR rat apoptosis-specific eIF-5A), or pHM6-Sense rF5A (pHM6-Full length rat apoptosis-specific eIF-5A). Five µg of protein from each sample was fractionated by SDS-PAGE, transferred to a PVDF membrane, and Western blotted with anti-[HA]-peroxidase. The bound antibody was detected by chemiluminescence and exposed to x-ray film for 30 seconds. Expression of LacZ (lane 2) and of sense rat apoptosis-specific eIF-5A (lane 4) is clearly visible.

As described above, COS-7 cells were either mock transfected or transfected with pHM6-Sense rF5A (pHM6-Full length rat apoptosis-specific eIF-5A). Forty hours after transfection, the cells were induced to undergo apoptosis by withdrawal of serum for 48 hours. The caspase proteolytic activity in the transfected cell extract was measured using a fluorometric homogenous caspase assay kit (Roche Diagnostics). DNA fragmentation was also measured using the FragEL DNA Fragmentation Apoptosis Detection kit (Oncogene) which labels the exposed 3'-OH ends of DNA fragments with fluorescein-labeled deoxynucleotides.

Additional COS-7 cells were either mock transfected or transfected with pHM6-Sense rF5A (pHM6-Full length rat apoptosis-specific eIF-5A). Forty hours after transfection, the cells were either grown for an additional 48 hours in regular medium containing serum (no further treatment), induced to undergo apoptosis by withdrawal of serum for 48 hours or induced to undergo apoptosis by treatment with 0.5 µg/ml of Actinomycin D for 48 hours. The cells were either stained with Hoescht 33258, which depicts nuclear fragmentation accompanying apoptosis, or stained with Annexin V-Cy3, which depicts phosphatidylserine exposure accompanying apoptosis. Stained cells were also viewed by fluorescence microscopy using a green filter and counted to determine the percentage of cells undergoing apoptosis. The total cell population was counted under visible light.

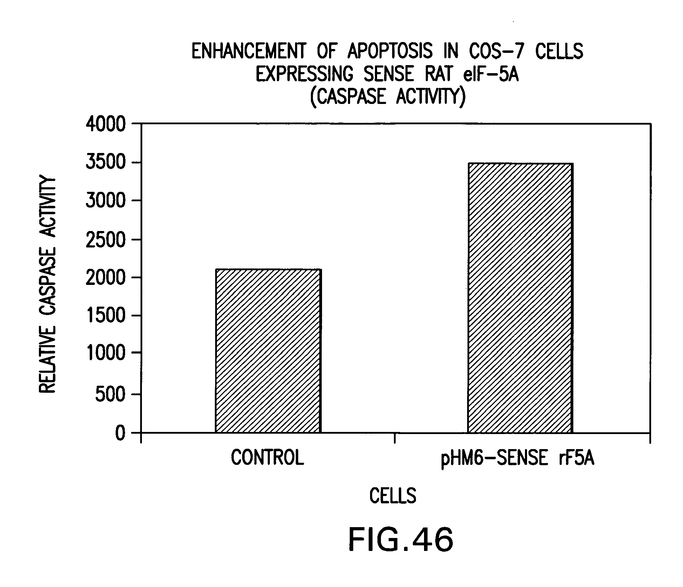

Figure 46 illustrates enhanced apoptosis as reflected by increased caspase activity when COS-7 cells were transiently transfected with pHM6 containing full-length rat apoptosis-specific eIF-5A in the sense orientation. Expression of rat apoptosis-specifice IF-5A resulted in a 60% increase in caspase activity.

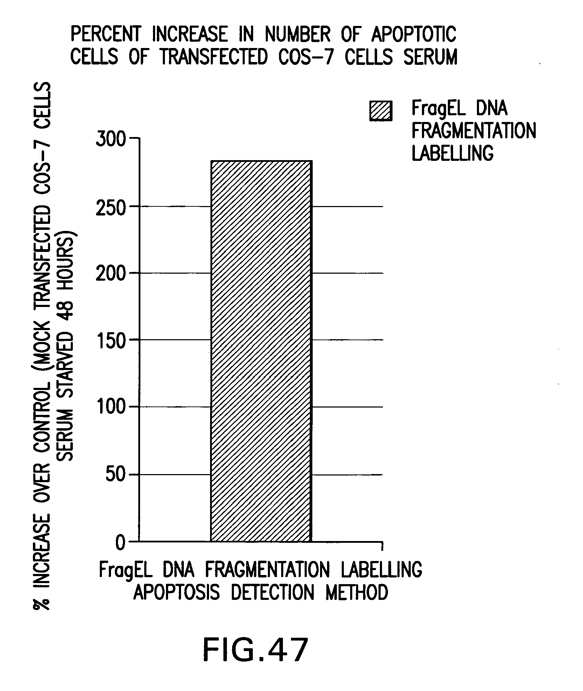

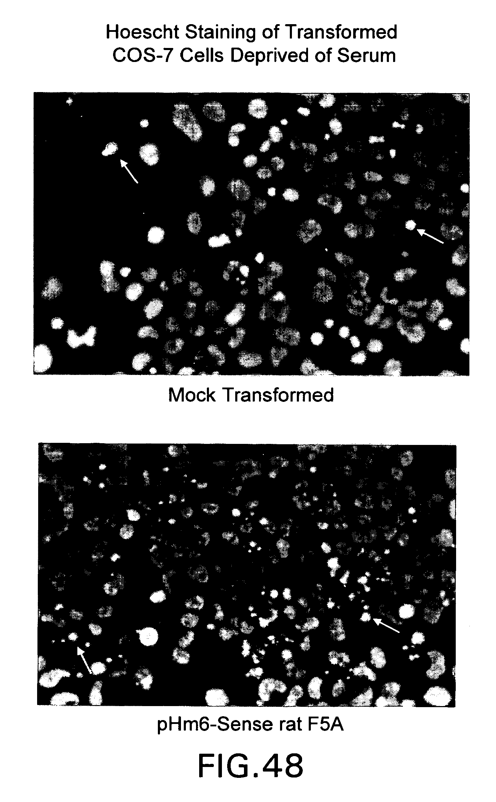

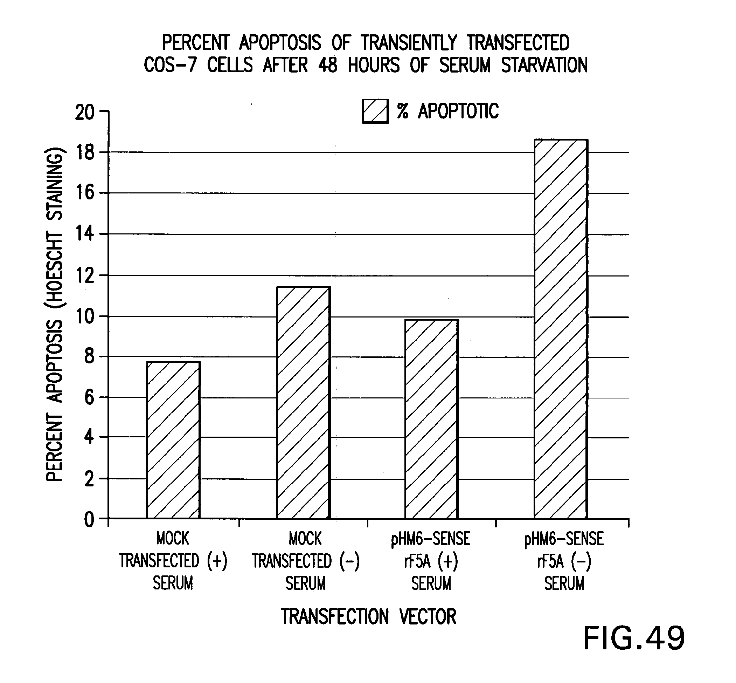

Figure 47 illustrates enhanced apoptosis as reflected by increased DNA fragmentation when COS-7 cells were transiently transfected with pHM6 containing full-length rat apoptosis-specific eIF-5A in the sense orientation. Expression of rat apoptosis-specific eIF-5A resulted in a 273% increase in DNA fragmentation. Figure 48 illustrates detection of apoptosis as reflected by increased nuclear fragmentation when COS-7 cells were transiently transfected with pHM6 containing full-length rat apoptosis-specific eIF-5A in the sense orientation. There is a greater incidence of fragmented nuclei in cells expressing rat apoptosis-specific eIF-5A. Figure 49 illustrates enhanced apoptosis as reflected by increased nuclear fragmentation when COS-7 cells were transiently transfected with pHM6 containing full-length rat apoptosis-specific eIF-5A in the sense orientation. Expression of rat apoptosis-specificeIF-5A resulted in a 27 % and 63 % increase in nuclear fragmentation over control in non-serum starved and serum starved samples, respectively.

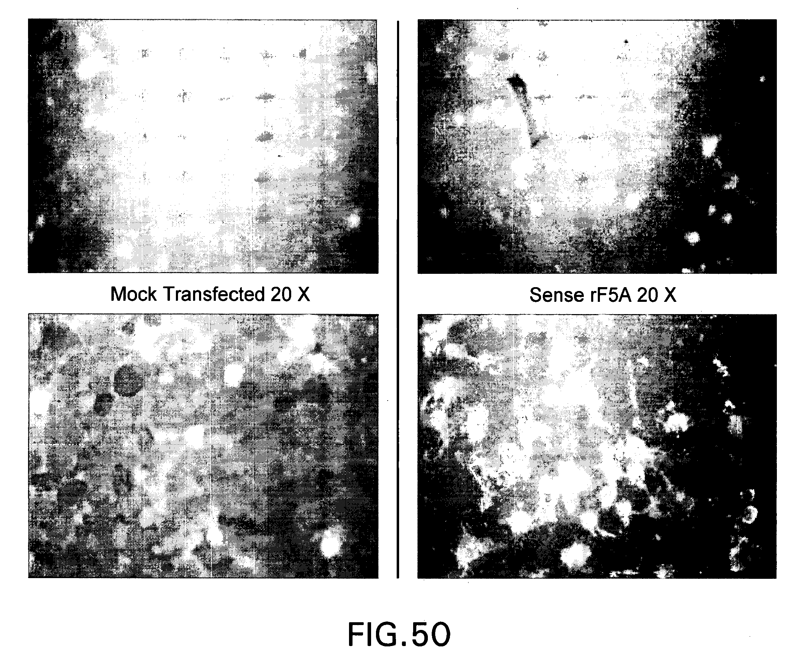

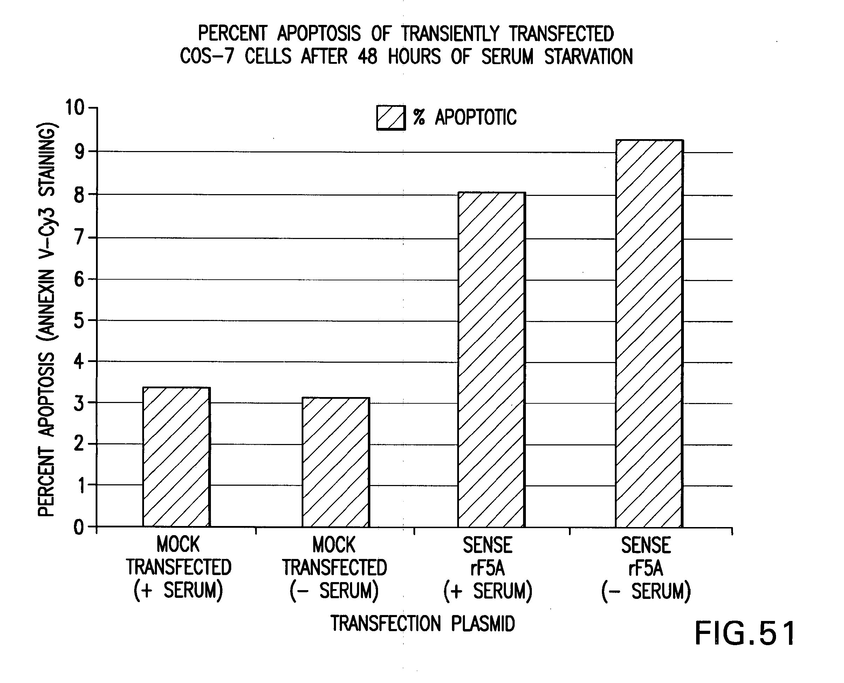

Figure 50 illustrates detection of apoptosis as reflected by phosphatidylserine exposure when COS-7 cells were transiently transfected with pHM6 containing full-length rat apoptosis-specific eIF-5A in the sense orientation. Figure 51 illustrates enhanced apoptosis as reflected by increased phosphatidylserine exposure when COS-7 cells were transiently transfected with pHM6 containing full-length rat apoptosis-specific eIF-5A in the sense orientation. Expression of rat apoptosis-specific eIF-5A resulted in a 140 % and 198 % increase in phosphatidylserine exposure over control, in non-serum starved and serum starved samples, respectively.

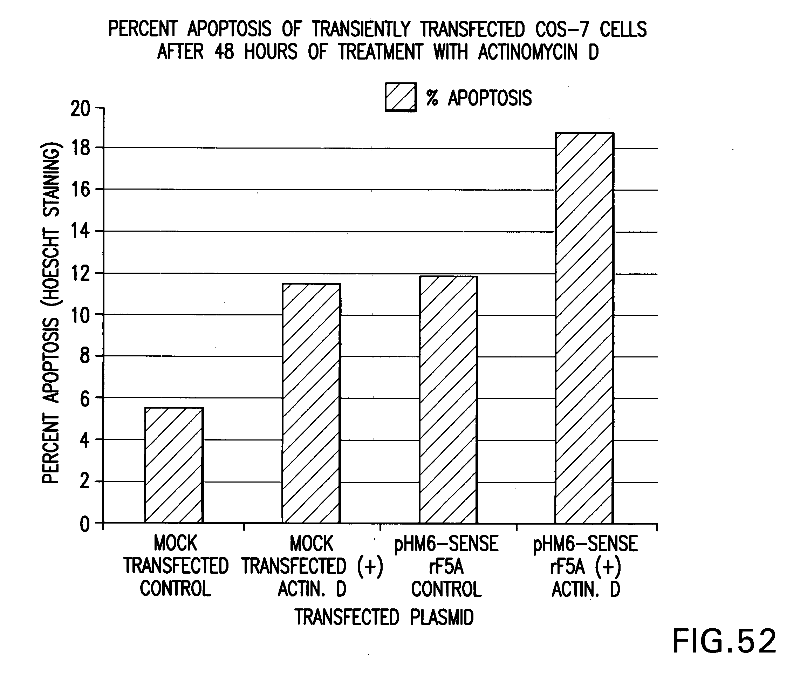

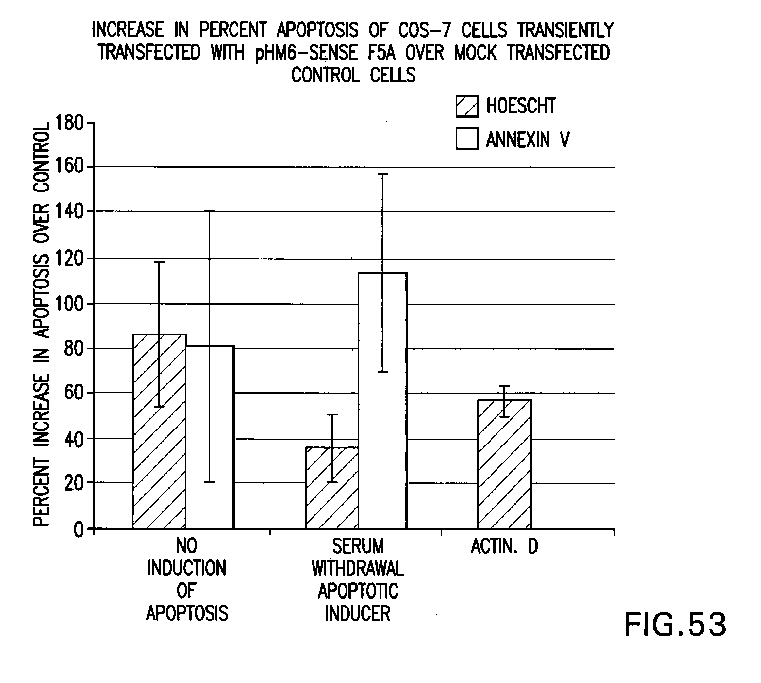

Figure 52 illustrates enhanced apoptosis as reflected by increased nuclear fragmentation when COS-7 cells were transiently transfected with pHM6 containing full-length rat apoptosis-specificeIF-5A in the sense orientation. Expression of rat apoptosis-specific eIF-5A resulted in a 115 % and 62 % increase in nuclear fragmentation over control in untreated and treated samples, respectively. Figure 53 illustrates a comparison of enhanced apoptosis under conditions in which COS-7 cells transiently transfected with pHM6 containing full-length rat apoptosis-specific eIF-5A in the sense orientation were either given no further treatment or treatment to induce apoptosis.

### EXAMPLE 4

The present example demonstrates modulation of apoptotic activity following administration of apoptosis-specific eIF-5A.

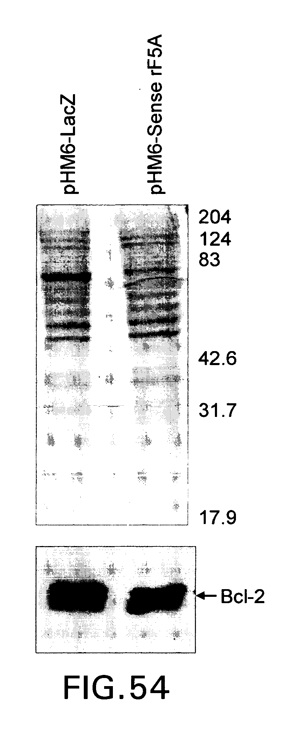

COS-7 cells were either mock transfected, transfected with pHM6-LacZ or transfected with pHM6-Sense rF5A (pHM6-Full length rat apoptosis-specific eIF-5A) and incubated for 40 hours. Five µg samples of protein extract from each sample were fractionated by SDS-PAGE, transferred to a PVDF membrane, and Western blotted with a monoclonal antibody that recognizes Bcl-2. Rabbit anti-mouse IgG conjugated to peroxidase was used as a secondary antibody, and bound antibody was detected by chemiluminescence and exposure to x-ray film. Results are shown in Figure 54. This figure illustrates down-regulation of Bcl-2 when COS-7 cells were transiently transfected with pHM6 containing full-length rat apoptosis-specific eIF-5A in the sense orientation. The upper panel illustrates the Coomassie-blue-stained protein blot; the lower panel illustrates the corresponding Western blot. Less Bcl-2 is detectable in cells transfected with pHM6-Sense rF5A than in those transfected with pHM6-LacZ; thus showing that Bcl-2 is down-regulated with the pHM6-sense rF5A construct.

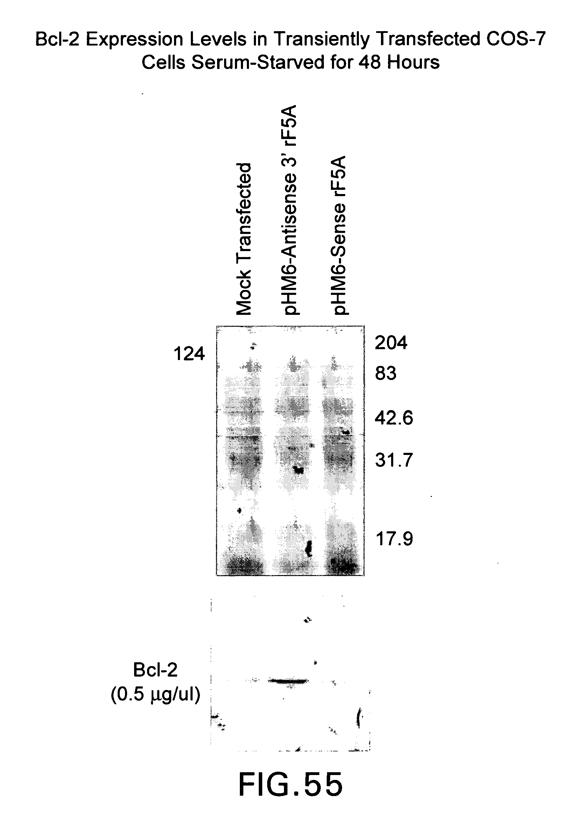

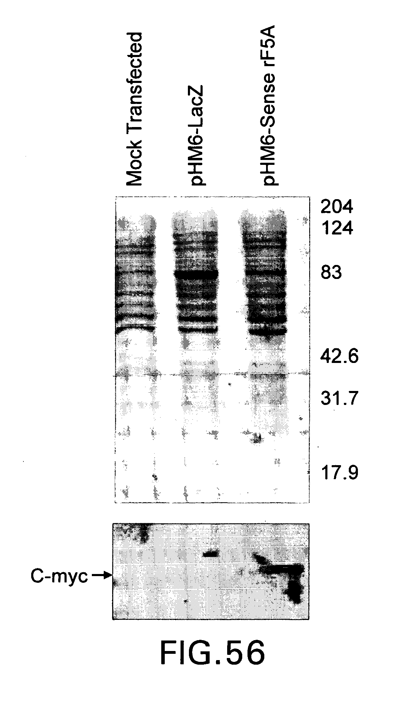

Additional COS-7 cells were either mock transfected, transfected with pHM6-antisense 3'rF5A (pHM6-antisense 3' UTR of rat apoptosis-specific eIF-5A) or transfected with pHM6-Sense rF5A (pHM6-Full length rat apoptosis-specific eIF-5A). Forty hours after transfection, the cells were induced to undergo apoptosis by withdrawal of serum for 48 hours. Five µg samples of protein extract from each sample were fractionated by SDS-PAGE, transferred to a PVDF membrane, and Western blotted with a monoclonal antibody that recognizes Bcl-2. Rabbit anti-mouse IgG conjugated to peroxidase was used as a secondary antibody, and bound antibody was detected by chemiluminescence and exposure to x-ray film. See figure 55. This figure up-regulation of Bcl-2 when COS-7 cells were transiently transfected with pHM6 containing the 3' end of apoptosis-specific eIF-5A in the antisense orientation. The upper panel illustrates the Coomassie-blue-stained protein blot; the lower panel illustrates the corresponding Western blot. More Bcl-2 is detectable in cells transfected with pHM6-antisense 3' rF5A than in those mock transfected or transfected with pHM6-Sense rF5A. Also additionally, COS-7 cells were either mock transfected, transfected with pHM6-LacZ or transfected with pHM6-Sense rF5A (pHM6-Full length rat apoptosis-specific eIF-5A) and incubated for 40 hours. Five µg samples of protein extract from each sample were fractionated by SDS-PAGE, transferred to a PVDF membrane, and Western blotted with a monoclonal antibody that recognizes p53. Goat anti-mouse IgG conjugated to alkaline phosphatase was used as a secondary antibody, and bound antibody was detected a colorimetrically. See figure 56. This figure shows up-regulation of c-Myc when COS-7 cells were transiently transfected with pHM6 containing full-length rat apoptosis-specific eIF-5A in the sense orientation. The upper panel illustrates the Coomassie-blue-stained protein blot; the lower panel illustrates the corresponding Western blot. Higher levels of c-Myc is detected in cells transfected with pHM6-Sense rF5A than in those transfected with pHM6-LacZ or the mock control.

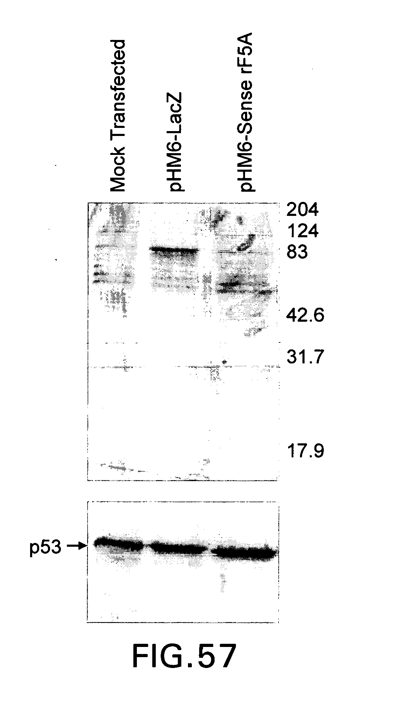

Finally, COS-7 cells were either mock transfected, transfected with pHM6-LacZ or transfected with pHM6-Sense rF5A (pHM6-Full length rat apoptosis-specific eIF-5A) and incubated for 40 hours. Five µg samples of protein extract from each sample were fractionated by SDS-PAGE, transferred to a PVDF membrane, and probed with a monoclonal antibody that recognizes p53. Corresponding protein blots were probed with anti-[HA]-peroxidase to determine the level of rat apoptosis-specific eIF-5A expression. Goat anti-mouse IgG conjugated to alkaline phosphatase was used as a secondary antibody, and bound antibody was detected by chemiluminescence. See figure 57. This figure shows up-regulation of p53 when COS-7 cells were transiently transfected with pHM6 containing full-length rat apoptosis-specific eIF-5A in the sense orientation. The upper panel illustrates the Coomassie-blue-stained protein blot; the lower panel illustrates the corresponding Western blot. Higher levels of p53 is detected in cells transfected with pHM6-Sense rF5A than in those transfected with pHM6-LacZ or the mock control.

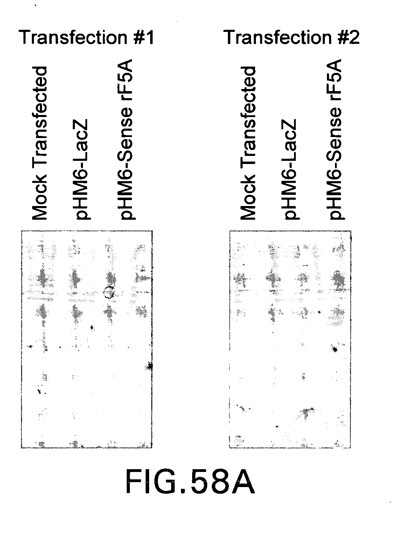

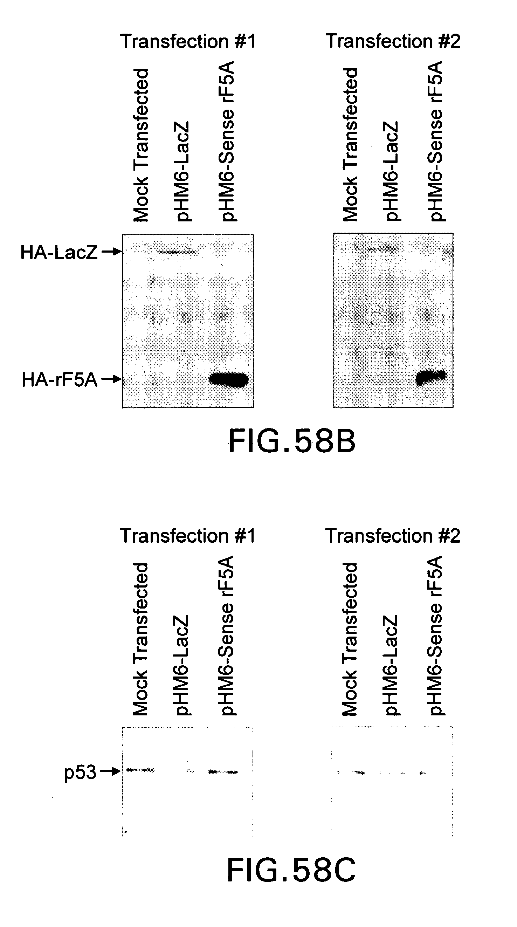

Figure 58-A-E illustrate the dependence of p53 upregulation upon the expression of pHM6-full length rat apoptosis-specificeIF-5A in COS-7 cells. More rat apoptosis-specificeIF-5A is detectable in the first transfection than in the second transfection. In the Western blot probed with anti-p53, the panel illustrates a corresponding Coomassie-blue-stained protein blot and the panel illustrates the Western blot with p53. For the first transfection, more p53 is detectable in cells transfected with pHM6-Sense rF5A than in those transfected with pHM6-LacZ or the mock control. For the second transfection in which there was less expression of rat apoptosis-specific eIF-5A, there was no detectable difference in levels of p53 between cells transfected with pHM6-Sense rF5A, pHM6-LacZ or the mock control.

### EXAMPLE 5

Heart tissue was exposed to normal oxygen levels and the expression levels apoptosis-specific eIF-5A and proliferating eIF-5A were measured. Later, the amount of oxygen delivered to the heart tissue was lowered, thus inducing hypoxia and ischemia, and ultimately, a heart attack in the heart tissue. The expression levels of apoptosis-specific eIF-5A and proliferating eIF-5A were measured and compared to the expression levels of the heart tissue before it was damaged by ischemia.

A slice of human heart tissue removed during valve replacement surgery was hooked up to electrodes. A small weight was attached to the heart tissue to ease in measuring the strength of the heart beats. The electrodes provided an electrical stimulus to get the tissue to start beating. The levels of gene expression for both apoptosis-specific eIF-5A and proliferating eIF-5A were measured in the heart tissue before ischemia was induced. See Figure 61. In the pre-ischemic heart tissue low levels both apoptosis-specific eIF-5A and proliferating eIF-5A were produced and their levels were in relative balance. During this time, oxygen and carbon dioxide were delivered in a buffer to the heart at 92.5% and 7.5 %, respectively. Later, the oxygen levels was reduced and the nitrogen levels was increased, to induce ischemia and finally a "heart attack." The heart tissue stopped beating. The oxygen levels were then returned to normal, the heart tissue was pulsed again with an electrical stimulus to start the heart beating again. After the "heart attack" the expression levels of apoptosis-specific eIF-5A and proliferating eIF-5A were again measured. This time, there was a significant increase in the level of expression of the apoptosis-specific eIF-5A levels, whereas the increase in the level of expression of proliferating eIF-5A was noticeably less. See Figure 61.

After the "heart attack" the heart did not beat as strong, as indicated by less compression/movement of the attached weight, thus indicating that the heart tissue cells were being killed rapidly due to the presence of apoptosis-specific eIF-5A.

### EXAMPLE 6

The following example provides cell culture conditions.

### Human Lamina Cribrosa and Astrocyte Culture

Paired human eyes were obtained within 48 hours *post mortem* from the Eye Bank of Canada, Ontario Division. Optic nerve heads (with attached pole) were removed and placed in Dulbecco's modified Eagle's medium (DMEM) supplemented with antibiotic/antimycotic, glutamine, and 10% FBS for 3 hours. The optic nerve head (ONH) button was retrieved from each tissue sample and minced with fine dissecting scissors into four small pieces. Explants were cultured in 12.5 cm² plastic culture flasks in DMEM medium. Growth was observed within one month in viable explants. Once the cells reached 90% confluence, they were trypsinized and subjected to differential subculturing to produce lamina cribrosa (LC) and astrocyte cell populations. Specifically, LC cells were subcultured in 25 cm² flasks in DMEM supplemented with gentamycin, glutamine, and 10% FBS, whereas astrocytes were expanded in 25cm² flasks containing EBM complete medium (Clonetics) with no FBS. FBS was added to astrocyte cultures following 10 days of subculture. Cells were maintained and subcultured as per this protocol.

Cell populations obtained by differential subculturing were characterized for identity and population purity using differential fluorescent antibody staining on 8 well culture slides. Cells were fixed in 10 % formalin solution and washed three times with Dulbecco's Phosphate Buffered Saline (DPBS). Following blocking with 2 % nonfat milk in DPBS, antibodies were diluted in 1% BSA in DPBS and applied to the cells in 6 of the wells. The remaining two wells were treated with only 1 % bovine serum albumin (BSA) solution and no primary antibody as controls. Cells were incubated with the primary antibodies for one hour at room temperature and then washed three times with DPBS. Appropriate secondary antibodies were diluted in 1 % BSA in DPBS, added to each well and incubated for 1 hour. Following washing with DPBS, the chambers separating the wells of the culture slide were removed from the slide, and the slide was immersed in double distilled water and then allowed to air-dry. Fluoromount (Vector Laboratories) was applied to each slide and overlayed by 22x60 mm coverglass slips.

Immunofluorescent staining was viewed under a fluorescent microscope with appropriate filters and compared to the control wells that were not treated with primary antibody. All primary antibodies were obtained from Sigma unless otherwise stated. All secondary antibodies were purchased from Molecular Probes. Primary antibodies used to identify LC cells were: anti-collagen I, anti-collagen IV, anti-laminin, anti-cellular fibronectin. Primary antibodies used to identify astrocytes were: anti-galactocerebroside (Chemicon International), anti-A2B5 (Chemicon International), anti-NCAM, anti-human Von willebrand Factor. Additional antibodies used for both cell populations included anti-glial fibrillary (GFAP) and anti-alpha-smooth muscle actin. Cell populations were determined to be comprised of LC cells if they stained positively for collagen I, collagen IV, laminin, cellular fibronectin, alpha smooth muscle actin and negatively for glial fibrillary (GFAP). Cell populations were determined to be comprised of astrocytes if they stained positively for NCAM, glial fibrillary (GFAP), and negatively for galactocerebroside, A2B5, human Von willebrand Factor, and alpha smooth muscle actin.

In this preliminary study, three sets of human eyes were used to initiate cultures. LC cell lines # 506, #517, and # 524 were established from the optic nerve heads of and 83-year old male, a 17-year old male, and a 26-year old female, respectively. All LC cell lines have been fully characterized and found to contain greater than 90 % LC cells.

### RKO Cell Culture

RKO (American Type Culture Collection CRL-2577), a human colon carcinoma cell line expressing wild-type p53, was used to test the antisense oligonucleotides for the ability to suppress apoptosis-specific eIF-5A protein expression. RKO were cultured in Minimum Essential Medium Eagle (MEM) with non-essential amino acids, Earle's salts, and L-glutamine. The culture media was supplemented with 10 % fetal bovine serum (FBS) and 100 units of penicillin/streptomycin. The cells were grown at 37 °C in a humidified environment of 5 % CO₂ and 95 % air. The cells were subcultured every 3 to 4 days by detaching the adherent cells with a solution of 0.25 % trypsin and 1 mM EDTA. The detached cells were dispensed at a split ratio of 1:10 to 1:12 into a new culture dish with fresh media.

### HepG2 Cell Culture

HepG2, a human hepatocellular carcinoma cell line, was used to test the ability of an antisense oligo directed against human apoptosis-specific eIF-5A to block production of TNF-α in response to treatment with IL-1β. HepG2 cells were cultured in DMEM supplemented with gentamycin, glutamine, and 10% FBS and grown at 37 °C in a humidified environment of 5 % CO₂ and 95 % air.

### EXAMPLE 7

### Induction ofApoptosis

Apoptosis was induced in RKO and lamina cribrosa cells using Actinomycin D, an RNA polymerase inhibitor, and camptothecin, a topoisomerase inhibitor, respectively. Actinomycin D was used at a concentration of 0.25 µg/ml and camptothecin was used at a concentration of 20, 40, or 50 µM. Apoptosis was also induced in lamina cribrosa cells using a combination of camptothecin (50 µM) and TNF-α (10 ng/ml). The combination of camptothecin and TNF-α was found to be more effective at inducing apoptosis than either camptothecin or TNF-α alone.

### Antisense Oligonucleotides

A set of three antisense oligonucleotides targeted against human apoptosis-specific eIF-5A were designed by, and purchased from, Molecula Research Labs. The sequence of the first antisense oligonucleotide targeted against human apoptosis-specific eIF-5A (#1) was 5' CCT GTC TCG AAG TCC AAG TC 3' (SEQ ID NO: 63). The sequence of the second antisense oligonucleotide targeted against human apoptosis-specific eIF-5A (#2) was 5' GGA CCT TGG CGT GGC CGT GC 3' (SEQ ID NO: 64). The sequence of the third antisense oligonucleotide targeted against human apoptosis-specific eIF-5A (#3) was 5' CTC GTA CCT CCC CGC TCT CC 3' (SEQ ID NO: 65). The control oligonucleotide had the sequence 5' CGT ACC GGT ACG GTT CCA GG 3' (SEQ ID NO: 66). A fluorescein isothiocyanate (FITC)-labeled antisense oligonucleotide (Molecula Research Labs) was used to monitor transfection efficiency and had the sequence 5' GGA CCT TGG CGT GGC CGT GCX 3' (SEQ ID NO: 67), where X is the FITC label. All antisense oligonucleotides were fully phosphorothioated.

### Transfection of Antisens Oligonucleotides

The ability of the apoptosis-specific eIF-5A antisense oligonucleotides to block apoptosis-specific eIF-5A protein expression was tested in RKO cells. RKO cells were transfected with antisense oligonucleotides using the transfection reagent, Oligofectamine (Invitrogen). Twenty four hours prior to transfection, the cells were split onto a 24 well plate at 157,000 per well in MEM media supplemented with 10 % FBS but lacking penicillin/streptomycin. Twenty four hours later the cells had generally reached a confluency of approximately 50%. RKO cells were either mock transfected, or transfected with 100 nM or 200 nM of antisense oligonucleotide. Transfection medium sufficient for one well of a 24 well plate was prepared by diluting 0, 1.25, or 2.5 µl of a 20 µM stock of antisense oligonucleotide with serum-free MEM to a final volume of 42.5 µl and incubating the mixture at room temperature for 15 minutes. 1.5 µl of Oligofectamine was diluted in 6 µl of serum-free MEM and incubated for 7.5 minutes at room temperature. After 5 minutes the diluted Oligofectamine mixture was added to the DNA mixture and incubated together at room temperature for 20 minutes. The cells were washed once with serum-free MEM before adding 200 µl of MEM to the cells and overlaying 50 µl of transfection medium. The cells were placed back in the growth chamber for 4 hours. After the incubation, 125 µl of MEM + 30 % FBS was added to the cells. The cells were then cultured for a further 48 hours, treated with 0.25 µg/ml Actinomycin D for 24 hours, and then cell extract was harvested for Western blot analysis.

Transfection of lamina cribrosa cells was also tested using 100 and 200 nM antisense oligonucleotide and Oligofectamine using the same procedure described for RKO cells. However, effective transfection of lamina cribrosa cells was achieved by simply adding antisense oligonucleotide, diluted from 1 µM to 10 µM in serum-free media, to the cells for 24 hours and thereafter replacing the media with fresh antisense oligonucleotides diluted in serum-containing media every 24 hours for a total of two to five days.

The efficiency of antisense oligonucleotide transfection was optimized and monitored by performing transfections with an FITC-labeled antisense oligonucleotide having the same sequence as apoptosis-specific eIF-5A antisense oligonucleotide # 2 (SEQ ID NO:64) but conjugated to FITC at the 3' end. RKO and lamina cribrosa cells were transfected with the FITC-labeled antisense oligonucleotide on an 8-well culture slide. Forty-eight hours later the cells were washed with PBS and fixed for 10 minutes in 3.7 % formaldehyde in PBS. The wells were removed and mounting media (Vectashield) was added, followed by a coverslip. The cells were then visualized under UV light on a fluorescent microscope nucleus using a fluorescein filter (Green H546, filter set 48915) and cells fluorescing bright green were determined to have taken up the oligonucleotide.

### Detection of Apoptosis

Following transfection of lamina cribosa cells with antisense oligonucleotides and induction of apoptosis with camptothecin, the percentage of cells undergoing apoptosis in cells treated with either control antisense oligonucleotide or antisense oligonucleotide apoptosis-specific eIF-5A SEQ ID NO:26 was determined. Two methods were used to detect apoptotic lamina cribosa cells - Hoescht staining and DeadEnd^{™} Fluorometric TUNEL. The nuclear stain, Hoescht, was used to label the nuclei of lamina cribosa cells in order to identify apoptotic cells based on morphological features such as nuclear fragmentation and condensation. A fixative, consisting of a 3:1 mixture of absolute methanol and glacial acetic acid, was prepared immediately before use. An equal volume of fixative was added to the media of cells growing on a culture slide and incubated for 2 minutes. The media/fixative mixture was removed from the cells and discarded and 1 ml of fixative was added to the cells. After 5 minutes the fixative was discarded and 1 ml of fresh fixative was added to the cells and incubated for 5 minutes. The fixative was discarded and the cells were air-dried for 4 minutes before adding 1 ml of Hoescht stain (0.5 µg/ml Hoescht 33258 in PBS). After a 10 minute incubation in the dark, the staining solution was discarded, the chambers separating the wells of the culture slide were removed, and the slide was washed 3 times for 1 minute with deionized water. After washing, a few drops of McIlvaine's buffer (0.021 M citric acid, 0.058 M Na₂HPO₄.7H₂O; pH 5.6) was added to the cells and overlaid with a coverslip. The stained cells were viewed under a fluorescent microscope using a UV filter. Cells with brightly stained or fragmented nuclei were scored as apoptotic. A minimum of 200 cells were counted per well.

The DeadEnd^{™} Fluorometric TUNEL (Promega) was used to detect the DNA fragmentation that is a characteristic feature of apoptotic cells. Following Hoescht staining, the culture slide was washed briefly with distilled water, and further washed by immersing the slide twice for 5 minutes in PBS (137mM NaCl, 2.68mM KCl, 1.47mM KH₂PO₄, 8.1mM Na₂HPO₄), blotting the slide on paper towel between washes. _The cells were permeabilized by immersing them in 0.2 % Triton X-100 in PBS for 5 minutes. The cells were then washed again by immersing the slide twice for 5 minutes in PBS and blotting the slide on paper towel between washes. 25 µl of equilibration buffer [200 mM potassium cacodylate (pH 6.6), 25 mM Tris-HCl (pH 6.6), 0.2 mM dithiothreitol, 0.25 mg/ml bovine serum albumin, and 2.5 mM cobalt chloride] was added per well and incubated for 5 to 10 minutes. During equilibration, 30 µl of reaction mixture was prepared for each well by mixing in a ratio of 45:5:1, respectively, equilibration buffer, nucleotide mix [50 µM fluorescein-12-dUTP, 100 µM dATP, 10 mM Tris-HCl (pH 7.6), and 1 mM EDTA], and terminal deoxynucleotidyl transferase enzyme (Tdt, 25 U/µl). After the incubation in equilibration buffer, 30 µl of reaction mixture was added per well and overlayed with a coverslip. The reaction was allowed to proceed in the dark at 37°C for 1 hour. The reaction was terminated by immersing the slide in 2 X SSC [0.3 M NaCl, and 30mM sodium citrate (pH 7.0)] and incubating for 15 minutes. The slide was then washed by immersion in PBS three times for 5 minutes. The PBS was removed by sponging around the wells with a Kim wipe, a drop of mounting media (Oncogene research project, JA1750-4ML) was added to each well, and the slide was overlayed with a coverslip. The cells were viewed under a fluorescent microscope using a UV filter (UV-G 365, filter set 487902) in order to count the Hoescht-stained nuclei. Any cells with brightly stained or fragmented nuclei were scored as apoptotic. Using the same field of view, the cells were then viewed using a fluorescein filter (Green H546, filter set 48915) and any nuclei fluorescing bright green were scored as apoptotic. The percentage of apoptotic cells in the field of view was calculated by dividing the number of bright green nuclei counted using the fluorescein filter by the total number of nuclei counted under the UV filter. A minimum of 200 cells were counted per well.

Figures 78-82 depict the results of these studies. The percentage of apoptotic cells in samples having been transfected with apoptosis-specific eIF-5A is clearly much less than seen in cells having been transfected with the control oligonucleotide.

### Protein Extraction and Western Blotting

Protein from transfected RKO cells was harvested for Western blot analysis by washing the cells with PBS, adding 40 µl of hot lysis buffer [0.5% SDS, 1 mM dithiothreitol, 50 mM Tris-HCl (pH 8.0)] per well. The cells were scraped and the resulting extract was transferred to a microfuge tube, boiled for 5 minutes, and stored at -20°C. The protein was quantitated using the Bio-Rad Protein Assay (Bio-Rad) according to the manufacturer's instructions.

For Western blotting 5 µg of total protein was separated on a 12 % SDS-polyacrylamide gel. The separated proteins were transferred to a polyvinylidene difluoride membrane. The membrane was then incubated for one hour in blocking solution (5 % skim milk powder in PBS) and washed three times for 15 minutes in 0.05 % Tween-20/PBS. The membrane was stored overnight in PBS-T at 4 °C. After being warmed to room temperature the next day, the membrane was blocked for 30 seconds in 1 µg/ml polyvinyl alcohol. The membrane was rinsed 5 times in deionized water and then blocked for 30 minutes in a solution of 5 % milk in 0.025 % Tween-20/PBS. The primary antibody was preincubated for 30 minutes in a solution of 5 % milk in 0.025% Tween-20/PBS prior to incubation with the membrane.

Several primary antibodies were used. A monoclonal antibody from Oncogene which recognizes p53 (Ab-6) and a polyclonal antibody directed against a synthetic peptide (amino-CRLPEGDLGKEIEQKYD-carboxy) (SEQ ID NO:68) homologous to the c-terminal end of human apoptosis-specific eIF-5A that was raised in chickens (Gallus Immunotech). An anti-β-actin antibody (Oncogene) was also used to demonstrate equal loading of protein. The monoclonal antibody to p53 was used at a dilution of 0.05 µg/ml, the antibody against apoptosis-specific eIF-5A was used at a dilution of 1:1000, and the antibody against actin was used at a dilution of 1:20,000. After incubation with primary antibody for 60 to 90 minutes, the membrane was washed 3 times for 15 minutes in 0.05% Tween-20/PBS. Secondary antibody was then diluted in 1 % milk in 0.025 % Tween-20/PBS and incubated with the membrane for 60 to 90 minutes. When p53 (Ab-6) was used as the primary antibody, the secondary antibody used was a rabbit anti-mouse IgG conjugated to peroxidase (Sigma) at a dilution of 1:5000. When anti-apoptosis-specific eIF-5A was used as the primary antibody, a rabbit anti-chicken IgY conjugated to peroxidase (Gallus Immunotech) was used at a dilution of 1:5000. The secondary antibody used with actin was a goat anti-mouse IgM conjugated to peroxidase (Calbiochem) used at a dilution of 1:5000. After incubation with the secondary antibody, the membrane was washed 3 times in PBS-T.

The ECL Plus Western blotting detection kit (Amersham Pharmacia Biotech) was used to detect peroxidase-conjugated bound antibodies. In brief, the membrane was lightly blotted dry and then incubated in the dark with a 40:1 mix of reagent A and reagent B for 5 minutes. The membrane was blotted dry, placed between sheets of acetate, and exposed to X-ray film for time periods varying from 10 seconds to 30 minutes. The membrane was stripped by submerging the membrane in stripping buffer [100 mM 2-Mercaptoethanol, 2 % SDS, and 62.5 mM Tris-HCl (pH 6.7)], and incubating at 50°C for 30 minutes. The membrane was then rinsed in deionized water and washed twice for 10 minutes in large volumes of 0.05 % Tween-20/PBS. Membranes were stripped and re-blotted up to three times.

### EXAMPLE 8

### Construction of siRNA

Small inhibitory RNAs (siRNAs) directed against human apoptosis-specific eIF-5A were used to specifically suppress expression of apoptosis-specific eIF-5A in RKO and lamina cribrosa cells. Six siRNAs were generated by *in vitro* transcription using the *Silencer*^{™} siRNA Construction Kit (Ambion Inc.). Four siRNAs were generated against human apoptosis-specific eIF-5A (siRNAs # 1 to # 4)(SEQ ID NO:30-33). Two siRNAs were used as controls; an siRNA directed against GAPDH provided in the kit, and an siRNA (siRNA # 5)(SEQ ID NO: 34) which had the reverse sequence of the apoptosis-specific eIF-5A siRNA # 1 (SEQ ID NO:30) but does not itself target apoptosis-specific eIF-5A. The siRNAs were generated according to the manufacturer's protocol. In brief, DNA oligonucleotides encoding the desired siRNA strands were used as templates for T7 RNA polymerase to generate individual strands of the siRNA following annealing of a T7 promoter primer and a fill-in reaction with Klenow fragment. Following transcription reactions for both the sense and antisense strands, the reactions were combined and the two siRNA strands were annealed, treated with DNase and RNase, and then column purified. The sequence of the DNA oligonucleotides (T7 primer annealing site underlined) used to generate the siRNAs were: siRNA # 1 antisense 5' AAAGGAATGACTTCCAGCTGACCTGTCTC 3' (SEQ ID NO:69) and siRNA # 1 sense 5' AATCAGCTGGAAGTCATTCCTCCTGTCTC 3' (SEQ ID NO:70); siRNA # 2 antisense 5' AAGATCGTCGAGATGTCTACTCCTGTCTC 3' (SEQ ID NO:71) and siRNA # 2 sense 5' AAAGTAGACATCTCGACGATCCCTGTCTC 3' (SEQ ID NO:72); siRNA # 3 antisense 5' AAGGTCCATCTGGTTGGTATTCCTGTCTC 3' (SEQ ID NO:73) and siRNA # 3 sense 5' AAAATACCAACCAGATGGACCCCTGTCTC 3' (SEQ ID NO:74) siRNA # 4 antisense 5' AAGCTGGACTCCTCCTACACACCTGTCTC 3' (SEQ ID NO:75) and siRNA # 4 sense 5' AATGTGTAGGAGGAGTCCAGCCCTGTCTC 3' (SEQ ID NO:76); siRNA # 5 antisense 5' AAAGTCGACCTTCAGTAAGGACCTGTCTC 3' (SEQ ID NO:77) and siRNA # 5 sense 5' AATCCTTACTGAAGGTCGACTCCTGTCTC 3' (SEQ ID NO:78).

The *Silencer*^{™} siRNA Labeling Kit - FAM (Ambion) was used to label GAPDH siRNA with FAM in order to monitor the uptake of siRNA into RKO and lamina cribrosa cells. After transfection on 8-well culture slides, cells were washed with PBS and fixed for 10 minutes in 3.7 % formaldehyde in PBS. The wells were removed and mounting media (Vectashield) was added, followed by a coverslip. Uptake of the FAM-labeled siRNA was visualized under a fluorescent microscope under UV light using a fluorescein filter. The GAPDH siRNA was labeled according to the manufacturer's protocol.

### Transfection of siRNA

RKO cells and lamina cribrosa cells were transfected with siRNA using the same transfection protocol. RKO cells were seeded the day before transfection onto 8-well culture slides or 24-well plates at a density of 46,000 and 105,800 cells per well, respectively. Lamina cribrosa cells were transfected when cell confluence was at 40 to 70 % and were generally seeded onto 8-well culture slides at 7500 to 10,000 cells per well three days prior to transfection. Transfection medium sufficient for one well of an 8-well culture slide was prepared by diluting 25.5 pmoles of siRNA stock to a final volume of 21.2 µl in Opti-Mem (Sigma). 0.425 µl of Lipofectamine 2000 was diluted to a final volume of 21.2 µl in Opti-Mem and incubated for 7 to 10 minutes at room temperature. The diluted Lipofectamine 2000 mixture was then added to the diluted siRNA mixture and incubated together at room temperature for 20 to 3 0 minutes. The cells were washed once with serum-free media before adding 135 µl of serum-free media to the cells and overlaying the 42.4 µl of transfection medium. The cells were placed back in the growth chamber for 4 hours. After the incubation, 65 µl of serum-free media + 30 % FBS was added to the cells. Transfection of siRNA into cells to be used for Western blot analysis were performed in 24-well plates using the same conditions as the transfections in 8-well slides except that the volumes were increased by 2.3 fold.

Following transfection, RKO and lamina cribrosa cells were incubated for 72 hours prior to collection of cellular extract for Western blot analysis. In order to determine the effectiveness of the siRNAs directed against apoptosis-specific eIF-5A to block apoptosis, lamina cribrosa cells were treated with 50 µM of camptothecin (Sigma) and 10 ng/ml of TNF-α (Leinco Technologies) to induce apoptosis either 48 or 72 hours after transfection. The cells were stained with Hoescht either 24 or 48 hours later in order to determine the percentage of cells undergoing apoptosis.

### EXAMPLE 9

### Quantification of HepG2 TNF-α Production

HepG2 cells were plated at 20,000 cells per well onto 48-well plates. Seventy two hours later the media was removed and fresh media containing either 2.5 µM control antisense oligonucleotide or 2.5 µM antisense oligonucleotide apoptosis-specific eIF-5A # 2 was added to the cells. Fresh media containing antisense oligonucleotides was added after twenty four hours. After a total of 48 hours incubation with the oligonucleotides, the media was replaced with media containing interleukin 1β (IL-1β, 1000 pg/ml; Leinco Technologies) and incubated for 6 hours. The media was collected and frozen (-20°C) for TNF-α quantification. Additional parallel incubations with untreated cells (without antisense oligonucleotide and IL-1β) and cells treated with only IL-1β were used for controls. All treatments were done in duplicate. TNF-α released into the media was measured by ELISA assays (Assay Designs Inc.) according to the manufacturer's protocol.

### EXAMPLE 10

The following experiments show that antisense apoptosis-specific eIF-5A nucleotides were able to inhibit expression of apoptosis-specific eIF-5A as well as p53.

RKO cells were either left untransfected, mock transfected, or transfected with 200 nM of antisense oligonucleotides apoptosis-specific eIF-5A # 1, # 2, or # 3 (SEQ ID NO: 25, 26, and 27). RKO cells were also transfected with 100 nM of antisense oligonucleotide apoptosis-specific eIF-5A # 2 (SEQ ID NO:26). Forty-eight hours after transfection, the cells were treated with 0.25 µg/ml Actinomycin D. Twenty-four hours later, the cell extract was harvested and 5 µg of protein from each sample was separated on an SDS-PAGE gel, transferred to a PVDF membrane, and Western blotted with an antibody against apoptosis-specific eIF-5A. After chemiluminescent detection, the membrane was stripped and reprobed with an antibody against p53. After chemiluminescent detection, the membrane was stripped again and reprobed with an antibody against actin. Figure 42 which shows the levels of protein produced by RKO cells after being treated with antisense oligo 1, 2 and 3 (to apoptosis-specific eIF-5a)(SEQ ID NO:25, 26, and 27, respectively). The RKO cells produced less apoptosis-specific eIF-5A as well as less p53 after having been transfected with the antisense apoptosis-specific eIF-5A nucleotides.

### EXAMPLE 11

The following experiments show that apoptosis-specific eIF-5A nucleotides were able to reduce apoptosis.

In one experiment, the lamina cribrosa cell line # 506 was either (A) transfected with 100 nM of FITC-labeled antisense oligonucleotide using Oligofectamine transfection reagent or (B) transfected with 10 µM of naked FITC-labeled antisense oligonucleotide diluted directly in serum-free media. After 24 hours fresh media containing 10 % FBS and fresh antisense oligonucleotide diluted to 10 µM was added to the cells. The cells, (A) and (B), were fixed after a total of 48 hours and visualized on a fluorescent microscope under UV light using a fluorescein filter. Figure 77A and B show uptake of the flourescently labeled antisense oligonucleotide.

In another experiment, the lamina cribrosa cell line # 506 was transfected with 10 µM of either the control antisense oligonucleotide or antisense oligonucleotide apoptosis-specific eIF-5A # 2 (SEQ ID NO:26) for a total of 4 days. Forty-eight hours after beginning antisense oligonucleotide treatment, the cells were treated with either 20 µM or 40 µM camptothecin for 48 hours. Antisense oligonucleotide and camptothecin-containing media was changed daily. The percentage of apoptotic cells was determined by labeling the cells with Hoescht and TUNEL. See figure 78.

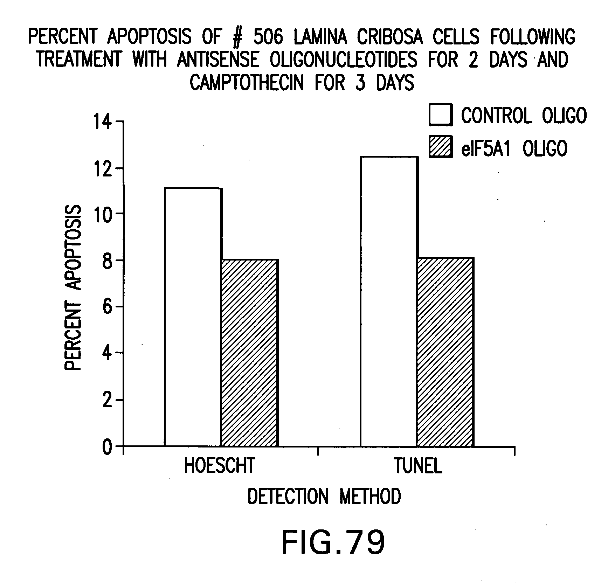

In another experiment, the lamina cribrosa cell line # 506 was transfected with 10 µM of either the control antisense oligonucleotide or antisense oligonucleotide apoptosis-specific eIF-5A # 2 (SEQ ID NO:26). Twenty-four hours later the media was changed and fresh antisense oligonucleotides were added. Forty-eight hours after beginning antisense oligonucleotide treatment, the antisense-oligonucleotides were removed and the cells were treated with 20 µM camptothecin for 3 days. The camptothecin-containing media was changed daily. The percentage of apoptotic cells was determined by labeling the cells with Hoescht and TUNEL. See Figure 79.

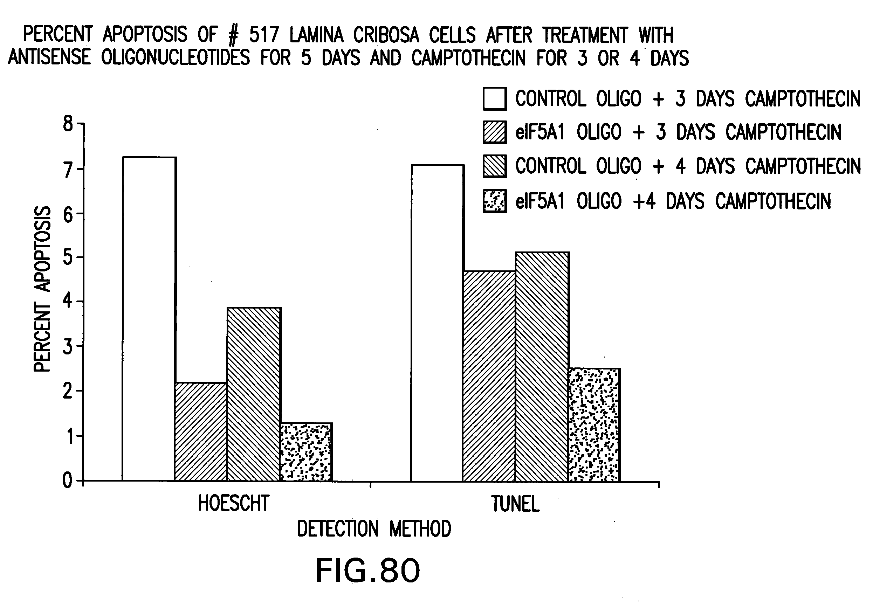

In yet another experiment, the lamina cribrosa cell line # 517 was transfected with 1 µM of either the control antisense oligonucleotide or antisense oligonucleotide apoptosis-specific eIF-5A # 2 (SEQ ID NO:26) for a total of five days. Forty-eight hours after beginning antisense oligonucleotide treatment, the cells were treated with 20 µM camptothecin for either 3 or 4 days. Antisense oligonucleotide and camptothecin-containing media was changed daily. The percentage of apoptotic cells was determined by labeling the cells with Hoescht and TUNEL. See Figure 80.

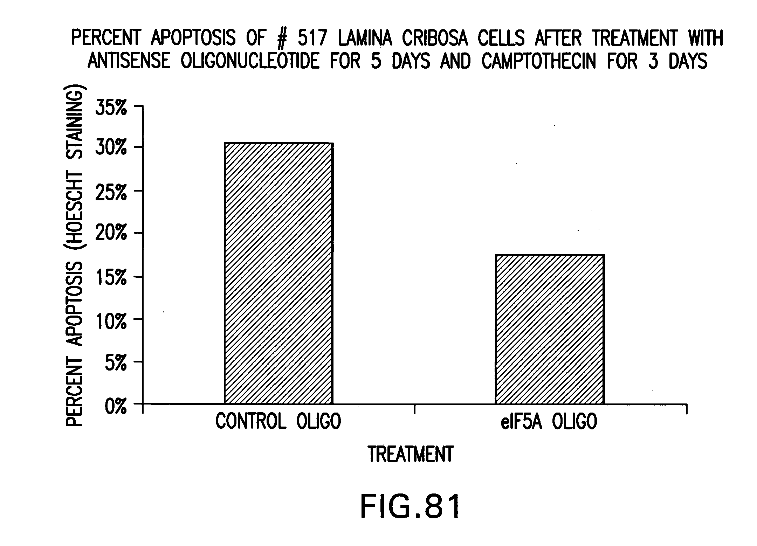

In another experiment, the lamina cribrosa cell line #517 was transfected with 2.5 µM of either the control antisense oligonucleotide or antisense oligonucleotide apoptosis-specific eIF-5A # 2 (SEQ ID NO:26) for a total of five days. Forty-eight hours after beginning antisense oligonucleotide treatment, the cells were treated with 40 µM camptothecin for 3 days. Antisense oligonucleotide and camptothecin-containing media was changed daily. The percentage of apoptotic cells was determined by labeling the cells with Hoescht. See figure 81.

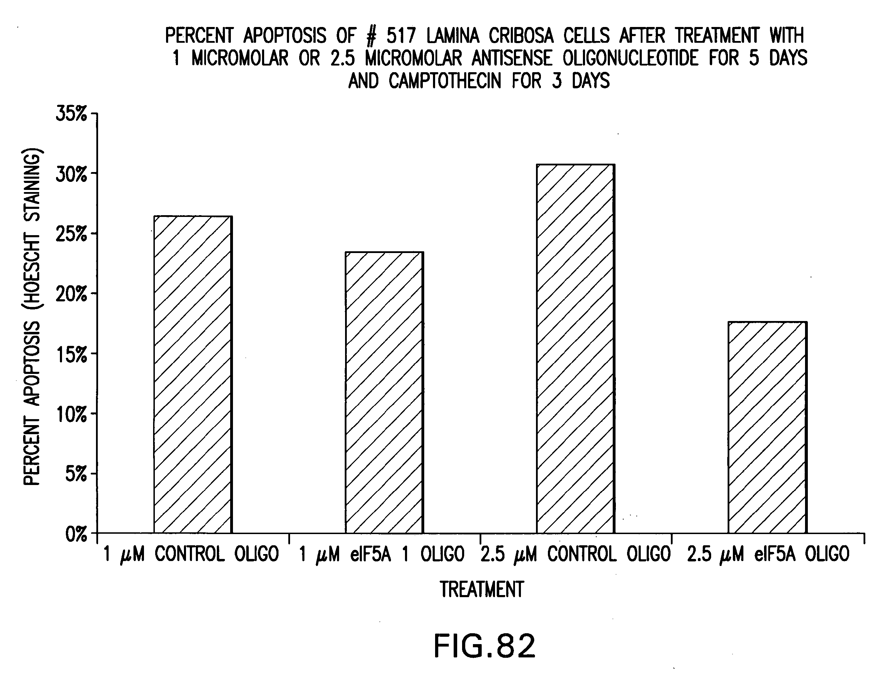

In another experiment, the lamina cribrosa cell line # 517 was transfected with either 1 µM or 2.5 µM of either the control antisense oligonucleotide or antisense oligonucleotide apoptosis-specific eIF-5A # 2 (SEQ ID NO:26) for a total of five days. Forty-eight hours after beginning antisense oligonucleotide treatment, the cells were treated with 40 µM camptothecin for 3 days. Antisense oligonucleotide and camptothecin-containing media was changed daily. The percentage of apoptotic cells was determined by labeling the cells with Hoescht. See figure 82.

In another experiment, the lamina cribrosa cell line #517 was left either untreated, or was treated with 10 ng/ml TNF-α, 50 µM camptothecin, or 10 ng/ml TNF-α and 50 µM camptothecin. The percentage of apoptotic cells was determined by labeling the cells with Hoescht. See figure 83.

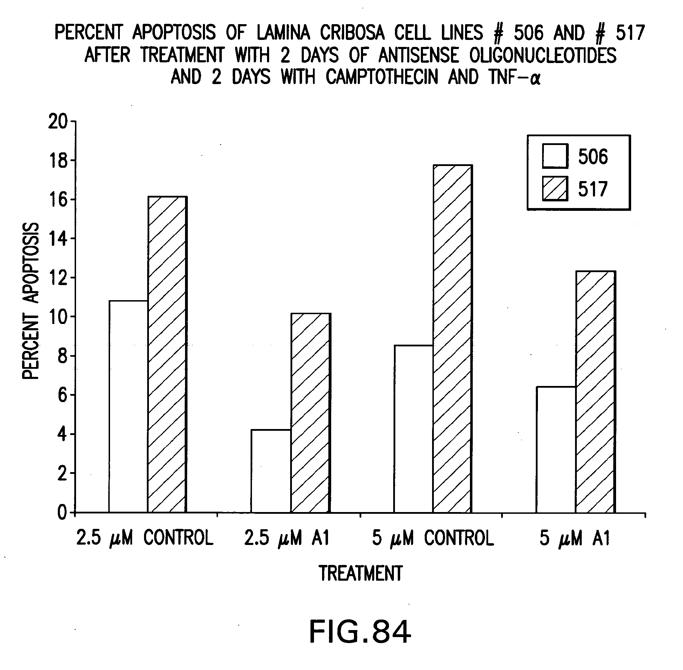

In another experiment, the lamina cribrosa cell lines # 506 and # 517 were transfected with either 2.5 µM or 5 µM of either the control antisense oligonucleotide or antisense oligonucleotide apoptosis-specific eIF-5A # 2 (SEQ ID NO:26) for a total of two days. Fresh media containing antisense oligonucleotides was added after 24 hours. Forty-eight hours after beginning antisense oligonucleotide treatment, the cells were treated with 50 µM camptothecin and 10 ng/ml TNF-α for 2 days. The percentage of apoptotic cells was determined by labeling the cells with Hoescht. See figure 84.

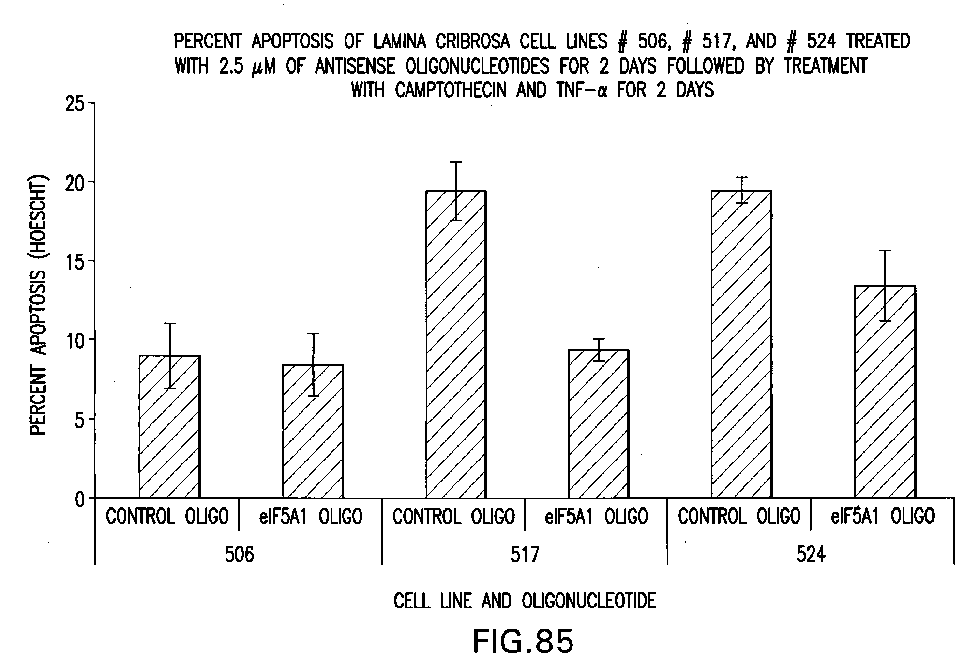

In another experiment, the lamina cribrosa cell lines # 506, # 517, and # 524 were transfected with 2.5 µM of either the control antisense oligonucleotide or antisense oligonucleotide apoptosis-specific eIF-5A # 2 (SEQ ID NO:26) for a total of two days. Fresh media containing antisense oligonucleotides was added after 24 hours. Forty-eight hours after beginning antisense oligonucleotide treatment, the cells were treated with 50 µM camptothecin and 10 ng/ml TNF-α for 2 days. The percentage of apoptotic cells was determined by labeling the cells with Hoescht. See figure 85.

### EXAMPLE 12

The following experiments show that cells transfected with siRNAs targeted against apoptosis-specific eIF-5A expressed less apoptosis-specific eIF-5A. The experiments also show that siRNAs targeted against apoptosis-specific eIF-5A were able to reduce apoptosis.

In one experiment, the lamina cribrosa cell line # 517 was transfected with 100 nM of FAM-labeled siRNA using Lipofectamine 2000 transfection reagent either with serum (A) or without serum (B) during transfection. The cells, (A) and (B), were fixed after a total of 24 hours and visualized on a fluorescent microscope under UV light using a fluorescein filter. See figure 86.

In another experiment, RKO cells were transfected with 100 nM of siRNA either in the presence or absence of serum during the transfection. Six siRNAs were transfected, two control siRNAs (siRNA # 5 (SEQ ID NO:34) and one targeted against GAPDH) and four targeted against apoptosis-specific eIF-5A (siRNA # 1 to # 4)(SEQ ID NO:30-33). Seventy-two hours after transfection, the cell extract was harvested and 5 µg of protein from each sample was separated on an SDS-PAGE gel, transferred to a PVDF membrane, and Western blotted with an antibody against apoptosis-specific eIF-5A. After chemiluminescent detection, the membrane was stripped and re-probed with an antibody against bcl-2. After chemiluminescent detection, the membrane was stripped again and re-probed with an antibody against actin. See figure 98.

In another experiment, lamina Cribrosa cell lines #506 and #517 were transfected with 100 nM of siRNA. Six siRNAs were transfected, two control siRNAs (siRNA # 5 (SEQ ID NO:34) and one targeted against GAPDH) and four targeted against apoptosis-specific eIF-5A (siRNA # 1 to # 4)(SEQ ID NO:30-33). Seventy-two hours after transfection, the cell extract was harvested and 5 µg of protein from each sample was separated on an SDS-PAGE gel, transferred to a PVDF membrane, and Western blotted with an antibody against apoptosis-specific eIF-5A. After chemiluminescent detection, the membrane was stripped and re-probed with an antibody against actin. See figure 99.

In another experiment, the lamina cribrosa cell line # 506 was transfected with 100 nm of siRNA. Six siRNAs were transfected, two control siRNAs (siRNA # 5 (SEQ ID NO:34) and one targeted against GAPDH) and four targeted against apoptosis-specific eIF-5A (siRNA # 1 to # 4)(SEQ ID NO:30-33). Forty-eight hours after transfection, the media was replaced with media containing 50 µM camptothecin and 10 ng/ml TNF-α. Twenty-four hours later, the percentage of apoptotic cells was determined by labeling the cells with Hoescht. See figure 87.

In another experiment, the lamina cribrosa cell line # 506 was transfected with 100 nm of siRNA. Six siRNAs were transfected, two control siRNAs (siRNA # 5 (SEQ ID NO:34) and one targeted against GAPDH) and four targeted against apoptosis-specific eIF-5A (siRNA # 1 to # 4)(SEQ ID NO:30-33). Seventy-two hours after transfection, the media was replaced with media containing 50 µM camptothecin and 10 ng/ml TNF-α. Twenty-four hours later, the percentage of apoptotic cells was determined by labeling the cells with Hoescht. See figure 88.

In another experiment, the lamina cribrosa cell line # 506 was either left untransfected or was transfected with 100 nm of siRNA. Six siRNAs were transfected, two control siRNAs (siRNA # 5 (SEQ ID NO:34) and one targeted against GAPDH) and four targeted against apoptosis-specific eIF-5A (siRNA # 1 to # 4)(SEQ ID NO:30-33). Seventy-two hours after transfection, the media was replaced with media containing 50 µM camptothecin and 10 ng/ml TNF-α. Fresh media was also added to the untransfected, untreated control cells. Forty-eight hours later, the percentage of apoptotic cells was determined by labeling the cells with Hoescht. See figure 89.

Photographs of Hoescht-stained lamina cribrosa cell line # 506 transfected with siRNA and treated with camptothecin and TNF-α from the experiment described in figure 67 and example 13 are provided in figure 90.

### EXAMPLE 13

This example shows that treating a human cell line with antisense oligonucleotides directed against apoptosis-specific eIF-5A causes the cells to produce less TNF-α.

HepG2 cells were treated with 2.5 µM of either the control antisense oligonucleotide or antisense oligonucleotide apoptosis-specific eIF-5A # 2 for a total of two days. Fresh media containing antisense oligonucleotides was added after 24 hours. Additional cells were left untreated for two days. Forty-eight hours after the beginning of treatment, the cells were treated with IL-1β (1000 pg/ml) in fresh media for 6 hours. At the end of the experiment, the media was collected and frozen (-20°C) for TNF-α quantification. TNF-α released into the media was measured using ELISA assays purchased from Assay Designs Inc. See figure 100. Cells that were transfected with antisense oligonucleotides of apoptosis-specific eIF-5A produced less TNF-α.

### EXAMPLE 14

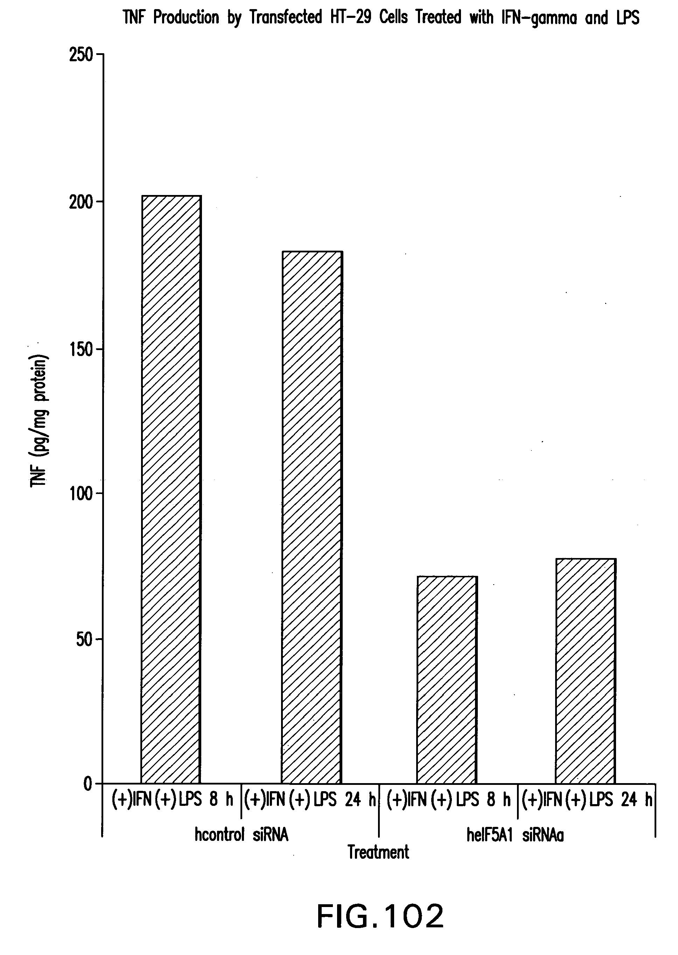

HT-29 cells (human colon adenocarcinoma) were transfected with either an siRNA against apoptosis-specific eIF-5A or with a control siRNA with the reverse sequence. The siRNA used is as follows:
Position 690 (3'UTR) % G/C=48
   5' AAGCUGGACUCCUCCUACACA 3' (SEQ ID NO: 79)
The control siRNA used is as follows:
   % G/C=39
   5' AAACACAUCCUCCUCAGGUCG 3' (SEQ ID NO: 80)
After 48 hours the cells were treated with interferon-gamma (IFN-gamma) for 16 hours. After 16 hours the cells were washed with fresh media and treated with lipopolysaccharide (LPS) for 8 or 24 hours. At each time point (8 or 24 hours) the cell culture media was removed from the cells, frozen, and the TNF-alpha present in the media was quantitated by ELISA. The cell lysate was also harvested, quantitated for protein, and used to adjust the TNF-alpha values to pg/mg protein (to adjust for differences in cell number in different wells). The results of the Western blot and Elisa are provided in Figures 101A and B. Figure 102 are the results of the same experiment except the cells were at a higher density.

### EXAMPLE 15

### Tissue culture conditions of U-937 cell line

U-937 is a human monocyte cell line that grows in suspension and will become adherent and differentiate into macrophages upon stimulation with PMA (ATCC Number CRL-1593.2)(cells not obtained directly from ATCC). Cells were maintained in RPMI 1640 media with 2 mM L-glutamine, 1.5 g/L sodium bicarbonate, 4.5 g/L glucose, 10 mM HEPES, 1.0 mM sodium pyruvate and 10% fetal bovine serum in a 37°C CO₂ (5%) incubator. Cells were split into fresh media (1:4 or 1:5 split ratio) twice a week and the cell density was always kept between 105 and 2 x 106 cells/ml. Cells were cultured in suspension in tissue culture-treated plastic T-25 flasks and experiments were conducted in 24-well plates.

### Time course experiment

Two days before the start of an experiment, the cell density was adjusted to 3 x 105 cells/ml media. On the day of the experiment, the cells were harvested in log phase. The cell suspension was transferred to 15ml tubes and centrifuged at 400 x g for 10 mins at room temperature. The supernatant was aspirated and the cell pellet was washed/resuspended with fresh media. The cells were again centrifuged at 400 x g for 10 mins, the supernatant was aspirated, and the cell pellet was finally resuspended in fresh media. Equal volumes of cell suspension and trypan blue solution (0.4% trypan blue dye in PBS) were mixed and the live cells were counted using a haemocytometer and a microscope. The cells were diluted to 4 x 10⁵ cells/ml.

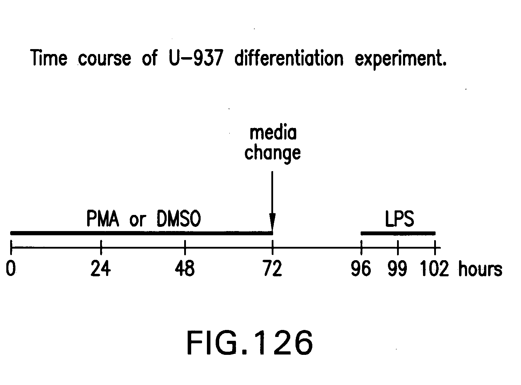

A 24-well plate was prepared by adding either PMA or DMSO (vehicle control) to each well. 1ml of cell suspension was added to each well so that each well contained 400,000 cells, 0.1 % DMSO +/- 162 nM PMA. The cells were maintained in a 37°C CO₂ (5%) incubator. Separate wells of cells were harvested at times 0, 24, 48, 72, 96, 99 and 102 h. See Figure 126 for a summary of the experimental time points and additions.

The media was changed at 72 h. Since some cells were adherent and others were in suspension, care was taken to avoid disrupting the adherent cells. The media from each well was carefully transferred into corresponding microcentrifuge tubes and the tubes were centrifuged at 14,000 xg for 3 min. The tubes were aspirated, the cell pellets were resuspended in fresh media (1 ml, (-) DMSO, (-) PMA), and returned to their original wells. The cells become quiescent in this fresh media without PMA. At 96 h, LPS (100 ng/ml) was added and cells were harvested at 3h (99h) and 6h (102h) later.

At the time points, the suspension cells and media were transferred from each well into microcentrifuge tubes. The cells were pelleted at 14,000 xg for 3 min. The media (supernatant) was transferred to clean tubes and stored (-20°C) for ELISA/cytokine analysis. The cells remaining in the wells were washed with PBS (1ml, 37°C) and this PBS was also used to wash the cell pellets in the corresponding microcentrifuge tubes. The cells were pelleted again at 14,000 xg for 3 min. The cells were lysed with boiling lysis buffer (50 mM Tris pH 7.4 and 2% SDS). The adherent cells and the suspension cells from each well were pooled. The samples were boiled and then stored at -20°C.

### Western Blotting

The protein concentration in each cell sample was determined by the BCA (bicinchoninic acid) method using BSA (bovine serum albumin) as the standard protein. Protein samples (5 µg total protein) were separated by 12% SDS-PAGE electrophoresis and transferred to PVDF membranes. The membranes were blocked with polyvinyl alcohol (1 µg/ml, 30 sec) and with 5% skim milk in PBS-t (1 h). The membranes were probed with a mouse monoclonal antibody raised against human eIF-5A (BD Biosciences cat # 611976; 1:20,000 in 5% skim milk, 1 h). The membranes were washed 3x10 mins PBS-t. The secondary antibody was a horseradish peroxidase-conjugated antimouse antibody (Sigma, 1:5000 in 1% skim milk, 1 h). The membranes were washed 3x10 mins PBS-t. The protein bands were visualized by chemiluminescence (ECL detection system, Amersham Pharmacia Biotech).

To demonstrate that similar amounts of protein were loaded on each gel lane, the membranes were stripped and reprobed for actin. Membranes were stripped (100 mM 2-mercaptoethanol, 2% SDS, 62.5 mM Tris-HCl pH 6.7; 50°C for 30 mins), washed, and then blocked as above. The membranes were probed with actin primary antibody (actin monoclonal antibody made in mouse; Oncogene, Ab-1; 1:20,000 in 5% skim milk). The secondary antibody, washing, and detection were the same as above.

Figure 127 shows that apoptosis-specific eIF-5A is upregulated during monocyte (U-397) differentiation and subsequent TNF-α secretion.

### EXAMPLE 16: Suppression of I1-8 production in response to interferon gamma by apoptosis-specific eIF-5A siRNA

HT-29 (human colon adenocarcinoma) cells were transfected with siRNA directed to apoptosis-specific eIF-5A. Approximately 48 hours after transfection the media was changed so that some of the test samples had media with interferon gamma and some of the samples had media without interferon gamma. 16 hours after interferon gamma addition, the cells were washed, and the media, with or without TNF-alpha, was placed on the cells. The media (used for ELISA detection of IL-8) and the cell lysate was harvested 8 or 24 hours later.

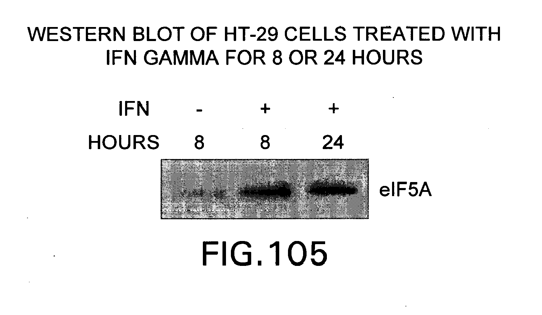

Figures 103 and 106 show that IL-8 is produced in response to TNF-alpha as well as in response to interferon. Priming the cells with interferon gamma prior to TNF treatment causes the cells to produce more IL-8 than either treatment alone. This may be due to the known upregulation of the TNF receptor 1 in response to interferon, so 'priming' the cells with interferon allows them to respond to TNF better since the cells have more receptors. siRNA against apoptosis-specific eIF-5A had no effect on IL-8 production in response to TNF alone (previous experiment) however, the siRNA blocked almost all IL-8 produced in response to interferon as well as a significant amount of the IL-8 produced as a result of the combined treatment of interferon and TNF. These results show that the by using siRNAs directed against apoptosis-specific eIF-5A, the inventors have the interferon signaling pathway leading to IL-8, but not the TNF pathway. Figure 105 is a western showing up-regulation (4 fold at 8 hours) of apoptosis-specific eIF-5A in response to interferon gamma in HT-29 cells.

### EXAMPLE 17

### Human Lamina Cribrosa Culture

Paired human eyes were obtained within 48 hours *post mortem* from the Eye Bank of Canada, Ontario Division. Optic nerve heads (with attached pole) were removed and placed in Dulbecco's modified Eagle's medium (DMEM) supplemented with antibiotic/antimycotic, glutamine, and 10% FBS for 3 hours. The optic nerve head (ONH) button was retrieved from each tissue sample and minced with fine dissecting scissors into four small pieces. Explants were cultured in 12.5 cm² plastic culture flasks in DMEM medium. Growth was observed within one month in viable explants. Once the cells reached 90% confluence, they were trypsinized and subjected to differential subculturing to produce lamina cribrosa (LC) and astrocyte cell populations. LC cells were enriched by subculture in 25 cm² flasks in DMEM supplemented with gentamycin, glutamine, and 10% FBS. Cells were maintained and subcultured as per this protocol.

The identity and population purity of cells populations obtained by differential subculturing was characterized using differential fluorescent antibody staining on 8 well culture slides. Cells were fixed in 10 % formalin solution and washed three times with Dulbecco's Phosphate Buffered Saline (DPBS). Following blocking with 2 % nonfat milk in DPBS, antibodies were diluted in 1% BSA in DPBS and applied to the cells in 6 of the wells. The remaining two wells were treated with only 1 % bovine serum albumin (BSA) solution and only secondary antibody as controls. Cells were incubated with the primary antibodies for one hour at room temperature and then washed three times with DPBS. Appropriate secondary antibodies were diluted in 1 % BSA in DPBS, added to each well and incubated for 1 hour. Following washing with DPBS, the slide was washed in water, air-dried, and overlayed with Fluoromount (Vector Laboratories). Immunofluorescent staining was viewed under a fluorescent microscope with appropriate filters and compared to the control wells that were not treated with primary antibody. All primary antibodies were obtained from Sigma unless otherwise stated. All secondary antibodies were purchased from Molecular Probes. Primary antibodies used to identify LC cells were: anti-collagen I, anti-collagen IV, anti-laminin, anti-cellular fibronectin, anti-glial fibrillary acidic protein (GFAP), and anti-alpha-smooth muscle actin. Cell populations were determined to be comprised of LC cells if they stained positively for collagen I, collagen IV, laminin, cellular fibronectin, alpha smooth muscle actin and negatively for glial fibrillary (GFAP). In this study, two sets of human eyes were used to initiate cultures. LC cell lines # 506 and # 517 were established from the optic nerve heads of and 83-year old male and a 17-year old male, respectively. All LC cell lines have been fully characterized and found to contain greater than 90 % LC cells.

### Treatment of LC cells

Apoptosis was induced in lamina cribrosa cells using a combination of 50 µM camptothecin (Sigma) and 10 ng/ml TNF-α (Leinco Technologies). The combination of camptothecin and TNF-α was found to be more effective at inducing apoptosis than either camptothecin or TNF-α alone.

### Construction and transfection of siRNAs

Small inhibitory RNAs (siRNAs) directed against human apoptosis-specific eIF-5A were used to specifically suppress expression of apoptosis-specific eIF-5A in lamina cribrosa cells. Six siRNAs were generated by *in vitro* transcription using the *Silencer*^{™} siRNA Construction Kit (Ambion Inc.). Four siRNAs were generated against human apoptosis-specific eIF-5A (siRNAs # 1 to # 4). Two siRNAs were used as controls; an siRNA directed against GAPDH provided in the kit, and an siRNA (siRNA # 5), which had the reverse sequence of the apoptosis-specific eIF-5A specific siRNA # 1, but does not itself target apoptosis-specific eIF-5A. The siRNAs were generated according to the manufacturer's protocol. The apoptosis-specific eIF-5A and control siRNA targets had the following sequences: siRNA # 1 5' AAAGGAATGACTTCCAGCTGA 3' (SEQ ID NO: 81); siRNA # 2 5' AAGATCGTCGAGATGTCTACT 3' (SEQ ID NO: 82); siRNA # 3 5' AAGGTCCATCTGGTTGGTATT 3' (SEQ ID NO: 83); siRNA # 4 5' AAGCTGGACTCCTCCTACACA 3' (SEQ ID NO: 84); siRNA # 5' AAAGTCGACCTTCAGTAAGGA 3 ' (SEQ ID NO: 85). Lamina cribrosa cells were transfected with siRNA using LipofectAMINE 2000.

Lamina cribrosa cells were transfected when cell confluence was at 40 to 70 % and were generally seeded onto 8-well culture slides at 7500 cells per well three days prior to transfection. Transfection medium sufficient for one well of an 8-well culture slide was prepared by diluting 25.5 pmoles of siRNA to a final volume of 21.2 µl in Opti-Mem (Sigma). 0.425 µl of Lipofectamine 2000 was diluted to a final volume of 21.2 µl in Opti-Mem and incubated for 7 to 10 minutes at room temperature. The diluted Lipofectamine 2000 mixture was then added to the diluted siRNA mixture and incubated together at room temperature for 20 to 30 minutes. The cells were washed once with serum-free media before adding 135 µl of serum-free media to the cells and overlaying 42.4 µl of transfection medium. The cells were placed back in the growth chamber for 4 hours. After the incubation, 65 µl of serum-free media plus 30 % FBS was added to the cells. Transfection of siRNA into cells to be used for Western blot analysis were performed in 24-well plates using the same conditions as the transfections in 8-well slides except that the volumes were increased by 2.3 fold. Following transfection, lamina cribrosa cells were incubated for 72 hours prior to treatment with 50 µM of camptothecin (Sigma) and 10 ng/ml of TNF-α (Leinco Technologies) to induce apoptosis. Cell lysates were then harvested for Western blotting or the cells were examined for apoptosis

### Detection of apoptotic cells

Transfected cells that had been treated with TNF-α and camptothecin for 24 hours were stained with Hoescht 33258 in order to determine the percentage of cells undergoing apoptosis. Briefly, cells were fixed with a 3:1 mixture of absolute methanol and glacial acetic acid and then incubated with Hoescht stain (0.5 µg/ml Hoescht 33258 in PBS). After a 10 minute incubation in the dark, the staining solution was discarded, the chambers separating the wells of the culture slide were removed, and the slide was washed 3 times for 1 minute with deionized water. After washing, a few drops of McIlvaine's buffer (0.021 M citric acid, 0.058 M Na₂HPO₄.7H₂O; pH 5.6) was added to the cells and overlaid with a coverslip. The stained cells were viewed under a fluorescent microscope using a UV filter. Cells with brightly stained or fragmented nuclei were scored as apoptotic. A minimum of 200 cells were counted per well. The DeadEnd^{™} Fluorometric TUNEL (Promega) was also used to detect the DNA fragmentation that is a characteristic feature of apoptotic cells. Following Hoescht staining, the culture slide was washed briefly with distilled water, and further washed by immersing the slide twice for 5 minutes in PBS (137mM NaCl, 2.68mM KCl, 1.47mM KH₂PO₄, 8.1mM Na₂HPO₄), blotting the slide on paper towel between washes. The cells were permeabilized by immersing them in 0.2 % Triton X-100 in PBS for 5 minutes. The cells were then washed again by immersing the slide twice for 5 minutes in PBS and blotting the slide on paper towel between washes. 25 µl of equilibration buffer [200 mM potassium cacodylate (pH 6.6), 25 mM Tris-HCl (pH 6.6), 0.2 mM dithiothreitol, 0.25 mg/ml bovine serum albumin, and 2.5 mM cobalt chloride] was added per well and incubated for 5 to 10 minutes. During equilibration, 30 µl of reaction mixture was prepared for each well by mixing in a ratio of 45:5:1, respectively, equilibration buffer, nucleotide mix [50 µM fluorescein-12-dUTP, 100 µM dATP, 10 mM Tris-HCl (pH 7.6), and 1 mM EDTA], and terminal deoxynucleotidyl transferase enzyme (Tdt, 25 U/µl). After the incubation in equilibration buffer, 30 µl of reaction mixture was added per well and overlayed with a coverslip. The reaction was allowed to proceed in the dark at 37°C for 1 hour. The reaction was terminated by immersing the slide in 2 X SSC [0.3 M NaCl, and 30mM sodium citrate (pH 7.0)] and incubating for 15 minutes. The slide was then washed by immersion in PBS three times for 5 minutes. The PBS was removed by sponging around the wells with a Kim wipe, a drop of mounting media (Oncogene research project, JA1750-4ML) was added to each well, and the slide was overlayed with a coverslip. The cells were viewed under a fluorescent microscope using a UV filter (UV-G 365, filter set 487902) in order to count the Hoescht-stained nuclei. Any cells with brightly stained or fragmented nuclei were scored as apoptotic. Using the same field of view, the cells were then viewed using a fluorescein filter (Green H546, filter set 48915) and any nuclei fluorescing bright green were scored as apoptotic. The percentage of apoptotic cells in the field of view was calculated by dividing the number of bright green nuclei counted using the fluorescein filter by the total number of nuclei counted under the UV filter. A minimum of 200 cells were counted per well.

### Protein extraction and Western blot analysis

Protein was isolated for Western blotting from lamina cribrosa cells growing on 24-well plates by washing the cells twice in PBS (8 g/L NaCl, 0.2 g/L KCl, 1.44 g/L Na₂HPO₄, and 0.24 g/L KH₂PO₄) and then adding 50 µl of lysis buffer [2 % SDS, 50 mM Tris-HCl (pH 7.4)]. The cell lysate was collected in a microcentrifuge tube, boiled for 5 minutes and stored at - 20 °C until ready for use. Protein concentrations were determined using the Bicinchoninic Acid Kit (BCA; Sigma). For Western blotting, 5 µg of total protein was separated on a 12 % SDS-polyacrylamide gel. The separated proteins were transferred to a polyvinylidene difluoride membrane. The membrane was then incubated for one hour in blocking solution (5 % skim milk powder, 0.02 % sodium azide in PBS) and washed three times for 15 minutes in PBS-T (PBS + 0.05 % Tween-20). The membrane was stored overnight in PBS-T at 4 °C. After being warmed to room temperature the next day, the membrane was blocked for 30 seconds in 1 µg/ml polyvinyl alcohol. The membrane was rinsed 5 times in deionized water and then blocked for 30 minutes in a solution of 5 % milk in PBS. The primary antibody was preincubated for 30 minutes in a solution of 5 % milk in PBS prior to incubation with the membrane. The primary antibodies used were anti-eIF-5A (BD Transduction Laboratories) at 1:20,000 and anti-β-actin (Oncogene). The membranes were washed three times in PBS-T and incubated for 1 hour with the appropriate HRP-conjugated secondary antibodies diluted in 1 % milk in PBS. The blot was washed and the ECL Plus Western blotting detection kit (Amersham Pharmacia Biotech) was used to detect the peroxidase-conjugated bound antibodies.

### Results

Two lamina cribrosa (LC) cell lines were established from optic nerve heads obtained from male donors ranging in age from 83 years (#506) to 17 years (#517). The cells isolated from the human lamina cribrosa had the same broad, flat morphology with prominent nucleus observed in other studies (Lambert *et al.,* 2001). Consistent with the characterizations of other groups, the LC cells showed immunoreactivity to alpha smooth muscle actin (figure 91a) as well as to a number of extracellular matrix proteins including cellular fibronectin (figure 91b), laminin (figure 91c), collagen I, and collagen IV (data not shown) (*Clark et al.*, 1995; Hernandez *et al.*, 1998; Hernandez and Yang, 2000; Lambert *et al.*; 2001). Negative immunoreactivity of the LC cells to glial fibrillary acidic protein (GFAP) was also observed consistent with previous findings (figure 91d) (Lambert *et al.,* 2001). These findings support the identification of the isolated cells as being LC cells rather than optic nerve head astrocytes.

Since TNF-α is believed to play an important role during the glaucomatous process, the susceptibility of LC cells to the cytotoxic effects of TNF-α was examined. Confluent LC cells were exposed to either camptothecin, TNF-α, or a combination of camptothecin and TNF-α for 48 hours (figure 92). Hoescht staining revealed that TNF-α alone was not cytotoxic to LC cells. Treatment with camptothecin resulted in approximately 30 % cell death of the LC cells. However, a synergistic increase in apoptosis was observed when LC cells were treated with both camptothecin and TNF-α, a treatment which resulted in the death of 45 % of LC cells by 48 hours. These results indicate that LC cells are capable of responding to the cytotoxic effects of TNF-α when primed for apoptosis by camptothecin.

eIF-5A is a nucleocytoplasmic shuttle protein known to be necessary for cell division and recently suggested to also be involved during apoptosis. The expression of apoptosis-specific eIF-5A protein in LC cells being induced to undergo apoptosis by either camptothecin, or camptothecin plus TNF-α. The expression of apoptosis-specific eIF-5A did not alter significantly upon treatment with camptothecin except perhaps to decrease slightly (figure 93). However, a significant upregulation of apoptosis-specific eIF-5A protein was observed after 8 and 24 hours of camptothecin plus TNF-α treatment (figure 93). These results indicate that of apoptosis-specific eIF-5A expression is induced specifically by exposure TNF-α and expression correlates to the induction of apoptosis. This points to a role for apoptosis-specific eIF-5A in the apoptotic pathway downstream of TNF-α receptor binding.

In order to examine the importance of apoptosis-specific eIF-5A expression during TNF-α-induced apoptosis in LC cells, a series of four siRNAs (siRNAs # 1 to # 4) (SEQ ID NO:81-84) targeting apoptosis-specific eIF-5A were designed and synthesized by *in vitro* transcription. To determine the effectiveness of the siRNAs in suppressing apoptosis-specific eIF-5A protein expression, LC cell lines # 506 and # 517 were transfected with each of the siRNAs and expression of apoptosis-specific eIF-5A protein in the cell lysate was examined 72 hours later (figure 94). For comparison, cells were also transfected with either an siRNA against GAPDH and/or a control siRNA (siRNA # 5) (SEQ ID NO:85) having the same chemical composition as siRNA #1 but which does not recognize apoptosis-specific eIF-5A. All siRNAs directed against apoptosis-specific eIF-5A were capable of significantly suppressing apoptosis-specific eIF-5A expression in both LC cell lines (figure 94). The GAPDH siRNA was used as an additional control because, unlike the control siRNA # 5 which simply has the reverse sequence of siRNA # 1 and does not have a cellular target, it is an active siRNA capable of suppressing the expression of it's target protein, GAPDH (data not shown). All four siRNAs against apoptosis-specific eIF-5A were also capable of protecting transfected LC cells (# 506) from apoptosis induced by 24 hour treatment with TNF-α and camptothecin (figure 95). Using Hoescht staining to detect cell death, the siRNAs (siRNAs # 1 to # 4) (SEQ ID NO:81-84) were found to be able to reduce apoptosis of LC cells by 59 % (siRNA # 1) (SEQ ID:81), 35 % (siRNA # 2) (SEQ ID NO:82), 50 % (siRNA # 3) (SEQ ID NO:83), and 69 % (siRNA # 4) (SEQ ID NO: 84). Interestingly, the siRNA against GAPDH was also able to reduce apoptosis of LC cells by 42 % (figure 95). GAPDH is known to have cellular functions outside of it's role as a glycolytic enzyme, including a proposed function during apoptosis of cerebellar neurons (Ishitani and Chuang, 1996; Ishitani *et al.*, 1996a; Ishitani *et al.,* 1996b). In a similar experiment we also demonstrated that siRNA # 1 (SEQ ID NO:81) was able to reduce apoptosis of the LC line # 517 by 53 % in response to TNF-α and camptothecin indicating that apoptosis-specific eIF-5A siRNAs are protective for LC cells isolated from different optic nerve heads (figure 96). These results indicate that apoptosis-specific eIF-5A does have a function during apoptosis and may be an important intermediate in the pathway leading to TNF-α-induced apoptosis in LC cells.

In order to confirm that LC cells exposed to TNF-a and camptothecin were dying by classical apoptosis, DNA fragmentation was evaluated in situ using the terminal deoxynucleotidyl transferase-mediated dUTP-digoxigenin nick end labeling (TUNEL) method. LC cells (# 506) were treated with TNF-α and camptothecin for 24 hours, 3 days after transfection with either an apoptosis-specific eIF-5A siRNA (siRNA # 1) (SEQ ID NO:81) or a control siRNA (siRNA # 5) (SEQ ID NO:85). The cells were also stained with Hoescht to facilitate visualization of the nuclei. 46 % of LC cells transfected with the control siRNA were positive for TUNEL staining while only 8 % of LC cells transfected with apoptosis-specific eIF-5A siRNA # 1 (SEQ ID NO:81) were positively labeled indicating that the apoptosis-specific eIF-5A siRNA provided greater than 80 % protection from apoptosis (figure 97). Similar results were obtained with apoptosis-specific eIF-5A siRNA # 4 which provided greater than 60 % protection from apoptosis relative to the control siRNA (data not shown).

### EXAMPLE 18

### Blood Collection and Preparation of PBMCs

Approximately 10 ml of blood was collected from each healthy donor. The blood was collected by venapuncture in a vacutainer containing sodium citrate as the anticoagulant. The samples were processed within 24 hours of collection.

A 60% SIP (9 parts v/v Percoll with 1 part v/v 1.5M NaCl) was cushioned on the bottom of 15ml conical tubes. The blood was then layered overtop with minimal mixing of the blood and Percoll cushion. The samples were centrifuged for 30 minutes total at 1000xg with slow acceleration in the first 5 minutes and slow deceleration in the last 5 minutes. The pure serum at the very top of the resulting gradient was removed and the white cushion (1-2ml) of PBMCs was collected and added dropwise to a tube containing 10ml of warm RPMI plus 15% FBS. The PBMCs were pelleted and counted.

### Stimulation to induce cytokine production in PBMCs over a time course

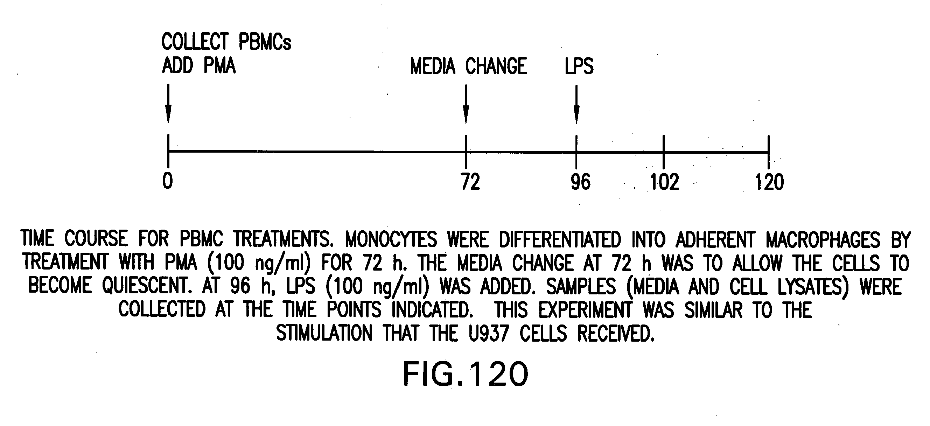

PBMCs were isolated and seeded at 2x10⁵ to 5x10⁵ cells/well. The cells were treated with phorbol 12-myristate 13-acetate (PMA; 100ng/well). At 72 hours the media was replaced and did not contain any stimulating factors. Then at 96 hours after PMA addition to PBMCs, lipopolysaccharide (LPS; 100 ng/well; from E. coli, serotype 0111) was added to the wells. Samples were collected before LPS addition (96h), and at various times after addition as outlined in figure 120. Both adherent cells (likely to be monocytes and macrophages) were collected with the floating cells (likely to be lymphocytes). To collect samples for analysis of cytokine secretion, the media from each well was transferred to clean microcentrifuge tubes and cleared of any debris by centrifugation at 13000 x g for 3 minutes. The resulting pellet was collected with the adherent cells. The media was stored at -20°C in 200-250 µl aliquots prior to analysis. The cells were washed with 1 ml of 37°C phosphate buffered saline (PBS) and then lysed in boiling lysis buffer (50 mM Tris pH 7, 2% SDS; 100µl per well). The cell lysates were boiled and stored frozen at -20°C. The Western blot is shown in figure 121 and the corresponding ELISA in figure 120.

### PBMC Stimulation to induce apoptosis-specific eIF-5A expression

PBMCs were collected and seeded at 2x10⁵ to 5x10⁵ cells/well. To determine which stimulators induce apoptosis-specific eIF-5A, as well as to see if they act synergistically, the PBMCs were stimulated with phytohemagglutinin (PHA; 100ng/ml), phorbol 12-myristate 13-acetate (PMA; 100ng/ml), lipopolysaccharide (LPS; 100ng/ml) or all three (each at 100ng/ml). The samples were collected 12 and 36 hours after stimulation and analyzed for apoptosis-specific eIF-5A expression (figure 123).

### Transfection of PBMCs

PBMCs were transfected the day they were prepared. Cells were seeded at 2x10⁵ to 5x10⁵ cells/well (150µl per well for transfection in a 24-well plate). They were either transfected individually in each well (Donors 77, 78 and 79; figures 124 and 125) or all at once in a conical tube before seeding (Donors 80 and 84; figure 125). For each well of cells to be transfected, 15 pmoles of siRNA was diluted in 50µl of Opti-MEM (Sigma). 1µl of Lipofectamine 2000 (Invitrogen) was diluted in 49µl of Opti-MEM, incubated for 7 to 10 minutes, added to the diluted siRNA and incubated 25 minutes. The transfection medium was overlayed onto the cells were placed in the 37°C growth chamber for 4 hours. The final transfection medium contained 9 % serum. After the incubation, 250µl of serum-free RPMI + 21 % FBS was added to the cells to make the final serum concentration 15%.

### Stimulation to induce cytokine production in PBMCs post transfection

72 hours after transfection of the PBMCs, as outlined above, lipopolysaccharide (LPS; 100 ng/well; from E. coli, serotype 0111) was added to the cells in 500µl of media. The samples were collected at 24 hours post stimulation. Both wells that were treated with LPS and wells that were transfected only (i.e. no stimulation) were collected. To collect samples for analysis of cytokine secretion, the media from each well was transferred to clean microcentrifuge tubes and cleared of any debris by centrifugation at 13000xg for 3 minutes. The resulting pellet was collected with the adherent cells. The media was stored at -20 °C in 200 - 250 µl aliquots prior to analysis. The cells were washed with 1 ml of 37 °C phosphate buffered saline (PBS) and then lysed in boiling lysis buffer (50 mM Tris pH 7, 2% SDS; 100µl per well). The cell lysates were boiled and stored frozen at -20°C for BCA protein quantitation.

### EXAMPLE 19

### Cell Culture

HT-29, a human colorectal adenocarcinoma cell line, was maintained in RPMI with 10 % fetal bovine serum (FBS). U937, a histiocytic lymphoma cell line, was grown in suspension in RPMI with 10% FBS. Both cell lines were maintained in a humidified environment at 37 °C and 5 % CO₂. For experiments with U937 cells, cells were counted and adjusted to 3 x 10⁵ cells/ml two days before the start of the experiment. On the first day of the experiment, cells were collected by centrifugation at 400 x g for 10 mins, the cell pellet was resuspended in fresh RPMI media with 10% FBS, the centrifugation was repeated, and the repelleted cells were resuspended in fresh RPMI media without FBS. The cells were counted and adjusted to 2 x 10⁶ cells/ml.

### siRNA

siRNA sequences were designed based on the human apoptosis-specific eIF-5A sequence and were synthesized by Dharmacon RNA Technologies. The apoptosis-specific eIF-5A siRNA (h5A1) target sequence was: 5' NNGCUGGACUCCUCCUACACA 3' (SEQ ID NO: 86). The corresponding double stranded siRNA sequence was:
5' GCUGGACUCCUCCUACACAdTdT 3' (SEQ ID NO: 87)
3' dTdTCGACCUGAGGAGGAUGUGU 5' (SEQ ID NO: 88)
The control siRNA (hcontrol) sequence was 5' NNACACAUCCUCCUCAGGUCG 3' (SEQ ID NO: 89). The corresponding double stranded siRNA sequence was:
5' ACACAUCCUCCUCAGGUCGdTdT 3' (SEQ ID NO: 90)
3' dTdTUGUGUAGGAGGAGUCCAGC 5' (SEQ ID NO: 91)

### Transfection of HT-29 Cells

The day before transfection, HT-29 cells were seeded at 105,000 cells per well onto a 24-well plate. For each well of cells to be transfected, 25.5 pmoles of siRNA was diluted in 50 µl of Opti-Mem (Sigma). 1 µl of Lipofectamine 2000 (Invitrogen) was diluted in 49 µl of Opti-Mem, incubated for 7 to 10 minutes and added to the diluted siRNA and incubated 25 minutes. The cells to be transfected were washed once with serum-free RPMI before adding 300 µl of serum-free RPMI and overlaying 100 µl of transfection medium. The cells were placed back in the growth chamber for 4 hours. After the incubation, 300 µl of serum-free RPMI + 30 % FBS was added to the cells.

### Electroporation of U937 Cells

apoptosis-specific eIF-5A and control siRNA were diluted in Opti-Mem media (Sigma). 400 µl cells (800,000 cells) and 100 pmoles siRNA were mixed in a 0.4mm electroporation cuvette. The cells were electroporated at 300 V, 10 mSec, 1 pulse with an ECM 830 Electrosquare porator (BTX, San Diego, CA). Following electroporation, the cells were gently mixed and added to wells containing RPMI and concentrated FBS so that the final FBS concentration was 10%.

### Treatment of HT-29 Cells

TNF-α production was induced in HT-29 cells according to the method developed by Suzuki et al. 2003. HT-29 cells were primed with 200 units/ml interferon gamma (Roche Diagnostics) 48 hours after transfection. After 16 hours of interferon gamma (Roche Diagnostics) 48 hours after transfection. After 16 hours of interferon gamma (IFNγ) priming the cells were washed with media and lipopolysaccharide (LPS; 100 ng/ml; from E. coli, serotype O111; Sigma) was added at 100 µg/ml. After 8 or 24 hours of LPS stimulation, the media from each well was transferred to microcentrifuge tubes and stored at -20°C until assayed for TNFα by ELISA. The cells were washed with 1 ml of phosphate buffered saline (PBS) heated to 37°C and then lysed in boiling lysis buffer (50 mM Tris pH 7, 2% SDS). The cell lysates were boiled and stored frozen at -20°C. The protein concentration in the cell lysates was determined by bicinchoninic acid assays (BCA) with bovine serum albumin used as the standard.

IL-8 production was induced in HT-29 cells by treatment with IFNγ. HT-29 cells were treated with 200 units/ml IFNγ 48 hours after transfection. After 24 hours of treatment, the media from each well was transferred to microcentrifuge tubes and stored at -20°C until assayed for IL-8 by liquid-phase electrochemiluminescence (ECL). The cells were washed with 1 ml of phosphate buffered saline (PBS) heated to 37°C and then lysed in boiling lysis buffer (50 mM Tris pH 7, 2% SDS). The cell lysates were boiled and stored frozen at -20°C. The protein concentration in the cell lysates was determined by bicinchoninic acid assays (BCA) with bovine serum albumin used as the standard.

### Induction of Differentiation in U937 Cells

U937 cells were collected and counted 16 hours after electroporation. 200,000 cells in 1ml of media were added to each well of 24-well plates. Macrophage differentiation was stimulated by adding phorbol 12-myristate 13-acetate (PMA; 100 ng/ml). After 48h with PMA, >80% of the monocytes had transformed from cells in suspension (monocytes) to adherent cells (macrophages). At 48 hours the media and any non-adherent cells were removed and fresh RPMI media with 10% FBS (1ml per well) was added. The cells were left for 24 hours in fresh media to become quiescent.

### Stimulation to induce cytokine production in U937 Cells

72 hours after PMA addition to U937 cells, lipopolysaccharide (LPS; 100 ng/ml; from E. coli, serotype 0111), interferonγ (IFNγ; 100 Units/ml), or a combination of LPS and IFNγ were added to the wells. Samples were collected before stimulator addition (72h), and at various times after addition as outlined in figure 131. To collect samples for analysis of cytokine secretion, the media from each well was transferred to clean microcentrifuge tubes and cleared of any debris by centrifugation at 13000 x g for 3 mins. The media was stored at -20°C in 200-250 ul aliquots prior to analysis. The cells were washed with 1 ml of 37°C phosphate buffered saline (PBS) and then lysed in boiling lysis buffer (50 mM Tris pH 7, 2% SDS; 75 µl per well). Like wells were pooled. The cell lysates were boiled and stored frozen at -20°C.

### Cytokine Quantification

All media samples were stored frozen at -20°C. TNFα was quantified using ELISA kits from Assay Designs according to the manufacturer's instructions with supplied standards for 0-250 pg TNFα/ml. For U937 experiments media samples for TNFα were diluted 20 fold (0h, 3h LPS) or 80 fold (6h, 24h, 30h LPS) with RPMI + 10% FBS. IL-1β, IL-8, and IL-6 were quantified by liquid-phase electrochemiluminescence (ECL). Media from HT-29 experiments were not diluted. All cytokine measurement results were corrected for the amount of total cellular protein (mg) per well.

IL-8, IL-1β, and IL-6 were assayed by liquid-phase. Briefly, a purified monoclonal mouse anti- mouse anti-human IL-8, IL-6 or IL-1β (R & D Systems) were labeled with biotin (Igen, Inc., Gaithersburg, MD). In addition, the goat anti-human IL-8, IL-6, or IL-1β antibody (R & D) were labeled with ruthenium (Igen) according to the manufacturer's instructions. The biotinylated antibodies were diluted to a final concentration of 1 mg/mL in PBS, pH 7.4, containing 0.25% BSA, 0.5% Tween-20 and 0.01 % azide, (ECL buffer). Per assay tube, 25 mL of the biotinylated antibodies were pre-incubated at room temperature with 25 mL of a 1 mg/mL solution of streptavidin-coated paramagnetic beads (Dynal Corp., Lake Success, NY) for 30 min by vigorous shaking. Samples to be tested (25 mL) which had been diluted in RPMI or standards were added to tubes followed by 25 mL of ruthenylated antibody (final concentration 1 mg/mL, diluted in ECL buffer). The tubes were then shaken for an additional 2 hours. The reaction was quenched by the addition of 200 mL/tube of PBS and the amount of chemiluminiscence determined using an Origen Analyzer (Igen).

### SDS-PAGE and Western Blotting

The protein concentration in the cell lysates was determined by bicinchoninic acid assays (BCA) with bovine serum albumin used as the standard. 5 µg of total cellular protein was separated by either 10% or 14% SDS-PAGE (sodium dodecyl sulfate polyacrylamide gel electrophoresis). 10% gels were used for analysis of proteins above 50 kDa (TLR4, IFNγ, TNF-R1, iNOS) while 14% gels were used for apoptosis-specific eIF-5A (17 kDa). Gels were transferred to polyvinylidene fluoride (PVDF) membranes with transfer buffer (48 mM Tris, 39 mM glycine, 1.3 mM SDS, pH 9.2; 15V for 18 mins) using a semi-dry transfer unit (Bio-Rad). Membranes were blocked for 1 hour with 5% skim milk in PBS-t (PBS with 0.1% Tween 20). Primary antibodies were diluted in the blocking solution and all blots were incubated at room temperature with shaking. Primary antibodies used were apoptosis-specific eIF-5A (BD Biosciences; 1:20,000; incubate 1 hour; recognizes both apoptosis-specific eIF-5A and eIF5-A2), TLR4 (Santa Cruz Biotechnology Inc; TLR4 (H-80): sc-10741; 1:1000; incubate 2 hours), IFN-γRα (Santa Cruz Biotechnology Inc; IFN-γRα (C-20): sc-700; 1:1000; incubate 1 hour), TNF-R1 (Santa Cruz Biotechnology Inc; TNF-R1 (H-5): sc-8436; 1:200; incubate 3 hours), iNOS (BD Transduction Laboratories:610431; 1:10,000; incubate 1 hour) and β-actin (Oncogene; actin (Ab-1); 1:20,000; incubate 1 hour). Following primary antibody incubations, blots were washed 3 times for 5-10 minutes with PBS-t. Horseradish peroxidase-conjugated (HRP) secondary antibodies were diluted in 1% skim milk and incubated with the membrane for 1 hour. Secondary antibodies used were anti-mouse IgG-HRP (Sigma; 1:5000; for apoptosis-specific eIF-5A and TNF-R1), anti-rabbit IgG-HRP (Amersham Pharmacia Biotech; 1: 2500; for TLR4 and IFNγ-Rα), anti-mouse IgMHRP (Calbiochem; 1:5000; for actin). Following secondary antibody incubations, blots were washed 4 times for 5-10 mins with PBS-t. Blots were developed with enhanced chemiluminescent detection reagent (ECL; Amersham Pharmacia Biotech) according to the manufacturers instructions and bands were visualized on X-ray film (Fuji).

### RT-PCR

RT-PCR was performed according to Medvedev *et al.* 2002 in order to observe changes in TLR4 mRNA expression in transfected HT-29 cells in response to IFNγ. Expression of GAPDH was used as a control to show that equal amounts of cDNA were being used between samples. Increasing PCR cycles (20, 25, 30, and 35) were used to determine the optimal cycle number that resulted in detectable amplified products under nonsaturating conditions. PCR products were detected by ethidium bromide-incorporation and were separated by agarose gel electrophoresis. RT-PCR of total mRNA isolated from siRNA-transfected HT-29 cells treated with or without IFNγ for 6 hours was used to detect TLR4 and GAPDH transcripts. HT-29 cells were transfected with siRNA as described above. 48 hours after transfection, the cells were treated with 200 units/ml IFNγ. Control cells which were not treated with IFNγ received only a media change. Total mRNA was isolated using the GenElute Mammalian RNA miniprep kit (Sigma) according to the manufacturer's protocol for adherent cells. The media was removed and the cells were washed twice with warm PBS. Lysis buffer was added to the cells and the lysate was transferred to a microcentrifuge tube and total RNA was isolated according to the manufacturer's protocol.
The primers for TLR4 (NM_003266) were:
Forward 5' CGGATGGCAACATTTAGAATTAGT 3' (SEQ ID NO: 92)
Reverse 5' TGATTGAGACTGTAATCAAGAACC 3' (SEQ ID NO: 93)
Expected fragment size: 674 bp
The primers for GAPDH (BC023632) were:
Forward 5' CTGATGCCCCCATGTTCGTCAT 3' (SEQ ID NO: 94)
Reverse 5' CCACCACCCTGTTGCTGTAG 3' (SEQ ID NO: 95)
Expected fragment size: 599 bp
The total RNA was reverse transcribed using the following conditions:
Mix:

| | |
|---|---|
| RNA | 2.5 µg |
| Poly (T) primer | 6.25 µl |
| Depc water | to 13.75 µl |
| | Heat 70°C 5min |
| | Chill on ice 5 min |

Add:

| | |
|---|---|
| 5X AMV Buffer | 5.0 µl |
| dNTPs (10 mM) | 2.5 µl |
| Rnase Inhibitor | 1.25 µl |
| AMV RT | 2.5 µl |
| | Heat 42°C 60 min |
| | Heat 70°C 10 min |

A single PCR reaction was performed using the following conditions:

| | |
|---|---|
| 10X Tsg buffer | 2.0 µl |
| dNTP (10 mM) | 0.4 µl |
| forward primer (25 pmol/ µl) | 0.4 µl |
| reverse primer (25 pmol/ µl) | 0.4 µl |
| MgCl₂(15 mM) | 2.0 µl |
| cDNA | 0.8 µl |
| H₂O | 13.88 µl |
| Tsg polymerase | 0.12 µl |

The PCR conditions for TLR4 were:
Heat to 95°C 5min
20, 25, 30, or 35 cycles of: 95 °C 1min
   55 °C 1 min
   72 °C 2 min
Extend at 72°C for 10 min
Sink to 4°C
The PCR conditions for GAPDH were:
Heat to 95°C 5min
20, 25, 30, or 35 cycles of: 95 °C 1min
   57°C 1 min
   72 °C 2 min
Extend at 72°C for 10 min
Sink to 4°C

### EXAMPLE 20: Material and Methods for NFκB Assay

The results of this assay show that when HT-29 cells exposed to IFN-γ and LPS are transfected with siRNAs against apoptosis-specific eIF-5A, there is a decrease in NFκB p50 activation and TNF-α production. See figure 114.

### Culture

HT-29, a human colorectal adenocarcinoma cell line, was maintained in RPMI plus 10 % FBS in a humidified environment at 37 °C and 5 % CO₂.

### Transfection

The day before transfection, HT-29 cells were seeded at 500,000 cells per well onto a 6-well plate. For each well of cells to be transfected, 155 pmoles of siRNA was diluted in 240 µl of Opti-Mem (Sigma). 4.8 µl of Lipofectamine 2000 (Invitrogen) was diluted to 240 µl with Opti-Mem, incubated for 7 to 10 minutes, added to the diluted siRNA and incubated 25 minutes. The cells to be transfected were washed once with serum-free RPMI before adding 1400 µl of serum-free RPMI and overlaying 480 µl of transfection medium. The cells were placed back in the growth chamber for 4 hours. After the incubation, 720 µl of serum-free RPMI plus 30 % FBS was added to the cells. Fresh media was added to the cells twenty-four hours after transfection.

### Treatment of Cells

Forty-eight hours after transfection, cells which were to be primed with interferon gamma (IFNγ; Roche Diagnostics) received 200 units/ml of IFNγ. All remaining wells received a change of media. 16 hours after IFNγ addition, cells were treated with either TNF-α (20 ng/ml; Leinco Technologies Inc., St. Louis, MO) or lipopolysaccharide (LPS; 100 ng/ml; from E. coli, serotype 0111; Sigma) or media with no additions for untreated controls. Cells which were to be treated with only IFNγ were primed overnight with IFNγ and received media with fresh IFNγ 16 hours later.

### Nuclear Extraction and NFκB Transcription Factor Assay

After one hour of treatment with the various stimulators, the nuclear proteins were harvested from the cells and used to measure NFκB activity. Nuclear extraction was carried out using the TransAM Nuclear Extract Kit (Active Motif, Carlsbad, CA) according to the manufacturer's protocol. The DNA-binding capacity of the p50 subunit of NFκB was measured using the TransAM NFκB Family Transcription Factor Assay Kit (Active Motif, Carlsbad, CA) using 20 µg of nuclear extract according to the manufacturer's protocol.

### EXAMPLE 21

### Treating Sepsis in a mammal-based on mouse septic model

Two types of groups of mice were used in the study. Balb/C mice and C57BL/6 mice were used. In both studies, the mice were given a dose of LPS that would induce sepsis and death within 48 hours 100 % of the time. The test was designed so that siRNA against eIF-5A1 (3'- GCC UUA CUG AAG GUC GAC U -5' (SEQ ID NO : 99)) was given intraperitoneal at different time courses. All doses of siRNA were 50 µg. In each study, 5 test groups and 1 control group were used. Each group started with 5 mice. The control group received no siRNA.

### Balb/C mouse model

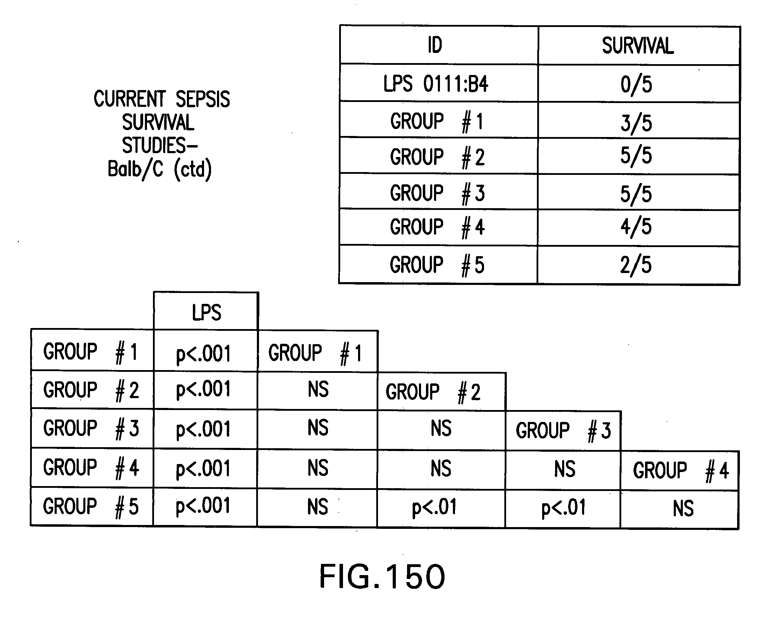

Figures 149 and 150 show the results of the test in Balb/C mice. All mice received the lethal dose of LPS at 48 hours. Group 1 mice received siRNA at 0 and 24 hours, and three out of five mice survived. Group 2 mice received siRNA at 0, 24, and 48 hours, and five out of five mice survived. Group 3 mice received siRNA at 48 hours and five out of five mice survived. Group 4 mice received siRNA at 50, 56, 64 and 72 hours, and four out of five mice survived. Group 5 mice received siRNA at 48, 56, 64 and 72 hours and two out of five mice survived. Group 6 mice, the control group, received no siRNA, and zero mice survived and all five died within 48 hours of LPS treatment (Day 4).

### C57BL/6 mouse model

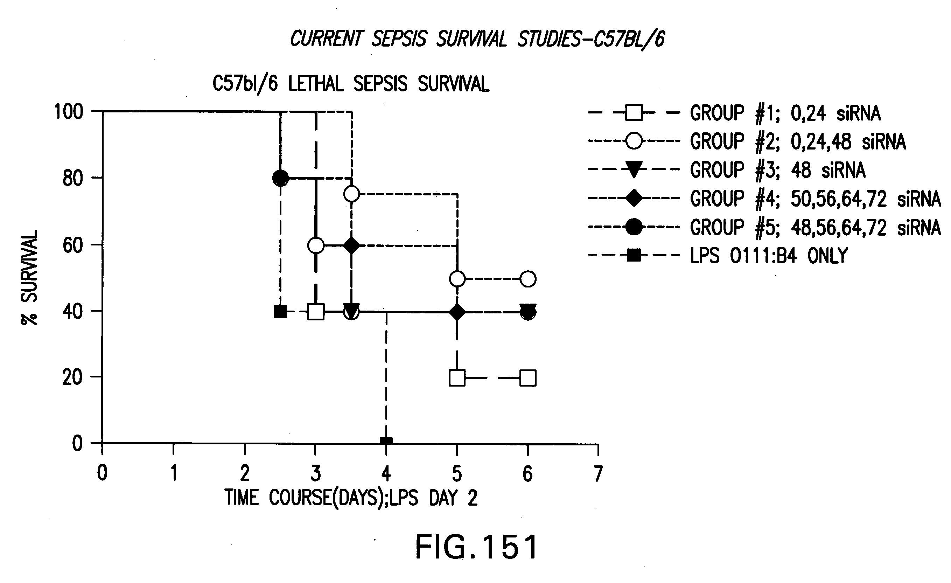

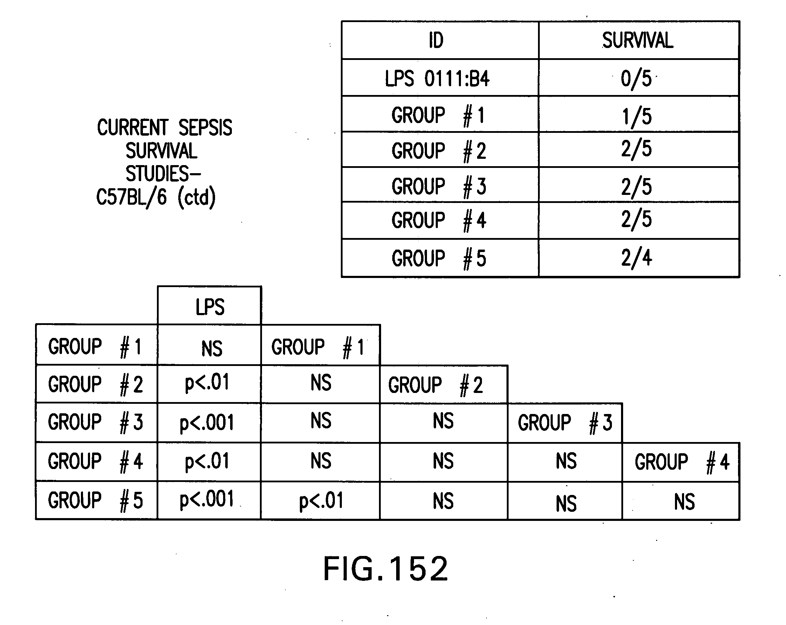

Figures 151 and 152 show the results of the test in C57BL/6 mice. All mice received the lethal dose of LPS at 48 hours. Group 1 mice received siRNA at 0 and 24 hours, and one out of five mice survived. Group 2 mice received siRNA at 0, 24, and 48 hours, and two out of five mice survived. Group 3 mice received siRNA at 48 hours and two out of five mice survived. Group 4 mice received siRNA at 50, 56, 64 and 72 hours, and two out of five mice survived. Group 5 mice received siRNA at 48, 56, 64 and 72 hours and two out of five mice survived. Group 6 mice, the control group, received no siRNA, and zero mice survived and all five died within 48 hours of LPS treatment (Day 4).

### EXAMPLE 22

### Chemicals

N1-guanyl-1,7-diaminoheptane (GC7; Biosearch Technologies), a potent inhibitor of DHS, was used at a concentration of 50 µM. Actinomycin D (Calbiochem) was used at 0.5 or 1.0 µg/ml.

### Cell Culture and Treatment

The human colon adenocarcinoma cell line, HT-29, was used for cell proliferation and eIF-5A localization studies and was a kind gift from Anita Antes (University of Medicine and Dentistry of New Jersey). HT-29 cells were maintained in RPMI 1640 supplemented with 1 mM sodium pyruvate, 10 mM HEPES, and 10% fetal bovine serum (FBS). All other cell lines were obtained from the American Type Culture Collection.

CCD112Co is a normal colon fibroblast cell line. RKO is a human colorectal carcinoma cell line (CRL-2577) containing a wild-type p53. The RKO-E6 cell line (CRL-2578) was derived from the RKO cell line. It contains a stably integrated human papilloma virus E6 oncogene and therefore lacks appreciable functional p53 tumor suppressor protein¹⁸. RKO, RKO-E6, and the cell line CCD112Co, were grown in Modified Eagle Minimum Essential Medium with 2 mM L-glutamine and Earle's Balanced Salt Solution adjusted to contain 1.5 g/L sodium bicarbonate, 0.1 mM non-essential amino acids, 1 mM sodium pyruvate and supplemented with 10 % FBS. Cells were maintained at 37°C in a humidified environment containing 5 % CO₂.

### Cloning and Construction of Plasmids

Human eIF5A1 was cloned by RT-PCR from total RNA isolated from RKO cells using the GenElute Mammalian RNA miniprep kit (Sigma) according to the manufacturer's protocol for adherent cells. The primers used were; forward, 5'-CGAGTTGGAATCGAAGCCTC-3' (SEQ ID NO: 100); and reverse, 5'-GGTTCAGAGGATCACTGCTG-3' (SEQ ID NO: 101). The resulting 532 base pair product was subcloned into pGEM-T Easy (Promega) and sequenced. The resulting plasmid was used as a template for PCR using the primers: forward, 5'-GCCAAGCTTAATGGCAGATGATTTGG-3' (SEQ ID NO: 102); and reverse, 5'-CCTGAATTCCAGTTATTTTGCCATGG-3' (SEQ ID NO: 103), and the PCR product was subcloned into the *Hind*III and *Eco*R1 sites of pHM6 (hemagglutinin [HA] tagged; Roche Molecular Biochemicals) to generate the pHM6-eIF5A1 vector. A C-terminal truncated construct of eIF5A1 (pHM6-eIF5A1Δ37) was generated by PCR using the following primers: forward, 5'- GCCAAGCTTAATGGCAGATGATTTGG-3' (SEQ ID NO: 104); and reverse, 5'- GCCGAATTCTCCCTCAGGCAGAGAAG-3' (SEQ ID NO: 105). The resulting PCR product was subcloned into the pHM6 vector. The pHM6-LacZ vector (Roche Molecular Biochemicals) was used to optimize transfection and as a control for the effects of transfection on apoptosis.

### Northern Blotting

RKO cells were grown to confluence on 6-well plates and treated for 0, 1, 4, or 8 hours with 1.0 µg/ml Actinomycin D. Total RNA was isolated from the cells using the GenElute Mammalian RNA miniprep kit (Sigma), and 5 µg of RNA was fractionated on a 1.2 % agarose/formaldehyde gel. The membrane was probed with a ³²P-labelled cDNA homologous to the 3'-untranslated region (3'-UTR) of eIF5A1 according to established methods. The eIF5A1 3'- UTR cDNA that was used for Northern blotting was cloned by RT-PCR from RKO cells using the following primers: forward, 5'-GAGGAATTCGCTGTTGCAATCAAGGC-3' (SEQ ID NO: 106); and reverse, 5'-TTTAAGCTTTGTGTCGGGGAGAGAGC-3' (SEQ ID NO: 107).

### Transfection of Plasmids and Detection of Apoptosis

RKO and RKO-E6 cells were transiently transfected with plasmid DNA using Lipofectamine 2000 (Invitrogen) according to the manufacturer's recommended protocol. Fortyeight hours after transfection, apoptotic cells containing fragmented DNA were detected by terminal deoxynucleotidyl transferase-mediated dUTP-digoxigenin nick end labelling (TUNEL) using a DNA Fragmentation Detection Kit (Oncogene Research Products) according to the manufacturer's protocol. Labeled cells were then analyzed by flow cytometry or by fluorescence microscopy. For flow cytometry analysis, harvested cells were fixed with 4% formaldehyde in PBS, labelled by TUNEL and analysed on a flow cytometer (Coulter Epics XL-MCL) with a 488 nm argon laser source and filters for fluorescein detection. For fluorescence microscopy analysis, cells were transfected on 8-well culture slides, fixed with 4% formaldehyde and then labelled by TUNEL and stained with Hoescht 33258 according to the methods described by Taylor *et al.* (2004).

### Transfection of siRNA

All siRNAs were obtained from Dharmacon. The eIF5A1 siRNA had the following sequence: sense strand, 5'- GCUGGACUCCUCCUACACAdTdT-3' (SEQ ID NO: 108); and antisense strand, 3'- dTdTCGACCUGAGGAGGAUGUGU-5' (SEQ ID NO: 109). The control siRNA that was used had the reverse sequence of the eIF5A1-specific siRNA and had no identity to any known human gene product. The control siRNA had the following sequence: sense strand, 5'-ACACAUCCUCCUCAGGUCGdTdT- 3' (SEQ ID NO: 110); and antisense strand, 3'-dTdTUGUGUAGGAGGAGUCCAGC-5' (SEQ ID NO: 111). Cells were transfected with siRNA¹² using Lipofectamine 2000 and used in proliferation studies or for Western blotting.

### Western Blotting

Protein for Western blotting was isolated using boiling lysis buffer [2 % SDS, 50 mM Tris-HCl (pH 7.4)]. Protein concentrations were determined using the Bicinchoninic Acid Kit (Sigma). For Western blotting, 5 µg of total protein was separated on a 12 % SDSpolyacrylamide gel and transferred to a polyvinylidene difluoride membrane. The primary antibodies used were anti-eIF5A1 (BD Transduction Laboratories; mouse IgG) and anti- β-actin (Oncogene; mouse IgM), both at a dilution of 1:20,000 in 5 % milk. The secondary antibodies were anti-mouse IgG conjugated to horseradish peroxidase (HRP; Sigma) and anti-mouse IgMHRP (Oncogene). Antibody-protein complexes were visualized using the enhanced chemiluminescence method (ECL, Amersham Biosciences). Following detection of eIF5A1, the blots were stripped according to the protocol provided by the ECL Plus Western blotting detection system and re-probed with anti-β-actin antibody to confirm equal loading.

### Proliferation assays

HT-29 cells were transfected with siRNA on 96-well plates using Lipofectamine 2000 (Invitrogen). Metabolic activity of proliferating cells was measured with the XTT Cell Proliferation Kit (Roche Applied Science). The BrdU Cell Proliferation Kit (Roche Applied Science) was used to measure DNA synthesis following the manufacturer's protocol. *Indirect Immunofluorescence* HT-29 cells were cultured on poly-L-lysine-coated glass coverslips. Subconfluent cells were incubated for 16 hours with 200 Units of interferon gamma (IFN-γ; Roche Applied Science) followed by TNF-α (100 ng/ml; Leinco Technologies) for times varying from 10 minutes to 8 hours. Alternatively, cells were treated with 1.0 µg/ml Actinomycin D for increasing lengths of time from 30 minutes to 16 hours. The treated cells were fixed with 3 % formaldehyde (methanol-free; Polysciences Inc.) for 20 minutes, washed twice for 5 minutes with PBS and once for 5 minutes with PBS containing 100 mM glycine, and permeabilized with 0.2% Triton X-100 in PBS for 4 minutes. Cells were then labeled for immunofluorescence using a standard protocol. The primary antibody was anti-eIF5A1 (BD Transduction Laboratories; mouse IgG) incubated at a dilution of 1:250 for 1 hour. The secondary antibody was anti-mouse IgG-AlexaFluor 488 (Molecular Probes) used at a dilution of 1:200 for 1 hour. Following antibody labeling, the nuclei were stained with Hoescht 33258, and the labelled cells were observed by fluorescent microscopy.

### Detection of Hypusine Modification

COS-7 cells were maintained in Dulbelcco's modified Eagles media (DMEM) containing 10% FBS and penicillin/streptomycin. COS-7 cells were cultured in T-25 flasks to 90-95% confluence. COS-7 cells were harvested after detachment with 0.25% Trypsin-EDTA (37°C for 3 min), diluted in DMEM containing 10% FBS, and centrifuged for 5 minutes at 1000 RPM. The pellet was resuspended in 1 ml DMEM and the total cell number counted with a hemacytometer. The cells were diluted with DMEM to a concentration of 1.5x10⁶ cells/ ml and 650 µL of diluted cells was used for electroporation. Fifteen micrograms of plasmid DNA (pHM6-eIF5A1) was diluted in 50 µL of Opti-MEM (Gibco), added to the 650 µL of diluted COS-7 cells and transfered to a cuvette. The cells were electroporated using a T820 ElectroSquarePorator at the following conditions:
i. Mode: High Voltage Mode (HV), 99 µsec;
ii. Voltage: 1.5 kV;
iii. Pulse Length: 90 µsec;
iv. Number of Pulses: 1 or 2;

After electroporation the cells were transfered from the cuvette to one well of a 6-well-plate which was pre-loaded with 1.2 ml of DMEM containing 16% FBS for each well and incubated at 37°C. Six hours after transfection, 100 µL of DMEM containing 10% FBS and 50 µCi [³H] spermidine was added to each well. Forty-eight hours after electroporation, the cells were washed with cold PBS and placed on ice. Two hundred microliters of cold lysis buffer [150 mM NaCl, 1% NP40, 50 mM Tris-HCl {pH 8.0}, protease inhibitor cocktail] was added to each well and incubated on ice for 30 minutes on a shaker. The cells were scraped from the plate, transferred to centrifuge tube and centrifuged for 10 minutes at 13,000 rpm at 4°C. 15. The lysate was transferred to a fresh tube and 20 µl of anti-HA antibody (Roche Applied Science) was added. The mixture was incubated at 4°C for 2 hours while rotating. Fifty microliters of protein A agarose (Roche Applied Science) was added and the mixture was incubated on rotator at 4 °C for 1 hour and then centrifuged at 13000 rpm at 4 °C for 15 seconds. The supernatant was removed, 800 µls of cold lysis buffer was added to the beads and resuspended by vortexing. The beads were washed twice more and then resuspended in 80 µls of 1 X SDS PAGE loading buffer. The beads were heated at 85 °C for 10 minutes and centrifuged at 13000 rpm for15 seconds. The supernatant was separated on a 15 % SDS-PAGE gel and transfered to a PVDF membrane. The membrane was incubated in Amplify^{™} Fluorographic Reagent (Amersham) for 30 minutes to increase detection efficiency for ³H and then exposed to Hyperfilm (Amersham) at -80°C for 10 days before developing. The membrane was then used for western blotting with anti-HA (Roche Applied Science) and anti-eIF5A (BD Transduction Laboratories) antibodies.

### Western blotting

Protein was isolated for Western blotting from normal colon fibroblast cells growing on 24-well plates by washing the cells twice in PBS (8 g/L NaCl, 0.2 g/L KCl, 1.44 g/L Na₂HPO₄, and 0.24 g/L KH₂PO₄) and then adding 50 µl of boiling lysis buffer [2 % SDS, 50 mM Tris-HCl (pH 7.4)]. The cell lysate was collected in a microcentrifuge tube, boiled for 5 minutes and stored at -20 °C. Protein concentrations were determined using the Bicinchoninic Acid Kit (BCA; Sigma). For Western blotting, 5 µg of total protein was fractionated on a 12 % SDS-polyacrylamide gel. The separated proteins were transferred to a polyvinylidene difluoride membrane. The primary antibodies used were anti-eIF5A (BD Transduction Laboratories; mouse IgG) and anti-HA (Roche Applied Science) at a dilution of 1:20,000 or 1:5000 in 5 % milk, respectively. The membranes were washed three times in PBS-T and incubated for 1 hour with the appropriate HRP-conjugated secondary antibodies diluted 1:5000 in 1% milk in PBS. The ECL Plus Western blotting detection kit (Amersham Pharmacia Biotech) was used to detect antibody-bound proteins.

### EXAMPLE 23

Because eIF5A is a conserved protein in all mammalian cells and plays role in cell survival, it was necessary to establish the effectiveness of siRNA to reduce the constitutive expression of eIF5A in vitro before testing in vivo. See Example 23. L929 were seeded onto a 24-well plate. The next day the cells were transfected with eIF5A1 siRNA or control scramble siRNA (CsiRNA). Three days after transfection, the cell lysates were assayed by Western-blotting with anti-eIF5A antibody. Specific inhibition of constitutive expression of the eIF5A protein was achieved following transfection with siRNA to murine eIF5A but not the CsiRNA.

Because systemic inflammation results in apoptosis in the thymus, the effect of intranasal LPS in inducing thymocyte apoptosis was studies. Mice received LPS intranasally and after 24 hours, exhibited the low numbers of thymocytes 24 hours compared to the numbers in untreated or vehicle treated mice. The number of thymocytes returned to baseline levels 48-72 hours after LPS administration. Since the reduction in thymocyte numbers could be due to apoptosis, early and late apoptosis of thymocytes, as measured by annexin-V and PI analysis, was determined.

Peak thymocyte apoptosis was observed 24 hours after LPS and returned to a physiological rate of apoptosis at 48-72 hours. This observation supports the concept that the atrophy that occurred in the thymus in mice treated with LPS was due to thymocyte apoptosis. Similar reduction in thymocyte numbers and increased PI and Annexin-V was reported in a model of systemic inflammation due to intravenous conA.

Intransal siRNA administration prevents LPS-induced thymocyte apoptosis Because peak thymocyte apoptosis was observed at 24 hours after intranasal LPS treatment, we studied the role of siRNA to eIF5A on LPS-induced thymocyte apoptosis at this time point. Administration of siRNA eIF5A did not significantly alter thymocyte number nor apoptosis events in vehicle-treated control mice. However, administration of siRNA eIF5A prior to LPS protected mice from thymocyte atrophy and induction of apoptosis, whereas CsiRNA had no significant effect.

### Cell culture

L929 cells were maintained in Minimum Essential Medium (Eagle) supplemented with 2 mM L-glutamine, 10 % of fetal bovine serum, and Earle's BSS adjusted to contain 1.5 g/L sodium bicarbonate, 0.1 mM non-essential amino acids, and 1.0 mM sodium pyruvate (all media components were purchased from Sigma-Aldrich, St. Louis, MO). L929 cells were subcultured twice per week using a subcultivation ratio of 1:5.

### siRNA

Target sequences for siRNA in the human eIF5A transcript (accession number NM-001970) were identified using the design guidelines suggested by Ambion (htto://www.ambion.com/techlib/tb/tb 506.html). siRNAs used to validate eIF5A suppression by Western blotting in L929 cells were generated by in vitro transcription using the SilencerTM siRNA Construction Kit (Ambion Inc.). For use in vivo, siRNAs having the same sequence were synthesized by Dharmacon, Lafayette, CO. The sequence of the eIF5A and control siRNA were: 5' CGGAAUGACUUCCAGCUGAdTdT 3' (SEQ ID NO: 112) and 5' AGUCGACCUUCAGUAAGGCdTdT 3' (SEQ ID NO: 113), respectively.

### Transfection of siRNA and Western blotting

L929 cells were transfected with 150 nM siRNA using Lipofectamine 2000 (Invitrogen Life Technologies, Carlsbad, CA) as described previously (29). Seventy-two hours after transfection, cell lysates were collected for Western blotting. Protein was harvested in hot lysis buffer [2 % SDS, 50 mM Tris-HCl (pH 7.4)], boiled for 5 minutes and stored at -20 °C. Protein concentrations were determined using the Bicinchoninic Acid Kit (BCA; Sigma). For Western blotting, 5 µg of total protein was fractionated on a 12 % SDS-polyacrylamide gel. The separated proteins were transferred to a polyvinylidene difluoride membrane. The primary antibodies used were anti-eIF5A (BD Transduction Laboratories; mouse IgG) and anti-13-actin (Oncogene; mouse IgM) each at a dilution of 1:20,000 in 5 % powdered skim milk. The ECL Plus Western blotting detection kit (Amersham Pharmacia Biotech) was used to detect antibody-bound proteins. The bound antibody was detected by electrochemiluminescence and exposed to x-ray film. Following detection for eIF5A, the blots were stripped according to the protocol provided by the ECL Plus Western blotting detection system and re-blotted with the actin antibody to confirm equal loading.

### Animals and treatments

Eight-week-old C57BL/6 mice (purchased from Jackson Laboratories, Bar Harbor, ME) were used. Mice were kept in pathogen-free conditions. The protocol was approved by the University of Colorado Health Sciences Center Institutional Animal Care and Use Committees. Fifty microliters of saline containing 75 µg of LPS (Escherichia coli K-234, Sigma) was administered intranasally by placement over the nostril of lightly anesthetized mice using a small pipette. Control mice received an equal amount of PBS intranasally (vehicle) or untreated. In some experiments, mice were pretreated with 50 µg of either siRNA or control siRNA intranasally 48 hours prior to LPS instillation. At various times after LPS, the thymus was removed and thymocytes were isolated as described below.

### Detection of T cell isolation and apoptosis

Thymocytes were freshly and isolated as described previously (7) using a 100µm cell strainer (Fisher Scientific, Pittsburgh, PA). The cells were maintained in complete cell culture medium (RPMI 1640 supplemented with 10% heat-inactivated fetal calf serum (FCS), 2 mM L-glutamine, 100 IU/ml penicillin, and 100 µg/ml streptomycin, Invitrogen). Thymocytes were washed once and cells were counted using a light microscope. Isolated thymocytes (105) were washed and suspended in 190 µl of binding buffer (0.1 M Hepes/NaOH (pH 7.4) 1.4 mM NaCl, 25 mM CaCl₂). Cells were incubated with 10µl of annexin-V and 5µl of propidium iodide (PI) (BD Pharmingen, San Jose, CA) for 10 minutes at room temperature and analyzed by flow cytometry.

### Statistical analysis

Data are expressed as mean ±SEM. The statistical significance of differences between treatment and control groups was determined by factorial ANOVA. Statistical analyses were performed using the XLStat software (Addinsoft, Brooklyn, NY, USA).

### EXAMPLE 24

### Method for mouse-VEGF determination

Mouse lungs were collected from each mouse as described below in Example 25, and were frozen at -70°C. A part of the lung tissue was cut off and ground using a mortal and pestil in liquid N2. The fine powder of the lung tissue was transferred to a clean tube and resuspended in 1 xTBS buffer containing 1% NP-40 and protease inhibitors. After vortexing, lung tissue suspensions were centrifuged at 4°C for 15 min at 13,000 x g. The supernatant containing mouse lung proteins was transferred to a new tube and stored at - 70°C. The concentration of protein in the samples was determined using the Bradford method with BSA as a standard.

The mouse-VEGF was determined quantitatively using ELISA kit (Cat # MMV00) provided by R&D Systems Inc. (Minneapolis, MN, USA). Briefly, 50 µg of protein in 50µl Assay Diluent RD1N was tested in each well, each sample was duplicated. The assay was conducted following the standard procedure provided by the manufacturer.

### EXAMPLE 25

### Lung cancer study

### Cell line and mice:

B16F10 murine melanoma cells were purchased from ATCC and cultured in DMEM-10%FBS. Cell monolayer was trypsinized and neutralized with MEM-10%FBS. Cells were washed with PBS for two times and determined for viability by trypan blue staining. B16F10 cells were diluted to 1x10⁶ viable cells/ml in PB S. 200ul of cells was injected into each mouse via tail vein.

C57BL/6NCRL mice were purchased from Charles River, Quebec, Canada at 5-7 weeks of age.

### Construction of eIF-5A1 vectors:

pCpG-lacZ vector lacking CpG dinucleotides was purchased from InvivoGen, San Diego, USA. To subclone eIF5A1 and eIF5A1mutant into pCpG-lacZ vector, pCpG-lacZ plasmid DNA was digested with NcoI and NheI, a 3.1kb of pCpG vector backbone without lacZ gene coding sequence was isolated and ligated with PCR amplified eIF5A1 or eIF5A1mutant using primers eIF5A1 for:
5'-GCTCCATGGCAGATGATTTGGACTTCG-3' (SEQ ID NO: 114)
and eIF5A1 rev: 5'-CGCGCTAGCCAGTTATTTTGCCATCGCC-3'. (SEQ ID NO: 115).
Constructed pCpG-eIF5A1 and pCpG-eIF5A1mutant were amplified in *E. coli* GT115 cultured in LB or 2XYT medium containing 25 µg/ml of zeocin. pCpG-HA-eIF5A1 was constructed using the same strategy with primers HA-5A1 for:
5'-GCTCCATGGATGTACCCATACGACGTCCC-3' (SEQ ID NO: 116) and eIF5A1 rev.

The plasmids were extracted and purified by QIAGEN Endofree Plasmid Giga kit. The DNA concentration was measured by UV absorption at 260nm and agarose gel electrophoresis.

### Tail vein injection:

Plasmid DNAs in 1x PBS (around 200 µl based on body weight) were injected into each mouse in groups 3-8 and 1x PBS into each mouse in groups 1-2 at day 2, 4, 7, 11, 16, 21; 26, 31. Plasmid DNA concentration was 660 ng/µl for 2x (6.6mg/kg), 330 ng/µl for 1x (3.3mg/kg), and 33 ng/µl for 0.1x (0.33mg/kg).

### Body and lung weights:

Body weights were measured before tail vein injection or every Monday and Friday. Mice were euthanized with CO₂ when they reached morbidity (lethargic, respiratory distress) and lungs were removed, weighed, photographed, frozen, and stored at-70°C.

Mammalian DNA differs from bacterial DNA in that in contains a low frequency of cytidine-phosphate-guanosine (CpG) dinucleotides which are usually methylated. Bacterial DNA on the other hand contains frequent, unmethylated CpG dinucleotides which, when present in a specific sequence context, can stimulate the vertebrate immune system. The unmethylated CpGs in bacterial DNA are recognized by the Toll-like receptor (TLR) 9 and initiate a signaling cascade that results in the production of proinflammatory cytokines such as IL-6 and IL-121,2. Since plasmids are produced in E. coli, any CpG motifs present in the plasmid will remain unmethylated and be potentially immunostimulatory if introduced in vivo. Immunostimulatory CpGs have also been shown to lead to the rapid decline of transgene expression in vivo. Another limitation of traditional gene therapy vectors is the loss of transcriptional activity of the cytomegalovirus early gene promoter (CMV) within a few weeks in vivo3,4,5, thus limiting the it's use for repeated administration and sustained transgene expression.

In order to circumvent some of the roadblocks of traditional DNA plasmid gene therapy, we have opted to use a plasmid from Invivogen called pCpG that is completely devoid of CpG dinucleotides. The vector also makes use of a robust cellular promoter (the human elongation factor 1 alpha core promoter with a mouse CMV enhancer) instead of the widely used CMV promoter which, in the context of a CpG-reduced backbone, should increase the duration of transgene expression in vivo as well as decrease the inflammatory response to plasmid DNA. The control plasmid CpG-LacZ is completely devoid of CpG dinucleotides while the vectors containing eIF5A1 have 14 CpG dinucleotides in the eIF5A cDNA which may or may not be immunostimulatory.

### REFERENCES

1. Park, M. H., Wolff, E. C. and Folk, J. E. (1993) BioFactors. 4, 95-104.
2. Schnier, J., Schwelberger, H. G., Smit-McBride, Z., Kang, H. A., and Hershey, J. W. (1991) Mol Cell Biol. 11, 3105-3114.
3. Jakus, J., Wolff, E. C., Park, M. H., and Folk, J. E. (1993) J Biol Chem. 268, 13151-13159.
4. Kang, H. A., and Hershey, J. W. (1994) J Biol Chem. 269, 3934-3940.
5. Hanauske-Abel, H. M., Slowinska, B., Zagulska, S., Wilson, R. C., Staiano-Coico, L., Hanauske, A. R., McCaffrey, T., and Szabo, P. (1995) FEBS Lett. 366, 92-98.
6. Chen, Z. P., Yan, Y. P., Ding, Q. J., Knapp, S., Potenza, J. A., Schugar, H. J., and Chen, K. Y. (1996) Cancer Lett. 105, 233-239.
7. Sasaki, K., Abid, M. R., and Miyazaki, M. (1996) FEBS Lett. 384, 151-154.
8. Shi, X. P., Yin, K. C., Ahern, J., Davis, L. J., Stem, A. M., and Waxman, L. (1996a) Biochim Biophys Acta. 1310, 119-126.
9. Park, M. H., Joe, Y. A., and Kang, K. R. (1998) J Biol Chem. 273, 1677-1683.
10. Xu, A. and Chen, K.Y. (2001) J. Biol. Chem. 276, 2555-2561.
11. Xu, A., Jao, D. L., and Chen, K. Y. (2004) Biochem J. 384, 585-590.
12. Taylor, C. A., Senchyna, M., Flanagan, J., Joyce, E. M., Cliche, D. O., Boone, A. N., Culp- Stewart, S., and Thompson, J. E. (2004) Invest Ophthalmol Vis Sci. 45, 3568-3576.
13. Li, A., Li, H.-Y., Jin, B.-F., Ye, Q.-N., Zhou, T., Yu, X.-D., Pan, X., Man, J.-H. et al. (2004) J Biol Chem. 279, 49251-49258.
14. Nigro, J. M., Baker, S. J., Preisinger, A. C., Jessup, J. M., Hostetter, R., Cleary, K., Bigner, S. H., Davidson, N., Baylin, S., Devilee, P., et al. (1989) Nature. 342, 705-708.
15. Shieh, S. Y., Ikeda, M., Taya, Y., and Prives, C. (1997) Cell. 91, 325-334.
16. Fu, L., Minden, M. D., and Benchimol, S. (1996) EMBO J. 15, 4392-4401.
17. Fu, L., and Benchimol, S. (1997) EMBO J. 16, 4117-4125.
18. Smith, M. L., Chen, I. T., Zhan, Q., O'Connor, P. M., and Fornace, A. J. Jr. (1995) Oncogene. 10, 1053-1059.
19. Caraglia, M., Marra, M., Giuberti, G., D'Alessandro, A. M., Baldi, A., Tassone, P., Venuta, S., Tagliaferri, P., and Abbruzzese, A. (2003) J Biochem (Tokyo) 133, 757-765.
20. Kim, K. K., Hung, L. W., Yokota, H., Kim, R., and Kim, S. H. (1998) Proc Natl Acad Sci U S A. 95, 10419-10424.
21. Shi, X. P., Yin, K. C., Zimolo, Z. A., Stem, A. M., and Waxman, L. (1996b) Exp Cell Res. 225, 348-356.
22. Shi, X. P., Yin, K. C., and Waxman, L. (1997) Biol Signals. 6, 143-149.
23. Valentini, S. R., Casolari, J. M., Oliveira, C. C., Silver, P. A., and McBride, A. E. (2002) Genetics. 160, 393-405.
24. Jin, B. F., He, K., Wang, H. X., Wang, J., Zhou, T., Lan, Y., Hu, M. R., Wei, K. H., Yang, S. C., Shen, B. F., and Zhang, X. M. (2003) Oncogene. 22, 4819-4830.
25. Cracchiolo, B. M., Heller, D. S., Clement, P. M., Wolff, E. C., Park, M. H., and Hanauske-Abel, H. M. (2004) Gynecol Oncol. 94, 217-222 8.
26. Abreu-Martin, M., Vidrich, A., Lynch, D., and Targan, S. (1995) J. Immunol. 155, 4147-4154.
27. Ossina, N. K., Cannas, A., Powers, V. C., Fitzpatrick, P. A., Knight, J. D., Gilbert, J. R., Shekhtman, E. M., Tomei, L. D., Umansky, S. R., and Kiefer, M. C. (1997) J Biol Chem. 272, 16351-16357.
28. Wright, K., Kolios, G., Westwick, J., and Ward, S. G. (1999) J Biol Chem. 274, 17193-17201.
29. Fiorucci, S., Distrutti, E., Ajuebor, M. N., Mencarelli, A., Mannucci, R., Palazzetti, B., Del Soldato, P., Morelli, A., and Wallace, J. L. (2001) Am J Physiol Gastrointest Liver Physiol. 281, G654-G665.
30. Lutz, N. W., Tome, M. E, and Cozzone, P. J. (2003) FEBS Lett. 544, 123-128.
31. Liu, Y. P., Nemeroff, M., Yan, Y. P. and Chen, K. Y. (1997) Biol. Signals 6, 166-174.
32. Chen, G., Gharib, T., Thomas, D., Huang, C., Misek, D., Kuick, R., Giordano, T., lannettoni, M., Orringer, M., Hanash, S., and Beer, D. (2003) Proteomics. 3, 496-504.
33. Hauber, I., Bevec, D., Heukeshoven, J., Kratzer, F., Horn, F., Choidas, A., Harrer, T., and Hauber, J. (2005) J Clin Invest. 115, 76-85.
34. Mazan-Mamczarz, K., Galban, S., Lopez de Silanes, I., Martindale, J. L., Atasoy, U., Keene, J. D., and Gorospe, M. (2003) Proc Natl Acad Sci USA. 100, 8354-8359.
35. Fu, L., Ma, W., and Benchimol, S. (1999) Oncogene. 18, 6419-6424.
36. Ruhl, M., Himmelspach, M., Bahr, G. M., Hammerschmid, F., Jaksche, H., Wolff, B., Aschauer, H., Farrington, G. K., Probst, H., Bevec, D., et al. (1993) J Cell Biol. 123, 1309-1320.
37. Rosorius, O., Reichart, B., Kratzer, F., Heger, P., Dabauvalle, M. C., and Hauber, J. (1999) J Cell Sci. 112, 2369-2380.
38. Jao, D. L.-E., and Chen, K. Y. (2002) J Cell Biochem. 86, 590-600.
39. Lipowsky, G., Bischoff, F., Schwarzmaier, P., Kraft, R., Kostka, S., Hartmann, E., Kutay, U., and Gorlich, D. (2000) EMBO J. 19, 4362-4371.
40. Tome, M., Fiser, S., Payne, C., and Gerner, E. (1997a) Biochem. J. 328, 847-854.
41. Tome, M. E., and Gerner E. W. (1997b) Biol Signals. 6, 150-156.
42. Caraglia, M., Passeggio, A., Beninati, S., Leardi, A., Nicolini, L., Improta, S., Pinto, A., Bianco, A. R., Tagliaferri, P., and Abbruzzese, A. (1997) Biochem J. 324, 737-741.
43. Beninati, S., Gentile, V., Caraglia, M., Lentini, A., Tagliaferri, P., and Abbruzzese, A. (1998) FEBS Lett. 437, 34-38.
44. Gordon, E. D., Mora, R., Meredith, S. C., and Lindquist; S. L. (1987) J. Biol. Chem. 262, 16590- 16595.

## Claims

1. Use of a polynucleotide encoding apoptosis-specific eIF-5A to manufacture a medicament to increase endogenous expression of apoptosis-specific eIF-5A in a cell of a mammal.

2. The use of claim 1 wherein the cell is a cancer cell.

3. The use of claim 2 wherein the cell is a lung cancer cell.

4. The use of claim 2 wherein the cell is a nasopharyngal cancer cell.

5. The use of claim 2 to 4, wherein the increase in endogenous expression of apoptosis-specific eIF-5A causes apoptosis in the cancer cell.

6. The use of claim 1 wherein the increase in endogenous expression of apoptosis-specific eIF-5A causes a decrease in expression of VEGF.

7. The use of claim 5 wherein the decrease in expression of VEGF leads to a decrease in angiogenesis of a tumor.


SEQUENCE LISTING

<110> TAYLOR, CATHERINE
      CLICHE, DOMINIC
      HEIKKILA, ELIZABETH MARGARET
      SENCHYNA, DIANE MICHELLE
      FLANAGAN, JOHN GERARD
      THOMPSON, JOHN E.

<120> INHIBITION OF APOPTOSIS-SPECIFIC eIF-5A ("eIF-5A1") WITH
      ANTISENSE OLIGONUCLEOTIDES AND siRNAs AS ANTI-INFLAMMATORY
      THERAPEUTICS

<130> 10799/125

<140> 10/861,980
<141> 2004-06-07

<150> 10/792,893
<151> 2004-03-05

<150> 10/383,614
<151> 2003-03-10

<150> 10/277,969
<151> 2002-10-23

<150> 10/200,148
<151> 2002-07-23

<150> 10/141,647
<151> 2002-05-07

<150> 09/909,796
<151> 2001-07-23

<150> 60/476,194
<151> 2003-06-06

<150> 60/504,731
<151> 2003-09-22

<150> 60/528,249
<151> 2003-12-10

<150> 60/557,671
<151> 2004-03-31

<150> 60/75,814
<151> 2004-06-02

<160> 95

<170> PatentIn Ver. 3.2

<210> 1
<211> 1139

<212> DNA
<213> Rattus sp.

<220>
<221> CDS
<222> (33)..(494)

<400> 1
   caggtctaga gttggaatcg aagcctctta aa atg gca gat gat ttg gac ttc 53
   Met Ala Asp Asp Leu Asp Phe
   1 5
   gag aca gga gat gca ggg gcc tca gcc acc ttc cca atg cag tgc tca 101
   Glu Thr Gly Asp Ala Gly Ala Ser Ala Thr Phe Pro Met Gln Cys Ser
   10 15 20
   gca tta cgt aag aat ggt ttt gtg gtg ctc aag ggc cgg cca tgt aag 149
   Ala Leu Arg Lys Asn Gly Phe Val Val Leu Lys Gly Arg Pro Cys Lys
   25 30 35
   atc gtc gag atg tct act tcg aag act ggc aag cat ggc cat gcc aag 197
   Ile Val Glu Met Ser Thr Ser Lys Thr Gly Lys His Gly His Ala Lys
   40 45 50 55
   gtc cat ctg gtt ggt att gat att ttt act ggg aag aaa tat gaa gat 245
   Val His Leu Val Gly Ile Asp Ile Phe Thr Gly Lys Lys Tyr Glu Asp
   60 65 70
   atc tgc ccg tcg act cat aac atg gat gtc ccc aac atc aaa agg aat 293
   Ile Cys Pro Ser Thr His Asn Met Asp Val Pro Asn Ile Lys Arg Asn
   75 80 85
   gat ttc cag ctg att ggc atc cag gat ggg tac cta tcc ctg ctc cag 341
   Asp Phe Gln Leu Ile Gly Ile Gln Asp Gly Tyr Leu Ser Leu Leu Gln
   90 95 100
   gac agt ggg gag gta cga gag gac ctt cgt ctg cct gag gga gac ctt 389
   Asp Ser Gly Glu Val Arg Glu Asp Leu Arg Leu Pro Glu Gly Asp Leu
   105 110 115
   ggc aag gag att gag cag aag tat gac tgt gga gaa gag atc ctg atc 437
   Gly Lys Glu Ile Glu Gln Lys Tyr Asp Cys Gly Glu Glu Ile Leu Ile
   120 125 130 135
   aca gtg ctg tcc gcc atg aca gag gag gca gct gtt gca atc aag gcc 485
   Thr Val Leu Ser Ala Met Thr Glu Glu Ala Ala Val Ala Ile Lys Ala
   140 145 150
   atg gca aaa taactggctt ccagggtggc ggtggtggca gcagtgatcc 534
   Met Ala Lys
   atgagcctac agaggcccct cccccagctc tggctgggcc cttggctgga ctcctatcca 594
   atttatttga cgttttattt tggttttcct caccccttca aactgtcggg gagaccctgc 654
   ccttcaccta gctcccttgg ccaggcatga gggagccatg gccttggtga agctacctgc 714
   ctcttctctc gcagccctga tgggggaaag ggagtgggta ctgcctgtgg tttaggttcc 774
   cctctccctt tttcttttta attcaatttg gaatcagaaa gctgtggatt ctggcaaatg 834
   gtcttgtgtc ctttatccca ctcaaaccca tctggtcccc tgttctccat agtccttcac 894
   ccccaagcac cactgacaga ctggggacca gcccccttcc ctgcctgtgt ctcttcccaa 954
   acccctctat aggggtgaca agaagaggag ggggggaggg gacacgatcc ctcctcaggc 1014
   atctgggaag gccttgcccc catgggcttt accctttcct gtgggctttc tccctgacac 1074
   atttgttaaa aatcaaacct gaataaaact acaagtttaa tatgaaaaaa aaaaaaaaaa 1134
   aaaaa 1139

<210> 2
<211> 154
<212> PRT
<213> Rattus sp.

<400> 2
Met Ala Asp Asp Leu Asp Phe Glu Thr Gly Asp Ala Gly Ala Ser Ala
1               5                   10                  15

Thr Phe Pro Met Gln Cys Ser Ala Leu Arg Lys Asn Gly Phe Val Val
            20                  25                  30

Leu Lys Gly Arg Pro Cys Lys Ile Val Glu Met Ser Thr Ser Lys Thr
        35                  40                  45

Gly Lys His Gly His Ala Lys Val His Leu Val Gly Ile Asp Ile Phe
    50                  55                  60

Thr Gly Lys Lys Tyr Glu Asp Ile Cys Pro Ser Thr His Asn Met Asp
65                  70                  75                  80

Val Pro Asn Ile Lys Arg Asn Asp Phe Gln Leu Ile Gly Ile Gln Asp
                85                  90                  95

Gly Tyr Leu Ser Leu Leu Gln Asp Ser Gly Glu Val Arg Glu Asp Leu
            100                 105                 110

Arg Leu Pro Glu Gly Asp Leu Gly Lys Glu Ile Glu Gln Lys Tyr Asp
        115                 120                 125

Cys Gly Glu Glu Ile Leu Ile Thr Val Leu Ser Ala Met Thr Glu Glu
    130                 135                 140

Ala Ala Val Ala Ile Lys Ala Met Ala Lys
145                 150

<210> 3
<211> 462

<212> DNA
<213> Homo sapiens

<400> 3
atggcagatg acttggactt cgagacagga gatgcagggg cctcagccac cttcccaatg 60
cagtgctcag cattacgtaa gaatggcttt gtggtgctca aaggccggcc atgtaagatc 120
gtcgagatgt ctacttcgaa gactggcaag cacggccacg ccaaggtcca tctggttggt 180
attgacatct ttactgggaa gaaatatgaa gatatctgcc cgtcaactca taatatggat 240
gtccccaaca tcaaaaggaa tgacttccag ctgattggca tccaggatgg gtacctatca 300
ctgctccagg acagcgggga ggtacgagag gaccttcgtc tccctgaggg agaccttggc 360
aaggagattg agcagaagta cgactgtgga gaagagatcc tgatcacggt gctgtctgcc 420
atgacagagg aggcagctgt tgcaatcaag gccatggcaa aa 462

<210> 4
<211> 460
<212> DNA
<213> Homo sapiens

<400> 4
atggcagacg aaattgattt cactactgga gatgccgggg cttccagcac ttaccctatg 60
cagtgctcgg ccttgcgcaa aaacggcttc gtggtgctca aaggacgacc atgcaaaata 120
gtggagatgt caacttccaa aactggaaag catggtcatg ccaaggttca ccttgttgga 180
attgatattt tcacgggcaa aaaatatgaa gatatttgtc cttctactca caacatggat 240
gttccaaata ttaagagaaa tgattatcaa ctgatatgca ttcaagatgg ttacctttcc 300
ctgctgacag aaactggtga agttcgtgag gatcttaaac tgccagaagg tgaactaggc 360
aaagaaatag agggaaaata caatgcaggt gaagatgtac aggtgtctgt catgtgtgca 420
atgagtgaag aatatgctgt agccataaaa ccctgcaaat 460

<210> 5
<211> 462
<212> DNA
<213> Rattus sp.

<400> 5
atggcagatg atttggactt cgagacagga gatgcagggg cctcagccac cttcccaatg 60
cagtgctcag cattacgtaa gaatggtttt gtggtgctca aaggccggcc atgtaagatc 120
gtcgagatgt ctacttcgaa gactggcaag catggccatg ccaaggtcca tctggttggc 180
attgacattt ttactgggaa gaaatatgaa gatatctgcc cgtcgactca taatatggat 240
gtccccaaca tcaaacggaa tgacttccag ctgattggca tccaggatgg gtacctatcc 300
ctgctccagg acagtgggga ggtacgagag gaccttcgtc tgcctgaagg agaccttggc 360
aaggagattg agcagaagta tgactgtgga gaagagatcc tgatcacagt gctgtctgcc 420
atgacagagg aggcagctgt tgcaatcaag gccatggcaa aa 462

<210> 6
<211> 606
<212> DNA
<213> Rattus sp.

<220>
<221> CDS
<222> (1)..(453)

<400> 6
gct gtg tat tat tgg gcc cat aag aac cac ata cct gtg ctg agt cct 48

Ala Val Tyr Tyr Trp Ala His Lys Asn His Ile Pro Val Leu Ser Pro
1               5                   10                  15

gca ctc aca gac ggc tca ctg ggt gac atg atc ttt ttc cat tcc tat     96
Ala Leu Thr Asp Gly Ser Leu Gly Asp Met Ile Phe Phe His Ser Tyr
            20                  25                  30

aaa aac cca ggc ttg gtc ctg gac atc gtt gaa gac ctg cgg ctc atc     144
Lys Asn Pro Gly Leu Val Leu Asp Ile Val Glu Asp Leu Arg Leu Ile
        35                  40                  45

aac atg cag gcc att ttc gcc aag cgc act ggg atg atc atc ctg ggt     192
Asn Met Gln Ala Ile Phe Ala Lys Arg Thr Gly Met Ile Ile Leu Gly
    50                  55                  60

gga ggc gtg gtc aag cac cac atc gcc aat gct aac ctc atg cgg aat     240
Gly Gly Val Val Lys His His Ile Ala Asn Ala Asn Leu Met Arg Asn
65                  70                  75                  80

gga gct gac tac gct gtt tat atc aac aca gcc cag gag ttt gat ggc     288
Gly Ala Asp Tyr Ala Val Tyr Ile Asn Thr Ala Gln Glu Phe Asp Gly
                85                  90                  95

tca gac tca gga gcc cgg cca gat gag gct gtc tcc tgg ggc aag atc     336
Ser Asp Ser Gly Ala Arg Pro Asp Glu Ala Val Ser Trp Gly Lys Ile
            100                 105                 110

cgg atg gat gca cag cca gta aag gtc tat gct gat gca tct ctg gtt     384
Arg Met Asp Ala Gln Pro Val Lys Val Tyr Ala Asp Ala Ser Leu Val
        115                 120                 125

ttc ccc ttg ctg gtg gct gag aca ttc gcc caa aag gca gat gcc ttc     432
Phe Pro Leu Leu Val Ala Glu Thr Phe Ala Gln Lys Ala Asp Ala Phe
    130                 135                 140

aga gct gag aag aat gag gac tgagcagatg ggtaaagacg gaggcttctg        483
Arg Ala Glu Lys Asn Glu Asp
145                 150

ccacaccttt atttattatt tgcataccaa cccctcctgg gccctctcct tggtcagcag 543

catcttgaga ataaatggcc tttttgttgg tttctgtaaa aaaaggactt taaaaaaaaa 603

aaa                                                               606

<210> 7
<211> 151
<212> PRT
<213> Rattus sp.

<400> 7
Ala Val Tyr Tyr Trp Ala His Lys Asn His Ile Pro Val Leu Ser Pro
1               5                   10                  15

Ala Leu Thr Asp Gly Ser Leu Gly Asp Met Ile Phe Phe His Ser Tyr
            20                  25                  30

Lys Asn Pro Gly Leu Val Leu Asp Ile Val Glu Asp Leu Arg Leu Ile
        35                  40                  45

Asn Met Gln Ala Ile Phe Ala Lys Arg Thr Gly Met Ile Ile Leu Gly
    50                  55                  60

Gly Gly Val Val Lys His His Ile Ala Asn Ala Asn Leu Met Arg Asn
65                  70                  75                  80

Gly Ala Asp Tyr Ala Val Tyr Ile Asn Thr Ala Gln Glu Phe Asp Gly
                85                  90                  95

Ser Asp Ser Gly Ala Arg Pro Asp Glu Ala Val Ser Trp Gly Lys Ile
            100                 105                 110

Arg Met Asp Ala Gln Pro Val Lys Val Tyr Ala Asp Ala Ser Leu Val
        115                 120                 125

Phe Pro Leu Leu Val Ala Glu Thr Phe Ala Gln Lys Ala Asp Ala Phe
    130                 135                 140

Arg Ala Glu Lys Asn Glu Asp
145                 150

<210> 8
<211> 453
<212> DNA
<213> Homo sapiens

<400> 8
tccgtgtatt actgggccca gaagaaccac atccctgtgt ttagtcccgc acttacagac 60
ggctcgctgg gcgacatgat cttcttccat tcctacaaga acccgggcct ggtcctggac 120
atcgttgagg acctgaggct catcaacaca caggccatct ttgccaagtg cactgggatg 180
atcattctgg gcgggggcgt ggtcaagcac cacattgcca atgccaacct catgcggaac 240
ggggccgact acgctgttta catcaacaca gcccaggagt ttgatggctc tgactcaggt 300
gcccgaccag acgaggctgt ctcctggggc aagatccggg tggatgcaca gcccgtcaag 360
gtctatgctg acgcctccct ggtcttcccc ctgcttgtgg ctgaaacctt tgcccagaag 420
atggatgcct tcatgcatga gaagaacgag gac                              453

<210> 9
<211> 20
<212> DNA
<213> Artificial Sequence

<220>
<223> Description of Artificial Sequence: Synthetic
      Primer

<220>
<221> modified_base
<222> (12)
<223> a, t, c or g

<400> 9
tcsaarachg gnaagcaygg                                                20

<210> 10
<211> 42
<212> DNA
<213> Artificial Sequence

<220>
<223> Description of Artificial Sequence: Synthetic
      Primer

<400> 10
gcgaagcttc catggctcga gttttttttt tttttttttt tt                        42

<210> 11
<211> 972
<212> DNA
<213> Rattus sp.

<220>
<221> CDS
<222> (1)..(327)

<400> 11
   tcg aag acc ggt aag cac ggc cat gcc aag gtc cat ctg gtt ggt att    48
   Ser Lys Thr Gly Lys His Gly His Ala Lys Val His Leu Val Gly Ile
     1               5                  10                  15
   gat att ttt act ggg aag aaa tat gaa gat atc tgc ccg tcg act cat    96
   Asp Ile Phe Thr Gly Lys Lys Tyr Glu Asp Ile Cys Pro Ser Thr His
                20                  25                  30
   aac atg gat gtc ccc aac atc aaa agg aat gat ttc cag ctg att ggc    144
   Asn Met Asp Val Pro Asn Ile Lys Arg Asn Asp Phe Gln Leu Ile Gly
            35                  40                  45
   atc cag gat ggg tac cta tcc ctg ctc cag gac agt ggg gag gta cga    192
   Ile Gln Asp Gly Tyr Leu Ser Leu Leu Gln Asp Ser Gly Glu Val Arg
        50                  55                  60
   gag gac ctt cgt ctg cct gag gga gac ctt ggc aag gag att gag cag    240
   Glu Asp Leu Arg Leu Pro Glu Gly Asp Leu Gly Lys Glu Ile Glu Gln
    65                  70                  75                  80
   aag tat gac tgt gga gaa gag atc ctg atc aca gtg ctg tcc gcc atg    288
   Lys Tyr Asp Cys Gly Glu Glu Ile Leu Ile Thr Val Leu Ser Ala Met
                    85                  90                  95
   aca gag gag gca gct gtt gca atc aag gcc atg gca aaa taactggctt    337
   Thr Glu Glu Ala Ala Val Ala Ile Lys Ala Met Ala Lys
               100                 105
   ccagggtggc ggtggtggca gcagtgatcc atgagcctac agaggcccct cccccagctc 397
   tggctgggcc cttggctgga ctcctatcca atttatttga cgttttattt tggttttcct 457
   caccccttca aactgtcggg gagaccctgc ccttcaccta gctcccttgg ccaggcatga 517
   gggagccatg gccttggtga agctacctgc ctcttctctc gcagccctga tgggggaaag 577
   ggagtgggta ctgcctgtgg tttaggttcc cctctccctt tttcttttta attcaatttg 637
   gaatcagaaa gctgtggatt ctggcaaatg gtcttgtgtc ctttatccca ctcaaaccca 697
   tctggtcccc tgttctccat agtccttcac ccccaagcac cactgacaga ctggggacca 757
   gcccccttcc ctgcctgtgt ctcttcccaa acccctctat aggggtgaca agaagaggag 817
   ggggggaggg gacacgatcc ctcctcaggc atctgggaag gccttgcccc catgggcttt 877
   accctttcct gtgggctttc tccctgacac atttgttaaa aatcaaacct gaataaaact 937
   acaagtttaa tatgaaaaaa aaaaaaaaaa aaaaa 972

<210> 12
<211> 109
<212> PRT
<213> Rattus sp.

<400> 12
Ser Lys Thr Gly Lys His Gly His Ala Lys Val His Leu Val Gly Ile
1               5                   10                  15

Asp Ile Phe Thr Gly Lys Lys Tyr Glu Asp Ile Cys Pro Ser Thr His
            20                  25                  30

Asn Met Asp Val Pro Asn Ile Lys Arg Asn Asp Phe Gln Leu Ile Gly
        35                  40                  45

Ile Gln Asp Gly Tyr Leu Ser Leu Leu Gln Asp Ser Gly Glu Val Arg
    50                  55                  60

Glu Asp Leu Arg Leu Pro Glu Gly Asp Leu Gly Lys Glu Ile Glu Gln
65                  70                  75                  80

Lys Tyr Asp Cys Gly Glu Glu Ile Leu Ile Thr Val Leu Ser Ala Met
                85                  90                  95

Thr Glu Glu Ala Ala Val Ala Ile Lys Ala Met Ala Lys
            100                 105

<210> 13
<211> 24
<212> DNA
<213> Artificial Sequence

<220>
<223> Description of Artificial Sequence: Synthetic

Primer

<400> 13
caggtctaga gttggaatcg aagc                                              24


<210> 14
<211> 30
<212> DNA
<213> Artificial Sequence

<220>
<223> Description of Artificial Sequence: Synthetic
      Primer

<400> 14
atatctcgag ccttgattgc aacagctgcc                                        30


<210> 15
<211> 489
<212> DNA
<213> Rattus sp.

<220>
<221> CDS
<222> (33)..(485)

<400> 15
caggtctaga gttggaatcg aagcctctta aa atg gca gat gat ttg gac ttc      53
                                    Met Ala Asp Asp Leu Asp Phe
                                      1               5

gag aca gga gat gca ggg gcc tca gcc acc ttc cca atg cag tgc tca      101
Glu Thr Gly Asp Ala Gly Ala Ser Ala Thr Phe Pro Met Gln Cys Ser
         10                  15                  20

gca tta cgt aag aat ggt ttt gtg gtg ctc aag ggc cgg cca tgt aag      149
Ala Leu Arg Lys Asn Gly Phe Val Val Leu Lys Gly Arg Pro Cys Lys
     25                  30                  35

atc gtc gag atg tct act tcg aag act ggc aag cat ggc cat gcc aag      197
Ile Val Glu Met Ser Thr Ser Lys Thr Gly Lys His Gly His Ala Lys
 40                  45                  50                  55

gtc cat ctg gtt ggt att gat att ttt act ggg aag aaa tat gaa gat      245
Val His Leu Val Gly Ile Asp Ile Phe Thr Gly Lys Lys Tyr Glu Asp
                 60                  65                  70

atc tgc ccg tcg act cat aac atg gat gtc ccc aac atc aaa agg aat      293
Ile Cys Pro Ser Thr His Asn Met Asp Val Pro Asn Ile Lys Arg Asn
             75                  80                  85

gat ttc cag ctg att ggc atc cag gat ggg tac cta tcc ctg ctc cag      341
Asp Phe Gln Leu Ile Gly Ile Gln Asp Gly Tyr Leu Ser Leu Leu Gln
         90                  95                  100

gac agt ggg gag gta cga gag gac ctt cgt ctg cct gag gga gac ctt     389
Asp Ser Gly Glu Val Arg Glu Asp Leu Arg Leu Pro Glu Gly Asp Leu
    105                 110                 115

ggc aag gag att gag cag aag tat gac tgt gga gaa gag atc ctg atc     437
Gly Lys Glu Ile Glu Gln Lys Tyr Asp Cys Gly Glu Glu Ile Leu Ile
120                 125                 130                 135

aca gtg ctg tcc gcc atg aca gag gag gca gct gtt gca atc aag gct     485
Thr Val Leu Ser Ala Met Thr Glu Glu Ala Ala Val Ala Ile Lys Ala
                140                 145                 150

cgag                                                                489

<210> 16
<211> 151
<212> PRT
<213> Rattus sp.

<400> 16
Met Ala Asp Asp Leu Asp Phe Glu Thr Gly Asp Ala Gly Ala Ser Ala
  1               5                  10                  15

Thr Phe Pro Met Gln Cys Ser Ala Leu Arg Lys Asn Gly Phe Val Val
             20                  25                  30

Leu Lys Gly Arg Pro Cys Lys Ile Val Glu Met Ser Thr Ser Lys Thr
         35                  40                  45

Gly Lys His Gly His Ala Lys Val His Leu Val Gly Ile Asp Ile Phe
     50                  55                  60

Thr Gly Lys Lys Tyr Glu Asp Ile Cys Pro Ser Thr His Asn Met Asp
 65                  70                  75                  80

Val Pro Asn Ile Lys Arg Asn Asp Phe Gln Leu Ile Gly Ile Gln Asp
                 85                  90                  95

Gly Tyr Leu Ser Leu Leu Gln Asp Ser Gly Glu Val Arg Glu Asp Leu
            100                 105                 110

Arg Leu Pro Glu Gly Asp Leu Gly Lys Glu Ile Glu Gln Lys Tyr Asp
        115                 120                 125

Cys Gly Glu Glu Ile Leu Ile Thr Val Leu Ser Ala Met Thr Glu Glu
    130                 135                 140

Ala Ala Val Ala Ile Lys Ala
145                 150

<210> 17
<211> 20
<212> DNA
<213> Artificial Sequence

<220>
<223> Description of Artificial Sequence: Synthetic
      Primer

<400> 17
gtctgtgtat tattgggccc                                                  20


<210> 18
<211> 42
<212> DNA
<213> Artificial Sequence

<220>
<223> Description of Artificial Sequence: Synthetic
      Primer

<400> 18
gcgaagcttc catggctcga gttttttttt tttttttttt tt                         42


<210> 19
<211> 1299
<212> DNA
<213> Homo sapiens

<400> 19
ggcacgaggg cggcggcggc ggtagaggcg gcggcggcgg cggcagcggg ctcggaggca 60
gcggttgggc tcgcggcgag cggacggggt cgagtcagtg cgttcgcgcg agttggaatc 120
gaagcctctt aaaatggcag atgacttgga cttcgagaca ggagatgcag gggcctcagc 180
caccttccca atgcagtgct cagcattacg taagaatggc tttgtggtgc tcaaaggccg 240
gccatgtaag atcgtcgaga tgtctacttc gaagactggc aagcacggcc acgccaaggt 300
ccatctggtt ggtattgaca tctttactgg gaagaaatat gaagatatct gcccgtcaac 360
tcataatatg gatgtcccca acatcaaaag gaatgacttc cagctgattg gcatccagga 420
tgggtaccta tcactgctcc aggacagcgg ggaggtacga gaggaccttc gtctccctga 480
gggagacctt ggcaaggaga ttgagcagaa gtacgactgt ggagaagaga tcctgatcac 540
ggtgctgtct gccatgacag aggaggcagc tgttgcaatc aaggccatgg caaaataact 600
ggctcccagg atggcggtgg tggcagcagt gatcctctga acctgcagag gccccctccc 660
cgagcctggc ctggctctgg cccggtccta agctggactc ctcctacaca atttatttga 720
cgttttattt tggttttccc caccccctca atctgtcggg gagcccctgc ccttcaccta 780
gctcccttgg ccaggagcga gcgaagctgt ggccttggtg aagctgccct cctcttctcc 840
cctcacacta cagccctggt gggggagaag ggggtgggtg ctgcttgtgg tttagtcttt 900
tttttttttt tttttttttt tttaaattca atctggaatc agaaagcggt ggattctggc 960
aaatggtcct tgtgccctcc ccactcatcc ctggtctggt cccctgttgc ccatagccct 1020
ttaccctgag caccacccca acagactggg gaccagcccc ctcgcctgcc tgtgtctctc 1080
cccaaacccc tttagatggg gagggaagag gaggagaggg gaggggacct gccccctcct 1140
caggcatctg ggagggccct gcccccatgg gctttaccct tccctgcggg ctctctcccc 1200
gacacatttg ttaaaatcaa acctgaataa aactacaagt ttaatatgaa aaaaaaaaaa 1260
aaaaaaaaaa aaaaaaaaaa aaaaaaaaaa aaaaaaaaa                             1299


<210> 20
<211> 462
<212> DNA
<213> Rattus sp.

<400> 20
atggcagatg atttggactt cgagacagga gatgcagggg cctcagccac cttcccaatg 60
cagtgctcag cattacgtaa gaatggtttt gtggtgctca agggccggcc atgtaagatc 120
gtcgagatgt ctacttcgaa gactggcaag catggccatg ccaaggtcca tctggttggt 180
attgatattt ttactgggaa gaaatatgaa gatatctgcc cgtcgactca taacatggat 240
gtccccaaca tcaaaaggaa tgatttccag ctgattggca tccaggatgg gtacctatcc 300
ctgctccagg acagtgggga ggtacgagag gaccttcgtc tgcctgaggg agaccttggc 360
aaggagattg agcagaagta tgactgtgga gaagagatcc tgatcacagt gctgtccgcc 420
atgacagagg aggcagctgt tgcaatcaag gccatggcaa aa 462

<210> 21
<211> 154
<212> PRT
<213> Homo sapiens

<400> 21
Met Ala Asp Asp Leu Asp Phe Glu Thr Gly Asp Ala Gly Ala Ser Ala
1               5                   10                  15

Thr Phe Pro Met Gln Cys Ser Ala Leu Arg Lys Asn Gly Phe Val Val
            20                  25                  30

Leu Lys Gly Arg Pro Cys Lys Ile Val Glu Met Ser Thr Ser Lys Thr
        35                  40                  45

Gly Lys His Gly His Ala Lys Val His Leu Val Gly Ile Asp Ile Phe
    50                  55                  60

Thr Gly Lys Lys Tyr Glu Asp Ile Cys Pro Ser Thr His Asn Met Asp
65                  70                  75                  80

Val Pro Asn Ile Lys Arg Asn Asp Phe Gln Leu Ile Gly Ile Gln Asp
                85                  90                  95

Gly Tyr Leu Ser Leu Leu Gln Asp Ser Gly Glu Val Arg Glu Asp Leu
            100                 105                 110

Arg Leu Pro Glu Gly Asp Leu Gly Lys Glu Ile Glu Gln Lys Tyr Asp
        115                 120                 125

Cys Gly Glu Glu Ile Leu Ile Thr Val Leu Ser Ala Met Thr Glu Glu
    130                 135                 140

Ala Ala Val Ala Ile Lys Ala Met Ala Lys
145                 150

<210> 22
<211> 153
<212> PRT
<213> Homo sapiens

<400> 22
Met Ala Asp Glu Ile Asp Phe Thr Thr Gly Asp Ala Gly Ala Ser Ser
1               5                   10                  15

Thr Tyr Pro Met Gln Cys Ser Ala Leu Arg Lys Asn Gly Phe Val Val
            20                  25                  30

Leu Lys Gly Arg Pro Cys Lys Ile Val Glu Met Ser Thr Ser Lys Thr
        35                  40                  45

Gly Lys His Gly His Ala Lys Val His Leu Val Gly Ile Asp Ile Phe
    50                  55                  60

Thr Gly Lys Lys Tyr Glu Asp Ile Cys Pro Ser Thr His Asn Met Asp
65                  70                  75                  80

Val Pro Asn Ile Lys Arg Asn Asp Tyr Gln Leu Ile Cys Ile Gln Asp
                85                  90                  95

Gly Tyr Leu Ser Leu Leu Thr Glu Thr Gly Glu Val Arg Glu Asp Leu
            100                 105                 110

Lys Leu Pro Glu Gly Glu Leu Gly Lys Glu Ile Glu Gly Lys Tyr Asn
        115                 120                 125

Ala Gly Glu Asp Val Gln Val Ser Val Met Cys Ala Met Ser Glu Glu
    130                 135                 140

Tyr Ala Val Ala Ile Lys Pro Cys Lys
145                 150

<210> 23
<211> 154
<212> PRT
<213> Mus musculus

<400> 23
Met Ala Asp Asp Leu Asp Phe Glu Thr Gly Asp Ala Gly Ala Ser Ala
1               5                   10                  15

Thr Phe Pro Met Gln Cys Ser Ala Leu Arg Lys Asn Gly Phe Val Val
            20                  25                  30

Leu Lys Gly Arg Pro Cys Lys Ile Val Glu Met Ser Thr Ser Lys Thr
        35                  40                  45

Gly Lys His Gly His Ala Lys Val His Leu Val Gly Ile Asp Ile Phe
    50                  55                  60

Thr Gly Lys Lys Tyr Glu Asp Ile Cys Pro Ser Thr His Asn Met Asp
65                  70                  75                  80

Val Pro Asn Ile Lys Arg Asn Asp Phe Gln Leu Ile Gly Ile Gln Asp
                85                  90                  95

Gly Tyr Leu Ser Leu Leu Gln Asp Ser Gly Glu Val Arg Glu Asp Leu
            100                 105                 110

Arg Leu Pro Glu Gly Asp Leu Gly Lys Glu Ile Glu Gln Lys Tyr Asp
        115                 120                 125

Cys Gly Glu Glu Ile Leu Ile Thr Val Leu Ser Ala Met Thr Glu Glu
    130                 135                 140

Ala Ala Val Ala Ile Lys Ala Met Ala Lys
145                 150

<210> 24
<211> 153
<212> PRT
<213> Homo sapiens

<400> 24
Met Ala Asp Glu Ile Asp Phe Thr Thr Gly Asp Ala Gly Ala Ser Ser
  1               5                  10                  15

Thr Tyr Pro Met Gln Cys Ser Ala Leu Arg Lys Asn Gly Phe Val Val
             20                  25                  30

Leu Lys Gly Arg Pro Cys Lys Ile Val Glu Met Ser Thr Ser Lys Thr
         35                  40                  45

Gly Lys His Gly His Ala Lys Val His Leu Val Gly Ile Asp Ile Phe
     50                  55                  60

Thr Gly Lys Lys Tyr Glu Asp Ile Cys Pro Ser Thr His Asn Met Asp
 65                  70                  75                  80

Val Pro Asn Ile Lys Arg Asn Asp Tyr Gln Leu Ile Cys Ile Gln Asp
                 85                  90                  95

Gly Cys Leu Ser Leu Leu Thr Glu Thr Gly Glu Val Arg Glu Asp Leu
            100                 105                 110

Lys Leu Pro Glu Gly Glu Leu Gly Lys Glu Ile Glu Gly Lys Tyr Asn
        115                 120                 125

Ala Gly Glu Asp Val Gln Val Ser Val Met Cys Ala Met Ser Glu Glu
    130                 135                 140

Tyr Ala Val Ala Ile Lys Pro Cys Lys
145                 150

<210> 25
<211> 20
<212> DNA
<213> Artificial Sequence

<220>
<223> Description of Artificial Sequence: Synthetic
      oligonucleotide

<400> 25
gacttggact tcgagacagg                                              20

<210> 26
<211> 19
<212> DNA
<213> Artificial Sequence

<220>
<223> Description of Artificial Sequence: Synthetic
      oligonucleotide

<400> 26
gcacggccac gccaaggtc                                                    19


<210> 27
<211> 20
<212> DNA
<213> Artificial Sequence

<220>
<223> Description of Artificial Sequence: Synthetic
      oligonucleotide

<400> 27
ggacagcggg gaggtacgag                                                   20


<210> 28
<211> 153
<212> PRT
<213> Artificial Sequence

<220>
<223> Description of Artificial Sequence: Illustrative
      consensus sequence

<400> 28
Met Ala Asp Glu Ile Asp Phe Thr Thr Gly Asp Ala Gly Ala Ser Ser
  1               5                  10                  15

Thr Tyr Pro Met Gln Cys Ser Ala Leu Arg Lys Asn Gly Phe Val Val
             20                  25                  30

Leu Lys Gly Arg Pro Cys Lys Ile Val Glu Met Ser Thr Ser Lys Thr
         35                  40                  45

Gly Lys His Gly His Ala Lys Val His Leu Val Gly Ile Asp Ile Phe
     50                  55                  60

Thr Gly Lys Lys Tyr Glu Asp Ile Cys Pro Ser Thr His Asn Met Asp
 65                  70                  75                  80

Val Pro Asn Ile Lys Arg Asn Asp Tyr Gln Leu Ile Cys Ile Gln Asp
                 85                  90                  95

Gly Cys Leu Ser Leu Leu Thr Glu Thr Gly Glu Val Arg Glu Asp Leu
            100                 105                 110

Lys Leu Pro Glu Gly Glu Leu Gly Lys Glu Ile Glu Gly Lys Tyr Asn
        115                 120                 125

Ala Gly Glu Asp Val Gln Val Ser Val Met Cys Ala Met Ser Glu Glu
    130                 135                 140

Tyr Ala Val Ala Ile Lys Pro Cys Lys
145                 150

<210> 29
<211> 1309
<212> DNA
<213> Homo sapiens

<400> 29
ggcacgaggg tagaggcggc ggcggcggcg gcagcgggct cggaggcagc ggttgggctc 60
gcggcgagcg gacggggtcg agtcagtgcg ttcgcgcgag ttggaatcga agcctcttaa 120
aatggcagat gacttggact tcgagacagg agatgcaggg gcctcagcca ccttcccaat 180
gcagtgctca gcattacgta agaatggctt tgtggtgctc aaaggccggc catgtaagat 240
cgtcgagatg tctacttcga agactggcaa gcacggccac gccaaggtcc atctggttgg 300
tattgacatc tttactggga agaaatatga agatatctgc ccgtcaactc ataatatgga 360
tgtccccaac atcaaaagga atgacttcca gctgattggc atccaggatg ggtacctatc 420
actgctccag gacagcgggg aggtacgaga ggaccttcgt ctccctgagg gagaccttgg 480
caaggagatt gagcagaagt acgactgtgg agaagagatc ctgatcacgg tgctgtctgc 540
catgacagag gaggcagctg ttgcaatcaa ggccatggca aaataactgg ctcccaggat 600
ggcggtggtg gcagcagtga tcctctgaac ctgcagaggc cccctccccg agcctggcct 660
ggctctggcc cggtcctaag ctggactcct cctacacaat ttatttgacg ttttattttg 720
gttttcccca ccccctcaat ctgtcgggga gcccctgccc ttcacctagc tcccttggcc 780
aggagcgagc gaagctgtgg ccttggtgaa gctgccctcc tcttctcccc tcacactaca 840
gccctggtgg gggagaaggg ggtgggtgct gcttgtggtt tagtcttttt tttttttttt 900
tttttttttt aaattcaatc tggaatcaga aagcggtgga ttctggcaaa tggtccttgt 960
gccctcccca ctcatccctg gtctggtccc ctgttgccca tagcccttta ccctgagcac 1020
caccccaaca gactggggac cagccccctc gcctgcctgt gtctctcccc aaaccccttt 1080
agatggggag ggaagaggag gagaggggag gggacctgcc ccctcctcag gcatctggga 1140
gggccctgcc cccatgggct ttacccttcc ctgcgggctc tctccccgac acatttgtta 1200
aaatcaaacc tgaataaaac tacaagttta atatgaaaaa aaaaaaaaaa aaaaaaaaaa 1260
aaaaaaaaaa aaaaaaaaaa aaaaaaaaaa aaaaaaaaaa aaaaaaaaa 1309

<210> 30
<211> 23
<212> DNA
<213> Homo sapiens

<400> 30
aaaggaatga cttccagctg att 23

<210> 31
<211> 23
<212> DNA
<213> Homo sapiens

<400> 31
aagatcgtcg agatgtctac ttc 23

<210> 32
<211> 23
<212> DNA
<213> Homo sapiens

<400> 32
aaggtccatc tggttggtat tga                                                23


<210> 33
<211> 23
<212> DNA
<213> Homo sapiens

<400> 33
aagctggact cctcctacac aat                                                23


<210> 34
<211> 23
<212> DNA
<213> Homo sapiens

<400> 34
aaagtcgacc ttcagtaagg att                                                23


<210> 35
<211> 20
<212> DNA
<213> Homo sapiens

<400> 35
cctgtctcga agtccaagtc                                                    20


<210> 36
<211> 20
<212> DNA
<213> Homo sapiens

<400> 36
gacttggact tcgagacagg                                                    20


<210> 37
<211> 20
<212> DNA
<213> Homo sapiens

<400> 37
ggaccttggc gtggccgtgc                                                    20


<210> 38

<211> 20
<212> DNA
<213> Homo sapiens

<400> 38
gcacggccac gccaaggtcc                                                    20


<210> 39
<211> 20
<212> DNA
<213> Homo sapiens

<400> 39
ctcgtacctc cccgctctcc                                                    20


<210> 40
<211> 19
<212> DNA
<213> Homo sapiens

<400> 40
ggacagcggg gaggtacga                                                     19


<210> 41
<211> 465
<212> DNA
<213> Homo sapiens

<400> 41
atggcagatg acttggactt cgagacagga gatgcagggg cctcagccac cttcccaatg 60
cagtgctcag cattacgtaa gaatggcttt gtggtgctca aaggccggcc atgtaagatc 120
gtcgagatgt ctacttcgaa gactggcaag cacggccacg ccaaggtcca tctggttggt 180
attgacatct ttactgggaa gaaatatgaa gatatctgcc cgtcaactca taatatggat 240
gtccccaaca tcaaaaggaa tgacttccag ctgattggca tccaggatgg gtacctatca 300
ctgctccagg acagcgggga ggtacgagag gaccttcgtc tccctgaggg agaccttggc 360
aaggagattg agcagaagta cgactgtgga gaagagatcc tgatcacggt gctgtctgcc 420
atgacagagg aggcagctgt tgcaatcaag gccatggcaa aataa                        465


<210> 42
<211> 462
<212> DNA
<213> Homo sapiens

<400> 42
atggcagacg aaattgattt cactactgga gatgccgggg cttccagcac ttaccctatg 60
cagtgctcgg ccttgcgcaa aaacggcttc gtggtgctca aaggacgacc atgcaaaata 120
gtggagatgt caacttccaa aactggaaag catggtcatg ccaaggttca ccttgttgga 180
attgatattt tcacgggcaa aaaatatgaa gatatttgtc cttctactca caacatggat 240
gttccaaata ttaagagaaa tgattatcaa ctgatatgca ttcaagatgg ttacctttcc 300
ctgctgacag aaactggtga agttcgtgag gatcttaaac tgccagaagg tgaactaggc 360
aaagaaatag agggaaaata caatgcaggt gaagatgtac aggtgtctgt catgtgtgca 420
atgagtgaag aatatgctgt agccataaaa ccctgcaaat aa                           462

<210> 43
<211> 154
<212> PRT
<213> Homo sapiens

<400> 43


<210> 44
<211> 21
<212> DNA
<213> Artificial Sequence

<220>
<223> Description of Artificial Sequence: Synthetic
      oligonucleotide

<400> 44
aaaggaatga cttccagctg a                                            21


<210> 45
<211> 23
<212> DNA
<213> Artificial Sequence

<220>

<223> Description of Combined DNA/RNA Molecule:
      Synthetic oligonucleotide

<220>
<223> Description of Artificial Sequence: Synthetic
      oligonucleotide

<400> 45
aaaggaauga cuuccagcug att                                              23


<210> 46
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 46
ucagcuggaa gucauuccuu utt                                              23


<210> 47
<211> 21
<212> DNA
<213> Artificial Sequence

<220>
<223> Description of Artificial Sequence: Synthetic
      oligonucleotide

<400> 47
aagatcgtcg agatgtctac t                                                21


<210> 48
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 48
aagaucgucg agaugucuac utt                                              23

<210> 49
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 49
aguagacauc ucgacgaucu utt                                              23


<210> 50
<211> 21
<212> DNA
<213> Artificial Sequence

<220>
<223> Description of Artificial Sequence: Synthetic
      oligonucleotide

<400> 50
aaggtccatc tggttggtat t                                                21


<210> 51
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 51
aagguccauc ugguugguau utt                                              23


<210> 52
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 52
aauaccaacc agauggaccu utt                                              23


<210> 53
<211> 21
<212> DNA
<213> Artificial Sequence

<220>
<223> Description of Artificial Sequence: Synthetic
      oligonucleotide

<400> 53
aagctggact cctcctacac a                                                21


<210> 54
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 54
aagcuggacu ccuccuacac att                                              23


<210> 55
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 55
uguguaggag gaguccagcu utt                                              23


<210> 56
<211> 21

<212> DNA
<213> Artificial Sequence

<220>
<223> Description of Artificial Sequence: Synthetic
      oligonucleotide

<400> 56
aaagtcgacc ttcagtaagg a                                                21


<210> 57
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 57
aaagucgacc uucaguaagg att                                              23


<210> 58
<211> 23
<212> DNA
<213> Artificial Sequence

<220>
<223> Description of Combined DNA/RNA Molecule:
      Synthetic oligonucleotide

<220>
<223> Description of Artificial Sequence: Synthetic
      oligonucleotide

<400> 58
uccuuacuga aggucgacuu utt                                              23


<210> 59
<211> 26
<212> DNA
<213> Artificial Sequence

<220>
<223> Description of Artificial Sequence: Synthetic
      primer

<400> 59
gccaagctta atggcagatg atttgg                                           26

<210> 60
<211> 25
<212> DNA
<213> Artificial Sequence

<220>
<223> Description of Artificial Sequence: Synthetic
      primer

<400> 60
ctgaattcca gttattttgc catgg                                          25


<210> 61
<211> 27
<212> DNA
<213> Artificial Sequence

<220>
<223> Description of Artificial Sequence: Synthetic
      primer

<400> 61
aatgaattcc gccatgacag aggaggc                                        27


<210> 62
<211> 42
<212> DNA
<213> Artificial Sequence

<220>
<223> Description of Artificial Sequence: Synthetic
      primer

<400> 62
gcgaagcttc catggctcga gttttttttt tttttttttt tt                       42


<210> 63
<211> 20
<212> DNA
<213> Artificial Sequence

<220>
<223> Description of Artificial Sequence: Synthetic
      oligonucleotide

<400> 63
cctgtctcga agtccaagtc                                                20


<210> 64
<211> 20
<212> DNA
<213> Artificial Sequence

<220>
<223> Description of Artificial Sequence: Synthetic
      oligonucleotide

<400> 64
ggaccttggc gtggccgtgc                                                    20


<210> 65
<211> 20
<212> DNA
<213> Artificial Sequence

<220>
<223> Description of Artificial Sequence: Synthetic
      oligonucleotide

<400> 65
ctcgtacctc cccgctctcc                                                    20


<210> 66
<211> 20
<212> DNA
<213> Artificial Sequence

<220>
<223> Description of Artificial Sequence: Synthetic
      oligonucleotide

<400> 66
cgtaccggta cggttccagg                                                    20


<210> 67
<211> 20
<212> DNA
<213> Artificial Sequence

<220>
<223> Description of Artificial Sequence: Synthetic
      oligonucleotide

<400> 67
ggaccttggc gtggccgtgc                                                    20


<210> 68
<211> 17
<212> PRT
<213> Artificial Sequence

<220>
<223> Description of Artificial Sequence: Synthetic
      peptide

<400> 68
Cys Arg Leu Pro Glu Gly Asp Leu Gly Lys Glu Ile Glu Gln Lys Tyr
1               5                   10                  15

Asp

<210> 69
<211> 29
<212> DNA
<213> Artificial Sequence

<220>
<223> Description of Artificial Sequence: Synthetic
      oligonucleotide

<400> 69
aaaggaatga cttccagctg acctgtctc                                   29

<210> 70
<211> 29
<212> DNA
<213> Artificial Sequence

<220>
<223> Description of Artificial Sequence: Synthetic
      oligonucleotide

<400> 70
aatcagctgg aagtcattcc tcctgtctc                                   29

<210> 71
<211> 29
<212> DNA
<213> Artificial Sequence

<220>
<223> Description of Artificial Sequence: Synthetic
      oligonucleotide

<400> 71
aagatcgtcg agatgtctac tcctgtctc                                   29

<210> 72
<211> 29
<212> DNA
<213> Artificial Sequence

<220>
<223> Description of Artificial Sequence: Synthetic
      oligonucleotide

<400> 72

aaagtagaca tctcgacgat ccctgtctc 29

<210> 73
<211> 29
<212> DNA
<213> Artificial Sequence

<220>
<223> Description of Artificial Sequence: Synthetic
      oligonucleotide

<400> 73
aaggtccatc tggttggtat tcctgtctc 29

<210> 74
<211> 29
<212> DNA
<213> Artificial Sequence

<220>
<223> Description of Artificial Sequence: Synthetic
      oligonucleotide

<400> 74
aaaataccaa ccagatggac ccctgtctc 29

<210> 75
<211> 29
<212> DNA
<213> Artificial Sequence

<220>
<223> Description of Artificial Sequence: Synthetic
      oligonucleotide

<400> 75
aagctggact cctcctacac acctgtctc 29

<210> 76
<211> 29
<212> DNA
<213> Artificial Sequence

<220>
<223> Description of Artificial Sequence: Synthetic
      oligonucleotide

<400> 76
aatgtgtagg aggagtccag ccctgtctc 29

<210> 77
<211> 29

<212> DNA
<213> Artificial Sequence

<220>
<223> Description of Artificial Sequence: Synthetic
      oligonucleotide

<400> 77
aaagtcgacc ttcagtaagg acctgtctc                                    29


<210> 78
<211> 29
<212> DNA
<213> Artificial Sequence

<220>
<223> Description of Artificial Sequence: Synthetic
      oligonucleotide

<400> 78
aatccttact gaaggtcgac tcctgtctc                                    29


<210> 79
<211> 20
<212> RNA
<213> Artificial Sequence

<220>
<223> Description of Artificial Sequence: Synthetic
      oligonucleotide

<400> 79
aagcuggacu ccuccuacac                                              20


<210> 80
<211> 21
<212> RNA
<213> Artificial Sequence

<220>
<223> Description of Artificial Sequence: Synthetic
      oligonucleotide

<400> 80
aaacacaucc uccucagguc g                                            21


<210> 81
<211> 21
<212> DNA
<213> Artificial Sequence

<220>
<223> Description of Artificial Sequence: Synthetic

oligonucleotide

<400> 81
aaaggaatga cttccagctg a                                                21

<210> 82
<211> 21
<212> DNA
<213> Artificial Sequence

<220>
<223> Description of Artificial Sequence: Synthetic
      oligonucleotide

<400> 82
aagatcgtcg agatgtctac t                                                21

<210> 83
<211> 21
<212> DNA
<213> Artificial Sequence

<220>
<223> Description of Artificial Sequence: Synthetic
      oligonucleotide

<400> 83
aaggtccatc tggttggtat t                                                21

<210> 84
<211> 21
<212> DNA
<213> Artificial Sequence

<220>
<223> Description of Artificial Sequence: Synthetic
      oligonucleotide

<400> 84
aagctggact cctcctacac a                                                21

<210> 85
<211> 21
<212> DNA
<213> Artificial Sequence

<220>
<223> Description of Artificial Sequence: Synthetic
      oligonucleotide

<400> 85
aaagtcgacc ttcagtaagg a                                                21

<210> 86
<211> 21
<212> RNA
<213> Artificial Sequence

<220>
<223> Description of Combined DNA/RNA Molecule:
      Synthetic oligonucleotide

<220>
<223> Description of Artificial Sequence: Synthetic
      siRNA sequence

<220>
<221> misc_feature
<222> (1)..(2)
<223> a, t, c, g or u

<400> 86
nngcuggacu ccuccuacac a                                                21


<210> 87
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic
      siRNA sequence

<400> 87
gcuggacucc uccuacacat t                                                21


<210> 88
<211> 21
<212> DNA
<213> Artificial Sequence

<220>
<223> Description of Artificial Sequence: Synthetic
      siRNA sequence

<400> 88
uguguaggag gaguccagct t                                                21


<210> 89
<211> 21
<212> RNA

<213> Artificial Sequence

<220>
<223> Description of Combined DNA/RNA Molecule:
      Synthetic oligonucleotide

<220>
<223> Description of Artificial Sequence: Synthetic
      siRNA sequence

<220>
<221> misc_feature
<222> (1)..(2)
<223> a, t, c, g or u

<400> 89
nnacacaucc uccucagguc g                                                21


<210> 90
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic
      siRNA sequence

<400> 90
acacauccuc cucaggucgt t                                                21


<210> 91
<211> 21
<212> DNA
<213> Artificial Sequence

<220>
<223> Description of Combined DNA/RNA Molecule:
      Synthetic oligonucleotide

<220>
<223> Description of Artificial Sequence: Synthetic
      siRNA sequence

<400> 91
cgaccugagg aggaugugut t                                                21


<210> 92
<211> 24
<212> DNA

<213> Artificial Sequence

<220>
<223> Description of Artificial Sequence: Synthetic
      siRNA sequence

<400> 92
cggatggcaa catttagaat tagt                                              24


<210> 93
<211> 24
<212> DNA
<213> Artificial Sequence

<220>
<223> Description of Artificial Sequence: Primer

<400> 93
tgattgagac tgtaatcaag aacc                                              24


<210> 94
<211> 22
<212> DNA
<213> Artificial Sequence

<220>
<223> Description of Artificial Sequence: Primer

<400> 94
ctgatgcccc catgttcgtc at                                                22


<210> 95
<211> 20
<212> DNA
<213> Artificial Sequence

<220>
<223> Description of Artificial Sequence: Primer

<400> 95
ccaccaccct gttgctgtag                                                   20